# EUROPEAN PATENT SPECIFICATION

(11) **EP 0 830 139 B1**
(45) Date of publication and mention of the grant of the patent: **02.01.2008**
(21) Application number: 96913035.0
(22) Date of filing: 22.04.1996
(51) Int. Cl.: A61K 39/00, C07H 21/04, C07K 14/47, G01N 33/53

(54) **MODIFIED MYELIN PROTEIN MOLECULES**
MODIFIZIERTE MYELIN PROTEINMOLEKÜLE
MOLECULES DE PROTEINE MYELINIQUE MODIFIEES

(30) Priority: 02.05.1995 US 431644; 02.05.1995 US 431648; 07.06.1995 US 482114
(43) Date of publication of application: 25.03.1998
(73) Proprietor: ALEXION PHARMACEUTICALS, INC., Cheshire, CT 06410 (US); THE UNITED STATES GOVERNMENT as represented by THE DEPARTMENT OF HEALTH AND HUMAN SERVICES, Rockville, MD 20852-3804 (US)
(72) Inventor: MUELLER, John, P., Old Lyme , CT 06371-1868 (US); LENARDO, Michael, J., Potomac, MD 20854 (US); MCFARLAND, Henry, F., Gaithersburg, MD 20882 (US); MATIS, Louis, Southport, CT 06490 (US); MUELLER, Eileen, Elliott, Old Lyme , CT 06371-1868 (US); NYE, Steven, H., Thiensville, WI 53092-5112 (US); PELFREY, Clara, M., Shaker Heights, OH 44122-5103 (US); SQUINTO, Stephen, P., Bethany, CT 06524 (US); WILKINS, James, A., Woodbridge, CT 06525 (US)
(74) Representative: Dempster, Robert Charles
(86) International application number: PCT/US1996/005611
(87) International publication number: WO 1996/034622

(56) References cited:
- NYE S.H. ET AL: "Purification of immunologically active recombinant 21.5 kDa isoform of human myelin basic protein." MOLECULAR IMMUNOLOGY, (1995) 32/14-15 (1131-1141)., XP000655158
- ZHAO M.-L. ET AL: "The immune response to a subdominant epitope in myelin basic protein exon - 2 results in immunity to intra- and intermolecular dominant epitopes." JOURNAL OF NEUROIMMUNOLOGY, (1995) 61/2 (179-184)., XP000991173
- MEINL E. ET AL: "Activation of a myelin basic protein-specific human T cell clone by antigen-presenting cells from rhesus monkeys." INTERNATIONAL IMMUNOLOGY, (1995) 7/9 (1489-1495)., XP000991134
- VOSKUHL R R ET AL: "A NOVEL CANDIDATE AUTOANTIGEN IN A MULTIPLEX FAMILY WITH MULTIPLE SCLEROSIS: PREVALENCE OF T-LYMPHOCYTES SPECIFIC FOR AN MBP EPITOPE UNIQUE TO MYELINATION" JOURNAL OF NEUROIMMUNOLOGY,ELSEVIER SCIENCE PUBLISHERS BV,XX, vol. 46, July 1993 (1993-07), pages 137-144, XP000991130 ISSN: 0165-5728
- VOSKUHL R.R. ET AL: "T-lymphocyte recognition of a portion of myelin basic protein encoded by an exon expressed during myelination." JOURNAL OF NEUROIMMUNOLOGY, (1993) 42/2 (187-192)., XP002046158
- SEGAL B.M. ET AL: "Experimental allergic encephalomyelitis induced by the peptide encoded by exon 2 of the MBP gene, a peptide implicated in remyelination." JOURNAL OF NEUROIMMUNOLOGY, (1994) 51/1 (7-19)., XP000991126
- FRITZ R.B. ET AL: "Encephalitogenicity of myelin basic protein exon - 2 peptide in mice." JOURNAL OF NEUROIMMUNOLOGY, (1994) 51/1 (1-6)., XP000992122
- NEWMAN S. ET AL: "Identification of a cDNA coding for a fifth form of myelin basic protein in mouse." PROCEEDINGS OF THE NATIONAL ACADEMY OF SCIENCES OF THE UNITED STATES OF AMERICA, (1987) 84/3 (886-890). CODEN: PNASA6, XP000990302
- ROTH H.J. ET AL: "Evidence for the expression of four myelin basic protein variants in the developing human spinal cord through cDNA cloning" JOURNAL OF NEUROSCIENCE RESEARCH, (1987) 17/4 (321-328). CODEN: JNREDK, XP000991117
- KAMHOLZ J. ET AL: "Identification of three forms of human myelin basic protein by cDNA cloning." PROCEEDINGS OF THE NATIONAL ACADEMY OF SCIENCES OF THE UNITED STATES OF AMERICA, (1986) 83/13 (4962-4966). CODEN: PNASA6, XP000990303
- H.L WEINER ET AL.: "Double-blind pilot trial of oral tolerization with myelin antigens in multiple sclerosis." SCIENCE, vol. 259, 26 February 1993 (1993-02-26), pages 1321-1324, XP000990295
- TSUCHIDA T ET AL: "AUTOREACTIVE CD8+ T-CELL RESPONSES TO HUMAN MYELIN PROTEIN-DERIVED PEPTIDES" PROCEEDINGS OF THE NATIONAL ACADEMY OF SCIENCES OF USA, NATIONAL ACADEMY OF SCIENCE. WASHINGTON, US, vol. 91, no. 23, November 1994 (1994-11), pages 10859-10863, XP001010361 ISSN: 0027-8424
- PELFREY C M ET AL: "IDENTIFICATION OF A SECOND T CELL EPITOPE OF HUMAN PROTEOLIPID PROTEIN (RESIDUES 89-106) RECOGNIZED BY PROLIFERATIVE AND CYTOLYTICCD4+ T CELLS FROM MULTIPLE SCLEROSIS PATIENTS" JOURNAL OF NEUROIMMUNOLOGY, ELSEVIER SCIENCE PUBLISHERS BV, XX, vol. 53, no. 2, September 1994 (1994-09), pages 153-161, XP001014180 ISSN: 0165-5728
- PELFREY C M ET AL: "IDENTIFICATION OF A NOVEL T CELL EPITOPE OF HUMAN PROTEOLIPID PROTEIN (RESIDUES 40-60) RECOGNIZED BY PROLIFERATIVE AND CYTOLYTIC CD4+ T CELLS FROM MULTIPLE SCLEROSIS PATIENTS" JOURNAL OF NEUROIMMUNOLOGY, ELSEVIER SCIENCE PUBLISHERS BV, XX, vol. 46, no. 1/2, July 1993 (1993-07), pages 33-42, XP001014181 ISSN: 0165-5728
- JEAN-LUC POPOT ET AL: "MAJOR MYELIN PROTEOLIPID: THE 4-ALPHA-HELIX TOPOLOGY" JOURNAL OF MEMBRANE BIOLOGY, NEW YORK, NY, US, vol. 120, 1991, pages 233-246, XP001014141
- WEIMBS T ET AL: "A POINT MUTATION AT THE X-CHROMOSOMAL PROTEOLIPID PROTEIN LOCUS IN PELIZAEUS-MERZBACHER DISEASE LEADS TO DISRUPTION OF MYELINOGENESIS" BIOLOGICAL CHEMISTRY HOPPE-SEYLER, WALTER DE GRUYTER, BERLIN, DE, vol. 371, no. 12, December 1990 (1990-12), pages 1175-1183, XP001014187 ISSN: 0177-3593
- SIMONS R ET AL: "THE MYELIN-DEFICIENT RAT HAS A SINGLE BASE SUBSTITUTION IN THE THIRD EXON OF THE MYELIN PROTEOLIPID PROTEIN GENE" JOURNAL OF NEUROCHEMISTRY, NEW YORK, NY, US, vol. 54, no. 3, March 1990 (1990-03), pages 1079-1081, XP001014182 ISSN: 0022-3042
- SIMONS R ET AL: "SINGLE BASE SUBSTITUTION IN CODON 74 OF THE MD RAT MYELIN PROTEOLIPID PROTEIN GENE" ANNALS OF THE NEW YORK ACADEMY OF SCIENCES, NEW YORK ACADEMY OF SCIENCES, NEW YORK, NY, US, vol. 605, 1990, pages 146-154, XP001014144 ISSN: 0077-8923
- HUDSON L D ET AL: "MUTATION OF THE PROTEOLIPID PROTEIN GENE PLP IN A HUMAN X CHROMOSOME-LINKED MYELIN DISORDER" PROCEEDINGS OF THE NATIONAL ACADEMY OF SCIENCES OF USA, NATIONAL ACADEMY OF SCIENCE. WASHINGTON, US, vol. 86, no. 20, October 1989 (1989-10), pages 8128-8131, XP001010360 ISSN: 0027-8424
- JOURNAL OF MEMBRANE BIOLOGY, Volume 120, issued 1991, J. POPOT et al., "Major Myelin Proteolipid: The 4-Alpha-Helix Topology", pages 233-246, XP001014141
- THE JOURNAL OF IMMUNOLOGY, issued 1994, R.R. VOSKUHL et al., "HLA Restriction and TCR Usage of T Lymphocytes Specific for Novel Candidate Autoantigen, X2 MBP, In Multiple Sclerosis", pages 4834-4844, XP002948987
- SCIENCE, Volume 263, issued 25 February 1994, J.M. CRITCHFIELD et al., "T Cell Deletion in High Antigen Dose Therapy of Autoimmune Encephalomyelitis", pages 1139-1143, XP002052787
- BIOCHEM. JOURNAL, Volume 123, issued 1971, P.R. CARNEGIE, "Amino Acid Sequence of the Encephalitogenic Basic Protein from Human Myelin", pages 57-67, XP002948988
- BIOCHEMICAL AND BIOPHYSICAL RESEARCH COMMUNICATIONS, Volume 192, Number 3, issued 14 May 1993, P. PROOST et al., "Leukocyte Gelatinase B Cleavage Releases Encephalitogens from Human Myelin Basic Protein", pages 1175-1181, XP002913652
- JOURNAL OF MOLECULAR BIOLOGY, Volume 188, issued 1986, E.G. SHPAER, "Constraints on Codon Context in Escherichia Coli Genes Their Possible Role in Modulating the Efficiency of Translation", pages 555-564, XP002948989
- CHEMICAL ABSTRACTS, vol. 123, 1995, Columbus, Ohio, US; abstract no. 218402N, DEVAUX ET AL: 'A cDNA for Human Myelin Oligodendrocyte Glycoprtein and Treatment of Autoimmune disease' page 107-108; XP002905506 & WO 95 06727 A 09 March 1995
- PROC. NATL. ACAD. SCI. U.S.A., Volume 83, issued December 1986, H. DIEHL et al., "Individual Exons Encode the Integral Membrane Domains of Human Myelin Proteolipid Protein", pages 9807-9811, XP002173463

### SEQUENCE LISTING

(1) GENERAL INFORMATION:
   (i) APPLICANT: Mueller, John P.
      Lenardo. Michael J.
      McFarland, Henry F.
      Matis, Louis A.
      Mueller, Eileen Elliott
      Nye, Steven H.
      Pelfrey, Clara M.
      Squinto, Stephen P.
      Wilkins, James A.
   (ii) TITLE OF INVENTION: Modified Myelin Protein Molecules
   (iii) NUMBER OF SEQUENCES: 29
   (iv) CORRESPONDENCE ADDRESS:
      (A) ADDRESSEE: Maurice M. Klee
      (B) STREET: 1951 Burr Street
      (C) CITY: Fairfield
      (D) STATE: Connecticut
      (E) COUNTRY: USA
      (F) ZIP: 06430
   (v) COMPUTER READABLE FORM:
      (A) MEDIUM TYPE: 3.5 inch, 0.8 Mb storage
      (B) COMPUTER: Macintosh Centris 610
      (C) OPERATING SYSTEM: System 7
      (D) SOFTWARE: Microsoft Word 6.0.1
   (vi) CURRENT APPLICATION DATA:
      (A) APPLICATION NUMBER:
      (B) FILING DATE: 02-MAY-1995
      (C) CLASSIFICATION:
   (vii) PRIOR APPLICATION DATA:
      (A) APPLICATION NUMBER: 08/431,644
      (B) FILING DATE: May 2, 1995

      (A) APPLICATION NUMBER: 08/431,648
      (B) FILING DATE: May 2, 1995

      (A) APPLICATION NUMBER: 08/482,114
      (B) FILING DATE: June 7, 1995
   (viii) ATTORNEY/AGENT INFORMATION:
      (A) NAME: Klee, Maurice M.
      (B) REGISTRATION NUMBER: 30,399
      (C) REFERENCE/DOCKET NUMBER: ALX-129
   (ix) TELECOMMUNICATION INFORMATION:
      (A) TELEPHONE: (203) 255 1400
      (B) TELEFAX: (203) 254 1101
(2) INFORMATION FOR SEQ ID NO:1:
   (i) SEQUENCE CHARACTERISTICS:
      (A) LENGTH: 594 base pairs
      (B) TYPE: Nucleic acid
      (C) STRANDEDNESS: Double
      (D) TOPOLOGY: Linear
   (ii) MOLECULE TYPE: cDNA to mRNA
      (A) DESCRIPTION: MBP+X2^{Cys81/hum}. (Human 21.5 kD form of MBP)
   (iii) HYPOTHETICAL: No
   (iv) ANTI-SENSE: No
   (x) PUBLICATION INFORMATION:
      (A) AUTHORS: Roth, H. J. Kronquist, K. E. Kerlero de Rosbo, N. Crandall, B. F. Campagnoni, A. T. (B) TITLE: Evidence for the Expression of Four Myelin Basic Protein Variants in the Developing Human Spinal Cord Through cDNA Cloning (C) JOURNAL: Journal of Neuroscience Research (D) VOLUME: 17 (F) PAGES: 312 - 328 (G) DATE:1987
   (xi) SEQUENCE DESCRIPTION: SEQ ID NO:1:
(2) INFORMATION FOR SEQ ID NO:2:
   (i) SEQUENCE CHARACTERISTICS:
      (A) LENGTH: 612 base pairs
      (B) TYPE: Nucleic acid
      (C) STRANDEDNESS: Double
      (D) TOPOLOGY: Linear
   (ii) MOLECULE TYPE: Other nucleic acid
      (A) DESCRIPTION: MBP+X2^{Cys81/bact}.
   (iii) HYPOTHETICAL: No
   (iv) ANTI-SENSE: No
   (xi) SEQUENCE DESCRIPTION: SEQ ID NO:2:
   ATG GCG TCT CAG AAA CGT CCG TCC CAG CGT CAC GGC TCC AAA          42
   TAC CTG GCC ACC GCC AGC ACC ATG GAC CAT GCC CGT CAT GGC          84
   TTC CTG CCG CGT CAC CGT GAC ACC GGC ATC CTG GAC TCC ATC         126
   GGC CGC TTC TTC GGC GGT GAC CGT GGT GCG CCG AAA CGT GGC         168
   TCT GGC AAA GTG CCG TGG CTG AAA CCG GGC CGT AGC CCG CTG         210
   CCG TCT CAT GCC CGT AGC CAG CCG GGC CTG TGC AAC ATG TAC         252
   AAA GAC TCC CAC CAC CCG GCT CGT ACC GCG CAC TAT GGC TCC         294
   CTG CCG CAG AAA TCC CAC GGC CGT ACC CAG GAT GAA AAC CCG         336
   GTG GTG CAC TTC TTC AAA AAC ATT GTG ACC CCG CGT ACC CCG         378
   CCG CCG TCT CAG GGC AAA GGC CGT GGC CTG TCC CTG AGC CGT         420
   TTC AGC TGG GGC GCC GAA GGC CAG CGT CCG GGC TTC GGT TAC         462
   GGC GGC CGT GCG TCC GAC TAT AAA TCT GCT CAC AAA GGC TTC        504
   AAA GGC GTG GAT GCC CAG GGT ACC TTG TCC AAA ATT TTC AAA        546
   CTG GGC GGC CGT GAT AGC CGT TCT GGC TCT CCG ATG GCT AGA        588
   CGT CAT CAC CAT CAC CAT CAC TAA                                612
(2) INFORMATION FOR SEQ ID NO:3:
   (i) SEQUENCE CHARACTERISTICS:
      (A) LENGTH: 612 base pairs
      (B) TYPE: Nucleic acid
      (C) STRANDEDNESS: Double
      (D) TOPOLOGY: Linear
   (ii) MOLECULE TYPE: cDNA to mRNA
      (A) DESCRIPTION: MBP+X2^{Ser81/bact.}
   (iii) HYPOTHETICAL: No
   (iv) ANTI-SENSE: No
   (xi) SEQUENCE DESCRIPTION: SEQ ID NO:3:
   ATG GCG TCT CAG AAA CGT CCG TCC CAG CGT CAC GGC TCC AAA        42
   Met Ala Ser Gln Lys Arg Pro Ser Gln Arg His Gly Ser Lys
    1              5                   10
   TAC CTG GCC ACC GCC AGC ACC ATG GAC CAT GCC CGT CAT GGC        84
   Tyr Leu Ala Thr Ala Ser Thr Met Asp His Ala Arg His Gly
   15                  20                  25
   TTC CTG CCG CGT CAC CGT GAC ACC GGC ATC CTG GAC TCC ATC        126
   Phe Leu Pro Arg His Arg Asp Thr Gly Ile Leu Asp Ser Ile
       30                  35                  40
   GGC CGC TTC TTC GGC GGT GAC CGT GGT GCG CCG AAA CGT GGC        168
   Gly Arg Phe Phe Gly Gly Asp Arg Gly Ala Pro Lys Arg Gly
           45                  50                  55
   TCT GGC AAA GTG CCG TGG CTG AAA CCG GGC CGT AGC CCG CTG        210
   Ser Gly Lys Val Pro Trp Leu Lys Pro Gly Arg Ser Pro Leu
               60                  65                  70
   CCG TCT CAT GCC CGT AGC CAG CCG GGC CTG TCG AAC ATG TAC        252
   Pro Ser His Ala Arg Ser Gln Pro Gly Leu Ser Asn Met Tyr
                    75                  80
   AAA GAC TCC CAC CAC CCG GCT CGT ACC GCG CAC TAT GGC TCC        294
   Lys Asp Ser His His Pro Ala Arg Thr Ala His Tyr Gly Ser
    85                  90                  95
   CTG CCG CAG AAA TCC CAC GGC CGT ACC CAG GAT GAA AAC CCG        336
   Leu Pro Gln Lys Ser His Gly Arg Thr Gln Asp Glu Asn Pro
       100                 105                 110
   GTG GTG CAC TTC TTC AAA AAC ATT GTG ACC CCG CGT ACC CCG        378
   Val Val His Phe Phe Lys Asn Ile Val Thr Pro Arg Thr Pro
           115                 120                 125
   CCG CCG TCT CAG GGC AAA GGC CGT GGC CTG TCC CTG AGC CGT        420
   Pro Pro Ser Gln Gly Lys Gly Arg Gly Leu Ser Leu Ser Arg
               130                 135                 140
   TTC AGC TGG GGC GCC GAA GGC CAG CGT CCG GGC TTC GGT TAC        462
   Phe Ser Trp Gly Ala Glu Gly Gln Arg Pro Gly Phe Gly Tyr
                   145                 150
   GGC GGC CGT GCG TCC GAC TAT AAA TCT GCT CAC AAA GGC TTC        504
   Gly Gly Arg Ala Ser Asp Tyr Lys Ser Ala His Lys Gly Phe
   155                 160                 165
   AAA GGC GTG GAT GCC CAG GGT ACC TTG TCC AAA ATT TTC AAA        546
   Lys Gly Val Asp Ala Gln Gly Thr Leu Ser Lys Ile Phe Lys
       170                 175                 180
   CTG GGC GGC CGT GAT AGC CGT TCT GGC TCT CCG ATG GCT AGA        588
   Leu Gly Gly Arg Asp Ser Arg Ser Gly Ser Pro Met Ala Arg
           185                 190                 195
   CGT CAT CAC CAT CAC CAT CAC TAA                                612
   Arg His His His His His His
               200
(2) INFORMATION FOR SEQ ID NO:4:
   (i) SEQUENCE CHARACTERISTICS:
      (A) LENGTH: 534 base pairs
      (B) TYPE: Nucleic acid
      (C) STRANDEDNESS: Double
      (D) TOPOLOGY: Linear
   (ii) MOLECULE TYPE: cDNA to mRNA
      (A) DESCRIPTION: Human 18.5 kDa form of MBP
   (iii) HYPOTHETICAL: No
   (iv) ANTI-SENSE: No
   (xi) SEQUENCE DESCRIPTION: SEQ ID NO:4:
(2) INFORMATION FOR SEQ ID NO:5 :
   (i) SEQUENCE CHARACTERISTICS:
      (A) LENGTH: 130 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: PCR primer oligonucleotide 1
   (iii) HYPOTHETICAL: No
   (iv) ANTI-SENSE: No
   (xi) SEQUENCE DESCRIPTION: SEQ ID NO:5:
   GGAATTCCGT AAGGAGGTAT AGCATATGGC GTCTCAGAAA CGTCCGTCCC      50
   AGCGTCACGG CTCCAAATAC CTGGCCACCG CCAGCACCAT GGACCATGCC     100
   CGTCATGGCT TCCTGCCGCG TCACCGTGAC                           130
(2) INFORMATION FOR SEQ ID NO:6:
   (i) SEQUENCE CHARACTERISTICS:
      (A) LENGTH: 129 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: PCR primer oligonucleotide 2
   (iii) HYPOTHETICAL: No
   (iv) ANTI-SENSE: Yes
   (xi) SEQUENCE DESCRIPTION: SEQ ID NO:6:
   GACGGCAGCG GGCTACGGCC CGGTTTCAGC CACGGCACTT TGCCAGAGCC      50
   ACGTTTCGGC GCACCACGGT CACCGCCGAA GAAGCGGCCG ATGGAGTCCA     100
   GGATGCCGGT GTCACGGTGA CGCGGCAGG                            129
(2) INFORMATION FOR SEQ ID NO:7:
   (i) SEQUENCE CHARACTERISTICS:
      (A) LENGTH: 133 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: PCR primer oligonucleotide 3
   (iii) HYPOTHETICAL: No
   (iv) ANTI-SENSE: No
   (xi) SEQUENCE DESCRIPTION: SEQ ID NO:7:
   CCGGGCCGTA GCCCGCTGCC GTCTCATGCC CGTAGCCAGC CGGGCCTGTG 50
   CAACATGTAC AAAGACTCCC ACCACCCGGC TCGTACCGCG CACTATGGCT 100
   CCCTGCCGCA GAAATCCCAC GGCCGTACCC AGG 133
(2) INFORMATION FOR SEQ ID NO:8:
   (i) SEQUENCE CHARACTERISTICS:
      (A) LENGTH: 131 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: PCR primer oligonucleotide 4
   (iii) HYPOTHETICAL: No
   (iv) ANTI-SENSE: Yes
   (xi) SEQUENCE DESCRIPTION: SEQ ID NO:8:
   CGGCGCCCCA GCTGAAACGG CTCAGGGACA GGCCACGGCC TTTGCCCTGA 50
   GACGGCGGCG GGGTACGCGG GGTCACAATG TTTTTGAAGA AGTGCACCAC 100
   CGGGTTTTCA TCCTGGGTAC GGCCGTGGGA T 131
(2) INFORMATION FOR SEQ ID NO:9:
   (i) SEQUENCE CHARACTERISTICS:
      (A) LENGTH: 119 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: PCR primer oligonucleotide 5
   (iii) HYPOTHETICAL: No
   (iv) ANTI-SENSE: No
   (xi) SEQUENCE DESCRIPTION: SEQ ID NO:9:
   GCCGTTTCAG CTGGGGCGCC GAAGGCCAGC GTCCGGGCTT CGGCTACGGC 50
   GGCCGTGCGT CCGACTATAA ATCTGCTCAC AAAGGCTTCA AAGGCGTGGA 100
   TGCCCAGGGC ACCCTGTCC 119
(2) INFORMATION FOR SEQ ID NO:10:
   (i) SEQUENCE CHARACTERISTICS:
      (A) LENGTH: 111 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: PCR primer oligonucleotide 6
   (iii) HYPOTHETICAL: No
   (iv) ANTI-SENSE: Yes
   (xi) SEQUENCE DESCRIPTION: SEQ ID NO:10:
   CCCCAAGCTT ATTAGTGATG GTGATGGTGA TGACGTCTAG CCATCGGAGA 50
   GCCAGAACGG CTATCACGGC CGCCCAGTTT GAAAATTTTG GACAGGGTGC 100
   CCTGGGCATC C 111
(2) INFORMATION FOR SEQ ID NO:11:
   (i) SEQUENCE CHARACTERISTICS:
      (A) LENGTH: 4059 base pairs
      (B) TYPE: Nucleic Acid
      (C) STRANDEDNESS: Double
      (D) TOPOLOGY: Circular
   (ii) MOLECULE TYPE: Other nucleic acid
      (A) DESCRIPTION: Apex-1 Eukaryotic Expression Vector
   (xi) SEQUENCE DESCRIPTION: SEQ ID NO:11:
   ACGCGTTGAC ATTGATTATT GACTAGTTAT TAATAGTAAT CAATTACGGG 50
   GTCATTAGTT CATAGCCCAT ATATGGAGTT CCGCGTTACA TAACTTACGG 100
   TAAATGGCCC CGCCTGGCTG ACCGCCCAAC GACCCCCGCC CATTGACGTC 150
   AATAATGACG TATGTTCCCA TAGTAACGCC AATAGGGACT TTCCATTGAC 200
   GTCAATGGGT GGACTATTTA CGGTAAACTG CCCACTTGGC AGTACATCAA 250
   GTGTATCATA TGCCAAGTAC GCCCCCTATT GACGTCAATG ACGGTAAATG 300
   GCCCGCCTGG CATTATGCCC AGTACATGAC CTTATGGGAC TTTCCTACTT 350
   GGCAGTACAT CTACGTATTA GTCATCGCTA TTACCATGGT GATGCGGTTT 400
   TGGCAGTACA TCAATGGGCG TGGATAGCGG TTTGACTCAC GGGGATTTCC 450
   AAGTCTCCAC CCCATTGACG TCAATGGGAG TTTGTTTTGG CACCAAAATC 500
   AACGGGACTT TCCAAAATGT CGTAACAACT CCGCCCCATT GACGCAAATG 550
   GGCGGTAGGC GTGTACGGTG GGAGGTCTAT ATAAGCAGAG CTCGTTTAGT 600
   GAACCGTCAG AATTCTGTTG GGCTCGCGGT TGATTACAAA CTCTTCGCGG 650
   TCTTTCCAGT ACTCTTGGAT CGGAAACCCG TCGGCCTCCG AACGGTACTC 700
   CGCCACCGAG GGACCTGAGC GAGTCCGCAT CGACCGGATC GGAAAACCTC 750
   TCGACTGTTG GGGTGAGTAC TCCCTCTCAA AAGCGGGCAT GACTTCTGCG 800
   CTAAGATTGT CAGTTTCCAA AAACGAGGAG GATTTGATAT TCACCTGGCC 850
   CGCGGTGATG CCTTTGAGGG TGGCCGCGTC CATCTGGTCA GAAAAGACAA 900
   TCTTTTTGTT GTCAAGCTTG AGGTGTGGCA GGCTTGAGAT CTGGCCATAC 950
   ACTTGAGTGA CAATGACATC CACTTTGCCT TTCTCTCCAC AGGTGTCCAC 1000
   TCCCAGGTCC AACTGCAGGT CGACCGGCTT GGTACCGAGC TCGGATCCAC 1050
   TAGTAACGGC CGCCAGTGTG CTGGAATTCT GCAGATATCC ATCACACTGG 1100
   CGGCCGCTCG AGCATGCATC TAGAACTTGT TTATTGCAGC TTATAATGGT 1150
   TACAAATAAA GCAATAGCAT CACAAATTTC ACAAATAAAG CATTTTTTTC 1200
   ACTGCATTCT AGTTGTGGTT TGTCCAAACT CATCAATGTA TCTTATCATG 1250
   TCTGGATCGA TCCCGCCATG GTATCAACGC CATATTTCTA TTTACAGTAG 1300
   GGACCTCTTC GTTGTGTAGG TACCGCTGTA TTCCTAGGGA AATAGTAGAG 1350
   GCACCTTGAA CTGTCTGCAT CAGCCATATA GCCCCCGCTG TTCGACTTAC 1400
   AAACACAGGC ACAGTACTGA CAAACCCATA CACCTCCTCT GAAATACCCA 1450
   TAGTTGCTAG GGCTGTCTCC GAACTCATTA CACCCTCCAA AGTCAGAGCT 1500
   GTAATTTCGC CATCAAGGGC AGCGAGGGCT TCTCCAGATA AAATAGCTTC 1550
   TGCCGAGAGT CCCGTAAGGG TAGACACTTC AGCTAATCCC TCGATGAGGT 1600
   CTACTAGAAT AGTCAGTGCG GCTCCCATTT TGAAAATTCA CTTACTTGAT 1650
   CAGCTTCAGA AGATGGCGGA GGGCCTCCAA CACAGTAATT TTCCTCCCGA 1700
   CTCTTAAAAT AGAAAATGTC AAGTCAGTTA AGCAGGAAGT GGACTAACTG 1750
   ACGCAGCTGG CCGTGCGACA TCCTCTTTTA ATTAGTTGCT AGGCAACGCC 1800
   CTCCAGAGGG CGTGTGGTTT TGCAAGAGGA AGCAAAAGCC TCTCCACCCA 1850
   GGCCTAGAAT GTTTCCACCC AATCATTACT ATGACAACAG CTGTTTTTTT 1900
   TAGTATTAAG CAGAGGCCGG GGACCCCTGG GCCCGCTTAC TCTGGAGAAA 1950
   AAGAAGAGAG GCATTGTAGA GGCTTCCAGA GGCAACTTGT CAAAACAGGA 2000
   CTGCTTCTAT TTCTGTCACA CTGTCTGGCC CTGTCACAAG GTCCAGCACC 2050
   TCCATACCCC CTTTAATAAG CAGTTTGGGA ACGGGTGCGG GTCTTACTCC 2100
   GCCCATCCCG CCCCTAACTC CGCCCAGTTC CGCCCATTCT CCGCCCCATG 2150
   GCTGACTAAT TTTTTTTATT TATGCAGAGG CCGAGGCCGC CTCGGCCTCT 2200
   GAGCTATTCC AGAAGTAGTG AGGAGGCTTT TTTGGAGGCC TAGGCTTTTG 2250
   CAAAAAGGAG CTCCCAGCAA AAGGCCAGGA ACCGTAAAAA GGCCGCGTTG 2300
   CTGGCGTTTT TCCATAGGCT CCGCCCCCCT GACGAGCATC ACAAAAATCG 2350
   ACGCTCAAGT CAGAGGTGGC GAAACCCGAC AGGACTATAA AGATACCAGG 2400
   CGTTTCCCCC TGGAAGCTCC CTCGTGCGCT CTCCTGTTCC GACCCTGCCG 2450
   CTTACCGGAT ACCTGTCCGC CTTTCTCCCT TCGGGAAGCG TGGCGCTTTC 2500
   TCAATGCTCA CGCTGTAGGT ATCTCAGTTC GGTGTAGGTC GTTCGCTCCA 2550
   AGCTGGGCTG TGTGCACGAA CCCCCCGTTC AGCCCGACCG CTGCGCCTTA 2600
   TCCGGTAACT ATCGTCTTGA GTCCAACCCG GTAAGACACG ACTTATCGCC 2650
   ACTGGCAGCA GCCACTGGTA ACAGGATTAG CAGAGCGAGG TATGTAGGCG 2700
   GTGCTACAGA GTTCTTGAAG TGGTGGCCTA ACTACGGCTA CACTAGAAGG 2750
   ACAGTATTTG GTATCTGCGC TCTGCTGAAG CCAGTTACCT TCGGAAAAAG 2800
   AGTTGGTAGC TCTTGATCCG GCAAACAAAC CACCGCTGGT AGCGGTGGTT 2850
   TTTTTGTTTG CAAGCAGCAG ATTACGCGCA GAAAAAAAGG ATCTCAAGAA 2900
   GATCCTTTGA TCTTTTCTAC GGGGTCTGAC GCTCAGTGGA ACGAAAACTC 2950
   ACGTTAAGGG ATTTTGGTCA TGAGATTATC AAAAAGGATC TTCACCTAGA 3000
   TCCTTTTAAA TTAAAAATGA AGTTTTAAAT CAATCTAAAG TATATATGAG 3050
   TAAACTTGGT CTGACAGTTA CCAATGCTTA ATCAGTGAGG CACCTATCTC 3100
   AGCGATCTGT CTATTTCGTT CATCCATAGT TGCCTGACTC CCCGTCGTGT 3150
   AGATAACTAC GATACGGGAG GGCTTACCAT CTGGCCCCAG TGCTGCAATG 3200
   ATACCGCGAG ACCCACGCTC ACCGGCTCCA GATTTATCAG CAATAAACCA 3250
   GCCAGCCGGA AGGGCCGAGC GCAGAAGTGG TCCTGCAACT TTATCCGCCT 3300
   CCATCCAGTC TATTAATTGT TGCCGGGAAG CTAGAGTAAG TAGTTCGCCA 3350
   GTTAATAGTT TGCGCAACGT TGTTGCCATT GCTACAGGCA TCGTGGTGTC 3400
   ACGCTCGTCG TTTGGTATGG CTTCATTCAG CTCCGGTTCC CAACGATCAA 3450
   GGCGAGTTAC ATGATCCCCC ATGTTGTGCA AAAAAGCGGT TAGCTCCTTC 3500
   GGTCCTCCGA TCGTTGTCAG AAGTAAGTTG GCCGCAGTGT TATCACTCAT 3550
   GGTTATGGCA GCACTGCATA ATTCTCTTAC TGTCATGCCA TCCGTAAGAT 3600
   GCTTTTCTGT GACTGGTGAG TACTCAACCA AGTCATTCTG AGAATAGTGT 3650
   ATGCGGCGAC CGAGTTGCTC TTGCCCGGCG TCAATACGGG ATAATACCGC 3700
   GCCACATAGC AGAACTTTAA AAGTGCTCAT CATTGGAAAA CGTTCTTCGG 3750
   GGCGAAAACT CTCAAGGATC TTACCGCTGT TGAGATCCAG TTCGATGTAA 3800
   CCCACTCGTG CACCCAACTG ATCTTCAGCA TCTTTTACTT TCACCAGCGT 3850
   TTCTGGGTGA GCAAAAACAG GAAGGCAAAA TGCCGCAAAA AAGGGAATAA 3900
   GGGCGACACG GAAATGTTGA ATACTCATAC TCTTCCTTTT TCAATATTAT 3950
   TGAAGCATTT ATCAGGGTTA TTGTCTCATG AGCGGATACA TATTTGAATG 4000
   TATTTAGAAA AATAAACAAA TAGGGGTTCC GCGCACATTT CCCCGAAAAG 4050
   TGCCACCTG 4059
(2) INFORMATION FOR SEQ ID NO:12:
   (i) SEQUENCE CHARACTERISTICS:
      (A) LENGTH: 8540 base pairs
      (B) TYPE: Nucleic Acid
      (C) STRANDEDNESS: Double
      (D) TOPOLOGY: Circular
   (ii) MOLECULE TYPE: Other nucleic acid
      (A) DESCRIPTION: Apex-3P Eukaryotic Expression Vector
   (xi) SEQUENCE DESCRIPTION: SEQ ID NO:12:
   GTGACCAATA CAAAACAAAA GCGCCCCTCG TACCAGCGAA GAAGGGGCAG 50
   AGATGCCGTA GTCAGGTTTA GTTCGTCCGG CGGCGGGGGA TCTGTATGGT 100
   GCACTCTCAG TACAATCTGC TCTGATGCCG CATAGTTAAG CCAGTATCTG 150
   CTCCCTGCTT GTGTGTTGGA GGTCGCTGAG TAGTGCGCGA GCAAAATTTA 200
   AGCTACAACA AGGCAAGGCT TGACCGACAA TTGCATGAAG AATCTGCTTA 250
   GGGTTAGGCG TTTTGCGCTG CTTCGCGATG TACGGGCCAG ATATACGCGT 300
   TGACATTGAT TATTGACTAG TTATTAATAG TAATCAATTA CGGGGTCATT 350
   AGTTCATAGC CCATATATGG AGTTCCGCGT TACATAACTT ACGGTAAATG 400
   GCCCGCCTGG CTGACCGCCC AACGACCCCC GCCCATTGAC GTCAATAATG 450
   ACGTATGTTC CCATAGTAAC GCCAATAGGG ACTTTCCATT GACGTCAATG 500
   GGTGGACTAT TTACGGTAAA CTGCCCACTT GGCAGTACAT CAAGTGTATC 550
   ATATGCCAAG TACGCCCCCT ATTGACGTCA ATGACGGTAA ATGGCCCGCC 600
   TGGCATTATG CCCAGTACAT GACCTTATGG GACTTTCCTA CTTGGCAGTA 650
   CATCTACGTA TTAGTCATCG CTATTACCAT GGTGATGCGG TTTTGGCAGT 700
   ACATCAATGG GCGTGGATAG CGGTTTGACT CACGGGGATT TCCAAGTCTC 750
   CACCCCATTG ACGTCAATGG GAGTTTGTTT TGGCACCAAA ATCAACGGGA 800
   CTTTCCAAAA TGTCGTAACA ACTCCGCCCC ATTGACGCAA ATGGGCGGTA 850
   GGCGTGTACG GTGGGAGGTC TATATAAGCA GAGCTCGTTT AGTGAACCGT 900
   CAGAATTCTG TTGGGCTCGC GGTTGATTAC AAACTCTTCG CGGTCTTTCC 950
   AGTACTCTTG GATCGGAAAC CCGTCGGCCT CCGAACGGTA CTCCGCCACC 1000
   GAGGGACCTG AGCGAGTCCG CATCGACCGG ATCGGAAAAC CTCTCGACTG 1050
   TTGGGGTGAG TACTCCCTCT CAAAAGCGGG CATGACTTCT GCGCTAAGAT 1100
   TGTCAGTTTC CAAAAACGAG GAGGATTTGA TATTCACCTG GCCCGCGGTG 1150
   ATGCCTTTGA GGGTGGCCGC GTCCATCTGG TCAGAAAAGA CAATCTTTTT 1200
   GTTGTCAAGC TTGAGGTGTG GCAGGCTTGA GATCTGGCCA TACACTTGAG 1250
   TGACAATGAC ATCCACTTTG CCTTTCTCTC CACAGGTGTC CACTCCCAGG 1300
   TCCAACTGCA GGTCGACCGG CTTGGTACCG AGCTCGGATC CTCTAGAGTC 1350
   GACCTGCAGG CATGCAAGCT TGGCACTGGC CGTCGTTTTA CAACGTCGTG 1400
   ACTGGGAAAA CCCTGGCGTT ACCCAACTTA ATCGCCTTGC AGCACATCCC 1450
   CCTTTCGCCA GCTGGCGTAA TAGCGAAGAG GCCCGCACCG ATCCAGACAT 1500
   GATAAGATAC ATTGATGAGT TTGGACAAAC CACAACTAGA ATGCAGTGAA 1550
   AAAAATGCTT TATTTGTGAA ATTTGTGATG CTATTGCTTT ATTTGTAACC 1600
   ATTATAAGCT GCAATAAACA AGTTAACAAC AACAATTGCA TTCATTTTAT 1650
   GTTTCAGGTT CAGGGGGAGG TGTGGGAGGT TTTTTAAAGC AAGTAAAACC 1700
   TCTACAAATG TGGTATGGCT GATTATGATC CCCAGGAAGC TCCTCTGTGT 1750
   CCTCATAAAC CCTAACCTCC TCTACTTGAG AGGACATTCC AATCATAGGC 1800
   TGCCCATCCA CCCTCTGTGT CCTCCTGTTA ATTAGGTCAC TTAACAAAAA 1850
   GGAAATTGGG TAGGGGTTTT TCACAGACCG CTTTCTAAGG GTAATTTTAA 1900
   AATATCTGGG AAGTCCCTTC CACTGCTGTG TTCCAGAAGT GTTGGTAAAC 1950
   AGCCCACAAA TGTCAACAGC AGAAACATAC AAGCTGTCAG CTTTGCACAA 2000
   GGGCCCAACA CCCTGCTCAT CAAGAAGCAC TGTGGTTGCT GTGTTAGTAA 2050
   TGTGCAAAAC AGGAGGCACA TTTTCCCCAC CTGTGTAGGT TCCAAAATAT 2100
   CTAGTGTTTT CATTTTTACT TGGATCAGGA ACCCAGCACT CCACTGGATA 2150
   AGCATTATCC TTATCCAAAA CAGCCTTGTG GTCAGTGTTC ATCTGCTGAC 2200
   TGTCAACTGT AGCATTTTTT GGGGTTACAG TTTGAGCAGG ATATTTGGTC 2250
   CTGTAGTTTG CTAACACACC CTGCAGCTCC AAAGGTTCCC CACCAACAGC 2300
   AAAAAAATGA AAATTTGACC CTTGAATGGG TTTTCCAGCA CCATTTTCAT 2350
   GAGTTTTTTG TGTCCCTGAA TGCAAGTTTA ACATAGCAGT TACCCCAATA 2400
   ACCTCAGTTT TAACAGTAAC AGCTTCCCAC ATCAAAATAT TTCCACAGGT 2450
   TAAGTCCTCA TTTGTAGAAT TCGCCAGCAC AGTGGTCGAC CCTGTGGATG 2500
   TGTGTCACTT AGGGTGTGGA AAGTCCCCAG GCTCCCCAGC AGGCAGAAGT 2550
   ATGCAAAGCA TGCATCTCAA TTAGTCAGCA ACCAGGTGTG GAAAGTCCCC 2600
   AGGCTCCCCA GCAGGCAGAA GTATGCAAAG CATGCATCTC AATTAGTCAG 2650
   CAACCATAGT CCCGCCCCTA ACTCCGCCCA TCCCGCCCCT AACTCCGCCC 2700
   AGTTCCGCCC ATTCTCCGCC CCATGGCTGA CTAATTTTTT TTATTTATGC 2750
   AGAGGCCGAG GCCGCCTCGG CCTCTGAGCT ATTCCAGAAG TAGTGAGGAG 2800
   GCTTTTTTGG AGGCCTAGGC TTTTGCAAAA GCTTACCATG ACCGAGTACA 2850
   AGCCCACGGT GCGCCTCGCC ACCCGCGACG ACGTCCCCCG GGCCGTACGC 2900
   ACCCTCGCCG CCGCGTTCGC CGACTACCCC GCCACGCGCC ACACCGTCGA 2950
   CCCGGACCGC CACATCGAGC GGGTCACCGA GCTGCAAGAA CTCTTCCTCA 3000
   CGCGCGTCGG GCTCGACATC GGCAAGGTGT GGGTCGCGGA CGACGGCGCC 3050
   GCGGTGGCGG TCTGGACCAC GCCGGAGAGC GTCGAAGCGG GGGCGGTGTT 3100
   CGCCGAGATC GGCCCGCGCA TGGCCGAGTT GAGCGGTTCC CGGCTGGCCG 3150
   CGCAGCAACA GATGGAAGGC CTCCTGGCGC CGCACCGGCC CAAGGAGCCC 3200
   GCGTGGTTCC TGGCCACCGT CGGCGTCTCG CCCGACCACC AGGGCAAGGG 3250
   TCTGGGCAGC GCCGTCGTGC TCCCCGGAGT GGAGGCGGCC GAGCGCGCCG 3300
   GGGTGCCCGC CTTCCTGGAG ACCTCCGCGC CCCGCAACCT CCCCTTCTAC 3350
   GAGCGGCTCG GCTTCACCGT CACCGCCGAC GTCGAGTGCC CGAAGGACCG 3400
   CGCGACCTGG TGCATGACCC GCAAGCCCGG TGCCTGACGC CCGCCCCACG 3450
   ACCCGCAGCG CCCGACCGAA AGGAGCGCAC GACCCCATGC ATCGATAAAA 3500
   TAAAAGATTT TATTTAGTCT CCAGAAAAAG GGGGGAATGA AAGACCCCAC 3550
   CTGTAGGTTT GGCAAGCTAG AACTTGTTTA TTGCAGCTTA TAATGGTTAC 3600
   AAATAAAGCA ATAGCATCAC AAATTTCACA AATAAAGCAT TTTTTTCACT 3650
   GCATTCTAGT TGTGGTTTGT CCAAACTCAT CAATGTATCT TATCATGTCT 3700
   GGATCGATCC CGCCATGGTA TCAACGCCAT ATTTCTATTT ACAGTAGGGA 3750
   CCTCTTCGTT GTGTAGGTAC CCCGGGTTCG AAATCGAATT CGCCAATGAC 3800
   AAGACGCTGG GCGGGGTTTG TGTCATCATA GAACTAAAGA CATGCAAATA 3850
   TATTTCTTCC GGGGACACCG CCAGCAAACG CGAGCAACGG GCCACGGGGA 3900
   TGAAGCAGCC CGGCGGCACC TCGCTAACGG ATTCACCACT CCAAGAATTG 3950
   GAGCCAATCA ATTCTTGCGG AGAACTGTGA ATGCGCAAAC CAACCCTTGG 4000
   CAGAACATAT CCATCGCGTC CGCCATCTCC AGCAGCCGCA CGCGGCGCAT 4050
   CTCGGGGCCG ACGCGCTGGG CTACGTCTTG CTGGCGTTCG CGACGCGAGG 4100
   CTGGATGGCC TTCCCCATTA TGATTCTTCT CGCTTCCGGC GGCATCGGGA 4150
   TGCCCGCGTT GCAGGCCATG CTGTCCAGGC AGGTAGATGA CGACCATCAG 4200
   GGACAGCTTC AAGGATCGCT CGCGGCTCTT ACCAGCGCCA GCAAAAGGCC 4250
   AGGAACCGTA AAAAGGCCGC GTTGCTGGCG TTTTTCCATA GGCTCCGCCC 4300
   CCCTGACGAG CATCACAAAA ATCGACGCTC AAGTCAGAGG TGGCGAAACC 4350
   CGACAGGACT ATAAAGATAC CAGGCGTTTC CCCCTGGAAG CTCCCTCGTG 4400
   CGCTCTCCTG TTCCGACCCT GCCGCTTACC GGATACCTGT CCGCCTTTCT 4450
   CCCTTCGGGA AGCGTGGCGC TTTCTCATAG CTCACGCTGT AGGTATCTCA 4500
   GTTCGGTGTA GGTCGTTCGC TCCAAGCTGG GCTGTGTGCA CGAACCCCCC 4550
   GTTCAGCCCG ACCGCTGCGC CTTATCCGGT AACTATCGTC TTGAGTCCAA 4600
   CCCGGTAAGA CACGACTTAT CGCCACTGGC AGCAGCCACT GGTAACAGGA 4650
   TTAGCAGAGC GAGGTATGTA GGCGGTGCTA CAGAGTTCTT GAAGTGGTGG 4700
   CCTAACTACG GCTACACTAG AAGGACAGTA TTTGGTATCT GCGCTCTGCT 4750
   GAAGCCAGTT ACCTTCGGAA AAAGAGTTGG TAGCTCTTGA TCCGGCAAAC 4800
   AAACCACCGC TGGTAGCGGT GGTTTTTTTG TTTGCAAGCA GCAGATTACG 4850
   CGCAGAAAAA AAGGATCTCA AGAAGATCCT TTGATCTTTT CTACGGGGTC 4900
   TGACGCTCAG TGGAACGAAA ACTCACGTTA AGGGATTTTG GTCATGAGAT 4950
   TATCAAAAAG GATCTTCACC TAGATCCTTT TAAATTAAAA ATGAAGTTTT 5000
   AAATCAATCT AAAGTATATA TGAGTAAACT TGGTCTGACA GTTACCAATG 5050
   CTTAATCAGT GAGGCACCTA TCTCAGCGAT CTGTCTATTT CGTTCATCCA 5100
   TAGTTGCCTG ACTCCCCGTC GTGTAGATAA CTACGATACG GGAGGGCTTA 5150
   CCATCTGGCC CCAGTGCTGC AATGATACCG CGAGACCCAC GCTCACCGGC 5200
   TCCAGATTTA TCAGCAATAA ACCAGCCAGC CGGAAGGGCC GAGCGCAGAA 5250
   GTGGTCCTGC AACTTTATCC GCCTCCATCC AGTCTATTAA TTGTTGCCGG 5300
   GAAGCTAGAG TAAGTAGTTC GCCAGTTAAT AGTTTGCGCA ACGTTGTTGC 5350
   CATTGCTGCA GGCATCGTGG TGTCACGCTC GTCGTTTGGT ATGGCTTCAT 5400
   TCAGCTCCGG TTCCCAACGA TCAAGGCGAG TTACATGATC CCCCATGTTG 5450
   TGCAAAAAAG CGGTTAGCTC CTTCGGTCCT CCGATCGTTG TCAGAAGTAA 5500
   GTTGGCCGCA GTGTTATCAC TCATGGTTAT GGCAGCACTG CATAATTCTC 5550
   TTACTGTCAT GCCATCCGTA AGATGCTTTT CTGTGACTGG TGAGTACTCA 5600
   ACCAAGTCAT TCTGAGAATA GTGTATGCGG CGACCGAGTT GCTCTTGCCC 5650
   GGCGTCAACA CGGGATAATA CCGCGCCACA TAGCAGAACT TTAAAAGTGC 5700
   TCATCATTGG AAAACGTTCT TCGGGGCGAA AACTCTCAAG GATCTTACCG 5750
   CTGTTGAGAT CCAGTTCGAT GTAACCCACT CGTGCACCCA ACTGATCTTC 5800
   AGCATCTTTT ACTTTCACCA GCGTTTCTGG GTGAGCAAAA ACAGGAAGGC 5850
   AAAATGCCGC AAAAAAGGGA ATAAGGGCGA CACGGAAATG TTGAATACTC 5900
   ATACTCTTCC TTTTTCAATA TTATTGAAGC ATTTATCAGG GTTATTGTCT 5950
   CATGAGCGGA TACATATTTG AATGTATTTA GAAAAATAAA CAAATAGGGG 6000
   TTCCGCGCAC ATTTCCCCGA AAAGTGCCAC CTGACGTCTA AGAAACCATT 6050
   ATTATCATGA CATTAACCTA TAAAAATAGG CGTATCACGA GGCCCTTTCG 6100
   TCTTCAAGAA TTCTCATGTT TGACAGCTTA TCGTAGACAT CATGCGTGCT 6150
   GTTGGTGTAT TTCTGGCCAT CTGTCTTGTC ACCATTTTCG TCCTCCCAAC 6200
   ATGGGGCAAT TGGGCATACC CATGTTGTCA CGTCACTCAG CTCCGCGCTC 6250
   AACACCTTCT CGCGTTGGAA AACATTAGCG ACATTTACCT GGTGAGCAAT 6300
   CAGACATGCG ACGGCTTTAG CCTGGCCTCC TTAAATTCAC CTAAGAATGG 6350
   GAGCAACCAG CAGGAAAAGG ACAAGCAGCG AAAATTCACG CCCCCTTGGG 6400
   AGGTGGCGGC ATATGCAAAG GATAGCACTC CCACTCTACT ACTGGGTATC 6450
   ATATGCTGAC TGTATATGCA TGAGGATAGC ATATGCTACC CGGATACAGA 6500
   TTAGGATAGC ATATACTACC CAGATATAGA TTAGGATAGC ATATGCTACC 6550
   CAGATATAGA TTAGGATAGC CTATGCTACC CAGATATAAA TTAGGATAGC 6600
   ATATACTACC CAGATATAGA TTAGGATAGC ATATGCTACC CAGATATAGA 6650
   TTAGGATAGC CTATGCTACC CAGATATAGA TTAGGATAGC ATATGCTACC 6700
   CAGATATAGA TTAGGATAGC ATATGCTATC CAGATATTTG GGTAGTATAT 6750
   GCTACCCAGA TATAAATTAG GATAGCATAT ACTACCCTAA TCTCTATTAG 6800
   GATAGCATAT GCTACCCGGA TACAGATTAG GATAGCATAT ACTACCCAGA 6850
   TATAGATTAG GATAGCATAT GCTACCCAGA TATAGATTAG GATAGCCTAT 6900
   GCTACCCAGA TATAAATTAG GATAGCATAT ACTACCCAGA TATAGATTAG 6950
   GATAGCATAT GCTACCCAGA TATAGATTAG GATAGCCTAT GCTACCCAGA 7000
   TATAGATTAG GATAGCATAT GCTATCCAGA TATTTGGGTA GTATATGCTA 7050
   CCCATGGCAA CATTAGCCCA CCGTGCTCTC AGCGACCTCG TGAATATGAG 7100
   GACCAACAAC CCTGTGCTTG GCGCTCAGGC GCAAGTGTGT GTAATTTGTC 7150
   CTCCAGATCG CAGCAATCGC GCCCCTATCT TGGCCCGCCC ACCTACTTAT 7200
   GCAGGTATTC CCCGGGGTGC CATTAGTGGT TTTGTGGGCA AGTGGTTTGA 7250
   CCGCAGTGGT TAGCGGGGTT ACAATCAGCC AAGTTATTAC ACCCTTATTT 7300
   TACAGTCCAA AACCGCAGGG CGGCGTGTGG GGGCTGACGC GTGCCCCCAC 7350
   TCCACAATTT CAAAAAAAAG AGTGGCCACT TGTCTTTGTT TATGGGCCCC 7400
   ATTGGCGTGG AGCCCCGTTT AATTTTCGGG GGTGTTAGAG ACAACCAGTG 7450
   GAGTCCGCTG CTGTCGGCGT CCACTCTCTT TCCCCTTGTT ACAAATAGAG 7500
   TGTAACAACA TGGTTCACCT GTCTTGGTCC CTGCCTGGGA CACATCTTAA 7550
   TAACCCCAGT ATCATATTGC ACTAGGATTA TGTGTTGCCC ATAGCCATAA 7600
   ATTCGTGTGA GATGGACATC CAGTCTTTAC GGCTTGTCCC CACCCCATGG 7650
   ATTTCTATTG TTAAAGATAT TCAGAATGTT TCATTCCTAC ACTAGTATTT 7700
   ATTGCCCAAG GGGTTTGTGA GGGTTATATT GGTGTCATAG CACAATGCCA 7750
   CCACTGAACC CCCCGTCCAA ATTTTATTCT GGGGGCGTCA CCTGAAACCT 7800
   TGTTTTCGAG CACCTCACAT ACACCTTACT GTTCACAACT CAGCAGTTAT 7850
   TCTATTAGCT AAACGAAGGA GAATGAAGAA GCAGGCGAAG ATTCAGGAGA 7900
   GTTCACTGCC CGCTCCTTGA TCTTCAGCCA CTGCCCTTGT GACTAAAATG 7950
   GTTCACTACC CTCGTGGAAT CCTGACCCCA TGTAAATAAA ACCGTGACAG 8000
   CTCATGGGGT GGGAGATATC GCTGTTCCTT AGGACCCTTT TACTAACCCT 8050
   AATTCGATAG CATATGCTTC CCGTTGGGTA ACATATGCTA TTGAATTAGG 8100
   GTTAGTCTGG ATAGTATATA CTACTACCCG GGAAGCATAT GCTACCCGTT 8150
   TAGGGTTAAC AAGGGGGCCT TATAAACACT ATTGCTAATG CCCTCTTGAG 8200
   GGTCCGCTTA TCGGTAGCTA CACAGGCCCC TCTGATTGAC GTTGGTGTAG 8250
   CCTCCCGTAG TCTTCCTGGG CCCCTGGGAG GTACATGTCC CCCAGCATTG 8300
   GTGTAAGAGC TTCAGCCAAG AGTTACACAT AAAGGCAATG TTGTGTTGCA 8350
   GTCCACAGAC TGCAAAGTCT GCTCCAGGAT GAAAGCCACT CAGTGTTGGC 8400
   AAATGTGCAC ATCCATTTAT AAGGATGTCA ACTACAGTCA GAGAACCCCT 8450
   TTGTGTTTGG TCCCCCCCCG TGTCACATGT GGAACAGGGC CCAGTTGGCA 8500
   AGTTGTACCA ACCAACTGAA GGGATTACAT GCACTGCCCC 8540
(2) INFORMATION FOR SEQ ID NO:13:
   (i) SEQUENCE CHARACTERISTICS:
      (A) LENGTH: 22 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: PCR primer N-terminus of hMBP18.5 (MASQKR)
   (iii) HYPOTHETICAL: No
   (iv) ANTI-SENSE: No
   (xi) SEQUENCE DESCRIPTION: SEQ ID NO:13:
      CATATGGCGT CACAGAAGAG AC 22
(2) INFORMATION FOR SEQ ID NO:14:
   (i) SEQUENCE CHARACTERISTICS:
      (A) LENGTH: 26 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: MBP C-terminal (PMARR) PCR primer
   (iii) HYPOTHETICAL: No
   (iv) ANTI-SENSE: Yes
   (xi) SEQUENCE DESCRIPTION: SEQ ID NO:14:
      GGATCCTTAG CGTCTAGCCA TGGGTG 26
(2) INFORMATION FOR SEQ ID NO:15:
   (i) SEQUENCE CHARACTERISTICS:
      (A) LENGTH: 36 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: PCR mutagenic Ser 81 primer
   (iii) HYPOTHETICAL: No
   (iv) ANTI-SENSE: Yes
   (xi) SEQUENCE DESCRIPTION: SEQ ID NO:15:
      GTCTTTGTAC ATGTTCGACA GGCCCGGCTG GCTACG 36
(2) INFORMATION FOR SEQ ID NO:16:
   (i) SEQUENCE CHARACTERISTICS:
      (A) LENGTH: 14 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: PCR primer for Ser mutagenesis
   (iii) HYPOTHETICAL: No
   (iv) ANTI-SENSE: No
   (xi) SEQUENCE DESCRIPTION: SEQ ID NO:16:
      CAGCACCATG GACC 14
(2) INFORMATION FOR SEQ ID NO:17:
   (i) SEQUENCE CHARACTERISTICS:
      (A) LENGTH: 128 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: X2 PCR primer
   (iii) HYPOTHETICAL: No
   (iv) ANTI-SENSE: No
   (xi) SEQUENCE DESCRIPTION: SEQ ID NO:17:
   GGTGCGCCAA AGCGGGGCTC TGGCAAGGTA CCCTGGCTAA AGCCGGGCCG 50
   GAGCCCTCTG CCCTCTCATG CCCGCAGCCA GCCTGGGCTG TGCAACATGT 100
   ACAAGGACTC ACACCACCCG GCAAGAAC   128
(2) INFORMATION FOR SEQ ID NO:18:
   (i) SEQUENCE CHARACTERISTICS:
      (A) LENGTH: 19 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: T7 terminator primer
   (iii) HYPOTHETICAL: No
   (iv) ANTI-SENSE: Yes
   (xi) SEQUENCE DESCRIPTION: SEQ ID NO:18:
      GCTAGTTATT GCTCAGCGG 19
(2) INFORMATION FOR SEQ ID NO:19:
   (i) SEQUENCE CHARACTERISTICS:
      (A) LENGTH: 20 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: T7 promoter primer T7PP
   (iii) HYPOTHETICAL: No
   (iv) ANTI-SENSE: No
   (xi) SEQUENCE DESCRIPTION: SEQ ID NO:19:
      TAATACGACT CACTATAGGG 20
(2) INFORMATION FOR SEQ ID NO:20:
   (i) SEQUENCE CHARACTERISTICS:
      (A) LENGTH: 39 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: 3' primer for X2 assembly
   (iii) HYPOTHETICAL: No
   (iv) ANTI-SENSE: No
   (xi) SEQUENCE DESCRIPTION: SEQ ID NO:20:
      GGCTTTAGCC AGGGTACCTT GCCAGAGCCC CGCTTTGGC 39
(2) INFORMATION FOR SEQ ID NO:21:
   (i) SEQUENCE CHARACTERISTICS:
      (A) LENGTH: 5248 base pairs
      (B) TYPE: Nucleic Acid
      (C) STRANDEDNESS: Double
      (D) TOPOLOGY: Circular
   (ii) MOLECULE TYPE: Other nucleic acid
      (A) DESCRIPTION: pET Trc S05/NI prokaryotic expression vector
   (xi) SEQUENCE DESCRIPTION: SEQ ID NO:21:
   TGGCGAATGG GACGCGCCCT GTAGCGGCGC ATTAAGCGCG GCGGGTGTGG 50
   TGGTTACGCG CAGCGTGACC GCTACACTTG CCAGCGCCCT AGCGCCCGCT 100
   CCTTTCGCTT TCTTCCCTTC CTTTCTCGCC ACGTTCGCCG GCTTTCCCCG 150
   TCAAGCTCTA AATCGGGGGC TCCCTTTAGG GTTCCGATTT AGTGCTTTAC 200
   GGCACCTCGA CCCCAAAAAA CTTGATTAGG GTGATGGTTC ACGTAGTGGG 250
   CCATCGCCCT GATAGACGGT TTTTCGCCCT TTGACGTTGG AGTCCACGTT 300
   CTTTAATAGT GGACTCTTGT TCCAAACTGG AACAACACTC AACCCTATCT 350
   CGGTCTATTC TTTTGATTTA TAAGGGATTT TGCCGATTTC GGCCTATTGG 400
   TTAAAAAATG AGCTGATTTA ACAAAAATTT AACGCGAATT TTAACAAAAT 450
   ATTAACGTTT ACAATTTCAG GTGGCACTTT TCGGGGAAAT GTGCGCGGAA 500
   CCCCTATTTG TTTATTTTTC TAAATACATT CAAATATGTA TCCGCTCATG 550
   AGACAATAAC CCTGATAAAT GCTTCAATAA TATTGAAAAA GGAAGAGTAT 600
   GAGTATTCAA CATTTCCGTG TCGCCCTTAT TCCCTTTTTT GCGGCATTTT 650
   GCCTTCCTGT TTTTGCTCAC CCAGAAACGC TGGTGAAAGT AAAAGATGCT 700
   GAAGATCAGT TGGGTGCACG AGTGGGTTAC ATCGAACTGG ATCTCAACAG 750
   CGGTAAGATC CTTGAGAGTT TTCGCCCCGA AGAACGTTTT CCAATGATGA 800
   GCACTTTTAA AGTTCTGCTA TGTGGCGCGG TATTATCCCG TATTGACGCC 850
   GGGCAAGAGC AACTCGGTCG CCGCATACAC TATTCTCAGA ATGACTTGGT 900
   TGAGTACTCA CCAGTCACAG AAAAGCATCT TACGGATGGC ATGACAGTAA 950
   GAGAATTATG CAGTGCTGCC ATAACCATGA GTGATAACAC TGCGGCCAAC 1000
   TTACTTCTGA CAACGATCGG AGGACCGAAG GAGCTAACCG CTTTTTTGCA 1050
   CAACATGGGG GATCATGTAA CTCGCCTTGA TCGTTGGGAA CCGGAGCTGA 1100
   ATGAAGCCAT ACCAAACGAC GAGCGTGACA CCACGATGCC TGCAGCAATG 1150
   GCAACAACGT TGCGCAAACT ATTAACTGGC GAACTACTTA CTCTAGCTTC 1200
   CCGGCAACAA TTAATAGACT GGATGGAGGC GGATAAAGTT GCAGGACCAC 1250
   TTCTGCGCTC GGCCCTTCCG GCTGGCTGGT TTATTGCTGA TAAATCTGGA 1300
   GCCGGTGAGC GTGGGTCTCG CGGTATCATT GCAGCACTGG GGCCAGATGG 1350
   TAAGCCCTCC CGTATCGTAG TTATCTACAC GACGGGGAGT CAGGCAACTA 1400
   TGGATGAACG AAATAGACAG ATCGCTGAGA TAGGTGCCTC ACTGATTAAG 1450
   CATTGGTAAC TGTCAGACCA AGTTTACTCA TATATACTTT AGATTGATTT 1500
   AAAACTTCAT TTTTAATTTA AAAGGATCTA GGTGAAGATC CTTTTTGATA 1550
   ATCTCATGAC CAAAATCCCT TAACGTGAGT TTTCGTTCCA CTGAGCGTCA 1600
   GACCCCGTAG AAAAGATCAA AGGATCTTCT TGAGATCCTT TTTTTCTGCG 1650
   CGTAATCTGC TGCTTGCAAA CAAAAAAACC ACCGCTACCA GCGGTGGTTT 1700
   GTTTGCCGGA TCAAGAGCTA CCAACTCTTT TTCCGAAGGT AACTGGCTTC 1750
   AGCAGAGCGC AGATACCAAA TACTGTCCTT CTAGTGTAGC CGTAGTTAGG 1800
   CCACCACTTC AAGAACTCTG TAGCACCGCC TACATACCTC GCTCTGCTAA 1850
   TCCTGTTACC AGTGGCTGCT GCCAGTGGCG ATAAGTCGTG TCTTACCGGG 1900
   TTGGACTCAA GACGATAGTT ACCGGATAAG GCGCAGCGGT CGGGCTGAAC 1950
   GGGGGGTTCG TGCACACAGC CCAGCTTGGA GCGAACGACC TACACCGAAC 2000
   TGAGATACCT ACAGCGTGAG CTATGAGAAA GCGCCACGCT TCCCGAAGGG 2050
   AGAAAGGCGG ACAGGTATCC GGTAAGCGGC AGGGTCGGAA CAGGAGAGCG 2100
   CACGAGGGAG CTTCCAGGGG GAAACGCCTG GTATCTTTAT AGTCCTGTCG 2150
   GGTTTCGCCA CCTCTGACTT GAGCGTCGAT TTTTGTGATG CTCGTCAGGG 2200
   GGGCGGAGCC TATGGAAAAA CGCCAGCAAC GCGGCCTTTT TACGGTTCCT 2250
   GGCCTTTTGC TGGCCTTTTG CTCACATGTT CTTTCCTGCG TTATCCCCTG 2300
   ATTCTGTGGA TAACCGTATT ACCGCCTTTG AGTGAGCTGA TACCGCTCGC 2350
   CGCAGCCGAA CGACCGAGCG CAGCGAGTCA GTGAGCGAGG AAGCGGAAGA 2400
   GCGCCTGATG CGGTATTTTC TCCTTACGCA TCTGTGCGGT ATTTCACACC 2450
   GCATATATGG TGCACTCTCA GTACAATCTG CTCTGATGCC GCATAGTTAA 2500
   GCCAGTATAC ACTCCGCTAT CGCTACGTGA CTGGGTCATG GCTGCGCCCC 2550
   GACACCCGCC AACACCCGCT GACGCGCCCT GACGGGCTTG TCTGCTCCCG 2600
   GCATCCGCTT ACAGACAAGC TGTGACCGTC TCCGGGAGCT GCATGTGTCA 2650
   GAGGTTTTCA CCGTCATCAC CGAAACGCGC GAGGCAGCTG CGGTAAAGCT 2700
   CATCAGCGTG GTCGTGAAGC GATTCACAGA TGTCTGCCTG TTCATCCGCG 2750
   TCCAGCTCGT TGAGTTTCTC CAGAAGCGTT AATGTCTGGC TTCTGATAAA 2800
   GCGGGCCATG TTAAGGGCGG TTTTTTCCTG TTTGGTCACT GATGCCTCCG 2850
   TGTAAGGGGG ATTTCTGTTC ATGGGGGTAA TGATACCGAT GAAACGAGAG 2900
   AGGATGCTCA CGATACGGGT TACTGATGAT GAACATGCCC GGTTACTGGA 2950
   ACGTTGTGAG GGTAAACAAC TGGCGGTATG GATGCGGCGG GACCAGAGAA 3000
   AAATCACTCA GGGTCAATGC CAGCGCTTCG TTAATACAGA TGTAGGTGTT 3050
   CCACAGGGTA GCCAGCAGCA TCCTGCGATG CAGATCCGGA ACATAATGGT 3100
   GCAGGGCGCT GACTTCCGCG TTTCCAGACT TTACGAAACA CGGAAACCGA 3150
   AGACCATTCA TGTTGTTGCT CAGGTCGCAG ACGTTTTGCA GCAGCAGTCG 3200
   CTTCACGTTC GCTCGCGTAT CGGTGATTCA TTCTGCTAAC CAGTAAGGCA 3250
   ACCCCGCCAG CCTAGCCGGG TCCTCAACGA CAGGAGCACG ATCATGCGCA 3300
   CCCGTGGGGC CGCCATGCCG GCGATAATGG CCTGCTTCTC GCCGAAACGT 3350
   TTGGTGGCGG GACCAGTGAC GAAGGCTTGA GCGAGGGCGT GCAAGATTCC 3400
   GAATACCGCA AGCGACAGGC CGATCATCGT CGCGCTCCAG CGAAAGCGGT 3450
   CCTCGCCGAA AATGACCCAG AGCGCTGCCG GCACCTGTCC TACGAGTTGC 3500
   ATGATAAAGA AGACAGTCAT AAGTGCGGCG ACGATAGTCA TGCCCCGCGC 3550
   CCACCGGAAG GAGCTGACTG GGTTGAAGGC TCTCAAGGGC ATCGGTCGAG 3600
   ATCCCGGTGC CTAATGAGTG AGCTAACTTA CATTAATTGC GTTGCGCTCA 3650
   CTGCCCGCTT TCCAGTCGGG AAACCTGTCG TGCCAGCTGC ATTAATGAAT 3700
   CGGCCAACGC GCGGGGAGAG GCGGTTTGCG TATTGGGCGC CAGGGTGGTT 3750
   TTTCTTTTCA CCAGTGAGAC GGGCAACAGC TGATTGCCCT TCACCGCCTG 3800
   GCCCTGAGAG AGTTGCAGCA AGCGGTCCAC GCTGGTTTGC CCCAGCAGGC 3850
   GAAAATCCTG TTTGATGGTG GTTAACGGCG GGATATAACA TGAGCTGTCT 3900
   TCGGTATCGT CGTATCCCAC TACCGAGATA TCCGCACCAA CGCGCAGCCC 3950
   GGACTCGGTA ATGGCGCGCA TTGCGCCCAG CGCCATCTGA TCGTTGGCAA 4000
   CCAGCATCGC AGTGGGAACG ATGCCCTCAT TCAGCATTTG CATGGTTTGT 4050
   TGAAAACCGG ACATGGCACT CCAGTCGCCT TCCCGTTCCG CTATCGGCTG 4100
   AATTTGATTG CGAGTGAGAT ATTTATGCCA GCCAGCCAGA CGCAGACGCG 4150
   CCGAGACAGA ACTTAATGGG CCCGCTAACA GCGCGATTTG CTGGTGACCC 4200
   AATGCGACCA GATGCTCCAC GCCCAGTCGC GTACCGTCTT CATGGGAGAA 4250
   AATAATACTG TTGATGGGTG TCTGGTCAGA GACATCAAGA AATAACGCCG 4300
   GAACATTAGT GCAGGCAGCT TCCACAGCAA TGGCATCCTG GTCATCCAGC 4350
   GGATAGTTAA TGATCAGCCC ACTGACGCGT TGCGCGAGAA GATTGTGCAC 4400
   CGCCGCTTTA CAGGCTTCGA CGCCGCTTCG TTCTACCATC GACACCACCA 4450
   CGCTGGCACC CAGTTGATCG GCGCGAGATT TAATCGCCGC GACAATTTGC 4500
   GACGGCGCGT GCAGGGCCAG ACTGGAGGTG GCAACGCCAA TCAGCAACGA 4550
   CTGTTTGCCC GCCAGTTGTT GTGCCACGCG GTTGGGAATG TAATTCAGCT 4600
   CCGCCATCGC CGCTTCCACT TTTTCCCGCG TTTTCGCAGA AACGTGGCTG 4650
   GCCTGGTTCA CCACGCGGGA AACGGTCTGA TAAGAGACAC CGGCATACTC 4700
   TGCGACATCG TATAACGTTA CTGGTTTCAC ATTCACCACC CTGAATTGAC 4750
   TCTCTTCCGG GCGCTATCAT GCCATACCGC GAAAGGTTTT GCGCCATTCG 4800
   ATGGTGTCCG GGATCTCGAC GCTCTCCCTT ATGCGACTCC TGCATTAGGA 4850
   AGCAGCCCAG TAGTAGGTTG AGGCCGTTGA GCACCGCCGC CGCAAGGAAT 4900
   GGTGCATGCG GTACCAGCTG TTGACAATTA ATCATCCGGC TCGTATAATA 4950
   GTACTGTGTG GAATTGTGAG CGCTCACAAT TCCACACATC TAGAAATAAT 5000
   TTTGTTTAAC TTTAAGAAGG AGATATACCA TGGAGATCTG GATCCATCGA 5050
   TGAATTCGAG CTCCGTCGAC AAGCTTGCGG CCGCACTCGA GCACCACCAC 5100
   CACCACCACT GAGATCCGGC TGCTAACAAA GCCCGAAAGG AAGCTGAGTT 5150
   GGCTGCTGCC ACCGCTGAGC AATAACTAGC ATAACCCCTT GGGGCCTCTA 5200
   AACGGGTCTT GAGGGGTTTT TTGCTGAAAG GAGGAACTAT ATCCGGAT 5248
(2) INFORMATION FOR SEQ ID NO:22:
   (i) SEQUENCE CHARACTERISTICS:
      (A) LENGTH: 277 amino acids
      (B) TYPE: Amino Acid
      (C) STRANDEDNESS: Single
      (D) TOPOLOGY: Linear
   (ii) MOLECULE TYPE: Other nucleic acid
      (A) DESCRIPTION: native human PLP
   (iii) HYPOTHETICAL: No
   (x) PUBLICATION INFORMATION:
      (A) AUTHORS: Kronquist, K. E. Crandall, B. F. Macklin, W.B. Campagnoni, A. T. (B) TITLE: Expression of Myelin Proteins in the Developing Human Spinal Cord: Cloning and Sequencing of Human Proteolipid Protein cDNA (C) JOURNAL: Journal of Neuroscience Research (D) VOLUME: 18 (F) PAGES: 395 - 401 (G) DATE: 1987
   (xi) SEQUENCE DESCRIPTION: SEQ ID NO:22:
(2) INFORMATION FOR SEQ ID NO:23:
   (i) SEQUENCE CHARACTERISTICS:
      (A) LENGTH: 561 base pairs
      (B) TYPE: Nucleic acid
      (C) STRANDEDNESS: Double
      (D) TOPOLOGY: Linear
   (ii) MOLECULE TYPE: Other nucleic acid
      (A) DESCRIPTION: Delta PLP3
   (iii) HYPOTHETICAL: No
   (iv) ANTI-SENSE: No
   (xi) SEQUENCE DESCRIPTION: SEQ ID NO:23:
   ATG CTC 6
   Met Leu
   1
   GAG GAT CCG GGA CAT GAA GCC CTC ACT GGC ACA GAA AAG CTA        48
   Glu Asp Pro Gly His Glu Ala Leu Thr Gly Thr Glu Lys Leu
               5                  10                  15
   ATT GAG ACC TAT TTC TCC AAA AAC TAC CAA GAC TAT GAG TAT        90
   Ile Glu Thr Tyr Phe Ser Lys Asn Tyr Gln Asp Tyr Glu Tyr
                   20                  25                  30
   CTC ATC AAT GTG ATC CAT GCC TTC CAG TAT GTC ATC TAT GGA       132
   Leu Ile Asn Val Ile His Ala Phe Gln Tyr Val Ile Tyr Gly
                       35                  40
   ACT GCC TCT TTC TTC TTC CTT TAT GGG GCC CTC CTG CTG GCT       174
   Thr Ala Ser Phe Phe Phe Leu Tyr Gly Ala Leu Leu Leu Ala
    45                     50                  55
   GAG GGC TTC TAC ACC ACC GGC GCA GTC AGG CAG ATC TTT GGC       216
   Glu Gly Phe Tyr Thr Thr Gly Ala Val Arg Gln Ile Phe Gly
       60                      65                  70
   GAC TAC AAG ACC ACC ATC TGC GGC AAG GGC CTG AGC GCA ACG       258
   Asp Tyr Lys Thr Thr Ile Cys Gly Lys Gly Leu Ser Ala Thr
               75                  80                  85
   GTA ACA GGG GGC CAG AAG GGG AGG GGT TCC AGA GGC CAA CAT       300
   Val Thr Gly Gly Gln Lys Gly Arg Gly Ser Arg Gly Gln His
                   90                  95                  100
   CAA GCT CAT TCT TTG GAG CGG GTG TGT CAT TGT TTG GGA AAA       342
   Gln Ala His Ser Leu Glu Arg Val Cys His Cys Leu Gly Lys
                       105                 110
   TGG CTA GGA CAT CCC GAC AAG TTT GTG GGC ATC TTC AAC ACC       384
   Trp Leu Gly His Pro Asp Lys Phe Val Gly Ile Phe Asn Thr
   115                     120                 125
   TGG ACC ACC TGC CAG TCT ATT GCC TTC CCC AGC AAG ACC TCT       426
   Trp Thr Thr Cys Gln Ser Ile Ala Phe Pro Ser Lys Thr Ser
       130                     135                 140
   GCC AGT ATA GGC AGT CTC TGT GCT GAC GCC AGA ATG TAT GGT       468
   Ala Ser Ile Gly Ser Leu Cys Ala Asp Ala Arg Met Tyr Gly
               145                 150                 155
   GTT CTC CCA TGG AAT GCT TTC CCT GGC AAG GTT TGT GGC TCC       510
   Val Leu Pro Trp Asn Ala Phe Pro Gly Lys Val Cys Gly Ser
                   160                 165                 170
   AAC CTT CTG TCC ATC TGC AAA ACA GCT GAG TTC CAA ATG ACC       552
   Asn Leu Leu Ser Ile Cys Lys Thr Ala Glu Phe Gln Met Thr
                       175                 180
   TTC CAC TAA
   Phe His                                                       561
   185
(2) INFORMATION FOR SEQ ID NO:24:
   (i) SEQUENCE CHARACTERISTICS:
      (A) LENGTH: 525 base pairs
      (B) TYPE: Nucleic acid
      (C) STRANDEDNESS: Double
      (D) TOPOLOGY: Linear
   (ii) MOLECULE TYPE: Other nucleic acid
      (A) DESCRIPTION: Delta PLP4
   (iii) HYPOTHETICAL: No
   (iv) ANTI-SENSE: No
   (xi) SEQUENCE DESCRIPTION: SEQ ID NO:24:
   ATG CTC GAG GAT CCG GGA CAT GAA GCC CTC ACT GGC ACA
   Met Leu Glu Asp Pro Gly His Glu Ala Leu Thr Gly Thr       39
    1               5                  10
   GAA AAG CTA ATT GAG ACC TAT TTC TCC AAA AAC TAC CAA GAC
   Glu Lys Leu Ile Glu Thr Tyr Phe Ser Lys Asn Tyr Gln Asp       81
    15                     20                  25
   TAT GAG TAT CTC ATC AAT GTG ATC CAT GCC TTC CAG TAT GCT
   Tyr Glu Tyr Leu Ile Asn Val Ile His Ala Phe Gln Tyr Ala      123
           30                  35                  40
   GAG GGC TTC TAC ACC ACC GGC GCA GTC AGG CAG ATC TTT GGC
   Glu Gly Phe Tyr Thr Thr Gly Ala Val Arg Gln Ile Phe Gly      165
               45                  50                  55
   GAC TAC AAG ACC ACC ATC TGC GGC AAG GGC CTG AGC GCA ACG
   Asp Tyr Lys Thr Thr Ile Cys Gly Lys Gly Leu Ser Ala Thr      207
                   60                  65
   GTA ACA GGG GGC CAG AAG GGG AGG GGT TCC AGA GGC CAA CAT
   Val Thr Gly Gly Gln Lys Gly Arg Gly Ser Arg Gly Gln His      249
    70                 75                  80
   CAA GCT CAT TCT TTG GAG CGG GTG TGT CAT TGT TTG GGA AAA
   Gln Ala His Ser Leu Glu Arg Val Cys His Cys Leu Gly Lys      291
        85                 90                  95
   TGG CTA GGA CAT CCC GAC AAG TTT GTG GGC ATC TTC AAC ACC
   Trp Leu Gly His Pro Asp Lys Phe Val Gly Ile Phe Asn Thr      333
           100                 105                 110
   TGG ACC ACC TGC CAG TCT ATT GCC TTC CCC AGC AAG ACC TCT
   Trp Thr Thr Cys Gln Ser Ile Ala Phe Pro Ser Lys Thr Ser      375
               115                 120                 125
   GCC AGT ATA GGC AGT CTC TGT GCT GAC GCC AGA ATG TAT GGT
   Ala Ser Ile Gly Ser Leu Cys Ala Asp Ala Arg Met Tyr Gly      417
                   130                 135
   GTT CTC CCA TGG AAT GCT TTC CCT GGC AAG GTT TGT GGC TCC
   Val Leu Pro Trp Asn Ala Phe Pro Gly Lys Val Cys Gly Ser     459
   140                 145                 150
   AAC CTT CTG TCC ATC TGC AAA ACA GCT GAG TTC CAA ATG ACC
   Asn Leu Leu Ser Ile Cys Lys Thr Ala Glu Phe Gln Met Thr     501
       155                 160                 165
   TTC CAC CAT CAC CAT CAC CAT TAA
   Phe His His His His His His                                525
           170
(2) INFORMATION FOR SEQ ID NO:25:
   (i) SEQUENCE CHARACTERISTICS:
      (A) LENGTH: 1155 base pairs
      (B) TYPE: Nucleic acid
      (C) STRANDEDNESS: Double
      (D) TOPOLOGY: Linear
   (ii) MOLECULE TYPE: Other nucleic acid
      (A) DESCRIPTION: MP3 chimera
   (iii) HYPOTHETICAL: No
   (iv) ANTI-SENSE: No
   (xi) SEQUENCE DESCRIPTION: SEQ ID NO:25:
   ATG GCG TCT CAG AAA CGT CCG TCC CAG CGT CAC GGC TCC AAA
   Met Ala Ser Gln Lys Arg Pro Ser Gln Arg His Gly Ser Lys     42
     1             5                  10
   TAC CTG GCC ACC GCC AGC ACC ATG GAC CAT GCC CGT CAT GGC
   Tyr Leu Ala Thr Ala Ser Thr Met Asp His Ala Arg His Gly     84
    15                 20                  25
   TTC CTG CCG CGT CAC CGT GAC ACC GGC ATC CTG GAC TCC ATC
   Phe Leu Pro Arg His Arg Asp Thr Gly Ile Leu Asp Ser Ile    126
        30                 35                  40
   GGC CGC TTC TTC GGC GGT GAC CGT GGT GCG CCG AAA CGT GGC
   Gly Arg Phe Phe Gly Gly Asp Arg Gly Ala Pro Lys Arg Gly    168
            45                 50                  55
   TCT GGC AAA GTG CCG TGG CTG AAA CCG GGC CGT AGC CCG CTG
   Ser Gly Lys Val Pro Trp Leu Lys Pro Gly Arg Ser Pro Leu    210
                60                 65                  70
   CCG TCT CAT GCC CGT AGC CAG CCG GGC CTG TGC AAC ATG TAC
   Pro Ser His Ala Arg Ser Gln Pro Gly Leu Cys Asn Met Tyr    252
                   75                  80
   AAA GAC TCC CAC CAC CCG GCT CGT ACC GCG CAC TAT GGC TCC
   Lys Asp Ser His His Pro Ala Arg Thr Ala His Tyr Gly Ser      294
    85                  90                  95
   CTG CCG CAG AAA TCC CAC GGC CGT ACC CAG GAT GAA AAC CCG
   Leu Pro Gln Lys Ser His Gly Arg Thr Gln Asp Glu Asn Pro      336
       100                 105                 110
   GTG GTG CAC TTC TTC AAA AAC ATT GTG ACC CCG CGT ACC CCG
   Val Val His Phe Phe Lys Asn Ile Val Thr Pro Arg Thr Pro      378
           115                 120                 125
   CCG CCG TCT CAG GGC AAA GGC CGT GGC CTG TCC CTG AGC CGT
   Pro Pro Ser Gln Gly Lys Gly Arg Gly Leu Ser Leu Ser Arg      420
               130                 135                 140
   TTC AGC TGG GGC GCC GAA GGC CAG CGT CCG GGC TTC GGT TAC
   Phe Ser Trp Gly Ala Glu Gly Gln Arg Pro Gly Phe Gly Tyr      462
                   145                 150
   GGC GGC CGT GCG TCC GAC TAT AAA TCT GCT CAC AAA GGC TTC
   Gly Gly Arg Ala Ser Asp Tyr Lys Ser Ala His Lys Gly Phe      504
   155                 160                 165
   AAA GGC GTG GAT GCC CAG GGT ACC TTG TCC AAA ATT TTC AAA
   Lys Gly Val Asp Ala Gln Gly Thr Leu Ser Lys Ile Phe Lys      546
       170                 175                 180
   CTG GGC GGC CGT GAT AGC CGT TCT GGC TCT CCG ATG GCT AGA
   Leu Gly Gly Arg Asp Ser Arg Ser Gly Ser Pro Met Ala Arg      588
           185                 190                 195
   CGT CTG GGA GGC CTC GAG GAT CCG GGA CAT GAA GCC CTC ACT
   Arg Leu Gly Gly Leu Glu Asp Pro Gly His Glu Ala Leu Thr      630
               200                 205                 210
   GGC ACA GAA AAG CTA ATT GAG ACC TAT TTC TCC AAA AAC TAC
   Gly Thr Glu Lys Leu Ile Glu Thr Tyr Phe Ser Lys Asn Tyr      672
                   215                 220
   CAA GAC TAT GAG TAT CTC ATC AAT GTG ATC CAT GCC TTC CAG
   Gln Asp Tyr Glu Tyr Leu Ile Asn Val Ile His Ala Phe Gln      714
   225                 230                 235
   TAT GTC ATC TAT GGA ACT GCC TCT TTC TTC TTC CTT TAT GGG
   Tyr Val Ile Tyr Gly Thr Ala Ser Phe Phe Phe Leu Tyr Gly      756
       240                 245                 250
   GCC CTC CTG CTG GCT GAG GGC TTC TAC ACC ACC GGC GCA GTC
   Ala Leu Leu Leu Ala Glu Gly Phe Tyr Thr Thr Gly Ala Val      798
           255                 260                 265
   AGG CAG ATC TTT GGC GAC TAC AAG ACC ACC ATC TGC GGC AAG
   Arg Gln Ile Phe Gly Asp Tyr Lys Thr Thr Ile Cys Gly Lys      840
               270                 275                 280
   GGC CTG AGC GCA ACG GTA ACA GGG GGC CAG AAG GGG AGG GGT
   Gly Leu Ser Ala Thr Val Thr Gly Gly Gln Lys Gly Arg Gly      882
                   285                 290
   TCC AGA GGC CAA CAT CAA GCT CAT TCT TTG GAG CGG GTG TGT
   Ser Arg Gly Gln His Gln Ala His Ser Leu Glu Arg Val Cys          924
   295                 300                     305
   CAT TGT TTG GGA AAA TGG CTA GGA CAT CCC GAC AAG TTT GTG
   His Cys Leu Gly Lys Trp Leu Gly His Pro Asp Lys Phe Val          966
       310                     315                     320
   GGC ATC TTC AAC ACC TGG ACC ACC TGC CAG TCT ATT GCC TTC
   Gly Ile Phe Asn Thr Trp Thr Thr Cys Gln Ser Ile Ala Phe         1008
           325                     330                     335
   CCC AGC AAG ACC TCT GCC AGT ATA GGC AGT CTC TGT GCT GAC
   Pro Ser Lys Thr Ser Ala Ser Ile Gly Ser Leu Cys Ala Asp         1050
               340                     345                     350
   GCC AGA ATG TAT GGT GTT CTC CCA TGG AAT GCT TTC CCT GGC
   Ala Arg Met Tyr Gly Val Leu Pro Trp Asn Ala Phe Pro Gly         1092
                   355                     360
   AAG GTT TGT GGC TCC AAC CTT CTG TCC ATC TGC AAA ACA GCT
   Lys Val Cys Gly Ser Asn Leu Leu Ser Ile Cys Lys Thr Ala         1134
   365                 370                     375
   GAG TTC CAA ATG ACC TTC CAC
   Glu Phe Gln Met Thr Phe His                                     1155
       380                 385
(2) INFORMATION FOR SEQ ID NO: 26 :
   (i) SEQUENCE CHARACTERISTICS:
      (A) LENGTH: 1122 base pairs
      (B) TYPE: Nucleic acid
      (C) STRANDEDNESS: Double
      (D) TOPOLOGY: Linear
   (ii) MOLECULE TYPE: Other nucleic acid
      (A) DESCRIPTION: MP4 chimera
   (iii) HYPOTHETICAL: No
   (iv) ANTI-SENSE: No
   (xi) SEQUENCE DESCRIPTION: SEQ ID NO:26:
   ATG GCG TCT CAG AAA CGT CCG TCC CAG CGT CAC GGC TCC
   Met Ala Ser Gln Lys Arg Pro Ser Gln Arg His Gly Ser               39
    1               5                  10
   AAA TAC CTG GCC ACC GCC AGC ACC ATG GAC CAT GCC CGT CAT
   Lys Tyr Leu Ala Thr Ala Ser Thr Met Asp His Ala Arg His           81
        15                  20                  25
   GGC TTC CTG CCG CGT CAC CGT GAC ACC GGC ATC CTG GAC TCC
   Gly Phe Leu Pro Arg His Arg Asp Thr Gly Ile Leu Asp Ser        123
           30                  35                  40
   ATC GGC CGC TTC TTC GGC GGT GAC CGT GGT GCG CCG AAA CGT
   Ile Gly Arg Phe Phe Gly Gly Asp Arg Gly Ala Pro Lys Arg        165
               45                  50                  55
   GGC TCT GGC AAA GTG CCG TGG CTG AAA CCG GGC CGT AGC CCG
   Gly Ser Gly Lys Val Pro Trp Leu Lys Pro Gly Arg Ser Pro        207
                   60                  65
   CTG CCG TCT CAT GCC CGT AGC CAG CCG GGC CTG TGC AAC ATG
   Leu Pro Ser His Ala Arg Ser Gln Pro Gly Leu Cys Asn Met        249
   70                  75                  80
   TAC AAA GAC TCC CAC CAC CCG GCT CGT ACC GCG CAC TAT GGC
   Tyr Lys Asp Ser His His Pro Ala Arg Thr Ala His Tyr Gly        291
       85                  90                  95
   TCC CTG CCG CAG AAA TCC CAC GGC CGT ACC CAG GAT GAA AAC
   Ser Leu Pro Gln Lys Ser His Gly Arg Thr Gln Asp Glu Asn        333
           100                 105                 110
   CCG GTG GTG CAC TTC TTC AAA AAC ATT GTG ACC CCG CGT ACC
   Pro Val Val His Phe Phe Lys Asn Ile Val Thr Pro Arg Thr        375
               115                 120                 125
   CCG CCG CCG TCT CAG GGC AAA GGC CGT GGC CTG TCC CTG AGC
   Pro Pro Pro Ser Gln Gly Lys Gly Arg Gly Leu Ser Leu Ser        417
                   130                 135
   CGT TTC AGC TGG GGC GCC GAA GGC CAG CGT CCG GGC TTC GGT
   Arg Phe Ser Trp Gly Ala Glu Gly Gln Arg Pro Gly Phe Gly        459
   140                 145                 150
   TAC GGC GGC CGT GCG TCC GAC TAT AAA TCT GCT CAC AAA GGC
   Tyr Gly Gly Arg Ala Ser Asp Tyr Lys Ser Ala His Lys Gly        501
       155                 160                 165
   TTC AAA GGC GTG GAT GCC CAG GGT ACC TTG TCC AAA ATT TTC
   Phe Lys Gly Val Asp Ala Gln Gly Thr Leu Ser Lys Ile Phe        543
           170                 175                 180
   AAA CTG GGC GGC CGT GAT AGC CGT TCT GGC TCT CCG ATG GCT
   Lys Leu Gly Gly Arg Asp Ser Arg Ser Gly Ser Pro Met Ala        585
               185                 190                 195
   AGA CGT CTG GGA GGC CTC GAG GAT CCG GGA CAT GAA GCC CTC
   Arg Arg Leu Gly Gly Leu Glu Asp Pro Gly His Glu Ala Leu        627
                   200                 205
   ACT GGC ACA GAA AAG CTA ATT GAG ACC TAT TTC TCC AAA AAC
   Thr Gly Thr Glu Lys Leu Ile Glu Thr Tyr Phe Ser Lys Asn        669
   210                 215                 220
   TAC CAA GAC TAT GAG TAT CTC ATC AAT GTG ATC CAT GCC TTC
   Tyr Gln Asp Tyr Glu Tyr Leu Ile Asn Val Ile His Ala Phe        713
       225                 230                 235
   CAG TAT GCT GAG GGC TTC TAC ACC ACC GGC GCA GTC AGG CAG
   Gln Tyr Ala Glu Gly Phe Tyr Thr Thr Gly Ala Val Arg Gln        753
           240                     245                     250
   ATC TTT GGC GAC TAC AAG ACC ACC ATC TGC GGC AAG GGC CTG
   Ile Phe Gly Asp Tyr Lys Thr Thr Ile Cys Gly Lys Gly Leu        795
               255                     260                     265
   AGC GCA ACG GTA ACA GGG GGC CAG AAG GGG AGG GGT TCC AGA
   Ser Ala Thr Val Thr Gly Gly Gln Lys Gly Arg Gly Ser Arg        837
                   270                     275
   GGC CAA CAT CAA GCT CAT TCT TTG GAG CGG GTG TGT CAT TGT
   Gly Gln His Gln Ala His Ser Leu Glu Arg Val Cys His Cys        879
   280                 285                     290
   TTG GGA AAA TGG CTA GGA CAT CCC GAC AAG TTT GTG GGC ATC
   Leu Gly Lys Trp Leu Gly His Pro Asp Lys Phe Val Gly Ile        921
       295                     300                     305
   TTC AAC ACC TGG ACC ACC TGC CAG TCT ATT GCC TTC CCC AGC
   Phe Asn Thr Trp Thr Thr Cys Gln Ser Ile Ala Phe Pro Ser        963
           310                     315                     320
   AAG ACC TCT GCC AGT ATA GGC AGT CTC TGT GCT GAC GCC AGA
   Lys Thr Ser Ala Ser Ile Gly Ser Leu Cys Ala Asp Ala Arg       1005
               325                     330                     335
   ATG TAT GGT GTT CTC CCA TGG AAT GCT TTC CCT GGC AAG GTT
   Met Tyr Gly Val Leu Pro Trp Asn Ala Phe Pro Gly Lys Val       1047
                   340                     345
   TGT GGC TCC AAC CTT CTG TCC ATC TGC AAA ACA GCT GAG TTC
   Cys Gly Ser Asn Leu Leu Ser Ile Cys Lys Thr Ala Glu Phe       1089
   350                 355                     360
   CAA ATG ACC TTC CAC CAT CAC CAT CAC CAT TAA
   Gln Met Thr Phe His His His His His His                       1122
       365                     370
(2) INFORMATION FOR SEQ ID NO:27:
   (i) SEQUENCE CHARACTERISTICS:
      (A) LENGTH: 1125 base pairs
      (B) TYPE: Nucleic acid
      (C) STRANDEDNESS: Double
      (D) TOPOLOGY: Linear
   (ii) MOLECULE TYPE: Other nucleic acid
      (A) DESCRIPTION: PM4 chimera
   (iii) HYPOTHETICAL: No
   (iv) ANTI-SENSE: No
   (xi) SEQUENCE DESCRIPTION: SEQ ID NO:27:
   ATG CTC GAG GAT CCG GGA CAT GAA GCC CTC ACT GGC ACA GAA
   Met Leu Glu Asp Pro Gly His Glu Ala Leu Thr Gly Thr Glu        42
    1               5                  10
   AAG CTA ATT GAG ACC TAT TTC TCC AAA AAC TAC CAA GAC TAT
   Lys Leu Ile Glu Thr Tyr Phe Ser Lys Asn Tyr Gln Asp Tyr        84
    15                 20                  25
   GAG TAT CTC ATC AAT GTG ATC CAT GCC TTC CAG TAT GCT GAG
   Glu Tyr Leu Ile Asn Val Ile His Ala Phe Gln Tyr Ala Glu       126
       30                  35                  40
   GGC TTC TAC ACC ACC GGC GCA GTC AGG CAG ATC TTT GGC GAC
   Gly Phe Tyr Thr Thr Gly Ala Val Arg Gln Ile Phe Gly Asp       168
           45                  50                  55
   TAC AAG ACC ACC ATC TGC GGC AAG GGC CTG AGC GCA ACG GTA
   Tyr Lys Thr Thr Ile Cys Gly Lys Gly Leu Ser Ala Thr Val       210
               60                  65                  70
   ACA GGG GGC CAG AAG GGG AGG GGT TCC AGA GGC CAA CAT CAA
   Thr Gly Gly Gln Lys Gly Arg Gly Ser Arg Gly Gln His Gln       252
                   75                  80
   GCT CAT TCT TTG GAG CGG GTG TGT CAT TGT TTG GGA AAA TGG
   Ala His Ser Leu Glu Arg Val Cys His Cys Leu Gly Lys Trp       294
    85                 90                  95
   CTA GGA CAT CCC GAC AAG TTT GTG GGC ATC TTC AAC ACC TGG
   Leu Gly His Pro Asp Lys Phe Val Gly Ile Phe Asn Thr Trp       336
       100                 105                 110
   ACC ACC TGC CAG TCT ATT GCC TTC CCC AGC AAG ACC TCT GCC
   Thr Thr Cys Gln Ser Ile Ala Phe Pro Ser Lys Thr Ser Ala       378
           115                 120                 125
   AGT ATA GGC AGT CTC TGT GCT GAC GCC AGA ATG TAT GGT GTT
   Ser Ile Gly Ser Leu Cys Ala Asp Ala Arg Met Tyr Gly Val       420
               130                 135                 140
   CTC CCA TGG AAT GCT TTC CCT GGC AAG GTT TGT GGC TCC AAC
   Leu Pro Trp Asn Ala Phe Pro Gly Lys Val Cys Gly Ser Asn       462
                   145                 150
   CTT CTG TCC ATC TGC AAA ACA GCT GAG TTC CAA ATG ACC TTC
   Leu Leu Ser Ile Cys Lys Thr Ala Glu Phe Gln Met Thr Phe       504
   155                 160                 165
   CAC GGC GGT GGC GGT GCG TCT CAG AAA CGT CCG TCC CAG CGT
   His Gly Gly Gly Gly Ala Ser Gln Lys Arg Pro Ser Gln Arg       546
       170                 175                 180
   CAC GGC TCC AAA TAC CTG GCC ACC GCC AGC ACC ATG GAC CAT
   His Gly Ser Lys Tyr Leu Ala Thr Ala Ser Thr Met Asp His       588
           185                 190                 195
   GCC CGT CAT GGC TTC CTG CCG CGT CAC CGT GAC ACC GGC ATC
   Ala Arg His Gly Phe Leu Pro Arg His Arg Asp Thr Gly Ile        630
               200                 205                 210
   CTG GAC TCC ATC GGC CGC TTC TTC GGC GGT GAC CGT GGT GCG
   Leu Asp Ser Ile Gly Arg Phe Phe Gly Gly Asp Arg Gly Ala        672
                   215                 220
   CCG AAA CGT GGC TCT GGC AAA GTG CCG TGG CTG AAA CCG GGC
   Pro Lys Arg Gly Ser Gly Lys Val Pro Trp Leu Lys Pro Gly        714
   225                 230                 235
   CGT AGC CCG CTG CCG TCT CAT GCC CGT AGC CAG CCG GGC CTG
   Arg Ser Pro Leu Pro Ser His Ala Arg Ser Gln Pro Gly Leu        756
       240                 245                 250
   TGC AAC ATG TAC AAA GAC TCC CAC CAC CCG GCT CGT ACC GCG
   Cys Asn Met Tyr Lys Asp Ser His His Pro Ala Arg Thr Ala        798
           255                 260                 265
   CAC TAT GGC TCC CTG CCG CAG AAA TCC CAC GGC CGT ACC CAG
   His Tyr Gly Ser Leu Pro Gln Lys Ser His Gly Arg Thr Gln        840
               270                 275                 280
   GAT GAA AAC CCG GTG GTG CAC TTC TTC AAA AAC ATT GTG ACC
   Asp Glu Asn Pro Val Val His Phe Phe Lys Asn Ile Val Thr        882
                   285                 290
   CCG CGT ACC CCG CCG CCG TCT CAG GGC AAA GGC CGT GGC CTG
   Pro Arg Thr Pro Pro Pro Ser Gln Gly Lys Gly Arg Gly Leu        924
   295                 300                 305
   TCC CTG AGC CGT TTC AGC TGG GGC GCC GAA GGC CAG CGT CCG
   Ser Leu Ser Arg Phe Ser Trp Gly Ala Glu Gly Gln Arg Pro        966
       310                 315                 320
   GGC TTC GGC TAC GGC GGC CGT GCG TCC GAC TAT AAA TCT GCT
   Gly Phe Gly Tyr Gly Gly Arg Ala Ser Asp Tyr Lys Ser Ala       1008
           325                 330                 335
   CAC AAA GGC TTC AAA GGC GTG GAT GCC CAG GGC ACC CTG TCC
   His Lys Gly Phe Lys Gly Val Asp Ala Gln Gly Thr Leu Ser       1050
               340                 345                 350
   AAA ATT TTC AAA CTG GGC GGC CGT GAT AGC CGT TCT GGC TCT
   Lys Ile Phe Lys Leu Gly Gly Arg Asp Ser Arg Ser Gly Ser       1092
   355                 360                 365
   CCG ATG GCT AGA CGT CAT CAC CAT CAC CAT CAC
   Pro Met Ala Arg Arg His His His His His His                   1125
       370                 375
(2) INFORMATION FOR SEQ ID NO:28:
   (i) SEQUENCE CHARACTERISTICS:
      (A) LENGTH: 1476 base pairs
      (B) TYPE: Nucleic acid
      (C) STRANDEDNESS: Double
      (D) TOPOLOGY: Linear
   (ii) MOLECULE TYPE: Other nucleic acid
      (A) DESCRIPTION: MMOGP4 chimera
   (iii) HYPOTHETICAL: No
   (iv) ANTI-SENSE: No
   (xi) SEQUENCE DESCRIPTION: SEQ ID NO:28:
   ATG GCG TCT CAG AAA CGT CCG TCC CAG CGT CAC GGC TCC AAA TAC CTG    48
   Met Ala Ser Gln Lys Arg Pro Ser Gln Arg His Gly Ser Lys Tyr Leu
                    5                  10                  15
   GCC ACC GCC AGC ACC ATG GAC CAT GCC CGT CAT GGC TTC CTG CCG CGT    96
   Ala Thr Ala Ser Thr Met Asp His Ala Arg His Gly Phe Leu Pro Arg
               20                  25                  30
   CAC CGT GAC ACC GGC ATC CTG GAC TCC ATC GGC CGC TTC TTC GGC GGT   144
   His Arg Asp Thr Gly Ile Leu Asp Ser Ile Gly Arg Phe Phe Gly Gly
           35                  40                  45
   GAC CGT GGT GCG CCG AAA CGT GGC TCT GGC AAA GTG CCG TGG CTG AAA   192
   Asp Arg Gly Ala Pro Lys Arg Gly Ser Gly Lys Val Pro Trp Leu Lys
       50                  55                  60
   CCG GGC CGT AGC CCG CTG CCG TCT CAT GCC CGT AGC CAG CCG GGC CTG   240
   Pro Gly Arg Ser Pro Leu Pro Ser His Ala Arg Ser Gln Pro Gly Leu
   65                  70                  75                  80
   TGC AAC ATG TAC AAA GAC TCC CAC CAC CCG GCT CGT ACC GCG CAC TAT   288
   Cys Asn Met Tyr Lys Asp Ser His His Pro Ala Arg Thr Ala His Tyr
                   85                  90                  95
   GGC TCC CTG CCG CAG AAA TCC CAC GGC CGT ACC CAG GAT GAA AAC CCG   336
   Gly Ser Leu Pro Gln Lys Ser His Gly Arg Thr Gln Asp Glu Asn Pro
               100                 105                 110
   GTG GTG CAC TTC TTC AAA AAC ATT GTG ACC CCG CGT ACC CCG CCG CCG   384
   Val Val His Phe Phe Lys Asn Ile Val Thr Pro Arg Thr Pro Pro Pro
           115                 120                 125
   TCT CAG GGC AAA GGC CGT GGC CTG TCC CTG AGC CGT TTC AGC TGG GGC   432
   Ser Gln Gly Lys Gly Arg Gly Leu Ser Leu Ser Arg Phe Ser Trp Gly
       130                 135                 140
   GCC GAA GGC CAG CGT CCG GGC TTC GGT TAC GGC GGC CGT GCG TCC GAC   480
   Ala Glu Gly Gln Arg Pro Gly Phe Gly Tyr Gly Gly Arg Ala Ser Asp
   145                 150                 155                 160
   TAT AAA TCT GCT CAC AAA GGC TTC AAA GGC GTG GAT GCC CAG GGT ACC   528
   Tyr Lys Ser Ala His Lys Gly Phe Lys Gly Val Asp Ala Gln Gly Thr
                   165                 170                 175
   TTG TCC AAA ATT TTC AAA CTG GGC GGC CGT GAT AGC CGT TCT GGC TCT  576
   Leu Ser Lys Ile Phe Lys Leu Gly Gly Arg Asp Ser Arg Ser Gly Ser
               180                 185                 190
   CCG ATG GCT AGA CGT CCC GGG CAG TTC AGA GTG ATA GGA CCA AGA CAC  624
   Pro Met Ala Arg Arg Pro Gly Gln Phe Arg Val Ile Gly Pro Arg His
           195                 200                 205
   CCT ATC CGG GCT CTG GTC GGG GAT GAA GTG GAA TTG CCA TGT CGC ATA  672
   Pro Ile Arg Ala Leu Val Gly Asp Glu Val Glu Leu Pro Cys Arg Ile
       210                 215                 220
   TCT CCT GGG AAG AAC GCT ACA GGC ATG GAG GTG GGG TGG TAC CGC CCC  720
   Ser Pro Gly Lys Asn Ala Thr Gly Met Glu Val Gly Trp Tyr Arg Pro
   225                 230                 235                 240
   CCC TTC TCT AGG GTG GTT CAT CTC TAC AGA AAT GGC AAG GAC CAA GAT  768
   Pro Phe Ser Arg Val Val His Leu Tyr Arg Asn Gly Lys Asp Gln Asp
                   245                 250                 255
   GGA GAC CAG GCA CCT GAA TAT CGG GGC CGG ACA GAG CTG CTG AAA GAT  816
   Gly Asp Gln Ala Pro Glu Tyr Arg Gly Arg Thr Glu Leu Leu Lys Asp
               260                 265                 270
   GCT ATT GGT GAG GGA AAG GTG ACT CTC AGG ATC CGG AAT GTA AGG TTC  864
   Ala Ile Gly Glu Gly Lys Val Thr Leu Arg Ile Arg Asn Val Arg Phe
           275                 280                 285
   TCA GAT GAA GGA GGT TTC ACC TGC TTC TTC CGA GAT CAT TCT TAC CAA  912
   Ser Asp Glu Gly Gly Phe Thr Cys Phe Phe Arg Asp His Ser Tyr Gln
       290                 295                 300
   GAG GAG GCA GCA ATG GAA TTG AAA GTA GAA GAT CCC TTC TAC TGG CTC  960
   Glu Glu Ala Ala Met Glu Leu Lys Val Glu Asp Pro Phe Tyr Trp Leu
   305                 310                 315                 320
   GAG GAT CCG GGA CAT GAA GCC CTC ACT GGC ACA GAA AAG CTA ATT GAG 1008
   Glu Asp Pro Gly His Glu Ala Leu Thr Gly Thr Glu Lys Leu Ile Glu
                   325                 330                 335
   ACC TAT TTC TCC AAA AAC TAC CAA GAC TAT GAG TAT CTC ATC AAT GTG 1056
   Thr Tyr Phe Ser Lys Asn Tyr Gln Asp Tyr Glu Tyr Leu Ile Asn Val
               340                 345                 350
   ATC CAT GCC TTC CAG TAT GCT GAG GGC TTC TAC ACC ACC GGC GCA GTC 1104
   Ile His Ala Phe Gln Tyr Ala Glu Gly Phe Tyr Thr Thr Gly Ala Val
           355                 360                 365
   AGG CAG ATC TTT GGC GAC TAC AAG ACC ACC ATC TGC GGC AAG GGC CTG 1152
   Arg Gln Ile Phe Gly Asp Tyr Lys Thr Thr Ile Cys Gly Lys Gly Leu
       370                 375                 380
   AGC GCA ACG GTA ACA GGG GGC CAG AAG GGG AGG GGT TCC AGA GGC CAA 1200
   Ser Ala Thr Val Thr Gly Gly Gln Lys Gly Arg Gly Ser Arg Gly Gln
   385                 390                 395                 400
   CAT CAA GCT CAT TCT TTG GAG CGG GTG TGT CAT TGT TTG GGA AAA TGG 1248
   His Gln Ala His Ser Leu Glu Arg Val Cys His Cys Leu Gly Lys Trp
   405                 410                 415
   CTA GGA CAT CCC GAC AAG TTT GTG GGC ATC TTC AAC ACC TGG ACC ACC 1296
   Leu Gly His Pro Asp Lys Phe Val Gly Ile Phe Asn Thr Trp Thr Thr
               420                 425                 430
   TGC CAG TCT ATT GCC TTC CCC AGC AAG ACC TCT GCC AGT ATA GGC AGT 1344
   Cys Gln Ser Ile Ala Phe Pro Ser Lys Thr Ser Ala Ser Ile Gly Ser
           435                 440                 445
   CTC TGT GCT GAC GCC AGA ATG TAT GGT GTT CTC CCA TGG AAT GCT TTC 1392
   Leu Cys Ala Asp Ala Arg Met Tyr Gly Val Leu Pro Trp Asn Ala Phe
       450                 455                 460
   CCT GGC AAG GTT TGT GGC TCC AAC CTT CTG TCC ATC TGC AAA ACA GCT 1440
   Pro Gly Lys Val Cys Gly Ser Asn Leu Leu Ser Ile Cys Lys Thr Ala
   465                 470                 475                 480
   GAG TTC CAA ATG ACC TTC CAC CAT CAC CAT CAC CAT                 1476
   Glu Phe Gln Met Thr Phe His His His His His His
                   485                 490
(2) INFORMATION FOR SEQ ID NO:29:
   (i) SEQUENCE CHARACTERISTICS:
      (A) LENGTH: 732 base pairs
      (B) TYPE: Nucleic acid
      (C) STRANDEDNESS: Double
      (D) TOPOLOGY: Linear
   (ii) MOLECULE TYPE: Other nucleic acid
      (A) DESCRIPTION: Delta PLP2
   (iii) HYPOTHETICAL: No
   (iv) ANTI-SENSE: No
   (xi) SEQUENCE DESCRIPTION: SEQ ID NO:29:

### SEQUENCE LISTING

(1) GENERAL INFORMATION:
   (i) APPLICANTS:
      (A) NAME: Alexion Pharmaceuticals, Inc.
      (B) STREET: Suite 360, 25 Science Park
      (C) CITY: New Haven
      (E) COUNTRY: U.S.
      (F) POSTAL CODE (ZIP): CT 06511

      (A) NAME: United States of America, represented by The Secretary, Department of Health and Human Services
      (B) STREET: National Institutes of Health, Director, Office of Technology Transfer, Suite 325, 6011 Executive Boulevard
      (C) CITY: Rockville
      (E) COUNTRY: U.S.
      (F) POSTAL CODE (ZIP): MD 20852
   (ii) TITLE OF INVENTION: Modified Myelin Protein Molecules
   (iii) NUMBER OF SEQUENCES: 29
   (iv) COMPUTER READABLE FORM:
      (A) MEDIUM TYPE: 3.5 inch, 0.8 Mb storage
      (B) COMPUTER: IBM PC Compatible
      (C) OPERATING SYSTEM: PC-DOS/MS-DOS
      (D) SOFTWARE: Microsoft Word 6.0.1
(2) INFORMATION FOR SEQ ID NO:1:
   (i) SEQUENCE CHARACTERISTICS:
      (A) LENGTH: 594 base pairs
      (B) TYPE: Nucleic acid
      (C) STRANDEDNESS: Double
      (D) TOPOLOGY: Linear
   (ii) MOLECULE TYPE: cDNA to mRNA
      (A) DESCRIPTION: MBP+X2Cys81/hum. (Human 21.5 kD form of MBP)
   (iii) HYPOTHETICAL: No
   (iv) ANTI-SENSE: No
   (x) PUBLICATION INFORMATION:
      (A) AUTHORS: Roth, H. J. Kronquist, K. E. Kerlero de Rosbo, N. Crandall, B. F. Campagnoni, A. T. (B) TITLE: Evidence for the Expression of Four Myelin Basic Protein Variants in the Developing Human Spinal Cord Through cDNA Cloning (C) JOURNAL: Journal of Neuroscience Research (D) VOLUME: 17 (F) PAGES: 312 - 328 (G) DATE: 1987
   (xi) SEQUENCE DESCRIPTION: SEQ ID NO:1:
   ATG GCG TCA CAG AAG AGA CCC TCC CAG AGG CAC GGA TCC        39
   Met Ala Ser Gln Lys Arg Pro Ser Gln Arg His Gly Ser
     1               5                   10
   AAG TAC CTG GCC ACA GCA AGT ACC ATG GAC CAT GCC AGG CAT    81
   Lys Tyr Leu Ala Thr Ala Ser Thr Met Asp His Ala Arg His
       15                  20                  25
   GGC TTC CTC CCA AGG CAC AGA GAC ACG GGC ATC CTT GAC TCC   123
   Gly Phe Leu Pro Arg His Arg Asp Thr Gly Ile Leu Asp Ser
           30                  35                  40
   ATC GGG CGC TTC TTT GGC GGT GAC AGG GGT GCG CCC AAG CGG   165
   Ile Gly Arg Phe Phe Gly Gly Asp Arg Gly Ala Pro Lys Arg
               45                  50                  55
   GGC TCT GGC AAG GTA CCC TGG CTA AAG CCG GGC CGG AGC CCT   207
   Gly Ser Gly Lys Val Pro Trp Leu Lys Pro Gly Arg Ser Pro
                   60                  65
   CTG CCC TCT CAT GCC CGC AGC CAG CCT GGG CTG TGC AAC ATG   249
   Leu Pro Ser His Ala Arg Ser Gln Pro Gly Leu Cys Asn Met
    70                  75                  80
   TAC AAG GAC TCA CAC CAC CCG GCA AGA ACT GCT CAC TAT GGC   291
   Tyr Lys Asp Ser His His Pro Ala Arg Thr Ala His Tyr Gly
       85                  90                  95
   TCC CTG CCC CAG AAG TCA CAC GGC CGG ACC CAA GAT GAA AAC   333
   Ser Leu Pro Gln Lys Ser His Gly Arg Thr Gln Asp Glu Asn
           100                 105                 110
   CCC GTA GTC CAC TTC TTC AAG AAC ATT GTG ACG CCT CGC ACA   375
   Pro Val Val His Phe Phe Lys Asn Ile Val Thr Pro Arg Thr
               115                 120                 125
   CCA CCC CCG TCG CAG GGA AAG GGG AGA GGA CTG TCC CTG AGC   417
   Pro Pro Pro Ser Gln Gly Lys Gly Arg Gly Leu Ser Leu Ser
                   130                 135
   AGA TTT AGC TGG GGG GCC GAA GGC CAG AGA CCA GGA TTT GGC   459
   Arg Phe Ser Trp Gly Ala Glu Gly Gln Arg Pro Gly Phe Gly
   140                 145                 150
   TAC GGA GGC AGA GCG TCC GAC TAT AAA TCG GCT CAC AAG GGA   501
   Tyr Gly Gly Arg Ala Ser Asp Tyr Lys Ser Ala His Lys Gly
       155                 160                 165
   TTC AAG GGA GTC GAT GCC CAG GGC ACG CTT TCC AAA ATT TTC   543
   Phe Lys Gly Val Asp Ala Gln Gly Thr Leu Ser Lys Ile Phe
           170                 175                 180
   AAG CTG GGA GGA AGA GAT AGT CGC TCT GGA TCA CCC ATG GCT   585
   Lys Leu Gly Gly Arg Asp Ser Arg Ser Gly Ser Pro Met Ala
               185                 190                 200
   AGA CGC TGA                                               594
   Arg Arg
(2) INFORMATION FOR SEQ ID NO:2:
   (i) SEQUENCE CHARACTERISTICS:
      (A) LENGTH: 612 base pairs
      (B) TYPE: Nucleic acid
      (C) STRANDEDNESS: Double
      (D) TOPOLOGY: Linear
   (ii) MOLECULE TYPE: Other nucleic acid
      (A) DESCRIPTION: MBP+X2Cys81/bact.
   (iii) HYPOTHETICAL: No
   (iv) ANTI-SENSE: No
   (xi) SEQUENCE DESCRIPTION: SEQ ID NO:2:
   ATG GCG TCT CAG AAA CGT CCG TCC CAG CGT CAC GGC TCC AAA      42
   TAC CTG GCC ACC GCC AGC ACC ATG GAC CAT GCC CGT CAT GGC      84
   TTC CTG CCG CGT CAC CGT GAC ACC GGC ATC CTG GAC TCC ATC     126
   GGC CGC TTC TTC GGC GGT GAC CGT GGT GCG CCG AAA CGT GGC     168
   TCT GGC AAA GTG CCG TGG CTG AAA CCG GGC CGT AGC CCG CTG     210
   CCG TCT CAT GCC CGT AGC CAG CCG GGC CTG TGC AAC ATG TAC     252
   AAA GAC TCC CAC CAC CCG GCT CGT ACC GCG CAC TAT GGC TCC     294
   CTG CCG CAG AAA TCC CAC GGC CGT ACC CAG GAT GAA AAC CCG     336
   GTG GTG CAC TTC TTC AAA AAC ATT GTG ACC CCG CGT ACC CCG     378
   CCG CCG TCT CAG GGC AAA GGC CGT GGC CTG TCC CTG AGC CGT     420
   TTC AGC TGG GGC GCC GAA GGC CAG CGT CCG GGC TTC GGT TAC     462
   GGC GGC CGT GCG TCC GAC TAT AAA TCT GCT CAC AAA GGC TTC     504
   AAA GGC GTG GAT GCC CAG GGT ACC TTG TCC AAA ATT TTC AAA     546
   CTG GGC GGC CGT GAT AGC CGT TCT GGC TCT CCG ATG GCT AGA     588
   CGT CAT CAC CAT CAC CAT CAC TAA                             612
(2) INFORMATION FOR SEQ ID NO:3:
   (i) SEQUENCE CHARACTERISTICS:
      (A) LENGTH: 612 base pairs
      (B) TYPE: Nucleic acid
      (C) STRANDEDNESS: Double
      (D) TOPOLOGY: Linear
   (ii) MOLECULE TYPE: cDNA to mRNA
      (A) DESCRIPTION: MBP+X2Ser81/bact.
   (iii) HYPOTHETICAL: No
   (iv) ANTI-SENSE: No
   (xi) SEQUENCE DESCRIPTION: SEQ ID NO:3:
   ATG GCG TCT CAG AAA CGT CCG TCC CAG CGT CAC GGC TCC AAA       42
   Met Ala Ser Gln Lys Arg Pro Ser Gln Arg His Gly Ser Lys
    1               5                  10
   TAC CTG GCC ACC GCC AGC ACC ATG GAC CAT GCC CGT CAT GGC       84
   Tyr Leu Ala Thr Ala Ser Thr Met Asp His Ala Arg His Gly
    15                 20                  25
   TTC CTG CCG CGT CAC CGT GAC ACC GGC ATC CTG GAC TCC ATC      126
   Phe Leu Pro Arg His Arg Asp Thr Gly Ile Leu Asp Ser Ile
        30                 35                  40
   GGC CGC TTC TTC GGC GGT GAC CGT GGT GCG CCG AAA CGT GGC      168
   Gly Arg Phe Phe Gly Gly Asp Arg Gly Ala Pro Lys Arg Gly
            45                 50                  55
   TCT GGC AAA GTG CCG TGG CTG AAA CCG GGC CGT AGC CCG CTG      210
   Ser Gly Lys Val Pro Trp Leu Lys Pro Gly Arg Ser Pro Leu
                60                 65                  70
   CCG TCT CAT GCC CGT AGC CAG CCG GGC CTG TCG AAC ATG TAC      252
   Pro Ser His Ala Arg Ser Gln Pro Gly Leu Ser Asn Met Tyr
                    75                 80
   AAA GAC TCC CAC CAC CCG GCT CGT ACC GCG CAC TAT GGC TCC      294
   Lys Asp Ser His His Pro Ala Arg Thr Ala His Tyr Gly Ser
    85                 90                  95
   CTG CCG CAG AAA TCC CAC GGC CGT ACC CAG GAT GAA AAC CCG      336
   Leu Pro Gln Lys Ser His Gly Arg Thr Gln Asp Glu Asn Pro
       100                105                 110
   GTG GTG CAC TTC TTC AAA AAC ATT GTG ACC CCG CGT ACC CCG      378
   Val Val His Phe Phe Lys Asn Ile Val Thr Pro Arg Thr Pro
           115                 120                 125
   CCG CCG TCT CAG GGC AAA GGC CGT GGC CTG TCC CTG AGC CGT      420
   Pro Pro Ser Gln Gly Lys Gly Arg Gly Leu Ser Leu Ser Arg
               130                 135                 140
   TTC AGC TGG GGC GCC GAA GGC CAG CGT CCG GGC TTC GGT TAC      462
   Phe Ser Trp Gly Ala Glu Gly Gln Arg Pro Gly Phe Gly Tyr
                   145                 150
   GGC GGC CGT GCG TCC GAC TAT AAA TCT GCT CAC AAA GGC TTC      504
   Gly Gly Arg Ala Ser Asp Tyr Lys Ser Ala His Lys Gly Phe
   155                 160                 165
   AAA GGC GTG GAT GCC CAG GGT ACC TTG TCC AAA ATT TTC AAA      546
   Lys Gly Val Asp Ala Gln Gly Thr Leu Ser Lys Ile Phe Lys
       170                 175                 180
   CTG GGC GGC CGT GAT AGC CGT TCT GGC TCT CCG ATG GCT AGA      588
   Leu Gly Gly Arg Asp Ser Arg Ser Gly Ser Pro Met Ala Arg
           185                 190                 195
   CGT CAT CAC CAT CAC CAT CAC TAA                              612
   Arg His His His His His His
               200
(2) INFORMATION FOR SEQ ID NO:4:
   (i) SEQUENCE CHARACTERISTICS:
      (A) LENGTH: 519 base pairs
      (B) TYPE: Nucleic acid
      (C) STRANDEDNESS: Double
      (D) TOPOLOGY: Linear
   (ii) MOLECULE TYPE: cDNA to mRNA
      (A) DESCRIPTION: Human 18.5 kDa form of MBP
   (iii) HYPOTHETICAL: No
   (iv) ANTI-SENSE: No
   (xi) SEQUENCE DESCRIPTION: SEQ ID NO:4:
   CAT ATG GCG TCA CAG AAG AGA CCC TCC CAG AGG CAC GGA TCC     42
   His Met Ala Ser Gln Lys Arg Pro Ser Gln Arg His Gly Ser
         1               5                  10
   AAG TAC CTG GCC ACA GCA AGT ACC ATG GAC CAT GCC AGG CAT     84
   Lys Tyr Leu Ala Thr Ala Ser Thr Met Asp His Ala Arg His
        15                  20                  25
   GGC TTC CTC CCA AGG CAC AGA GAC ACG GGC ATC CTT GAC TCC    126
   Gly Phe Leu Pro Arg His Arg Asp Thr Gly Ile Leu Asp Ser
            30                  35                  40
   ATC GGG CGC TTC TTT GGC GGT GAC AGG GGT GCG CCA AAG CGG    168
   Ile Gly Arg Phe Phe Gly Gly Asp Arg Gly Ala Pro Lys Arg
                45                  50                  55
   GGC TCT GGC AAG GAC TCA CAC CAC CCG GCA AGA ACT GCT CAC    210
   Gly Ser Gly Lys Asp Ser His His Pro Ala Arg Thr Ala His
                    60                  65
   TAT GGC TCC CTG CCC CAG AAG TCA CAC GGC CGG ACC CAA GAT    252
   Tyr Gly Ser Leu Pro Gln Lys Ser His Gly Arg Thr Gln Asp
    70                  75                  80
   GAA AAC CCC GTA GTC CAC TTC TTC AAG AAC ATT GTG ACG CCT    294
   Glu Asn Pro Val Val His Phe Phe Lys Asn Ile Val Thr Pro
        85                  90                  95
   CGC ACA CCA CCC CCG TCG CAG GGA AAG GGG AGA GGA CTG TCC    336
   Arg Thr Pro Pro Pro Ser Gln Gly Lys Gly Arg Gly Leu Ser
           100                 105                 110
   CTG AGC AGA TTT AGC TGG GGG GCC GAA GGC CAG AGA CCA GGA    378
   Leu Ser Arg Phe Ser Trp Gly Ala Glu Gly Gln Arg Pro Gly
               115                 120                 125
   TTT GGC TAC GGA GGC AGA GCG TCC GAC TAT AAA TCG GCT CAC    420
   Phe Gly Tyr Gly Gly Arg Ala Ser Asp Tyr Lys Ser Ala His
                   130                 135
   AAG GGA TTC AAG GGA GTC GAT GCC CAG GGC ACG CTT TCC AAA    462
   Lys Gly Phe Lys Gly Val Asp Ala Gln Gly Thr Leu Ser Lys
   140                 145                 150
   ATT TTT AAG CTG GGA GGA AGA GAT AGT CGC TCT GGA TCA CCC    504
   Ile Phe Lys Leu Gly Gly Arg Asp Ser Arg Ser Gly Ser Pro
       155                 160                 165
   ATG GCT AGA CGC TAA                                        519
   Met Ala Arg Arg
           170
(2) INFORMATION FOR SEQ ID NO:5:
   (i) SEQUENCE CHARACTERISTICS:
      (A) LENGTH: 130 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: PCR primer oligonucleotide 1
   (iii) HYPOTHETICAL: No
   (iv) ANTI-SENSE: No
   (xi) SEQUENCE DESCRIPTION: SEQ ID NO:5:
   GGAATTCCGT AAGGAGGTAT AGCATATGGC GTCTCAGAAA CGTCCGTCCC    50
   AGCGTCACGG CTCCAAATAC CTGGCCACCG CCAGCACCAT GGACCATGCC   100
   CGTCATGGCT TCCTGCCGCG TCACCGTGAC                         130
(2) INFORMATION FOR SEQ ID NO:6:
   (i) SEQUENCE CHARACTERISTICS:
      (A) LENGTH: 129 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: PCR primer oligonucleotide 2
   (iii) HYPOTHETICAL: No
   (iv) ANTI-SENSE: Yes
   (xi) SEQUENCE DESCRIPTION: SEQ ID NO:6:
   GACGGCAGCG GGCTACGGCC CGGTTTCAGC CACGGCACTT TGCCAGAGCC    50
   ACGTTTCGGC GCACCACGGT CACCGCCGAA GAAGCGGCCG ATGGAGTCCA   100
   GGATGCCGGT GTCACGGTGA CGCGGCAGG                          129
(2) INFORMATION FOR SEQ ID NO:7:
   (i) SEQUENCE CHARACTERISTICS:
      (A) LENGTH: 133 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: PCR primer oligonucleotide 3
   (iii) HYPOTHETICAL: No
   (iv) ANTI-SENSE: No
   (xi) SEQUENCE DESCRIPTION: SEQ ID NO:7:
   CCGGGCCGTA GCCCGCTGCC GTCTCATGCC CGTAGCCAGC CGGGCCTGTG      50
   CAACATGTAC AAAGACTCCC ACCACCCGGC TCGTACCGCG CACTATGGCT     100
   CCCTGCCGCA GAAATCCCAC GGCCGTACCC AGG                       133
(2) INFORMATION FOR SEQ ID NO:8:
   (i) SEQUENCE CHARACTERISTICS:
      (A) LENGTH: 131 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: PCR primer oligonucleotide 4
   (iii) HYPOTHETICAL: No
   (iv) ANTI-SENSE: Yes
   (xi) SEQUENCE DESCRIPTION: SEQ ID NO:8:
   CGGCGCCCCA GCTGAAACGG CTCAGGGACA GGCCACGGCC TTTGCCCTGA      50
   GACGGCGGCG GGGTACGCGG GGTCACAATG TTTTTGAAGA AGTGCACCAC     100
   CGGGTTTTCA TCCTGGGTAC GGCCGTGGGA T                         131
(2) INFORMATION FOR SEQ ID NO:9:
   (i) SEQUENCE CHARACTERISTICS:
      (A) LENGTH: 119 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: PCR primer oligonucleotide 5
   (iii) HYPOTHETICAL: No
   (iv) ANTI-SENSE: No
   (xi) SEQUENCE DESCRIPTION: SEQ ID NO:9:
   GCCGTTTCAG CTGGGGCGCC GAAGGCCAGC GTCCGGGCTT CGGCTACGGC     50
   GGCCGTGCGT CCGACTATAA ATCTGCTCAC AAAGGCTTCA AAGGCGTGGA    100
   TGCCCAGGGC ACCCTGTCC                                      119
(2) INFORMATION FOR SEQ ID NO:10:
   (i) SEQUENCE CHARACTERISTICS:
      (A) LENGTH: 111 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: PCR primer oligonucleotide 6
   (iii) HYPOTHETICAL: No
   (iv) ANTI-SENSE: Yes
   (xi) SEQUENCE DESCRIPTION: SEQ ID NO:10:
   CCCCAAGCTT ATTAGTGATG GTGATGGTGA TGACGTCTAG CCATCGGAGA     50
   GCCAGAACGG CTATCACGGC CGCCCAGTTT GAAAATTTTG GACAGGGTGC    100
   CCTGGGCATC C                                              111
(2) INFORMATION FOR SEQ ID NO:11:
   (i) SEQUENCE CHARACTERISTICS:
      (A) LENGTH: 4059 base pairs
      (B) TYPE: Nucleic Acid
      (C) STRANDEDNESS: Double
      (D) TOPOLOGY: Circular
   (ii) MOLECULE TYPE: Other nucleic acid
      (A) DESCRIPTION: Apex-1 Eukaryotic Expression Vector
   (xi) SEQUENCE DESCRIPTION: SEQ ID NO:11:
   ACGCGTTGAC ATTGATTATT GACTAGTTAT TAATAGTAAT CAATTACGGG    50
   GTCATTAGTT CATAGCCCAT ATATGGAGTT CCGCGTTACA TAACTTACGG   100
   TAAATGGCCC CGCCTGGCTG ACCGCCCAAC GACCCCCGCC CATTGACGTC   150
   AATAATGACG TATGTTCCCA TAGTAACGCC AATAGGGACT TTCCATTGAC   200
   GTCAATGGGT GGACTATTTA CGGTAAACTG CCCACTTGGC AGTACATCAA   250
   GTGTATCATA TGCCAAGTAC GCCCCCTATT GACGTCAATG ACGGTAAATG   300
   GCCCGCCTGG CATTATGCCC AGTACATGAC CTTATGGGAC TTTCCTACTT   350
   GGCAGTACAT CTACGTATTA GTCATCGCTA TTACCATGGT GATGCGGTTT   400
   TGGCAGTACA TCAATGGGCG TGGATAGCGG TTTGACTCAC GGGGATTTCC   450
   AAGTCTCCAC CCCATTGACG TCAATGGGAG TTTGTTTTGG CACCAAAATC   500
   AACGGGACTT TCCAAAATGT CGTAACAACT CCGCCCCATT GACGCAAATG   550
   GGCGGTAGGC GTGTACGGTG GGAGGTCTAT ATAAGCAGAG CTCGTTTAGT   600
   GAACCGTCAG AATTCTGTTG GGCTCGCGGT TGATTACAAA CTCTTCGCGG   650
   TCTTTCCAGT ACTCTTGGAT CGGAAACCCG TCGGCCTCCG AACGGTACTC   700
   CGCCACCGAG GGACCTGAGC GAGTCCGCAT CGACCGGATC GGAAAACCTC 750
   TCGACTGTTG GGGTGAGTAC TCCCTCTCAA AAGCGGGCAT GACTTCTGCG 800
   CTAAGATTGT CAGTTTCCAA AAACGAGGAG GATTTGATAT TCACCTGGCC 850
   CGCGGTGATG CCTTTGAGGG TGGCCGCGTC CATCTGGTCA GAAAAGACAA 900
   TCTTTTTGTT GTCAAGCTTG AGGTGTGGCA GGCTTGAGAT CTGGCCATAC 950
   ACTTGAGTGA CAATGACATC CACTTTGCCT TTCTCTCCAC AGGTGTCCAC 1000
   TCCCAGGTCC AACTGCAGGT CGACCGGCTT GGTACCGAGC TCGGATCCAC 1050
   TAGTAACGGC CGCCAGTGTG CTGGAATTCT GCAGATATCC ATCACACTGG 1100
   CGGCCGCTCG AGCATGCATC TAGAACTTGT TTATTGCAGC TTATAATGGT 1150
   TACAAATAAA GCAATAGCAT CACAAATTTC ACAAATAAAG CATTTTTTTC 1200
   ACTGCATTCT AGTTGTGGTT TGTCCAAACT CATCAATGTA TCTTATCATG 1250
   TCTGGATCGA TCCCGCCATG GTATCAACGC CATATTTCTA TTTACAGTAG 1300
   GGACCTCTTC GTTGTGTAGG TACCGCTGTA TTCCTAGGGA AATAGTAGAG 1350
   GCACCTTGAA CTGTCTGCAT CAGCCATATA GCCCCCGCTG TTCGACTTAC 1400
   AAACACAGGC ACAGTACTGA CAAACCCATA CACCTCCTCT GAAATACCCA 1450
   TAGTTGCTAG GGCTGTCTCC GAACTCATTA CACCCTCCAA AGTCAGAGCT 1500
   GTAATTTCGC CATCAAGGGC AGCGAGGGCT TCTCCAGATA AAATAGCTTC 1550
   TGCCGAGAGT CCCGTAAGGG TAGACACTTC AGCTAATCCC TCGATGAGGT 1600
   CTACTAGAAT AGTCAGTGCG GCTCCCATTT TGAAAATTCA CTTACTTGAT 1650
   CAGCTTCAGA AGATGGCGGA GGGCCTCCAA CACAGTAATT TTCCTCCCGA 1700
   CTCTTAAAAT AGAAAATGTC AAGTCAGTTA AGCAGGAAGT GGACTAACTG 1750
   ACGCAGCTGG CCGTGCGACA TCCTCTTTTA ATTAGTTGCT AGGCAACGCC 1800
   CTCCAGAGGG CGTGTGGTTT TGCAAGAGGA AGCAAAAGCC TCTCCACCCA 1850
   GGCCTAGAAT GTTTCCACCC AATCATTACT ATGACAACAG CTGTTTTTTT 1900
   TAGTATTAAG CAGAGGCCGG GGACCCCTGG GCCCGCTTAC TCTGGAGAAA 1950
   AAGAAGAGAG GCATTGTAGA GGCTTCCAGA GGCAACTTGT CAAAACAGGA 2000
   CTGCTTCTAT TTCTGTCACA CTGTCTGGCC CTGTCACAAG GTCCAGCACC 2050
   TCCATACCCC CTTTAATAAG CAGTTTGGGA ACGGGTGCGG GTCTTACTCC 2100
   GCCCATCCCG CCCCTAACTC CGCCCAGTTC CGCCCATTCT CCGCCCCATG 2150
   GCTGACTAAT TTTTTTTATT TATGCAGAGG CCGAGGCCGC CTCGGCCTCT 2200
   GAGCTATTCC AGAAGTAGTG AGGAGGCTTT TTTGGAGGCC TAGGCTTTTG 2250
   CAAAAAGGAG CTCCCAGCAA AAGGCCAGGA ACCGTAAAAA GGCCGCGTTG 2300
   CTGGCGTTTT TCCATAGGCT CCGCCCCCCT GACGAGCATC ACAAAAATCG 2350
   ACGCTCAAGT CAGAGGTGGC GAAACCCGAC AGGACTATAA AGATACCAGG 2400
   CGTTTCCCCC TGGAAGCTCC CTCGTGCGCT CTCCTGTTCC GACCCTGCCG 2450
   CTTACCGGAT ACCTGTCCGC CTTTCTCCCT TCGGGAAGCG TGGCGCTTTC 2500
   TCAATGCTCA CGCTGTAGGT ATCTCAGTTC GGTGTAGGTC GTTCGCTCCA 2550
   AGCTGGGCTG TGTGCACGAA CCCCCCGTTC AGCCCGACCG CTGCGCCTTA 2600
   TCCGGTAACT ATCGTCTTGA GTCCAACCCG GTAAGACACG ACTTATCGCC 2650
   ACTGGCAGCA GCCACTGGTA ACAGGATTAG CAGAGCGAGG TATGTAGGCG 2700
   GTGCTACAGA GTTCTTGAAG TGGTGGCCTA ACTACGGCTA CACTAGAAGG 2750
   ACAGTATTTG GTATCTGCGC TCTGCTGAAG CCAGTTACCT TCGGAAAAAG 2800
   AGTTGGTAGC TCTTGATCCG GCAAACAAAC CACCGCTGGT AGCGGTGGTT 2850
   TTTTTGTTTG CAAGCAGCAG ATTACGCGCA GAAAAAAAGG ATCTCAAGAA 2900
   GATCCTTTGA TCTTTTCTAC GGGGTCTGAC GCTCAGTGGA ACGAAAACTC 2950
   ACGTTAAGGG ATTTTGGTCA TGAGATTATC AAAAAGGATC TTCACCTAGA 3000
   TCCTTTTAAA TTAAAAATGA AGTTTTAAAT CAATCTAAAG TATATATGAG 3050
   TAAACTTGGT CTGACAGTTA CCAATGCTTA ATCAGTGAGG CACCTATCTC 3100
   AGCGATCTGT CTATTTCGTT CATCCATAGT TGCCTGACTC CCCGTCGTGT 3150
   AGATAACTAC GATACGGGAG GGCTTACCAT CTGGCCCCAG TGCTGCAATG 3200
   ATACCGCGAG ACCCACGCTC ACCGGCTCCA GATTTATCAG CAATAAACCA 3250
   GCCAGCCGGA AGGGCCGAGC GCAGAAGTGG TCCTGCAACT TTATCCGCCT 3300
   CCATCCAGTC TATTAATTGT TGCCGGGAAG CTAGAGTAAG TAGTTCGCCA 3350
   GTTAATAGTT TGCGCAACGT TGTTGCCATT GCTACAGGCA TCGTGGTGTC 3400
   ACGCTCGTCG TTTGGTATGG CTTCATTCAG CTCCGGTTCC CAACGATCAA 3450
   GGCGAGTTAC ATGATCCCCC ATGTTGTGCA AAAAAGCGGT TAGCTCCTTC 3500
   GGTCCTCCGA TCGTTGTCAG AAGTAAGTTG GCCGCAGTGT TATCACTCAT 3550
   GGTTATGGCA GCACTGCATA ATTCTCTTAC TGTCATGCCA TCCGTAAGAT 3600
   GCTTTTCTGT GACTGGTGAG TACTCAACCA AGTCATTCTG AGAATAGTGT 3650
   ATGCGGCGAC CGAGTTGCTC TTGCCCGGCG TCAATACGGG ATAATACCGC 3700
   GCCACATAGC AGAACTTTAA AAGTGCTCAT CATTGGAAAA CGTTCTTCGG 3750
   GGCGAAAACT CTCAAGGATC TTACCGCTGT TGAGATCCAG TTCGATGTAA 3800
   CCCACTCGTG CACCCAACTG ATCTTCAGCA TCTTTTACTT TCACCAGCGT 3850
   TTCTGGGTGA GCAAAAACAG GAAGGCAAAA TGCCGCAAAA AAGGGAATAA 3900
   GGGCGACACG GAAATGTTGA ATACTCATAC TCTTCCTTTT TCAATATTAT 3950
   TGAAGCATTT ATCAGGGTTA TTGTCTCATG AGCGGATACA TATTTGAATG 4000
   TATTTAGAAA AATAAACAAA TAGGGGTTCC GCGCACATTT CCCCGAAAAG 4050
   TGCCACCTG 4059
(2) INFORMATION FOR SEQ ID NO:12:
   (i) SEQUENCE CHARACTERISTICS:
      (A) LENGTH: 8540 base pairs
      (B) TYPE: Nucleic Acid
      (C) STRANDEDNESS: Double
      (D) TOPOLOGY: Circular
   (ii) MOLECULE TYPE: Other nucleic acid
      (A) DESCRIPTION: Apex-3P Eukaryotic Expression Vector
   (xi) SEQUENCE DESCRIPTION: SEQ ID NO:12:
   GTGACCAATA CAAAACAAAA GCGCCCCTCG TACCAGCGAA GAAGGGGCAG 50
   AGATGCCGTA GTCAGGTTTA GTTCGTCCGG CGGCGGGGGA TCTGTATGGT 100
   GCACTCTCAG TACAATCTGC TCTGATGCCG CATAGTTAAG CCAGTATCTG 150
   CTCCCTGCTT GTGTGTTGGA GGTCGCTGAG TAGTGCGCGA GCAAAATTTA 200
   AGCTACAACA AGGCAAGGCT TGACCGACAA TTGCATGAAG AATCTGCTTA 250
   GGGTTAGGCG TTTTGCGCTG CTTCGCGATG TACGGGCCAG ATATACGCGT 300
   TGACATTGAT TATTGACTAG TTATTAATAG TAATCAATTA CGGGGTCATT 350
   AGTTCATAGC CCATATATGG AGTTCCGCGT TACATAACTT ACGGTAAATG 400
   GCCCGCCTGG CTGACCGCCC AACGACCCCC GCCCATTGAC GTCAATAATG 450
   ACGTATGTTC CCATAGTAAC GCCAATAGGG ACTTTCCATT GACGTCAATG 500
   GGTGGACTAT TTACGGTAAA CTGCCCACTT GGCAGTACAT CAAGTGTATC 550
   ATATGCCAAG TACGCCCCCT ATTGACGTCA ATGACGGTAA ATGGCCCGCC 600
   TGGCATTATG CCCAGTACAT GACCTTATGG GACTTTCCTA CTTGGCAGTA 650
   CATCTACGTA TTAGTCATCG CTATTACCAT GGTGATGCGG TTTTGGCAGT 700
   ACATCAATGG GCGTGGATAG CGGTTTGACT CACGGGGATT TCCAAGTCTC 750
   CACCCCATTG ACGTCAATGG GAGTTTGTTT TGGCACCAAA ATCAACGGGA 800
   CTTTCCAAAA TGTCGTAACA ACTCCGCCCC ATTGACGCAA ATGGGCGGTA 850
   GGCGTGTACG GTGGGAGGTC TATATAAGCA GAGCTCGTTT AGTGAACCGT 900
   CAGAATTCTG TTGGGCTCGC GGTTGATTAC AAACTCTTCG CGGTCTTTCC 950
   AGTACTCTTG GATCGGAAAC CCGTCGGCCT CCGAACGGTA CTCCGCCACC 1000
   GAGGGACCTG AGCGAGTCCG CATCGACCGG ATCGGAAAAC CTCTCGACTG 1050
   TTGGGGTGAG TACTCCCTCT CAAAAGCGGG CATGACTTCT GCGCTAAGAT 1100
   TGTCAGTTTC CAAAAACGAG GAGGATTTGA TATTCACCTG GCCCGCGGTG 1150
   ATGCCTTTGA GGGTGGCCGC GTCCATCTGG TCAGAAAAGA CAATCTTTTT 1200
   GTTGTCAAGC TTGAGGTGTG GCAGGCTTGA GATCTGGCCA TACACTTGAG 1250
   TGACAATGAC ATCCACTTTG CCTTTCTCTC CACAGGTGTC CACTCCCAGG 1300
   TCCAACTGCA GGTCGACCGG CTTGGTACCG AGCTCGGATC CTCTAGAGTC 1350
   GACCTGCAGG CATGCAAGCT TGGCACTGGC CGTCGTTTTA CAACGTCGTG 1400
   ACTGGGAAAA CCCTGGCGTT ACCCAACTTA ATCGCCTTGC AGCACATCCC 1450
   CCTTTCGCCA GCTGGCGTAA TAGCGAAGAG GCCCGCACCG ATCCAGACAT 1500
   GATAAGATAC ATTGATGAGT TTGGACAAAC CACAACTAGA ATGCAGTGAA 1550
   AAAAATGCTT TATTTGTGAA ATTTGTGATG CTATTGCTTT ATTTGTAACC 1600
   ATTATAAGCT GCAATAAACA AGTTAACAAC AACAATTGCA TTCATTTTAT 1650
   GTTTCAGGTT CAGGGGGAGG TGTGGGAGGT TTTTTAAAGC AAGTAAAACC 1700
   TCTACAAATG TGGTATGGCT GATTATGATC CCCAGGAAGC TCCTCTGTGT 1750
   CCTCATAAAC CCTAACCTCC TCTACTTGAG AGGACATTCC AATCATAGGC 1800
   TGCCCATCCA CCCTCTGTGT CCTCCTGTTA ATTAGGTCAC TTAACAAAAA 1850
   GGAAATTGGG TAGGGGTTTT TCACAGACCG CTTTCTAAGG GTAATTTTAA 1900
   AATATCTGGG AAGTCCCTTC CACTGCTGTG TTCCAGAAGT GTTGGTAAAC 1950
   AGCCCACAAA TGTCAACAGC AGAAACATAC AAGCTGTCAG CTTTGCACAA 2000
   GGGCCCAACA CCCTGCTCAT CAAGAAGCAC TGTGGTTGCT GTGTTAGTAA 2050
   TGTGCAAAAC AGGAGGCACA TTTTCCCCAC CTGTGTAGGT TCCAAAATAT 2100
   CTAGTGTTTT CATTTTTACT TGGATCAGGA ACCCAGCACT CCACTGGATA 2150
   AGCATTATCC TTATCCAAAA CAGCCTTGTG GTCAGTGTTC ATCTGCTGAC 2200
   TGTCAACTGT AGCATTTTTT GGGGTTACAG TTTGAGCAGG ATATTTGGTC 2250
   CTGTAGTTTG CTAACACACC CTGCAGCTCC AAAGGTTCCC CACCAACAGC 2300
   AAAAAAATGA AAATTTGACC CTTGAATGGG TTTTCCAGCA CCATTTTCAT 2350
   GAGTTTTTTG TGTCCCTGAA TGCAAGTTTA ACATAGCAGT TACCCCAATA 2400
   ACCTCAGTTT TAACAGTAAC AGCTTCCCAC ATCAAAATAT TTCCACAGGT 2450
   TAAGTCCTCA TTTGTAGAAT TCGCCAGCAC AGTGGTCGAC CCTGTGGATG 2500
   TGTGTCACTT AGGGTGTGGA AAGTCCCCAG GCTCCCCAGC AGGCAGAAGT 2550
   ATGCAAAGCA TGCATCTCAA TTAGTCAGCA ACCAGGTGTG GAAAGTCCCC 2600
   AGGCTCCCCA GCAGGCAGAA GTATGCAAAG CATGCATCTC AATTAGTCAG 2650
   CAACCATAGT CCCGCCCCTA ACTCCGCCCA TCCCGCCCCT AACTCCGCCC 2700
   AGTTCCGCCC ATTCTCCGCC CCATGGCTGA CTAATTTTTT TTATTTATGC 2750
   AGAGGCCGAG GCCGCCTCGG CCTCTGAGCT ATTCCAGAAG TAGTGAGGAG 2800
   GCTTTTTTGG AGGCCTAGGC TTTTGCAAAA GCTTACCATG ACCGAGTACA 2850
   AGCCCACGGT GCGCCTCGCC ACCCGCGACG ACGTCCCCCG GGCCGTACGC 2900
   ACCCTCGCCG CCGCGTTCGC CGACTACCCC GCCACGCGCC ACACCGTCGA 2950
   CCCGGACCGC CACATCGAGC GGGTCACCGA GCTGCAAGAA CTCTTCCTCA 3000
   CGCGCGTCGG GCTCGACATC GGCAAGGTGT GGGTCGCGGA CGACGGCGCC 3050
   GCGGTGGCGG TCTGGACCAC GCCGGAGAGC GTCGAAGCGG GGGCGGTGTT 3100
   CGCCGAGATC GGCCCGCGCA TGGCCGAGTT GAGCGGTTCC CGGCTGGCCG 3150
   CGCAGCAACA GATGGAAGGC CTCCTGGCGC CGCACCGGCC CAAGGAGCCC 3200
   GCGTGGTTCC TGGCCACCGT CGGCGTCTCG CCCGACCACC AGGGCAAGGG 3250
   TCTGGGCAGC GCCGTCGTGC TCCCCGGAGT GGAGGCGGCC GAGCGCGCCG 3300
   GGGTGCCCGC CTTCCTGGAG ACCTCCGCGC CCCGCAACCT CCCCTTCTAC 3350
   GAGCGGCTCG GCTTCACCGT CACCGCCGAC GTCGAGTGCC CGAAGGACCG 3400
   CGCGACCTGG TGCATGACCC GCAAGCCCGG TGCCTGACGC CCGCCCCACG 3450
   ACCCGCAGCG CCCGACCGAA AGGAGCGCAC GACCCCATGC ATCGATAAAA 3500
   TAAAAGATTT TATTTAGTCT CCAGAAAAAG GGGGGAATGA AAGACCCCAC 3550
   CTGTAGGTTT GGCAAGCTAG AACTTGTTTA TTGCAGCTTA TAATGGTTAC 3600
   AAATAAAGCA ATAGCATCAC AAATTTCACA AATAAAGCAT TTTTTTCACT 3650
   GCATTCTAGT TGTGGTTTGT CCAAACTCAT CAATGTATCT TATCATGTCT 3700
   GGATCGATCC CGCCATGGTA TCAACGCCAT ATTTCTATTT ACAGTAGGGA 3750
   CCTCTTCGTT GTGTAGGTAC CCCGGGTTCG AAATCGAATT CGCCAATGAC 3800
   AAGACGCTGG GCGGGGTTTG TGTCATCATA GAACTAAAGA CATGCAAATA 3850
   TATTTCTTCC GGGGACACCG CCAGCAAACG CGAGCAACGG GCCACGGGGA 3900
   TGAAGCAGCC CGGCGGCACC TCGCTAACGG ATTCACCACT CCAAGAATTG 3950
   GAGCCAATCA ATTCTTGCGG AGAACTGTGA ATGCGCAAAC CAACCCTTGG 4000
   CAGAACATAT CCATCGCGTC CGCCATCTCC AGCAGCCGCA CGCGGCGCAT 4050
   CTCGGGGCCG ACGCGCTGGG CTACGTCTTG CTGGCGTTCG CGACGCGAGG 4100
   CTGGATGGCC TTCCCCATTA TGATTCTTCT CGCTTCCGGC GGCATCGGGA 4150
   TGCCCGCGTT GCAGGCCATG CTGTCCAGGC AGGTAGATGA CGACCATCAG 4200
   GGACAGCTTC AAGGATCGCT CGCGGCTCTT ACCAGCGCCA GCAAAAGGCC 4250
   AGGAACCGTA AAAAGGCCGC GTTGCTGGCG TTTTTCCATA GGCTCCGCCC 4300
   CCCTGACGAG CATCACAAAA ATCGACGCTC AAGTCAGAGG TGGCGAAACC 4350
   CGACAGGACT ATAAAGATAC CAGGCGTTTC CCCCTGGAAG CTCCCTCGTG 4400
   CGCTCTCCTG TTCCGACCCT GCCGCTTACC GGATACCTGT CCGCCTTTCT 4450
   CCCTTCGGGA AGCGTGGCGC TTTCTCATAG CTCACGCTGT AGGTATCTCA 4500
   GTTCGGTGTA GGTCGTTCGC TCCAAGCTGG GCTGTGTGCA CGAACCCCCC 4550
   GTTCAGCCCG ACCGCTGCGC CTTATCCGGT AACTATCGTC TTGAGTCCAA 4600
   CCCGGTAAGA CACGACTTAT CGCCACTGGC AGCAGCCACT GGTAACAGGA 4650
   TTAGCAGAGC GAGGTATGTA GGCGGTGCTA CAGAGTTCTT GAAGTGGTGG 4700
   CCTAACTACG GCTACACTAG AAGGACAGTA TTTGGTATCT GCGCTCTGCT 4750
   GAAGCCAGTT ACCTTCGGAA AAAGAGTTGG TAGCTCTTGA TCCGGCAAAC 4800
   AAACCACCGC TGGTAGCGGT GGTTTTTTTG TTTGCAAGCA GCAGATTACG 4850
   CGCAGAAAAA AAGGATCTCA AGAAGATCCT TTGATCTTTT CTACGGGGTC 4900
   TGACGCTCAG TGGAACGAAA ACTCACGTTA AGGGATTTTG GTCATGAGAT 4950
   TATCAAAAAG GATCTTCACC TAGATCCTTT TAAATTAAAA ATGAAGTTTT 5000
   AAATCAATCT AAAGTATATA TGAGTAAACT TGGTCTGACA GTTACCAATG 5050
   CTTAATCAGT GAGGCACCTA TCTCAGCGAT CTGTCTATTT CGTTCATCCA 5100
   TAGTTGCCTG ACTCCCCGTC GTGTAGATAA CTACGATACG GGAGGGCTTA 5150
   CCATCTGGCC CCAGTGCTGC AATGATACCG CGAGACCCAC GCTCACCGGC 5200
   TCCAGATTTA TCAGCAATAA ACCAGCCAGC CGGAAGGGCC GAGCGCAGAA 5250
   GTGGTCCTGC AACTTTATCC GCCTCCATCC AGTCTATTAA TTGTTGCCGG 5300
   GAAGCTAGAG TAAGTAGTTC GCCAGTTAAT AGTTTGCGCA ACGTTGTTGC 5350
   CATTGCTGCA GGCATCGTGG TGTCACGCTC GTCGTTTGGT ATGGCTTCAT 5400
   TCAGCTCCGG TTCCCAACGA TCAAGGCGAG TTACATGATC CCCCATGTTG 5450
   TGCAAAAAAG CGGTTAGCTC CTTCGGTCCT CCGATCGTTG TCAGAAGTAA 5500
   GTTGGCCGCA GTGTTATCAC TCATGGTTAT GGCAGCACTG CATAATTCTC 5550
   TTACTGTCAT GCCATCCGTA AGATGCTTTT CTGTGACTGG TGAGTACTCA 5600
   ACCAAGTCAT TCTGAGAATA GTGTATGCGG CGACCGAGTT GCTCTTGCCC 5650
   GGCGTCAACA CGGGATAATA CCGCGCCACA TAGCAGAACT TTAAAAGTGC 5700
   TCATCATTGG AAAACGTTCT TCGGGGCGAA AACTCTCAAG GATCTTACCG 5750
   CTGTTGAGAT CCAGTTCGAT GTAACCCACT CGTGCACCCA ACTGATCTTC 5800
   AGCATCTTTT ACTTTCACCA GCGTTTCTGG GTGAGCAAAA ACAGGAAGGC 5850
   AAAATGCCGC AAAAAAGGGA ATAAGGGCGA CACGGAAATG TTGAATACTC 5900
   ATACTCTTCC TTTTTCAATA TTATTGAAGC ATTTATCAGG GTTATTGTCT 5950
   CATGAGCGGA TACATATTTG AATGTATTTA GAAAAATAAA CAAATAGGGG 6000
   TTCCGCGCAC ATTTCCCCGA AAAGTGCCAC CTGACGTCTA AGAAACCATT 6050
   ATTATCATGA CATTAACCTA TAAAAATAGG CGTATCACGA GGCCCTTTCG 6100
   TCTTCAAGAA TTCTCATGTT TGACAGCTTA TCGTAGACAT CATGCGTGCT 6150
   GTTGGTGTAT TTCTGGCCAT CTGTCTTGTC ACCATTTTCG TCCTCCCAAC 6200
   ATGGGGCAAT TGGGCATACC CATGTTGTCA CGTCACTCAG CTCCGCGCTC 6250
   AACACCTTCT CGCGTTGGAA AACATTAGCG ACATTTACCT GGTGAGCAAT 6300
   CAGACATGCG ACGGCTTTAG CCTGGCCTCC TTAAATTCAC CTAAGAATGG 6350
   GAGCAACCAG CAGGAAAAGG ACAAGCAGCG AAAATTCACG CCCCCTTGGG 6400
   AGGTGGCGGC ATATGCAAAG GATAGCACTC CCACTCTACT ACTGGGTATC 6450
   ATATGCTGAC TGTATATGCA TGAGGATAGC ATATGCTACC CGGATACAGA 6500
   TTAGGATAGC ATATACTACC CAGATATAGA TTAGGATAGC ATATGCTACC 6550
   CAGATATAGA TTAGGATAGC CTATGCTACC CAGATATAAA TTAGGATAGC 6600
   ATATACTACC CAGATATAGA TTAGGATAGC ATATGCTACC CAGATATAGA 6650
   TTAGGATAGC CTATGCTACC CAGATATAGA TTAGGATAGC ATATGCTACC 6700
   CAGATATAGA TTAGGATAGC ATATGCTATC CAGATATTTG GGTAGTATAT 6750
   GCTACCCAGA TATAAATTAG GATAGCATAT ACTACCCTAA TCTCTATTAG 6800
   GATAGCATAT GCTACCCGGA TACAGATTAG GATAGCATAT ACTACCCAGA 6850
   TATAGATTAG GATAGCATAT GCTACCCAGA TATAGATTAG GATAGCCTAT 6900
   GCTACCCAGA TATAAATTAG GATAGCATAT ACTACCCAGA TATAGATTAG 6950
   GATAGCATAT GCTACCCAGA TATAGATTAG GATAGCCTAT GCTACCCAGA 7000
   TATAGATTAG GATAGCATAT GCTATCCAGA TATTTGGGTA GTATATGCTA 7050
   CCCATGGCAA CATTAGCCCA CCGTGCTCTC AGCGACCTCG TGAATATGAG 7100
   GACCAACAAC CCTGTGCTTG GCGCTCAGGC GCAAGTGTGT GTAATTTGTC 7150
   CTCCAGATCG CAGCAATCGC GCCCCTATCT TGGCCCGCCC ACCTACTTAT 7200
   GCAGGTATTC CCCGGGGTGC CATTAGTGGT TTTGTGGGCA AGTGGTTTGA 7250
   CCGCAGTGGT TAGCGGGGTT ACAATCAGCC AAGTTATTAC ACCCTTATTT 7300
   TACAGTCCAA AACCGCAGGG CGGCGTGTGG GGGCTGACGC GTGCCCCCAC 7350
   TCCACAATTT CAAAAAAAAG AGTGGCCACT TGTCTTTGTT TATGGGCCCC 7400
   ATTGGCGTGG AGCCCCGTTT AATTTTCGGG GGTGTTAGAG ACAACCAGTG 7450
   GAGTCCGCTG CTGTCGGCGT CCACTCTCTT TCCCCTTGTT ACAAATAGAG 7500
   TGTAACAACA TGGTTCACCT GTCTTGGTCC CTGCCTGGGA CACATCTTAA 7550
   TAACCCCAGT ATCATATTGC ACTAGGATTA TGTGTTGCCC ATAGCCATAA 7600
   ATTCGTGTGA GATGGACATC CAGTCTTTAC GGCTTGTCCC CACCCCATGG 7650
   ATTTCTATTG TTAAAGATAT TCAGAATGTT TCATTCCTAC ACTAGTATTT 7700
   ATTGCCCAAG GGGTTTGTGA GGGTTATATT GGTGTCATAG CACAATGCCA 7750
   CCACTGAACC CCCCGTCCAA ATTTTATTCT GGGGGCGTCA CCTGAAACCT 7800
   TGTTTTCGAG CACCTCACAT ACACCTTACT GTTCACAACT CAGCAGTTAT 7850
   TCTATTAGCT AAACGAAGGA GAATGAAGAA GCAGGCGAAG ATTCAGGAGA 7900
   GTTCACTGCC CGCTCCTTGA TCTTCAGCCA CTGCCCTTGT GACTAAAATG 7950
   GTTCACTACC CTCGTGGAAT CCTGACCCCA TGTAAATAAA ACCGTGACAG 8000
   CTCATGGGGT GGGAGATATC GCTGTTCCTT AGGACCCTTT TACTAACCCT 8050
   AATTCGATAG CATATGCTTC CCGTTGGGTA ACATATGCTA TTGAATTAGG 8100
   GTTAGTCTGG ATAGTATATA CTACTACCCG GGAAGCATAT GCTACCCGTT 8150
   TAGGGTTAAC AAGGGGGCCT TATAAACACT ATTGCTAATG CCCTCTTGAG 8200
   GGTCCGCTTA TCGGTAGCTA CACAGGCCCC TCTGATTGAC GTTGGTGTAG 8250
   CCTCCCGTAG TCTTCCTGGG CCCCTGGGAG GTACATGTCC CCCAGCATTG 8300
   GTGTAAGAGC TTCAGCCAAG AGTTACACAT AAAGGCAATG TTGTGTTGCA 8350
   GTCCACAGAC TGCAAAGTCT GCTCCAGGAT GAAAGCCACT CAGTGTTGGC 8400
   AAATGTGCAC ATCCATTTAT AAGGATGTCA ACTACAGTCA GAGAACCCCT 8450
   TTGTGTTTGG TCCCCCCCCG TGTCACATGT GGAACAGGGC CCAGTTGGCA 8500
   AGTTGTACCA ACCAACTGAA GGGATTACAT GCACTGCCCC 8540
(2) INFORMATION FOR SEQ ID NO:13:
   (i) SEQUENCE CHARACTERISTICS:
      (A) LENGTH: 22 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: PCR primer N-terminus of hMBP18.5 (MASQKR)
   (iii) HYPOTHETICAL: No
   (iv) ANTI-SENSE: No
   (xi) SEQUENCE DESCRIPTION: SEQ ID NO:13:
      CATATGGCGT CACAGAAGAG AC 22
(2) INFORMATION FOR SEQ ID NO:14:
   (i) SEQUENCE CHARACTERISTICS:
      (A) LENGTH: 26 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: MBP C-terminal (PMARR) PCR primer
   (iii) HYPOTHETICAL: No
   (iv) ANTI-SENSE: Yes
   (xi) SEQUENCE DESCRIPTION: SEQ ID NO:14:
      GGATCCTTAG CGTCTAGCCA TGGGTG 26
(2) INFORMATION FOR SEQ ID NO:15:
   (i) SEQUENCE CHARACTERISTICS:
      (A) LENGTH: 36 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: PCR mutagenic Ser 81 primer
   (iii) HYPOTHETICAL: No
   (iv) ANTI-SENSE: Yes
   (xi) SEQUENCE DESCRIPTION: SEQ ID NO:15:
      GTCTTTGTAC ATGTTCGACA GGCCCGGCTG GCTACG 36
(2) INFORMATION FOR SEQ ID NO:16:
   (i) SEQUENCE CHARACTERISTICS:
      (A) LENGTH: 14 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: PCR primer for Ser mutagenesis
   (iii) HYPOTHETICAL: No
   (iv) ANTI-SENSE: No
   (xi) SEQUENCE DESCRIPTION: SEQ ID NO:16:
      CAGCACCATG GACC 14
(2) INFORMATION FOR SEQ ID NO:17:
   (i) SEQUENCE CHARACTERISTICS:
      (A) LENGTH: 128 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: X2 PCR primer
   (iii) HYPOTHETICAL: No
   (iv) ANTI-SENSE: No
   (xi) SEQUENCE DESCRIPTION: SEQ ID NO:17:
   GGTGCGCCAA AGCGGGGCTC TGGCAAGGTA CCCTGGCTAA AGCCGGGCCG 50
   GAGCCCTCTG CCCTCTCATG CCCGCAGCCA GCCTGGGCTG TGCAACATGT 100
   ACAAGGACTC ACACCACCCG GCAAGAAC 128
(2) INFORMATION FOR SEQ ID NO:18:
   (i) SEQUENCE CHARACTERISTICS:
      (A) LENGTH: 19 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: T7 terminator primer
   (iii) HYPOTHETICAL: No
   (iv) ANTI-SENSE: Yes
   (xi) SEQUENCE DESCRIPTION: SEQ ID NO:18 :
      GCTAGTTATT GCTCAGCGG 19
(2) INFORMATION FOR SEQ ID NO:19:
   (i) SEQUENCE CHARACTERISTICS:
      (A) LENGTH: 20 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: T7 promoter primer T7PP
   (iii) HYPOTHETICAL: No
   (iv) ANTI-SENSE: No
   (xi) SEQUENCE DESCRIPTION: SEQ ID NO:19:
      TAATACGACT CACTATAGGG 20
(2) INFORMATION FOR SEQ ID NO:20:
   (i) SEQUENCE CHARACTERISTICS:
      (A) LENGTH: 39 bases
      (B) TYPE: Nucleic acid
      (C) STRANDEDNESS: Single
      (D) TOPOLOGY: Linear
   (ii) MOLECULE TYPE: Other nucleic acid
      (A) DESCRIPTION: 3' primer for X2 assembly
   (iii) HYPOTHETICAL: No
   (iv) ANTI-SENSE: No
   (xi) SEQUENCE DESCRIPTION: SEQ ID NO:20:
      GGCTTTAGCC AGGGTACCTT GCCAGAGCCC CGCTTTGGC 39
(2) INFORMATION FOR SEQ ID NO:21:
   (i) SEQUENCE CHARACTERISTICS:
      (A) LENGTH: 5248 base pairs
      (B) TYPE: Nucleic Acid
      (C) STRANDEDNESS: Double
      (D) TOPOLOGY: Circular
   (ii) MOLECULE TYPE: Other nucleic acid
      (A) DESCRIPTION: pET Trc SO5/NI prokaryotic expression vector
   (xi) SEQUENCE DESCRIPTION: SEQ ID NO:21:
   TGGCGAATGG GACGCGCCCT GTAGCGGCGC ATTAAGCGCG GCGGGTGTGG 50
   TGGTTACGCG CAGCGTGACC GCTACACTTG CCAGCGCCCT AGCGCCCGCT 100
   CCTTTCGCTT TCTTCCCTTC CTTTCTCGCC ACGTTCGCCG GCTTTCCCCG 150
   TCAAGCTCTA AATCGGGGGC TCCCTTTAGG GTTCCGATTT AGTGCTTTAC 200
   GGCACCTCGA CCCCAAAAAA CTTGATTAGG GTGATGGTTC ACGTAGTGGG 250
   CCATCGCCCT GATAGACGGT TTTTCGCCCT TTGACGTTGG AGTCCACGTT 300
   CTTTAATAGT GGACTCTTGT TCCAAACTGG AACAACACTC AACCCTATCT 350
   CGGTCTATTC TTTTGATTTA TAAGGGATTT TGCCGATTTC GGCCTATTGG 400
   TTAAAAAATG AGCTGATTTA ACAAAAATTT AACGCGAATT TTAACAAAAT 450
   ATTAACGTTT ACAATTTCAG GTGGCACTTT TCGGGGAAAT GTGCGCGGAA 500
   CCCCTATTTG TTTATTTTTC TAAATACATT CAAATATGTA TCCGCTCATG 550
   AGACAATAAC CCTGATAAAT GCTTCAATAA TATTGAAAAA GGAAGAGTAT 600
   GAGTATTCAA CATTTCCGTG TCGCCCTTAT TCCCTTTTTT GCGGCATTTT 650
   GCCTTCCTGT TTTTGCTCAC CCAGAAACGC TGGTGAAAGT AAAAGATGCT 700
   GAAGATCAGT TGGGTGCACG AGTGGGTTAC ATCGAACTGG ATCTCAACAG 750
   CGGTAAGATC CTTGAGAGTT TTCGCCCCGA AGAACGTTTT CCAATGATGA 800
   GCACTTTTAA AGTTCTGCTA TGTGGCGCGG TATTATCCCG TATTGACGCC 850
   GGGCAAGAGC AACTCGGTCG CCGCATACAC TATTCTCAGA ATGACTTGGT 900
   TGAGTACTCA CCAGTCACAG AAAAGCATCT TACGGATGGC ATGACAGTAA 950
   GAGAATTATG CAGTGCTGCC ATAACCATGA GTGATAACAC TGCGGCCAAC 1000
   TTACTTCTGA CAACGATCGG AGGACCGAAG GAGCTAACCG CTTTTTTGCA 1050
   CAACATGGGG GATCATGTAA CTCGCCTTGA TCGTTGGGAA CCGGAGCTGA 1100
   ATGAAGCCAT ACCAAACGAC GAGCGTGACA CCACGATGCC TGCAGCAATG 1150
   GCAACAACGT TGCGCAAACT ATTAACTGGC GAACTACTTA CTCTAGCTTC 1200
   CCGGCAACAA TTAATAGACT GGATGGAGGC GGATAAAGTT GCAGGACCAC 1250
   TTCTGCGCTC GGCCCTTCCG GCTGGCTGGT TTATTGCTGA TAAATCTGGA 1300
   GCCGGTGAGC GTGGGTCTCG CGGTATCATT GCAGCACTGG GGCCAGATGG 1350
   TAAGCCCTCC CGTATCGTAG TTATCTACAC GACGGGGAGT CAGGCAACTA 1400
   TGGATGAACG AAATAGACAG ATCGCTGAGA TAGGTGCCTC ACTGATTAAG 1450
   CATTGGTAAC TGTCAGACCA AGTTTACTCA TATATACTTT AGATTGATTT 1500
   AAAACTTCAT TTTTAATTTA AAAGGATCTA GGTGAAGATC CTTTTTGATA 1550
   ATCTCATGAC CAAAATCCCT TAACGTGAGT TTTCGTTCCA CTGAGCGTCA 1600
   GACCCCGTAG AAAAGATCAA AGGATCTTCT TGAGATCCTT TTTTTCTGCG 1650
   CGTAATCTGC TGCTTGCAAA CAAAAAAACC ACCGCTACCA GCGGTGGTTT 1700
   GTTTGCCGGA TCAAGAGCTA CCAACTCTTT TTCCGAAGGT AACTGGCTTC 1750
   AGCAGAGCGC AGATACCAAA TACTGTCCTT CTAGTGTAGC CGTAGTTAGG 1800
   CCACCACTTC AAGAACTCTG TAGCACCGCC TACATACCTC GCTCTGCTAA 1850
   TCCTGTTACC AGTGGCTGCT GCCAGTGGCG ATAAGTCGTG TCTTACCGGG 1900
   TTGGACTCAA GACGATAGTT ACCGGATAAG GCGCAGCGGT CGGGCTGAAC 1950
   GGGGGGTTCG TGCACACAGC CCAGCTTGGA GCGAACGACC TACACCGAAC 2000
   TGAGATACCT ACAGCGTGAG CTATGAGAAA GCGCCACGCT TCCCGAAGGG 2050
   AGAAAGGCGG ACAGGTATCC GGTAAGCGGC AGGGTCGGAA CAGGAGAGCG 2100
   CACGAGGGAG CTTCCAGGGG GAAACGCCTG GTATCTTTAT AGTCCTGTCG 2150
   GGTTTCGCCA CCTCTGACTT GAGCGTCGAT TTTTGTGATG CTCGTCAGGG 2200
   GGGCGGAGCC TATGGAAAAA CGCCAGCAAC GCGGCCTTTT TACGGTTCCT 2250
   GGCCTTTTGC TGGCCTTTTG CTCACATGTT CTTTCCTGCG TTATCCCCTG 2300
   ATTCTGTGGA TAACCGTATT ACCGCCTTTG AGTGAGCTGA TACCGCTCGC 2350
   CGCAGCCGAA CGACCGAGCG CAGCGAGTCA GTGAGCGAGG AAGCGGAAGA 2400
   GCGCCTGATG CGGTATTTTC TCCTTACGCA TCTGTGCGGT ATTTCACACC 2450
   GCATATATGG TGCACTCTCA GTACAATCTG CTCTGATGCC GCATAGTTAA 2500
   GCCAGTATAC ACTCCGCTAT CGCTACGTGA CTGGGTCATG GCTGCGCCCC 2550
   GACACCCGCC AACACCCGCT GACGCGCCCT GACGGGCTTG TCTGCTCCCG 2600
   GCATCCGCTT ACAGACAAGC TGTGACCGTC TCCGGGAGCT GCATGTGTCA 2650
   GAGGTTTTCA CCGTCATCAC CGAAACGCGC GAGGCAGCTG CGGTAAAGCT 2700
   CATCAGCGTG GTCGTGAAGC GATTCACAGA TGTCTGCCTG TTCATCCGCG 2750
   TCCAGCTCGT TGAGTTTCTC CAGAAGCGTT AATGTCTGGC TTCTGATAAA 2800
   GCGGGCCATG TTAAGGGCGG TTTTTTCCTG TTTGGTCACT GATGCCTCCG 2850
   TGTAAGGGGG ATTTCTGTTC ATGGGGGTAA TGATACCGAT GAAACGAGAG 2900
   AGGATGCTCA CGATACGGGT TACTGATGAT GAACATGCCC GGTTACTGGA 2950
   ACGTTGTGAG GGTAAACAAC TGGCGGTATG GATGCGGCGG GACCAGAGAA 3000
   AAATCACTCA GGGTCAATGC CAGCGCTTCG TTAATACAGA TGTAGGTGTT 3050
   CCACAGGGTA GCCAGCAGCA TCCTGCGATG CAGATCCGGA ACATAATGGT 3100
   GCAGGGCGCT GACTTCCGCG TTTCCAGACT TTACGAAACA CGGAAACCGA 3150
   AGACCATTCA TGTTGTTGCT CAGGTCGCAG ACGTTTTGCA GCAGCAGTCG 3200
   CTTCACGTTC GCTCGCGTAT CGGTGATTCA TTCTGCTAAC CAGTAAGGCA 3250
   ACCCCGCCAG CCTAGCCGGG TCCTCAACGA CAGGAGCACG ATCATGCGCA 3300
   CCCGTGGGGC CGCCATGCCG GCGATAATGG CCTGCTTCTC GCCGAAACGT 3350
   TTGGTGGCGG GACCAGTGAC GAAGGCTTGA GCGAGGGCGT GCAAGATTCC 3400
   GAATACCGCA AGCGACAGGC CGATCATCGT CGCGCTCCAG CGAAAGCGGT 3450
   CCTCGCCGAA AATGACCCAG AGCGCTGCCG GCACCTGTCC TACGAGTTGC 3500
   ATGATAAAGA AGACAGTCAT AAGTGCGGCG ACGATAGTCA TGCCCCGCGC 3550
   CCACCGGAAG GAGCTGACTG GGTTGAAGGC TCTCAAGGGC ATCGGTCGAG 3600
   ATCCCGGTGC CTAATGAGTG AGCTAACTTA CATTAATTGC GTTGCGCTCA 3650
   CTGCCCGCTT TCCAGTCGGG AAACCTGTCG TGCCAGCTGC ATTAATGAAT 3700
   CGGCCAACGC GCGGGGAGAG GCGGTTTGCG TATTGGGCGC CAGGGTGGTT 3750
   TTTCTTTTCA CCAGTGAGAC GGGCAACAGC TGATTGCCCT TCACCGCCTG 3800
   GCCCTGAGAG AGTTGCAGCA AGCGGTCCAC GCTGGTTTGC CCCAGCAGGC 3850
   GAAAATCCTG TTTGATGGTG GTTAACGGCG GGATATAACA TGAGCTGTCT 3900
   TCGGTATCGT CGTATCCCAC TACCGAGATA TCCGCACCAA CGCGCAGCCC 3950
   GGACTCGGTA ATGGCGCGCA TTGCGCCCAG CGCCATCTGA TCGTTGGCAA 4000
   CCAGCATCGC AGTGGGAACG ATGCCCTCAT TCAGCATTTG CATGGTTTGT 4050
   TGAAAACCGG ACATGGCACT CCAGTCGCCT TCCCGTTCCG CTATCGGCTG 4100
   AATTTGATTG CGAGTGAGAT ATTTATGCCA GCCAGCCAGA CGCAGACGCG 4150
   CCGAGACAGA ACTTAATGGG CCCGCTAACA GCGCGATTTG CTGGTGACCC 4200
   AATGCGACCA GATGCTCCAC GCCCAGTCGC GTACCGTCTT CATGGGAGAA 4250
   AATAATACTG TTGATGGGTG TCTGGTCAGA GACATCAAGA AATAACGCCG 4300
   GAACATTAGT GCAGGCAGCT TCCACAGCAA TGGCATCCTG GTCATCCAGC 4350
   GGATAGTTAA TGATCAGCCC ACTGACGCGT TGCGCGAGAA GATTGTGCAC 4400
   CGCCGCTTTA CAGGCTTCGA CGCCGCTTCG TTCTACCATC GACACCACCA 4450
   CGCTGGCACC CAGTTGATCG GCGCGAGATT TAATCGCCGC GACAATTTGC 4500
   GACGGCGCGT GCAGGGCCAG ACTGGAGGTG GCAACGCCAA TCAGCAACGA 4550
   CTGTTTGCCC GCCAGTTGTT GTGCCACGCG GTTGGGAATG TAATTCAGCT 4600
   CCGCCATCGC CGCTTCCACT TTTTCCCGCG TTTTCGCAGA AACGTGGCTG 4650
   GCCTGGTTCA CCACGCGGGA AACGGTCTGA TAAGAGACAC CGGCATACTC 4700
   TGCGACATCG TATAACGTTA CTGGTTTCAC ATTCACCACC CTGAATTGAC 4750
   TCTCTTCCGG GCGCTATCAT GCCATACCGC GAAAGGTTTT GCGCCATTCG 4800
   ATGGTGTCCG GGATCTCGAC GCTCTCCCTT ATGCGACTCC TGCATTAGGA 4850
   AGCAGCCCAG TAGTAGGTTG AGGCCGTTGA GCACCGCCGC CGCAAGGAAT 4900
   GGTGCATGCG GTACCAGCTG TTGACAATTA ATCATCCGGC TCGTATAATA  4950
   GTACTGTGTG GAATTGTGAG CGCTCACAAT TCCACACATC TAGAAATAAT  5000
   TTTGTTTAAC TTTAAGAAGG AGATATACCA TGGAGATCTG GATCCATCGA  5050
   TGAATTCGAG CTCCGTCGAC AAGCTTGCGG CCGCACTCGA GCACCACCAC  5100
   CACCACCACT GAGATCCGGC TGCTAACAAA GCCCGAAAGG AAGCTGAGTT  5150
   GGCTGCTGCC ACCGCTGAGC AATAACTAGC ATAACCCCTT GGGGCCTCTA  5200
   AACGGGTCTT GAGGGGTTTT TTGCTGAAAG GAGGAACTAT ATCCGGAT    5248
(2) INFORMATION FOR SEQ ID NO:22:
   (i) SEQUENCE CHARACTERISTICS:
      (A) LENGTH: 277 amino acids
      (B) TYPE: Amino Acid
      (C) STRANDEDNESS: Single
      (D) TOPOLOGY: Linear
   (ii) MOLECULE TYPE: Other nucleic acid
      (A) DESCRIPTION: native human PLP
   (iii) HYPOTHETICAL: No
   (x) PUBLICATION INFORMATION:
      (A) AUTHORS: Kronquist, K. E. Crandall, B. F. Macklin, W.B. Campagnoni, A. T. (B) TITLE: Expression of Myelin Proteins in the Developing Human Spinal Cord: Cloning and Sequencing of Human Proteolipid Protein cDNA (C) JOURNAL: Journal of Neuroscience Research (D) VOLUME: 18 (F) PAGES: 395 - 401 (G) DATE: 1987
   (xi) SEQUENCE DESCRIPTION: SEQ ID NO:22:
(2) INFORMATION FOR SEQ ID NO:23:
   (i) SEQUENCE CHARACTERISTICS:
      (A) LENGTH: 561 base pairs
      (B) TYPE: Nucleic acid
      (C) STRANDEDNESS: Double
      (D) TOPOLOGY: Linear
   (ii) MOLECULE TYPE: Other nucleic acid
      (A) DESCRIPTION: Delta PLP3
   (iii) HYPOTHETICAL: No
   (iv) ANTI-SENSE: No
   (xi) SEQUENCE DESCRIPTION: SEQ ID NO:23:
   ATG CTC      6
   Met Leu
    1
   GAG GAT CCG GGA CAT GAA GCC CTC ACT GGC ACA GAA AAG CTA     48
   Glu Asp Pro Gly His Glu Ala Leu Thr Gly Thr Glu Lys Leu
            5                  10                  15
   ATT GAG ACC TAT TTC TCC AAA AAC TAC CAA GAC TAT GAG TAT     90
   Ile Glu Thr Tyr Phe Ser Lys Asn Tyr Gln Asp Tyr Glu Tyr
               20                  25                  30
   CTC ATC AAT GTG ATC CAT GCC TTC CAG TAT GTC ATC TAT GGA    132
   Leu Ile Asn Val Ile His Ala Phe Gln Tyr Val Ile Tyr Gly
                   35                  40
   ACT GCC TCT TTC TTC TTC CTT TAT GGG GCC CTC CTG CTG GCT    174
   Thr Ala Ser Phe Phe Phe Leu Tyr Gly Ala Leu Leu Leu Ala
    45                  50                  55
   GAG GGC TTC TAC ACC ACC GGC GCA GTC AGG CAG ATC TTT GGC    216
   Glu Gly Phe Tyr Thr Thr Gly Ala Val Arg Gln Ile Phe Gly
        60                  65                  70
   GAC TAC AAG ACC ACC ATC TGC GGC AAG GGC CTG AGC GCA ACG      258
   Asp Tyr Lys Thr Thr Ile Cys Gly Lys Gly Leu Ser Ala Thr
            75                  80                  85
   GTA ACA GGG GGC CAG AAG GGG AGG GGT TCC AGA GGC CAA CAT      300
   Val Thr Gly Gly Gln Lys Gly Arg Gly Ser Arg Gly Gln His
               90                  95                  100
   CAA GCT CAT TCT TTG GAG CGG GTG TGT CAT TGT TTG GGA AAA      342
   Gln Ala His Ser Leu Glu Arg Val Cys His Cys Leu Gly Lys
                   105                 110
   TGG CTA GGA CAT CCC GAC AAG TTT GTG GGC ATC TTC AAC ACC      384
   Trp Leu Gly His Pro Asp Lys Phe Val Gly Ile Phe Asn Thr
   115                 120                 125
   TGG ACC ACC TGC CAG TCT ATT GCC TTC CCC AGC AAG ACC TCT      426
   Trp Thr Thr Cys Gln Ser Ile Ala Phe Pro Ser Lys Thr Ser
       130                 135                 140
   GCC AGT ATA GGC AGT CTC TGT GCT GAC GCC AGA ATG TAT GGT      468
   Ala Ser Ile Gly Ser Leu Cys Ala Asp Ala Arg Met Tyr Gly
           145                 150                 155
   GTT CTC CCA TGG AAT GCT TTC CCT GGC AAG GTT TGT GGC TCC      510
   Val Leu Pro Trp Asn Ala Phe Pro Gly Lys Val Cys Gly Ser
               160                 165                 170
   AAC CTT CTG TCC ATC TGC AAA ACA GCT GAG TTC CAA ATG ACC      552
   Asn Leu Leu Ser Ile Cys Lys Thr Ala Glu Phe Gln Met Thr
                   175                 180
   TTC CAC TAA                                                  561
   Phe His
   185
(2) INFORMATION FOR SEQ ID NO:24:
   (i) SEQUENCE CHARACTERISTICS:
      (A) LENGTH: 525 base pairs
      (B) TYPE: Nucleic acid
      (C) STRANDEDNESS: Double
      (D) TOPOLOGY: Linear
   (ii) MOLECULE TYPE: Other nucleic acid
      (A) DESCRIPTION: Delta PLP4
   (iii) HYPOTHETICAL: No
   (iv) ANTI-SENSE: No
   (xi) SEQUENCE DESCRIPTION: SEQ ID NO:24:
   ATG CTC GAG GAT CCG GGA CAT GAA GCC CTC ACT GGC ACA     39
   Met Leu Glu Asp Pro Gly His Glu Ala Leu Thr Gly Thr
    1               5                  10
   GAA AAG CTA ATT GAG ACC TAT TTC TCC AAA AAC TAC CAA GAC     81
   Glu Lys Leu Ile Glu Thr Tyr Phe Ser Lys Asn Tyr Gln Asp
       15                  20                  25
   TAT GAG TAT CTC ATC AAT GTG ATC CAT GCC TTC CAG TAT GCT    123
   Tyr Glu Tyr Leu Ile Asn Val Ile His Ala Phe Gln Tyr Ala
           30                  35                  40
   GAG GGC TTC TAC ACC ACC GGC GCA GTC AGG CAG ATC TTT GGC    165
   Glu Gly Phe Tyr Thr Thr Gly Ala Val Arg Gln Ile Phe Gly
               45                  50                  55
   GAC TAC AAG ACC ACC ATC TGC GGC AAG GGC CTG AGC GCA ACG    207
   Asp Tyr Lys Thr Thr Ile Cys Gly Lys Gly Leu Ser Ala Thr
                   60                  65
   GTA ACA GGG GGC CAG AAG GGG AGG GGT TCC AGA GGC CAA CAT    249
   Val Thr Gly Gly Gln Lys Gly Arg Gly Ser Arg Gly Gln His
   70                  75                  80
   CAA GCT CAT TCT TTG GAG CGG GTG TGT CAT TGT TTG GGA AAA    291
   Gln Ala His Ser Leu Glu Arg Val Cys His Cys Leu Gly Lys
       85                  90                  95
   TGG CTA GGA CAT CCC GAC AAG TTT GTG GGC ATC TTC AAC ACC    333
   Trp Leu Gly His Pro Asp Lys Phe Val Gly Ile Phe Asn Thr
           100                 105                 110
   TGG ACC ACC TGC CAG TCT ATT GCC TTC CCC AGC AAG ACC TCT    375
   Trp Thr Thr Cys Gln Ser Ile Ala Phe Pro Ser Lys Thr Ser
               115                 120                 125
   GCC AGT ATA GGC AGT CTC TGT GCT GAC GCC AGA ATG TAT GGT    417
   Ala Ser Ile Gly Ser Leu Cys Ala Asp Ala Arg Met Tyr Gly
                   130                 135
   GTT CTC CCA TGG AAT GCT TTC CCT GGC AAG GTT TGT GGC TCC    459
   Val Leu Pro Trp Asn Ala Phe Pro Gly Lys Val Cys Gly Ser
   140                 145                 150
   AAC CTT CTG TCC ATC TGC AAA ACA GCT GAG TTC CAA ATG ACC    501
   Asn Leu Leu Ser Ile Cys Lys Thr Ala Glu Phe Gln Met Thr
       155                 160                 165
   TTC CAC CAT CAC CAT CAC CAT TAA                            525
   Phe His His His His His His
           170
(2) INFORMATION FOR SEQ ID NO:25:
   (i) SEQUENCE CHARACTERISTICS:
      (A) LENGTH: 1155 base pairs
      (B) TYPE: Nucleic acid
      (C) STRANDEDNESS: Double
      (D) TOPOLOGY: Linear
   (ii) MOLECULE TYPE: Other nucleic acid
      (A) DESCRIPTION: MP3 chimera
   (iii) HYPOTHETICAL: No
   (iv) ANTI-SENSE: No
   (xi) SEQUENCE DESCRIPTION: SEQ ID NO:25:
   ATG GCG TCT CAG AAA CGT CCG TCC CAG CGT CAC GGC TCC AAA      42
   Met Ala Ser Gln Lys Arg Pro Ser Gln Arg His Gly Ser Lys
    1               5                  10
   TAC CTG GCC ACC GCC AGC ACC ATG GAC CAT GCC CGT CAT GGC      84
   Tyr Leu Ala Thr Ala Ser Thr Met Asp His Ala Arg His Gly
    15                  20                  25
   TTC CTG CCG CGT CAC CGT GAC ACC GGC ATC CTG GAC TCC ATC     126
   Phe Leu Pro Arg His Arg Asp Thr Gly Ile Leu Asp Ser Ile
        30                  35                  40
   GGC CGC TTC TTC GGC GGT GAC CGT GGT GCG CCG AAA CGT GGC     168
   Gly Arg Phe Phe Gly Gly Asp Arg Gly Ala Pro Lys Arg Gly
            45                  50                  55
   TCT GGC AAA GTG CCG TGG CTG AAA CCG GGC CGT AGC CCG CTG     210
   Ser Gly Lys Val Pro Trp Leu Lys Pro Gly Arg Ser Pro Leu
                60                  65                  70
   CCG TCT CAT GCC CGT AGC CAG CCG GGC CTG TGC AAC ATG TAC     252
   Pro Ser His Ala Arg Ser Gln Pro Gly Leu Cys Asn Met Tyr
                    75                  80
   AAA GAC TCC CAC CAC CCG GCT CGT ACC GCG CAC TAT GGC TCC     294
   Lys Asp Ser His His Pro Ala Arg Thr Ala His Tyr Gly Ser
    85                 90                  95
   CTG CCG CAG AAA TCC CAC GGC CGT ACC CAG GAT GAA AAC CCG     336
   Leu Pro Gln Lys Ser His Gly Arg Thr Gln Asp Glu Asn Pro
       100                 105                 110
   GTG GTG CAC TTC TTC AAA AAC ATT GTG ACC CCG CGT ACC CCG     378
   Val Val His Phe Phe Lys Asn Ile Val Thr Pro Arg Thr Pro
           115                 120                 125
   CCG CCG TCT CAG GGC AAA GGC CGT GGC CTG TCC CTG AGC CGT     420
   Pro Pro Ser Gln Gly Lys Gly Arg Gly Leu Ser Leu Ser Arg
               130                 135                 140
   TTC AGC TGG GGC GCC GAA GGC CAG CGT CCG GGC TTC GGT TAC     462
   Phe Ser Trp Gly Ala Glu Gly Gln Arg Pro Gly Phe Gly Tyr
                   145                 150
   GGC GGC CGT GCG TCC GAC TAT AAA TCT GCT CAC AAA GGC TTC     504
   Gly Gly Arg Ala Ser Asp Tyr Lys Ser Ala His Lys Gly Phe
   155                 160                 165
   AAA GGC GTG GAT GCC CAG GGT ACC TTG TCC AAA ATT TTC AAA     546
   Lys Gly Val Asp Ala Gln Gly Thr Leu Ser Lys Ile Phe Lys
       170                 175                 180
   CTG GGC GGC CGT GAT AGC CGT TCT GGC TCT CCG ATG GCT AGA     588
   Leu Gly Gly Arg Asp Ser Arg Ser Gly Ser Pro Met Ala Arg
           185                 190                 195
   CGT CTG GGA GGC CTC GAG GAT CCG GGA CAT GAA GCC CTC ACT     630
   Arg Leu Gly Gly Leu Glu Asp Pro Gly His Glu Ala Leu Thr
               200                 205                 210
   GGC ACA GAA AAG CTA ATT GAG ACC TAT TTC TCC AAA AAC TAC     672
   Gly Thr Glu Lys Leu Ile Glu Thr Tyr Phe Ser Lys Asn Tyr
                   215                 220
   CAA GAC TAT GAG TAT CTC ATC AAT GTG ATC CAT GCC TTC CAG     714
   Gln Asp Tyr Glu Tyr Leu Ile Asn Val Ile His Ala Phe Gln
   225                 230                 235
   TAT GTC ATC TAT GGA ACT GCC TCT TTC TTC TTC CTT TAT GGG     756
   Tyr Val Ile Tyr Gly Thr Ala Ser Phe Phe Phe Leu Tyr Gly
       240                 245                 250
   GCC CTC CTG CTG GCT GAG GGC TTC TAC ACC ACC GGC GCA GTC     798
   Ala Leu Leu Leu Ala Glu Gly Phe Tyr Thr Thr Gly Ala Val
           255                 260                 265
   AGG CAG ATC TTT GGC GAC TAC AAG ACC ACC ATC TGC GGC AAG     840
   Arg Gln Ile Phe Gly Asp Tyr Lys Thr Thr Ile Cys Gly Lys
               270                 275                 280
   GGC CTG AGC GCA ACG GTA ACA GGG GGC CAG AAG GGG AGG GGT     882
   Gly Leu Ser Ala Thr Val Thr Gly Gly Gln Lys Gly Arg Gly
                   285                 290
   TCC AGA GGC CAA CAT CAA GCT CAT TCT TTG GAG CGG GTG TGT     924
   Ser Arg Gly Gln His Gln Ala His Ser Leu Glu Arg Val Cys
   295                 300                 305
   CAT TGT TTG GGA AAA TGG CTA GGA CAT CCC GAC AAG TTT GTG     966
   His Cys Leu Gly Lys Trp Leu Gly His Pro Asp Lys Phe Val
       310                 315                 320
   GGC ATC TTC AAC ACC TGG ACC ACC TGC CAG TCT ATT GCC TTC    1008
   Gly Ile Phe Asn Thr Trp Thr Thr Cys Gln Ser Ile Ala Phe
               325                 330                 335
   CCC AGC AAG ACC TCT GCC AGT ATA GGC AGT CTC TGT GCT GAC    1050
   Pro Ser Lys Thr Ser Ala Ser Ile Gly Ser Leu Cys Ala Asp
               340                 345                 350
   GCC AGA ATG TAT GGT GTT CTC CCA TGG AAT GCT TTC CCT GGC    1092
   Ala Arg Met Tyr Gly Val Leu Pro Trp Asn Ala Phe Pro Gly
                   355                 360
   AAG GTT TGT GGC TCC AAC CTT CTG TCC ATC TGC AAA ACA GCT    1134
   Lys Val Cys Gly Ser Asn Leu Leu Ser Ile Cys Lys Thr Ala
   365                 370                 375
   GAG TTC CAA ATG ACC TTC CAC                                1155
   Glu Phe Gln Met Thr Phe His
       380                 385
(2) INFORMATION FOR SEQ ID NO:26:
   (i) SEQUENCE CHARACTERISTICS:
      (A) LENGTH: 1122 base pairs
      (B) TYPE: Nucleic acid
      (C) STRANDEDNESS: Double
      (D) TOPOLOGY: Linear
   (ii) MOLECULE TYPE: Other nucleic acid
      (A) DESCRIPTION: MP4 chimera
   (iii) HYPOTHETICAL: No
   (iv) ANTI-SENSE: No
   (xi) SEQUENCE DESCRIPTION: SEQ ID NO:26:
   ATG GCG TCT CAG AAA CGT CCG TCC CAG CGT CAC GGC TCC      39
   Met Ala Ser Gln Lys Arg Pro Ser Gln Arg His Gly Ser
    1              5                   10
   AAA TAC CTG GCC ACC GCC AGC ACC ATG GAC CAT GCC CGT CAT      81
   Lys Tyr Leu Ala Thr Ala Ser Thr Met Asp His Ala Arg His
        15                 20                  25
   GGC TTC CTG CCG CGT CAC CGT GAC ACC GGC ATC CTG GAC TCC     123
   Gly Phe Leu Pro Arg His Arg Asp Thr Gly Ile Leu Asp Ser
            30                  35                  40
   ATC GGC CGC TTC TTC GGC GGT GAC CGT GGT GCG CCG AAA CGT     165
   Ile Gly Arg Phe Phe Gly Gly Asp Arg Gly Ala Pro Lys Arg
                45                  50                  55
   GGC TCT GGC AAA GTG CCG TGG CTG AAA CCG GGC CGT AGC CCG     207
   Gly Ser Gly Lys Val Pro Trp Leu Lys Pro Gly Arg Ser Pro
                    60                  65
   CTG CCG TCT CAT GCC CGT AGC CAG CCG GGC CTG TGC AAC ATG     249
   Leu Pro Ser His Ala Arg Ser Gln Pro Gly Leu Cys Asn Met
    70                  75                  80
   TAC AAA GAC TCC CAC CAC CCG GCT CGT ACC GCG CAC TAT GGC     291
   Tyr Lys Asp Ser His His Pro Ala Arg Thr Ala His Tyr Gly
        85                  90                  95
   TCC CTG CCG CAG AAA TCC CAC GGC CGT ACC CAG GAT GAA AAC     333
   Ser Leu Pro Gln Lys Ser His Gly Arg Thr Gln Asp Glu Asn
           100                 105                 110
   CCG GTG GTG CAC TTC TTC AAA AAC ATT GTG ACC CCG CGT ACC     375
   Pro Val Val His Phe Phe Lys Asn Ile Val Thr Pro Arg Thr
               115                 120                 125
   CCG CCG CCG TCT CAG GGC AAA GGC CGT GGC CTG TCC CTG AGC     417
   Pro Pro Pro Ser Gln Gly Lys Gly Arg Gly Leu Ser Leu Ser
                   130                 135
   CGT TTC AGC TGG GGC GCC GAA GGC CAG CGT CCG GGC TTC GGT      459
   Arg Phe Ser Trp Gly Ala Glu Gly Gln Arg Pro Gly Phe Gly
   140                 145                 150
   TAC GGC GGC CGT GCG TCC GAC TAT AAA TCT GCT CAC AAA GGC      501
   Tyr Gly Gly Arg Ala Ser Asp Tyr Lys Ser Ala His Lys Gly
       155                 160                 165
   TTC AAA GGC GTG GAT GCC CAG GGT ACC TTG TCC AAA ATT TTC      543
   Phe Lys Gly Val Asp Ala Gln Gly Thr Leu Ser Lys Ile Phe
           170                 175                 180
   AAA CTG GGC GGC CGT GAT AGC CGT TCT GGC TCT CCG ATG GCT      585
   Lys Leu Gly Gly Arg Asp Ser Arg Ser Gly Ser Pro Met Ala
               185                 190                 195
   AGA CGT CTG GGA GGC CTC GAG GAT CCG GGA CAT GAA GCC CTC      627
   Arg Arg Leu Gly Gly Leu Glu Asp Pro Gly His Glu Ala Leu
                   200                 205
   ACT GGC ACA GAA AAG CTA ATT GAG ACC TAT TTC TCC AAA AAC      669
   Thr Gly Thr Glu Lys Leu Ile Glu Thr Tyr Phe Ser Lys Asn
   210                 215                 220
   TAC CAA GAC TAT GAG TAT CTC ATC AAT GTG ATC CAT GCC TTC      711
   Tyr Gln Asp Tyr Glu Tyr Leu Ile Asn Val Ile His Ala Phe
       225                 230                 235
   CAG TAT GCT GAG GGC TTC TAC ACC ACC GGC GCA GTC AGG CAG      753
   Gln Tyr Ala Glu Gly Phe Tyr Thr Thr Gly Ala Val Arg Gln
           240                 245                 250
   ATC TTT GGC GAC TAC AAG ACC ACC ATC TGC GGC AAG GGC CTG      795
   Ile Phe Gly Asp Tyr Lys Thr Thr Ile Cys Gly Lys Gly Leu
               255                 260                 265
   AGC GCA ACG GTA ACA GGG GGC CAG AAG GGG AGG GGT TCC AGA      837
   Ser Ala Thr Val Thr Gly Gly Gln Lys Gly Arg Gly Ser Arg
                   270                 275
   GGC CAA CAT CAA GCT CAT TCT TTG GAG CGG GTG TGT CAT TGT      879
   Gly Gln His Gln Ala His Ser Leu Glu Arg Val Cys His Cys
   280                 285                 290
   TTG GGA AAA TGG CTA GGA CAT CCC GAC AAG TTT GTG GGC ATC      921
   Leu Gly Lys Trp Leu Gly His Pro Asp Lys Phe Val Gly Ile
       295                 300                 305
   TTC AAC ACC TGG ACC ACC TGC CAG TCT ATT GCC TTC CCC AGC      963
   Phe Asn Thr Trp Thr Thr Cys Gln Ser Ile Ala Phe Pro Ser
           310                 315                 320
   AAG ACC TCT GCC AGT ATA GGC AGT CTC TGT GCT GAC GCC AGA     1005
   Lys Thr Ser Ala Ser Ile Gly Ser Leu Cys Ala Asp Ala Arg
               325                 330                 335
   ATG TAT GGT GTT CTC CCA TGG AAT GCT TTC CCT GGC AAG GTT     1047
   Met Tyr Gly Val Leu Pro Trp Asn Ala Phe Pro Gly Lys Val
                   340                 345
   TGT GGC TCC AAC CTT CTG TCC ATC TGC AAA ACA GCT GAG TTC     1089
   Cys Gly Ser Asn Leu Leu Ser Ile Cys Lys Thr Ala Glu Phe
   350                 355                 360
   CAA ATG ACC TTC CAC CAT CAC CAT CAC CAT TAA                 1122
   Gln Met Thr Phe His His His His His His
       365                 370
(2) INFORMATION FOR SEQ ID NO:27:
   (i) SEQUENCE CHARACTERISTICS:
      (A) LENGTH: 1125 base pairs
      (B) TYPE: Nucleic acid
      (C) STRANDEDNESS: Double
      (D) TOPOLOGY: Linear
   (ii) MOLECULE TYPE: Other nucleic acid
      (A) DESCRIPTION: PM4 chimera
   (iii) HYPOTHETICAL: No
   (iv) ANTI-SENSE: No
   (xi) SEQUENCE DESCRIPTION: SEQ ID NO:27:
   ATG CTC GAG GAT CCG GGA CAT GAA GCC CTC ACT GGC ACA GAA      42
   Met Leu Glu Asp Pro Gly His Glu Ala Leu Thr Gly Thr Glu
    1               5                  10
   AAG CTA ATT GAG ACC TAT TTC TCC AAA AAC TAC CAA GAC TAT      84
   Lys Leu Ile Glu Thr Tyr Phe Ser Lys Asn Tyr Gln Asp Tyr
    15                 20                  25
   GAG TAT CTC ATC AAT GTG ATC CAT GCC TTC CAG TAT GCT GAG     126
   Glu Tyr Leu Ile Asn Val Ile His Ala Phe Gln Tyr Ala Glu
        30                  35                  40
   GGC TTC TAC ACC ACC GGC GCA GTC AGG CAG ATC TTT GGC GAC     168
   Gly Phe Tyr Thr Thr Gly Ala Val Arg Gln Ile Phe Gly Asp
            45                  50                  55
   TAC AAG ACC ACC ATC TGC GGC AAG GGC CTG AGC GCA ACG GTA     210
   Tyr Lys Thr Thr Ile Cys Gly Lys Gly Leu Ser Ala Thr Val
                60                  65                  70
   ACA GGG GGC CAG AAG GGG AGG GGT TCC AGA GGC CAA CAT CAA     252
   Thr Gly Gly Gln Lys Gly Arg Gly Ser Arg Gly Gln His Gln
                    75                  80
   GCT CAT TCT TTG GAG CGG GTG TGT CAT TGT TTG GGA AAA TGG     294
   Ala His Ser Leu Glu Arg Val Cys His Cys Leu Gly Lys Trp
    85                 90                  95
   CTA GGA CAT CCC GAC AAG TTT GTG GGC ATC TTC AAC ACC TGG     336
   Leu Gly His Pro Asp Lys Phe Val Gly Ile Phe Asn Thr Trp
       100                 105                 110
   ACC ACC TGC CAG TCT ATT GCC TTC CCC AGC AAG ACC TCT GCC     378
   Thr Thr Cys Gln Ser Ile Ala Phe Pro Ser Lys Thr Ser Ala
           115                 120                 125
   AGT ATA GGC AGT CTC TGT GCT GAC GCC AGA ATG TAT GGT GTT     420
   Ser Ile Gly Ser Leu Cys Ala Asp Ala Arg Met Tyr Gly Val
               130                 135                 140
   CTC CCA TGG AAT GCT TTC CCT GGC AAG GTT TGT GGC TCC AAC     462
   Leu Pro Trp Asn Ala Phe Pro Gly Lys Val Cys Gly Ser Asn
                   145                 150
   CTT CTG TCC ATC TGC AAA ACA GCT GAG TTC CAA ATG ACC TTC     504
   Leu Leu Ser Ile Cys Lys Thr Ala Glu Phe Gln Met Thr Phe
   155                 160                 165
   CAC GGC GGT GGC GGT GCG TCT CAG AAA CGT CCG TCC CAG CGT     546
   His Gly Gly Gly Gly Ala Ser Gln Lys Arg Pro Ser Gln Arg
       170                 175                 180
   CAC GGC TCC AAA TAC CTG GCC ACC GCC AGC ACC ATG GAC CAT     588
   His Gly Ser Lys Tyr Leu Ala Thr Ala Ser Thr Met Asp His
               185                     190                     195
   GCC CGT CAT GGC TTC CTG CCG CGT CAC CGT GAC ACC GGC ATC        630
   Ala Arg His Gly Phe Leu Pro Arg His Arg Asp Thr Gly Ile
                   200                     205                 210
   CTG GAC TCC ATC GGC CGC TTC TTC GGC GGT GAC CGT GGT GCG        672
   Leu Asp Ser Ile Gly Arg Phe Phe Gly Gly Asp Arg Gly Ala
                       215                     220
   CCG AAA CGT GGC TCT GGC AAA GTG CCG TGG CTG AAA CCG GGC        714
   Pro Lys Arg Gly Ser Gly Lys Val Pro Trp Leu Lys Pro Gly
   225                 230                     235
   CGT AGC CCG CTG CCG TCT CAT GCC CGT AGC CAG CCG GGC CTG        756
   Arg Ser Pro Leu Pro Ser His Ala Arg Ser Gln Pro Gly Leu
       240                 245                     250
   TGC AAC ATG TAC AAA GAC TCC CAC CAC CCG GCT CGT ACC GCG        798
   Cys Asn Met Tyr Lys Asp Ser His His Pro Ala Arg Thr Ala
           255                     260                     265
   CAC TAT GGC TCC CTG CCG CAG AAA TCC CAC GGC CGT ACC CAG        840
   His Tyr Gly Ser Leu Pro Gln Lys Ser His Gly Arg Thr Gln
               270                     275                     280
   GAT GAA AAC CCG GTG GTG CAC TTC TTC AAA AAC ATT GTG ACC        882
   Asp Glu Asn Pro Val Val His Phe Phe Lys Asn Ile Val Thr
                   285                     290
   CCG CGT ACC CCG CCG CCG TCT CAG GGC AAA GGC CGT GGC CTG        924
   Pro Arg Thr Pro Pro Pro Ser Gln Gly Lys Gly Arg Gly Leu
   295                 300                     305
   TCC CTG AGC CGT TTC AGC TGG GGC GCC GAA GGC CAG CGT CCG        966
   Ser Leu Ser Arg Phe Ser Trp Gly Ala Glu Gly Gln Arg Pro
       310                     315                     320
   GGC TTC GGC TAC GGC GGC CGT GCG TCC GAC TAT AAA TCT GCT       1008
   Gly Phe Gly Tyr Gly Gly Arg Ala Ser Asp Tyr Lys Ser Ala
           325                     330                     335
   CAC AAA GGC TTC AAA GGC GTG GAT GCC CAG GGC ACC CTG TCC       1050
   His Lys Gly Phe Lys Gly Val Asp Ala Gln Gly Thr Leu Ser
               340                     345                     350
   AAA ATT TTC AAA CTG GGC GGC CGT GAT AGC CGT TCT GGC TCT       1092
   Lys Ile Phe Lys Leu Gly Gly Arg Asp Ser Arg Ser Gly Ser
   355                 360                     365
   CCG ATG GCT AGA CGT CAT CAC CAT CAC CAT CAC                   1125
   Pro Met Ala Arg Arg His His His His His His
       370                 375
(2) INFORMATION FOR SEQ ID NO:28:
   (i) SEQUENCE CHARACTERISTICS:
      (A) LENGTH: 1476 base pairs
      (B) TYPE: Nucleic acid
      (C) STRANDEDNESS: Double
      (D) TOPOLOGY: Linear
   (ii) MOLECULE TYPE: Other nucleic acid
      (A) DESCRIPTION: MMOGP4 chimera
   (iii) HYPOTHETICAL: No
   (iv) ANTI-SENSE: No
   (xi) SEQUENCE DESCRIPTION: SEQ ID NO:28:
   ATG GCG TCT CAG AAA CGT CCG TCC CAG CGT CAC GGC TCC AAA TAC CTG 48
   Met Ala Ser Gln Lys Arg Pro Ser Gln Arg His Gly Ser Lys Tyr Leu
                   5                  10                  15
   GCC ACC GCC AGC ACC ATG GAC CAT GCC CGT CAT GGC TTC CTG CCG CGT    96
   Ala Thr Ala Ser Thr Met Asp His Ala Arg His Gly Phe Leu Pro Arg
               20                  25                  30
   CAC CGT GAC ACC GGC ATC CTG GAC TCC ATC GGC CGC TTC TTC GGC GGT   144
   His Arg Asp Thr Gly Ile Leu Asp Ser Ile Gly Arg Phe Phe Gly Gly
           35                  40                  45
   GAC CGT GGT GCG CCG AAA CGT GGC TCT GGC AAA GTG CCG TGG CTG AAA   192
   Asp Arg Gly Ala Pro Lys Arg Gly Ser Gly Lys Val Pro Trp Leu Lys
       50                  55                  60
   CCG GGC CGT AGC CCG CTG CCG TCT CAT GCC CGT AGC CAG CCG GGC CTG   240
   Pro Gly Arg Ser Pro Leu Pro Ser His Ala Arg Ser Gln Pro Gly Leu
   65                  70                  75                  80
   TGC AAC ATG TAC AAA GAC TCC CAC CAC CCG GCT CGT ACC GCG CAC TAT   288
   Cys Asn Met Tyr Lys Asp Ser His His Pro Ala Arg Thr Ala His Tyr
                   85                  90                  95
   GGC TCC CTG CCG CAG AAA TCC CAC GGC CGT ACC CAG GAT GAA AAC CCG   336
   Gly Ser Leu Pro Gln Lys Ser His Gly Arg Thr Gln Asp Glu Asn Pro
               100                 105                 110
   GTG GTG CAC TTC TTC AAA AAC ATT GTG ACC CCG CGT ACC CCG CCG CCG   384
   Val Val His Phe Phe Lys Asn Ile Val Thr Pro Arg Thr Pro Pro Pro
           115                 120                 125
   TCT CAG GGC AAA GGC CGT GGC CTG TCC CTG AGC CGT TTC AGC TGG GGC   432
   Ser Gln Gly Lys Gly Arg Gly Leu Ser Leu Ser Arg Phe Ser Trp Gly
       130                 135                 140
   GCC GAA GGC CAG CGT CCG GGC TTC GGT TAC GGC GGC CGT GCG TCC GAC   480
   Ala Glu Gly Gln Arg Pro Gly Phe Gly Tyr Gly Gly Arg Ala Ser Asp
   145                 150                 155                 160
   TAT AAA TCT GCT CAC AAA GGC TTC AAA GGC GTG GAT GCC CAG GGT ACC   528
   Tyr Lys Ser Ala His Lys Gly Phe Lys Gly Val Asp Ala Gln Gly Thr
                   165                 170                 175
   TTG TCC AAA ATT TTC AAA CTG GGC GGC CGT GAT AGC CGT TCT GGC TCT   576
   Leu Ser Lys Ile Phe Lys Leu Gly Gly Arg Asp Ser Arg Ser Gly Ser
               180                 185                 190
   CCG ATG GCT AGA CGT CCC GGG CAG TTC AGA GTG ATA GGA CCA AGA CAC   624
   Pro Met Ala Arg Arg Pro Gly Gln Phe Arg Val Ile Gly Pro Arg His
           195                 200                 205
   CCT ATC CGG GCT CTG GTC GGG GAT GAA GTG GAA TTG CCA TGT CGC ATA   672
   Pro Ile Arg Ala Leu Val Gly Asp Glu Val Glu Leu Pro Cys Arg Ile
       210                 215                 220
   TCT CCT GGG AAG AAC GCT ACA GGC ATG GAG GTG GGG TGG TAC CGC CCC   720
   Ser Pro Gly Lys Asn Ala Thr Gly Met Glu Val Gly Trp Tyr Arg Pro
   225                 230                 235                 240
   CCC TTC TCT AGG GTG GTT CAT CTC TAC AGA AAT GGC AAG GAC CAA GAT   768
   Pro Phe Ser Arg Val Val His Leu Tyr Arg Asn Gly Lys Asp Gln Asp
                   245                 250                 255
   GGA GAC CAG GCA CCT GAA TAT CGG GGC CGG ACA GAG CTG CTG AAA GAT   816
   Gly Asp Gln Ala Pro Glu Tyr Arg Gly Arg Thr Glu Leu Leu Lys Asp
               260                 265                 270
   GCT ATT GGT GAG GGA AAG GTG ACT CTC AGG ATC CGG AAT GTA AGG TTC   864
   Ala Ile Gly Glu Gly Lys Val Thr Leu Arg Ile Arg Asn Val Arg Phe
           275                 280                 285
   TCA GAT GAA GGA GGT TTC ACC TGC TTC TTC CGA GAT CAT TCT TAC CAA   912
   Ser Asp Glu Gly Gly Phe Thr Cys Phe Phe Arg Asp His Ser Tyr Gln
       290                 295                 300
   GAG GAG GCA GCA ATG GAA TTG AAA GTA GAA GAT CCC TTC TAC TGG CTC   960
   Glu Glu Ala Ala Met Glu Leu Lys Val Glu Asp Pro Phe Tyr Trp Leu
   305                 310                 315                 320
   GAG GAT CCG GGA CAT GAA GCC CTC ACT GGC ACA GAA AAG CTA ATT GAG  1008
   Glu Asp Pro Gly His Glu Ala Leu Thr Gly Thr Glu Lys Leu Ile Glu
                   325                 330                 335
   ACC TAT TTC TCC AAA AAC TAC CAA GAC TAT GAG TAT CTC ATC AAT GTG  1056
   Thr Tyr Phe Ser Lys Asn Tyr Gln Asp Tyr Glu Tyr Leu Ile Asn Val
               340                 345                 350
   ATC CAT GCC TTC CAG TAT GCT GAG GGC TTC TAC ACC ACC GGC GCA GTC  1104
   Ile His Ala Phe Gln Tyr Ala Glu Gly Phe Tyr Thr Thr Gly Ala Val
           355                 360                 365
   AGG CAG ATC TTT GGC GAC TAC AAG ACC ACC ATC TGC GGC AAG GGC CTG  1152
   Arg Gln Ile Phe Gly Asp Tyr Lys Thr Thr Ile Cys Gly Lys Gly Leu
       370                 375                 380
   AGC GCA ACG GTA ACA GGG GGC CAG AAG GGG AGG GGT TCC AGA GGC CAA  1200
   Ser Ala Thr Val Thr Gly Gly Gln Lys Gly Arg Gly Ser Arg Gly Gln
   385                 390                 395                 400
   CAT CAA GCT CAT TCT TTG GAG CGG GTG TGT CAT TGT TTG GGA AAA TGG  1248
   His Gln Ala His Ser Leu Glu Arg Val Cys His Cys Leu Gly Lys Trp
   405                 410                 415
   CTA GGA CAT CCC GAC AAG TTT GTG GGC ATC TTC AAC ACC TGG ACC ACC  1296
   Leu Gly His Pro Asp Lys Phe Val Gly Ile Phe Asn Thr Trp Thr Thr
               420                 425                 430
   TGC CAG TCT ATT GCC TTC CCC AGC AAG ACC TCT GCC AGT ATA GGC AGT  1344
   Cys Gln Ser Ile Ala Phe Pro Ser Lys Thr Ser Ala Ser Ile Gly Ser
           435                 440                 445
   CTC TGT GCT GAC GCC AGA ATG TAT GGT GTT CTC CCA TGG AAT GCT TTC  1392
   Leu Cys Ala Asp Ala Arg Met Tyr Gly Val Leu Pro Trp Asn Ala Phe
       450                 455                 460
   CCT GGC AAG GTT TGT GGC TCC AAC CTT CTG TCC ATC TGC AAA ACA GCT  1440
   Pro Gly Lys Val Cys Gly Ser Asn Leu Leu Ser Ile Cys Lys Thr Ala
   465                 470                 475                 480
   GAG TTC CAA ATG ACC TTC CAC CAT CAC CAT CAC CAT                  1476
   Glu Phe Gln Met Thr Phe His His His His His His
                   485                 490
(2) INFORMATION FOR SEQ ID NO:29:
   (i) SEQUENCE CHARACTERISTICS:
      (A) LENGTH: 732 base pairs
      (B) TYPE: Nucleic acid
      (C) STRANDEDNESS: Double
      (D) TOPOLOGY: Linear
   (ii) MOLECULE TYPE: Other nucleic acid
      (A) DESCRIPTION: Delta PLP2
   (iii) HYPOTHETICAL: No
   (iv) ANTI-SENSE: No
   (xi) SEQUENCE DESCRIPTION: SEQ ID NO:29:

## Description

### FIELD OF THE INVENTION

The present invention relates to the treatment of autoimmune diseases. In particular, the invention provides compositions and methods facilitating the diagnosis and treatment of Multiple Sclerosis (MS). More particularly, engineered human Myelin Basic Protein (MBP) molecules, i.e., MBP polypeptides and nucleic acid molecules encoding MBP polypeptides, and Proteolipid Protein (PLP) molecules, i.e., polypeptides comprising PLP sequences and nucleic acid molecules encoding such polypeptides, are provided, as well as methods for the use of such polypeptides for the diagnosis, clinical assessment, and therapeutic treatment of multiple sclerosis.

### BACKGROUND OF THE INVENTION

The discussion in this section is not limited to subject matter that qualifies as "prior art" against the present invention. Therefore, no admission of such prior art status shall be implied or inferred by reason of inclusion of particular subject matter in this discussion, and no declaration against the present inventors' interests shall be implied by reason of such inclusion.

### Autoimmune Diseases

Autoimmune diseases result from the loss of tolerance to certain self antigens, resulting in an inappropriate attack by the immune system upon these antigens. Numerous mechanisms normally function to maintain immune self-tolerance in both the antibody-mediated (humoral) and cellular aspects of the immune system. It is when these mechanisms malfunction that autoimmune diseases occur.

Illnesses resulting from such misdirected immune system activity affect more than 10 million patients in the U.S. alone. Therapies that treat the causes, rather than the symptoms of these diseases have long been sought. While agents have been found that provide beneficial reductions in autoimmune activity, such treatments, in general, have the undesirable and dangerous effect of also compromising normal immune functions, and are thus considered sub optimal.

### Multiple Sclerosis

Multiple Sclerosis (MS) is a progressive neurodegenerative autoimmune disorder affecting about 350,000 Americans (see, for example, Hauser, 1994). Females are twice as likely as males to develop the disease. MS usually affects patients who are between the ages of 15 and 50 years, most commonly young women between the ages of 20 and 40. MS derives its name from the multiple scarred (sclerotic) areas of degeneration visible on macroscopic examination of the central nervous system (CNS) of affected individuals. The degeneration associated with MS includes demyelination, chronic inflammation, and gliosis (scarring) of affected areas of the brain, optic nerve, and spinal cord.

MS is characterized by different types and stages of disease progression. Patients are diagnosed as having relapsing and remitting MS when they experience periods of exacerbations and remissions. Rapidly progressive or chronically progressive MS is diagnosed depending upon the pace of disease progression. These stages usually occur later in the course of the disease when the extent of recovery from individual attacks decreases and there are clinically stable periods between periods of deterioration. Inactive MS typically occurs late in disease progression and is characterized by fixed neurologic deficits of variable magnitude.

MS is always debilitating and may sometimes lead to paralysis and death. Although the factors triggering the initial onset of MS remain unknown, evidence is persuasive that MS pathology results from an abnormal immune response against the myelin sheath. This immune response involves autoimmune actions of certain white blood cells. It is believed that neuroantigen-specific T cells are especially important in this regard.

Pathologically, MS is characterized by chronic inflammation, demyelination, and gliosis of white matter. The classic lesions of MS, termed plaques, are well-demarcated gray or pink areas easily distinguished from surrounding white matter. (The coloration of white matter is due to the high concentrations of myelin in this tissue.) The acute MS lesion is characterized by demyelination associated with tissue infiltration by mononuclear cells, predominantly T cells (both helper and cytotoxic) and macrophages, with B cells and plasma cells rarely being present. These inflammatory infiltrates appear to mediate the demyelination that is characteristic of the disease. Since activated T cells release cytokines that promote macrophage infiltration and activation, T cells are considered the primary mediators of pathogenic autoimmune attack in MS. More detailed discussions of T cells and myelin are found below under "T Cell Physiology," "T Cells and Autoimmune Pathogenesis," and "T Cells Target Defined Autoantigens in MS."

Current treatments for MS vary. Depending on the severity of disease and the response to treatment, a variety of options for drug therapy are available. Drugs used to treat MS include steroids such as prednisone and methylprednisolone, hormones such as adrenocorticotropic hormone (ACTH), antimetabolites such as azathioprine, alkylating agents such as cyclophosphamide, and T-cell inhibitory agents such as cyclosporine. The administration of any of these drugs is dangerous, as they all typically produce some level of generalized immunosuppression and leave the patient more prone to infection. Patients may also experience side effects such as nausea, hair loss, hypertension, and renal dysfunction when treated with such drugs. In addition, some of these drugs are carcinogenic.

New approaches to treating MS include interferon-beta therapy, which can lessen the frequency of MS attacks and may slow disease progression. Other new approaches include administration of antigens involved in MS autoimmune responses, as discussed below.

### Diagnosis of MS

MS is typically diagnosed based on medical history and physical examinations. No clinical signs or diagnostic tests are unique to MS. Diagnosis of a patient with a single, initial symptom commonly associated with MS cannot be definitive, although symptoms of relapsing and remitting disease increases the likelihood of an MS diagnosis. Two or more episodes of worsening each lasting 24 hours or occurring at least a month apart, or slow stepwise progression of signs and symptoms over at least six months are considered strong indications of MS. MRI findings implicating involvement in two or more areas of CNS white matter and evidence of systemic disease are also indicative of MS.

Currently, various laboratory tests are performed to confirm the diagnosis and assess the progression of the disease. Such tests include analysis of human cerebrospinal fluid (CSF) and blood for chemical and cellular signs of MS pathology.

CSF abnormalities associated with MS consist of mononuclear cell pleocytosis and the presence of autoreactive (typically myelin reactive) T cells, an elevation in the level of total Ig, and the presence of oligoclonal Ig, typically seen as two or more oligoclonal bands. In approximately 80 percent of patients, the CSF content of IgG is increased in the presence of a normal concentration of total protein. This results from the selective production of IgG within the CNS.

Oligoclonal banding of CSF IgG is detected by agarose gel electrophoresis techniques. Two or more oligoclonal bands are found in 75 to 90 percent of MS patients. The presence of oligoclonal banding correlates with an elevated total IgG level in MS. Other Ig abnormalities in MS CSF include free kappa or lambda light chains and elevated levels of other Ig isotypes including IgA. -

Metabolites derived from myelin breakdown also may be detected in CSF. Elevated levels of PLP or its fragments may be detected, e.g., by radioimmunoassay, both in MS and in some patients with other neurologic diseases.

In addition to many of the pathologic signs described above for CSF, blood of MS patients may show increased levels of IgG synthesis, polymorphonuclear leukocytes, decreased serum B₁₂ levels, elevated erythrocyte sedimentation rate, and presence of autoantibodies or autoreactive T cells. As discussed below, the "reactive T cell index" is a particularly useful cellular finding for monitoring the clinical course of MS.

While these various indicators of MS disease are clinically useful, other means of following the course and extent of autoimmune activity in MS patients using relatively inexpensive and easily quantifiable tests, such as blood or cerebrospinal fluid tests (as opposed to expensive imaging techniques such as MRI) are needed.

### T Cells. Antigen Presenting Cells, and T Cell Epitopes

As mentioned above, MS pathogenesis is believed to be mediated by the inappropriate actions of white blood cells (leukocytes), most importantly T cells. T cells are mononuclear white blood cells that provide many essential immune functions. The importance of T cells in human autoimmune diseases has been increasingly appreciated in the past decade. Studies using treatments that result in generalized immunosuppression have defined a critical role for a subset of T cells, known as CD4⁺ or helper T cells, as primary regulators of all immune responses (both cellular and humoral) to protein or peptide antigens.

T cells mediate tissue injury by indirect and direct means. T cells.of both CD8⁺ (cytotoxic) and CD4⁺ (helper) subsets secrete a variety of inflammatory cytokines that can damage tissues indirectly by activating various other types of white blood cells. Examples of such T cell effects include activation of antibody secreting B cells (stimulating humoral immune activity) and activation of macrophages, which can cause acute tissue damage and inflammation by releasing hydrolytic enzymes, reactive oxygen species, and additional pro-inflammatory cytokines. In addition to these indirect effects of T cell activity, direct tissue damage can be mediated by CD8⁺ cytotoxic T cells attacking cells displaying target antigens.

One unique aspect of the physiology of T cells is the presence of membrane bound antibody-like binding structures called T cell receptors (TCRs) on their cell surfaces. Like antibodies, TCRs bind with high specificity to particular antigens. Like antibody-producing cells, which develop as multitudinous clones of cells, each clone producing antibodies with unique specificities, T cells develop as a vast number of distinct clones, and any particular T cell clone expresses a single type of TCR with a defined binding specificity. T cell clones with TCRs that bind specifically to self antigens are responsible for the development of autoimmune diseases.

In addition to being cell surface, rather than soluble molecules, TCRs differ from antibodies in the way they recognize antigens. While antibodies bind to antigens in various contexts (e.g., antigens that are native, denatured, soluble, or membrane bound), TCRs only bind to most antigens after the antigens have been broken down (processed) by certain cells known as antigen presenting cells (APCs) and the resulting peptides displayed (presented) on the cell surfaces of the APCs in association with class II or class I proteins of the major histocompatibility complex (MHC). In a human population, different individuals may display very different MHC molecules of these classes. Therefore, many different epitopes may be preferentially presented in such individuals.

The details of the mechanism by which antigen processing is carried out by APCs are poorly understood. There is consequently considerable uncertainty regarding the ability of APCs to process a given antigen in such a way as to produce and display a particular peptide unless that antigen has already been characterized in this respect.

One exception to the requirement that APCs process and present antigens in order for the antigens to stimulate T cells via their TCRs is the case of small peptide antigens. Such peptides can bind directly to MHC class I molecules on cell surfaces without being processed by APCs, and may then be "recognized" and bound by specific TCRs and thereby stimulate T cells.

Studies of the interactions of antibodies and TCRs with their specific antigens have shown that a particular polypeptide antigen typically comprises numerous submolecular features, known as epitopes, that each can serve as a distinct binding site for a particular antibody or (subsequent to APC processing of the polypeptide and MHC display of a derived peptide comprising the T cell epitope) a particular TCR.

Thus, TCRs and antibodies are similar in that each only recognizes a small portion of a polypeptide antigen. They differ in that an antibody typically recognizes its specific epitope within the context of the intact polypeptide, while a TCR only recognizes a specific epitope as an MHC class II or class I associated peptide fragment of a processed polypeptide on the surface of an APC. Importantly, this TCR epitope recognition process can only occur if an APC can process the polypeptide antigen so as to generate and display the appropriate peptide. Thus, even though a peptide that is recognized by a specific TCR may be present in a particular polypeptide antigen, it is uncertain whether peptides capable of stimulating T cells expressing that specific TCR will be derived from that polypeptide antigen *in vivo*. This is because it is uncertain whether APCs can generate the peptide recognized by the specific TCR by processing the particular polypeptide antigen.

This lack of certainty regarding the results of APC processing of a particular polypeptide antigen stems from several factors. One reason why an APC may not process a particular polypeptide antigen so as to display a specific peptide epitope contained within the polypeptide is that the APC efficiently cleaves the polypeptide at a site within the epitope and thereby destroys it. A second reason is that the polypeptide cannot enter into or be effectively broken down by the subcellular compartments of APCs responsible for polypeptide processing.

Certain aspects of the primary structure (linear amino acid sequence), secondary structure (3D structure resulting from interactions of amino acid residues that are close to one another in the linear amino acid sequence), or tertiary structure (3D structure resulting from interactions of amino acid residues that are far from one another in the linear amino acid sequence but come into proximity with each other as a result of folding of the polypeptide chain) can impact APC processing. The amino acid sequence of a polypeptide is clearly the most important factor in determining its potential to be processed and displayed by APCs so as to stimulate specific T cells. The peptide recognized by the specific T cell's TCRs must be contained within the amino acid sequence of the polypeptide. The amino acid sequence also determines the potential secondary and tertiary structure (i.e., the folding) of the polypeptide.

The folding of a polypeptide can also significantly impact APC processing. Both the first and second reasons given above for the uncertainty of the display by APCs of a specific epitope derived from a particular polypeptide can result from the way in which the polypeptide is folded. Proteolytic cleavage during processing within the APC can be influenced by the exposure or masking of a cleavage site due to folding. Entry of polypeptides into subcellular compartments is well known to be influenced by the 3D structure of the polypeptide, which structure is a function of folding.

### T Cells and Autoimmune Diseases

In autoimmune diseases, only a limited number of T cell clones, reactive with various epitopes of a small number of autoantigens, become activated and are involved in pathogenesis. Various mechanisms have been postulated to play a role in this pathogenic activation of disease-causing autoreactive T cells. Primary activation of antigen presenting cells (APCs) by infection or local inflammation is implicated in one such mechanism. APCs activated in this way can then provide powerful co-stimulation for hitherto unreactive T cells.

Other proposed mechanisms involve the polyclonal activation of previously quiescent autoreactive T cells by superantigens, such as bacterial toxins; or a coincidental molecular mimicry between foreign and self antigens (Abbas et. al. 1994). In this last case, the host immune system mounts a response to an epitope on a protein expressed by a pathogen, such as a virus, that resembles a homologous epitope on a host protein. Autoimmune attack then results from the cross-reactive immune response that ensues.

In addition to external factors, underlying the emergence of all T cell-mediated autoimmune disease is a complex pattern of inherited susceptibility determined by multigenic factors. For further discussions of these various factors, Steinman, 1995, reviews current theories of autoimmunity.

In several autoimmune diseases, including MS (as discussed in detail immediately below under "T Cells Target Defined Autoantigens in MS"), some or all of the autoantigens targeted by abnormal immune responses have been identified. Knowledge of these self antigens and the specific epitopes within these antigens that are targeted by autoreactive T cells in an autoimmune disorder such as MS provides an approach to therapy, as discussed in detail below under "treatment of MS by Administration of Antigens" and "therapeutic Induction of Apoptosis".

### T Cells Target Defined Autoantigens in MS

Although, as discussed above, the precise etiology of MS remains unknown, autoimmune attack is clearly responsible for the destruction of central nervous system (CNS) myelin that is the hallmark of the disease. Myelin is the characteristic component of the myelin sheath that surrounds the axons of certain neurons, acts as an electrical insulator, and is essential for the proper signal transmission functions of these neurons. The demyelination associated with MS thus causes a loss of function in affected neurons, disrupting neuronal signaling and leading to paralysis and severe impairment of sensory functions.

The myelin sheath is made by oligodendrocytes (in the central nervous system) and Schwann cells (in the peripheral nervous system). Myelin is composed of regularly alternating layers of lipids (e.g., cholesterol, phospholipids, and sphingolipids) and proteins.

The four major protein components of myelin, i.e., myelin basic protein (MBP), proteolipid protein (PLP), myelin associated glycoprotein (MAG) and myelin oligodendrocyte protein (MOG), are recognized by autoreactive T lymphocytes isolated from MS patients (Endoh et al. 1986; Martin et al. 1992; Kerlero de Rosbo et al. 1993; Amor et al. 1994; Johns et al. 1995).

Myelin basic protein (MBP) and proteolipid protein (PLP) are major protein components of myelin, comprising approximately 30% and 50% respectively of the total protein content of the myelin sheath. MBP and PLP have been shown to be major target autoantigens in MS, and T cells reactive with MBP and PLP play key roles in its pathogenesis (see, for example, Schwartz, 1993; Brown and McFarlin 1981. Lab Invest 45, pp. 278-284; Allegretta et al. 1990; Lehmann et al. 1992; Martin et al. 1992; Sprent 1994; Su and Sriram. 1991. J of Neuroimmunol 34, pp. 181-190; and Weimbs and Stoffel. 1992).

MBP-specific and PLP-specific T lymphocytes are found in the blood of MS patients. While they can sometimes be found in the blood of healthy individuals, they are typically present in the cerebrospinal fluid (CSF) of patients with MS. Significantly, such T cells are not found in CSF from healthy individuals (Kerlero de Rosbo et al. 1993; Zhang et al. 1994).

The immune responses of MS patients towards MBP and PLP clearly differ from those of healthy individuals. MBP and PLP reactive T cells are preferentially activated in MS patients, as demonstrated by the observation that the frequency of MBP-specific and PLP-specific T cells expressing markers of activation (e.g., IL-2 receptors) is elevated in MS patients (see, for example, Zhang, et al., 1994).

Gene mutation frequency analysis also provides evidence that MBP reactive T lymphocytes are specifically activated in MS patients. Since gene mutation is more frequent in dividing than in resting T cells, an increased mutation frequency in. T cells of a particular specificity provides an indication of the specific activation of those cells *in vivo* (Allegretta et al. 1990).

T lymphocytes from MS patients were cultured in thioguanine to test the frequency of mutations in the hprt gene that would render them resistant to this purine analogue. A high frequency of thioguanine resistant T cell clones, up to 10 times the frequency of T cells from normal individuals, was found in MS patients, and a significant percentage of these mutant clones proliferated in response to brain MBP, although they had never been intentionally exposed to this antigen. In contrast, no resistant clones obtained from normal subjects recognized MBP.

MBP, PLP, and MOG are also considered to be primary autoantigens in MS because of their ability to induce experimental allergic encephalomyelitis (EAE) in animals. EAE is an experimentally induced condition that closely resembles MS and is the benchmark animal model of MS. In addition, transfer of T cells from an individual suffering from EAE (or MS) to a healthy animal can produce EAE in the recipient, a method of disease induction referred to as "adoptive transfer". For example, in a human to animal transfer study, CSF mononuclear cells (including T cells) from MS patients caused paralysis, ataxia, and inflammatory brain lesions when injected into the CSF in the brain ventricles of severe combined immunodeficiency (SCID) mice (Saeki et al. 1992). Also, immunization of animals with MBP and/or PLP and/or MOG can elicit the CNS inflammation, paralysis, and other signs and symptoms of EAE (see, for example, Alvord et al. 1984; Abbas et al. 1994; Amor et al. 1994; and Johns et al. 1995).

Although it is clear that MBP, PLP, and MOG are primary antigens targeted by the abnormal immune response in MS, studies have revealed a marked heterogeneity of MBP and PLP epitopes that can induce T cell proliferative responses. These studies have not consistently revealed a single epitope that is recognized with higher frequency by reactive T cells of MS patients than those of normal healthy individuals (Chou et al. 1989; Richert et al. 1989; Martin et al. 1990; Ota et al. 1990; Pette et al. 1990; Martin et al. 1992; Meinl et al. 1993). This heterogeneity in antigen targeting may, in part, be a function of the variety of the MHC molecules and TCRs expressed by different individuals in a human population.

Different molecular forms (isoforms) of MBP are generated by differential splicing of MBP hnRNAs, resulting in the presence in the encoded protein of some or all of the seven exons of the single MBP gene. In healthy adults, MBP is found almost exclusively as an 18.5 kDa molecule which is produced from an mRNA comprising all exons of the MBP gene except exon 2 (Kamholtz et al. 1988). Other forms of MBP include a full length (all 7 exons) 21.5 kDa isoform, and two other minor isoforms (17.2 and 20.2 kDa). The expression of the two exon 2 containing isoforms (21.5 kDa and 20.2 kDa) appears to increase with myelin formation, during both early fetal development and remyelination of damaged tissue (Kamholtz et al. 1988; Roth et al. 1987). These two isoforms are referred to in the art, and herein, as "fetal" isoforms, although they are also found in remyelinating damaged adult tissue.

MS plaques contain areas of remyelination and thus should contain higher levels of the 21.5 isoform of MBP than found in healthy adult CNS tissue, suggesting that an immune response to an epitope within the common 26 amino acid region (corresponding to the sequence spanning amino acid residue 60 to amino acid residue 85 of SEQ ID NO:1) of each of the two fetal isoforms of MBP coded for by exon 2 (which regions are referred to as "X2MBP" or simply "X2") could exacerbate the clinical course of established disease (Prineas et al. 1993; Raine and Wu, 1993; Bruck et al. 1994).

Since remyelination may occur cyclically in the course of MS, each cycle of remyelination could theoretically serve to drive an ongoing immune response by activating resting X2MBP specific T cells in the CNS. Supporting this hypothesis, several lines of evidence suggest the involvement of an epitope encoded by exon 2 of the MBP gene (i.e., an epitope within X2MBP) in MS pathogenesis.

Studies of the role of alternate isoforms of MBP in MS require the availability of quantities of purified myelin antigens in order to evaluate their immunological properties. Such studies have therefore generally been limited to utilizing synthetically-derived peptides, e.g., peptides comprising X2MBP. Recently, CD4⁺ MHC class II-restricted T cells reactive with peptides containing exon 2 encoded sequences of human MBP were isolated from peripheral blood of both MS patients and normal healthy controls (Voskuhl et al., 1993a; Voskuhl et al. 1994). In a family afflicted with MS, the frequency of T lymphocytes specific for an X2 comprising peptide was higher than the frequency of T cells specific for epitopes within the 18.5 kDa isoform of MBP that does not contain X2 (Voskuhl et al., 1993b). In addition to this data from human subjects, a murine X2 comprising peptide was recently found to be immunogenic in SJL/J mice, and severe EAE was induced by adoptive transfer of exon 2 peptide-sensitized lymphocytes (Segal et al., 1994; Fritz and Zhao, 1994).

Taken together, these human and animal findings demonstrate that an in *vivo* cellular immune response to the myelin derived antigen MBP causes at least some of the pathogenesis associated with multiple sclerosis. It should be noted, however, that all of the studies regarding X2 epitopes used synthetic peptides as antigens and none of them used full length MBP 21.5 protein. In light of the uncertainty regarding processing and display of particular epitopes of untested proteins by APCs, it has been questioned in the art whether these results are truly relevant to in *vivo* MS pathogenesis.

PLP is a highly hydrophobic integral myelin membrane protein whose physical and chemical properties render it difficult to isolate, study, or administer to a patient (see, for example, Sobel et al. 1994; Tuohy 1994; Van der Venn et al. 1989; Van der Venn et al. 1990; Van der Venn et al. 1992; van Noort et al. 1994). The primary amino acid sequence of PLP is highly conserved between species. Typically, the mature PLP polypeptide does not include the initiator methionine coded for by the PLP gene; this amino acid appears to be removed in mammalian cells by a post-translational processing event. Accordingly, as used herein, the amino acid numbering of human PLP is that shown in SEQ ID NO:22, and is numbered starting with a glycine residue as amino acid number 1.

The 276 amino acid PLP polypeptide contains approximately 50% hydrophobic residues and is described as being structured into five hydrophilic domains and four extremely hydrophobic domains, which are numbered one to four starting at the amino terminus of the protein. Protein domains may be defined as having different extents, depending upon the criteria used to define the domain boundaries. Thus, by the most stringent criteria, the hydrophobic domains of the human PLP molecule span amino acid residues 10 to 36 (hydrophobic domain 1), 59 to 87 (hydrophobic domain 2), 151 to 178 (hydrophobic domain 3), and 238 to 267 (hydrophobic domain 4) of the amino acid sequence of human PLP (SEQ ID NO: 22). Less stringent criteria are also used to define these domains, so that the hydrophobic domains may alternatively be said to span amino acid residues 10 to 18 (hydrophobic domain 1), 70 to 80 (hydrophobic domain 2), 162 to 170 (hydrophobic domain 3), and 250 to 258 (hydrophobic domain 4) of the amino acid sequence of human PLP (SEQ ID NO: 22).

Accordingly, the hydrophilic domains of PLP may be defined as amino acid residues 1 to 9 (hydrophilic domain 1), 37 to 58 (hydrophilic domain 2), 88 to 150 (hydrophilic domain 3), 179 to 237 (hydrophilic domain 4), and 267 to 276 (hydrophilic domain 5) of the amino acid sequence of human PLP (SEQ ID NO: 22).

PLP-reactive T cell lines react strongly to PLP peptides. Synthetic peptides with sequences based on the PLP sequence have been used to identify murine and human encephalitogenic epitopes. See, for example, Fritz et al. 1983. J Immunol 130, pp. 191-194; Endoh et al. 1986; Greer et al. 1992; Kuchroo et al. 1992; Kuchroo et al. 1994; McRae et al. 1992; Pelfrey et al. 1993; Pelfrey et al. 1994; Sobel et al. 1992; Tuohy et al. 1988; Tuohy et al. 1989; Tuohy et al. 1992; Whitham et al. 1991. J Immunol 147, pp. 101-107; Whitham et al. 1991. J Immunol 147, pp. 3803-3808; and Correale et al. 1995. The human peptide-defined epitopes are shown in table 1.

In accordance with a recently proposed structure of PLP (Weimbs and Stoffel. 1992), these encephalitogenic epitopes are found in the both intramembrane and extramembrane domains of PLP.

PLP peptides have been shown to be encephalitogenic, and can induce disease in rabbits, rats, guinea pigs, and a variety of mouse strains (see, for example, Trotter et al. 1987). Murine PLP is identical, in sequence to human PLP (SEQ ID NO:22). Encephalitogenic epitopes in mouse models include those shown in Table 2. In at least some mouse strains, PLP represents the major encephalitogen within the CNS (Kennedy et al. 1990). In various rodent models, significantly more demyelination was observed with PLP-induced EAE compared to MBP-induced disease (Tabira 1988) . In clinical studies, significant differences in the number of PLP-peptide-reactive T cells in MS patients versus normal healthy control individuals have been reported (Sun et al. 1991; Trotter et al. 1991; Chou et al. 1992; Zhang et al. 1994).

In addition to these observations, the importance of PLP in MS pathogenesis is suggested by the observation that PLP, unlike MBP, is found solely in the CNS and not in the peripheral nervous system, where relatively little damage occurs in MS (Lees and Mackin. 1988).

### Treatment of MS by Administration of Antigens

The ideal therapeutic treatment for any disease is one that specifically blocks pathogenesis without affecting normal physiology. In the case of autoimmune diseases, an approach to such ideal therapy is a treatment that specifically induces immune tolerance to autoimmune disease-associated self antigens without affecting immune responses to foreign antigens. New therapeutic agents and treatment strategies are being sought that will allow the induction of tolerance to specific autoantigens, while leaving all other aspects of immune function unaltered.

Attempts have been made to therapeutically modify T cell responses via the administration of antigens to suppress specific autoreactive lymphocytes, especially T cells, and thereby elicit tolerance to disease-associated autoantigens. A distinct advantage of such antigen-specific therapy is that it can achieve the therapeutic modulation of the activities of only those T cells that, by reacting with the self antigens, are responsible for the development of pathology. This specificity provides therapeutic benefits without altering the important immune activities of T cells reactive with other antigens.

MS antigens have been studied as tolerization inducers for the treatment of MS/EAE, since therapies that suppress autoreactive T cells may significantly alleviate nervous tissue demyelination and resulting symptoms (see, for example, Adronni et al. 1993; Critchfield et al. 1994; Miller and Karpus 1994; Racke et al. 1995). A number of treatment protocols and antigens have been used in these studies, with animal rather than human forms of the antigens predominantly being used. For example, Weiner et al. 1993 used MBP purified from bovine myelin and Miller et al. 1992 used guinea pig, rat, and mouse MBPs. In studies using human MBP antigen, MBP was purified from cadaveric human brain (See, for example, Zhang, et al. 1994).

Oral tolerance involves regulatory CD8⁺ T cells that suppress immune responses both *in vitro* and *in vivo* through the secretion of cytokines, including TGF-beta (Chen et al. 1994 Science 265:1237-1240). The down-regulation of the activity of T cells mediated by this mechanism is not specific to particular T cell clones, and does not involve antigen-specific immunosuppression, but acts on any T cells in close enough proximity to the suppressive T cells to be affected by their secreted cytokines. This phenomenon has been termed "bystander suppression".

Recent studies have investigated the tolerizing effects of oral administration of bovine myelin to MS patients (Weiner et al. 1993 Science 259:1321-1324; Yoon et al. 1993). While fewer of the patients treated with oral myelin developed exacerbations of their MS symptoms than the patients treated with placebo, the results of the study were inconclusive, as the patients were not properly randomized. In fact, the authors cautioned that "It must be strongly emphasized that this study does not demonstrate efficacy of oral myelin in the treatment of MS." Thus, while oral tolerization studies support the usefulness of myelin proteins as immunomodulatory agents for the treatment of MS, new, more effective antigens, and alternative modes of administration of such antigens for the immunomodulatory treatment of MS continue to be sought.

Clearly, for the treatment of human disease, human-derived antigens have advantages over animal-derived antigens, as they are the actual autoantigens targeted for autoimmune attack in human disease, and suppression of disease should be most effective when homologous protein is administered (Miller et al. 1992). This is because the human protein will have the same MHC binding specificity and be subject to the same antigen processing as the endogenous protein targeted by the autoimmune response.

In fact, it is known that immunodominant epitopes (i.e. the antigenic regions of the protein most often recognized by CD4⁺ autoreactive T cells) of important MS autoantigens differ depending on the species from which the antigen is derived, even though many myelin antigens exhibit high interspecies homology at the amino acid sequence level. For example, as determined by analysis of T cells obtained from MS patients, an immunodominant epitope of human MBP is contained with the region spanning amino acids 84-102 and another is found in the region spanning amino acids 143-168. In contrast, a major immunodominant eptiope of murine MBP is found in the region spanning amino acids 1-9 (Zamvil et al. Nature 324:258, 1986) and a major immunodominant epitope of rat MBP is found in the region spanning amino acids 68-88 (Burns, et al. J. Ex. Med. 169:27, 1989).

The use of antigens isolated from human CNS tissue as therapeutic agents is, however, undesirable. This is due not only to problems associated with purifying antigens from CNS tissue generally and the difficulty of obtaining human raw materials, but, more importantly, to the problem of eliminating the possibility of pathogenic contamination. One example of potential contaminants in the purification of CNS-derived proteins are prion particles that transmit the spongiform encephalopathies Creutzfeldt-Jakob disease and kuru. The prion particles present a particularly intractable problem because they are resistant to any known means of sterilization that will not also destroy the proteins being purified.

A useful approach to obtaining human antigens that avoids these problems is the production of protein antigens using recombinant DNA technology, typically by preparing DNA molecules encoding the antigens and using the DNAs to drive expression of the antigens in non-human host cells. Oettinger et al. (1993) have prepared a recombinant DNA molecule comprising unmodified human sequences encoding the 18.5 kDa form of human MBP and used this DNA to express recombinant human 18.5 kDa MBP in *Escherichia coli*. The expression of PLP polypeptides in *E coli*, however, has proven an intractable problem until now, as at least some PLP sequences appear to have toxic effects upon bacteria.

In fact, the hydrophobicity of PLP severely limits aqueous solubility (Tuohy 1994), rendering native PLP from any source difficult to prepare and to administer intravenously.

### T Cell Deletion

Alterations in the T cell repertoire occur naturally during T cell development. Only a small fraction of thymocytes (immature T cells) survive the development and selection events in the thymus that result in emigration of developing T cells to the peripheral circulation where they complete their maturation (von Boehmer, 1988; Marrack and Kappler, 1987). Experimental evidence strongly suggests that a large number of thymocytes that bear receptors for autoantigens are initially present in the thymus. During T cell development in the thymus, those cells reactive with self antigens are deleted (killed) as part of the normal developmental pathway. This intrathymic tolerization process is referred to as "thymic tolerance".

Developing T cells do not encounter certain autoantigens in the thymus, but may encounter them as mature peripheral T cells. For example, it may be that neural antigens are never presented in the thymus. Tolerance to such autoantigens is normally produced outside the thymus, and is referred to as "Peripheral tolerance". Peripheral tolerance can occur by at least two mechanisms, one of which is a similar but distinct process to thymic tolerization that results in the deletion of those mature peripheral T cells that are specifically reactive with a previously unencountered autoantigen. In addition, T cells with certain specific reactivities can be induced to become inactive (anergic). Peripheral deletion and the induction of anergy are physiologic mechanisms that result in the development of "peripheral tolerance". As a result of thymic and peripheral tolerization, mature T cells are normally tolerant to most autoantigens, however, autoreactive T cells may persist because their antigen is not presented with the required costimulation or is found in an immunologically privileged site.

The mechanism by which tolerization via T cell deletion is generated has recently been shown to depend upon repeated exposure to an antigen under certain defined conditions. Specific T cell deletion can therefore be induced by the appropriate administration of exogenous compounds comprising the relevant epitopes. As only a limited number of autoantigens (notably comprising a much greater number of epitopes) are involved in the pathogenesis of any individual autoimmune disease, it is possible, when they are known, to administer the self epitopes targeted in a disease to sufferers in the form of one or more isolated autoantigen-derived compounds containing the epitopes involved in pathogenesis. To have an optimal clinical effect, it may be necessary to have a comprehensive mixture of MBP and PLP epitopes, perhaps together with MOG epitopes, because of the large degree of human MHC and TCR polymorphism, and because new epitope reactivities may appear during autoimmune disease progression (McCarron et al. 1990; Lehmann et al. 1992; Kaufman et al. 1993).

### Apoptosis

The deletion of autoreactive T cells is an example of programmed cell death, which represents an important process in the regulation of many biological systems (Singer et al. 1994). Programmed cell death occurs by a mechanism referred to as apoptosis, in which cells respond to certain stimuli by undergoing a specific sequence of predetermined events that effectively constitute cellular suicide. Apoptosis clearly plays a large role in shaping and maintaining the T cell repertoire and contributes to the establishment of self-tolerance by actively eliminating cells expressing autoreactive TCRs.

It has recently been discovered that T cells are sensitive to apoptotic cell death induced by a variety of stimuli at multiple points in their lifespan (see, for example, Lenardo 1991; Boehme and Lenardo 1993; Critchfield et al. 1994). Positive selection factors are also believed to play a role in regulating the survival of specific T cell clones. The reduction or expansion of the number of individual T cells of a particular clone in an organism by these and other mechanisms serve to modulate the responsiveness of the organism's immune system to a particular antigen. It is now firmly established in several autoimmune disease models, as well as in certain viral infections, that apoptosis can be induced (upon exposure to antigen under certain defined conditions) in mature peripheral antigen-specific T lymphocytes as well as in immature thymocytes.

Apoptosis occurs in many biological systems (see, for example, Kerr et al. 1991; Lockshin and Zakeri, 1991; Cohen et al. 1992; Duvall and Wyllie, 1986; Cotter et al. 1990). A cell undergoing apoptosis undergoes a specific program of eventscellular and biochemical processes that depend upon active metabolism and contribute to the cell's self-destruction. In apoptotic T cells, the nucleus shrinks, the chromatin condenses, the genetic material (DNA) progressively degrades into small (nucleosomal repeat sized) fragments, there is cytoplasmic compaction, the cell membrane forms blebs, and the cell eventually collapses (Kawabe and Ochi, 1991; Smith et al. 1989). Cells cannot recover from apoptosis, it results in irreversible cell death (Kawabe and Ochi, 1991; Smith et al. 1989).

Recent reports have indicated a role for the TNF-related cytokine known as the FAS ligand and its receptor, CD95 (the FAS receptor), in the induction of apoptosis in T cells (Crispe et al. 1994; Nagata and Suda, 1995; Strasser, 1995; Dhein et al., 1995; Brunner et al., 1995; and Ju et al., 1995).

T cells that do not undergo apoptosis, but which have become activated, will carry out their "effector" functions by causing cytolysis, or by secreting lymphokines that cause B cell responses or other immune effects (Paul, 1989). These effector functions are the cause of tissue damage in autoimmune and other diseases.

### Therapeutic Induction of Apoptosis

A powerful approach to avoiding or treating autoimmune diseases is to permanently eliminate lymphocytes involved in the autoimmune response by apoptosis. For example, a therapeutic effect can be achieved by eliminating only those T cells reactive with autoantigens targeted in the particular autoimmune disease being treated, while leaving the vast majority of the T cell repertoire intact. *In vivo* studies have demonstrated that EAE can be treated by administration of myelin antigens at a dose and interval effective to induce apoptosis of T cells reactive with the antigens (se, for example, Critchfield et al. 1994).

This approach is described in co-pending U.S. patent application No. 07/751,090, filed in the name of Michael J. Lenardo, and entitled Interleukin-2 Stimulated T Lymphocyte Cell Death for the Treatment of Autoimmune Diseases, Allergic Disorders, and Graft Rejection and co-pending U.S. patent application No. 07/926,290, filed in the name of Michael J. Lenardo, and entitled Interleukin-4 Stimulated T Lymphocyte Cell Death for the Treatment of Autoimmune Diseases, Allergic Disorders, and Graft Rejection.

The accompanying figures, which are incorporated in and constitute part of the specification, illustrate certain aspects of the invention, and together with the description, serve to explain the principles of the invention. It is to be understood, of course, that both the figures and the description are explanatory only and are not restrictive of the invention.

### Brief Description of the Drawings

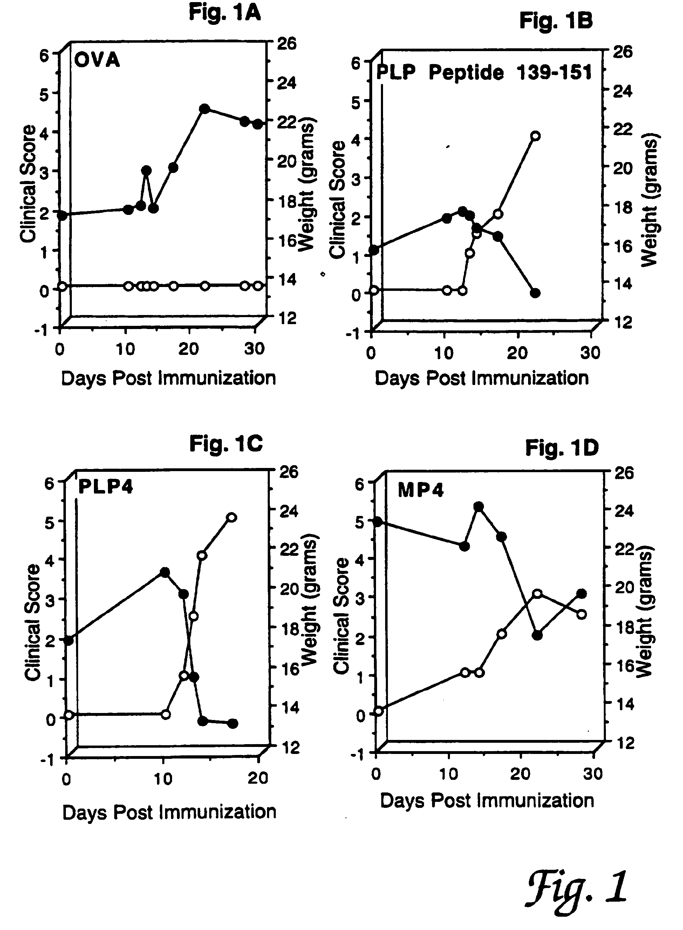

Fig. 1. Clinical course of active (antigen-induced) EAE in four individual SJL/J mice treated with ovalbumin (Fig. 1A - OVA), PLP peptide 139-151 (Fig. 1B - a peptide with an amino acid sequence corresponding to amino acid residues 139-151 of SEQ IS NO:22), ΔPLP4 (Fig. 1C - PLP4), or MP4 (Fig. 1D) - which are administered in CFA adjuvant. Disease was graded 0, no abnormality; 1, limp tail; 2, limp tail with inability to right upon being turned over; 3, hind limb weakness or dragging one hind limb; 4, paralysis of both hind limbs; 5, moribund; and 6, death. Clinical score -- open circles; weight in grams -- closed circles.

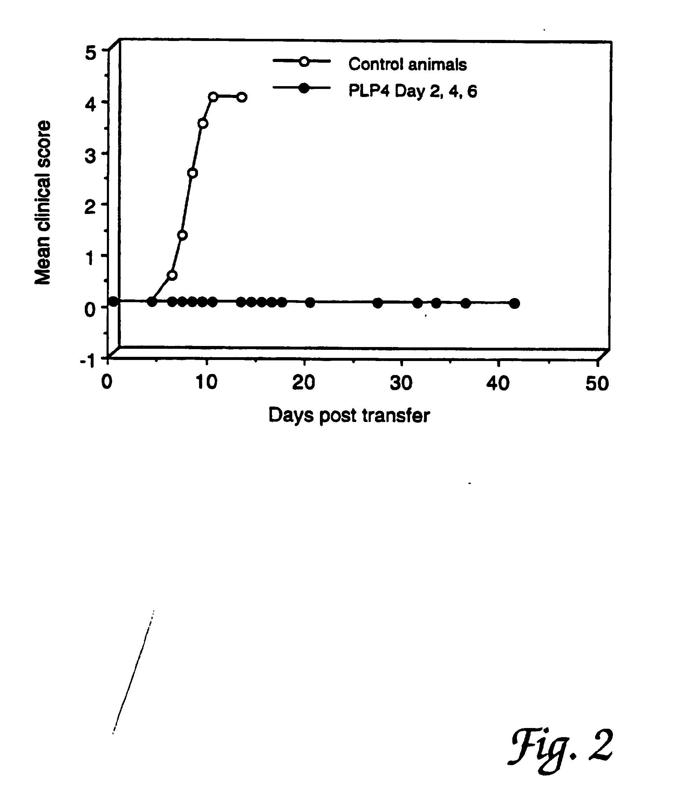

Fig. 2. Prevention / treatment of adoptive EAE by intravenous APLP4 administration. PLP-specific lymph node cells from ΔPLP4/CFA immunized mice were stimulated *in vitro* with PLP peptide 139-151 (described above for Fig. 1). T cells from these animals were transferred by intravenous injection into naive recipients at 10⁷ cells/mouse on day 0. The five mice in the treated group (PLP4 Day 2, 4, 6) received two intravenous injections (separated by 6-8 h) of 125 µg of ΔPLP4 on days 2, 4, and 6 post transfer. The five untreated mice (Control animals) received an equal volume (100 µl) of sterile water. Mice were monitored daily and a mean clinical score for each group was determined (scored as in Fig. 1).

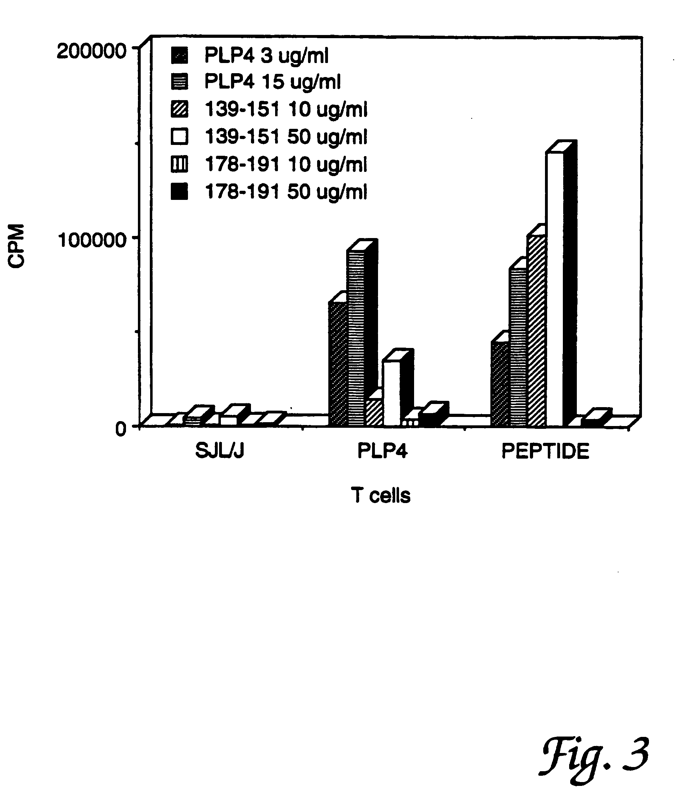

Fig. 3 Proliferative responses of T cell enriched lymph node cells from, as indicated on the x axis, naive mice (SJL/J) and mice immunized with PLP peptide 139-151, described above for Fig. 1, (PEPTIDE) or ΔPLP4 (PLP4) in response to *in vitro* stimulation with synthetic PLP peptides (139-151 or 178-191 -- a peptide with an amino acid sequence corresponding to amino acid residues 178-191 of SEQ ID NO:22) or intact ΔPLP4 (PLP4) at the concentrations indicated. T cells were incubated for 72 h in media alone or with antigens. Proliferation was measured by ³H-thymidine incorporation following an 18 h pulse. All assays were replicated with triplicate cultures.

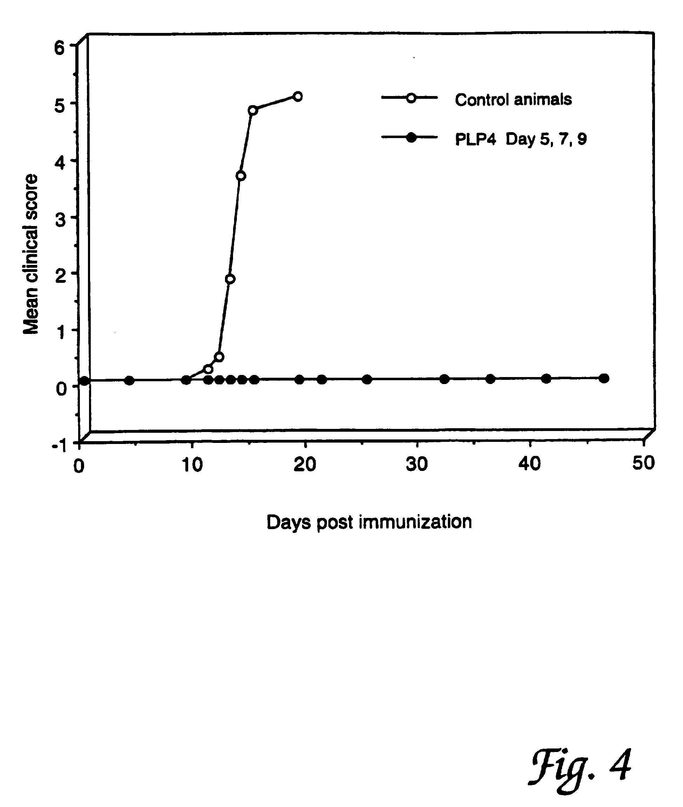

Fig. 4 Prevention / treatment of ΔPLP4-induced active EAE by intravenous ΔPLP4 administration. EAE was induced in SJL/J mice by subcutaneous injection on days 0 and 3 with 100 µg of ΔPLP4 in CFA followed by 300 ng of pertussis toxin. The five experimental mice received two intravenous injections (separated by 6-8 h) of 125 µg of ΔPLP4 on days 5, 7, and 9 post-immunization (PLP4 Day 5, 7, 9, closed circles). The five untreated mice (Control animals, open circles) were given an equal volume (100 µl) of sterile water on the same schedule. Mice were monitored daily and a mean clinical score (determined as in Fig. 1) was assigned for each group.

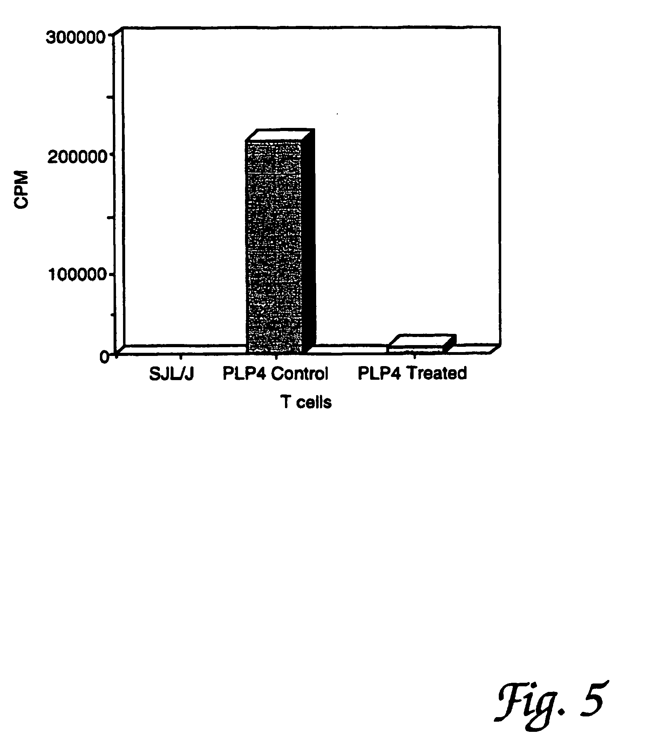

Fig. 5 PLP treatment eliminates T cell proliferation in response to PLP and MBP antigens. T cell proliferation assays were performed on lymph node cells obtained from mice immunized to induce EAE and treated with APLP4. Induction and treatment were as described for Fig. 4. Antigens used in the in vitro T cell proliferation assays at 50 ug/ml were ΔPLP4 (PLP4) or MP4, as indicated.

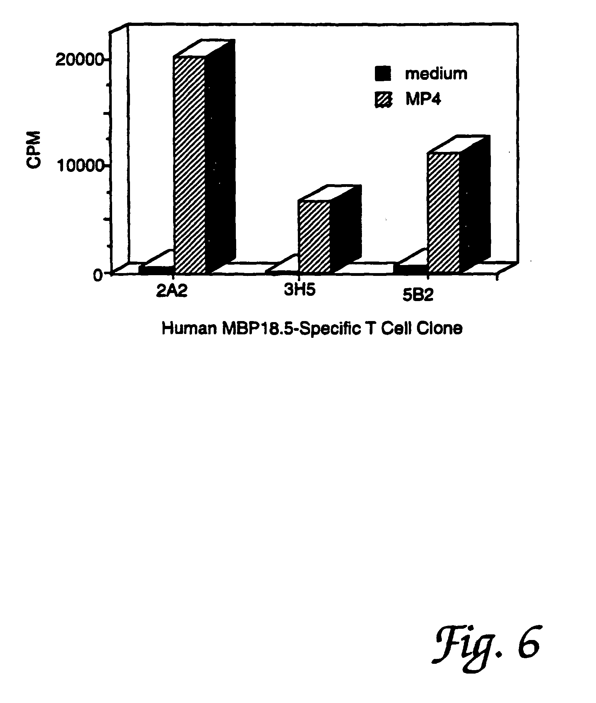

Fig. 6 Proliferation of human MBP-specific T cell lines in response to stimulation with recombinant MP4. MP4 was used at a concentration of 10 µg/ml. Human T cell lines 2A2 (reactive with MBP peptide 31-50), 3H5 (reactive with MBP peptide 87-106) and 5B2 (reactive with MBP peptide 151-170) were initially obtained from a healthy individual. These cell lines are specific for MBP epitopes indicated by the corresponding amino acid positions of adult human brain (18.5 kDa) MBP (SEQ ID NO:4) displayed in parentheses.

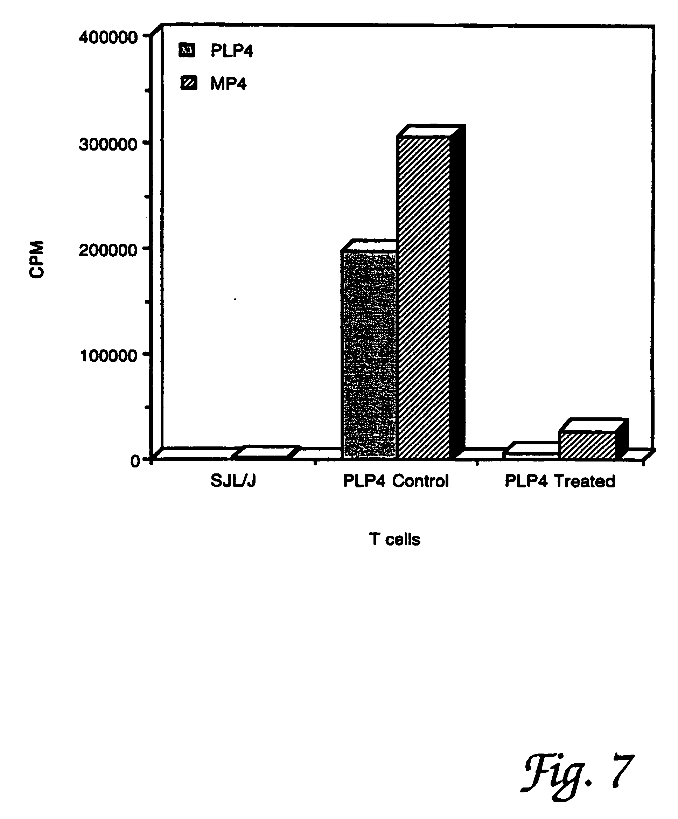

Fig. 7 MP4 stimulates murine T-cells after disease induction with PLP and PLP treatment eliminates T cell proliferation in response to MP4 antigens. T cell proliferation assays were performed on lymph node cells obtained from mice immunized with ΔPLP4 to induce EAE and treated with ΔPLP4. MP4 was used at 50 µg/ml in the *in vitro* T cell proliferation assays.

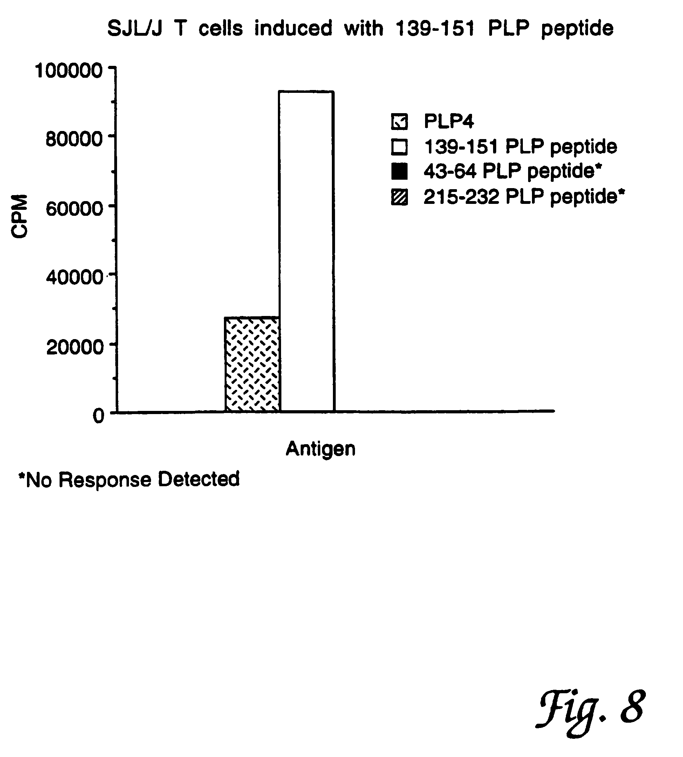

Fig. 8 Proliferative responses of T cell enriched lymph node cells from SJL/J mice immunized with PLP peptide 139-151 (described above for Fig. 1) in response to *in vitro* stimulation with synthetic PLP peptides (139-151, 43-64 -- a peptide with an amino acid sequence corresponding to amino acid residues 43-64 of SEQ ID NO:22, or 215-232 -- a peptide with an amino acid sequence corresponding to amino acid residues 215-232 of SEQ ID NO:22) or intact ΔPLP4 (PLP4) at 10µg/ml. T cells were incubated for 72 h in media alone or with antigens. Proliferation was measured by ³H-thymidine incorporation following an 18 h pulse. All assays were replicated with triplicate cultures.

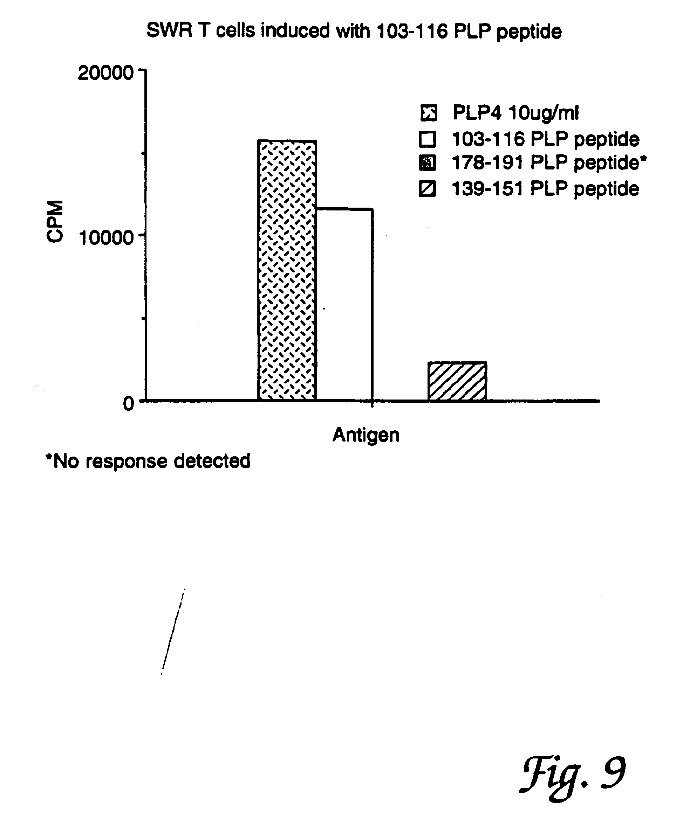

Fig. 9 Proliferative responses of T cell enriched lymph node cells from SWR mice immunized with PLP peptide 103-116 in response to *in vitro* stimulation with synthetic PLP peptides (178-191 -- discussed above, 139-151 -- discussed above, or 103-116 -- a peptide with an amino acid sequence corresponding to amino acid residues 103-116 of SEQ ID NO:22) or intact ΔPLP4 (PLP4) at 10µg/ml. T cells were incubated for 72 h in media alone or with antigens. Proliferation was measured by ³H-thymidine incorporation following an 18 h pulse. All assays were replicated with triplicate cultures.

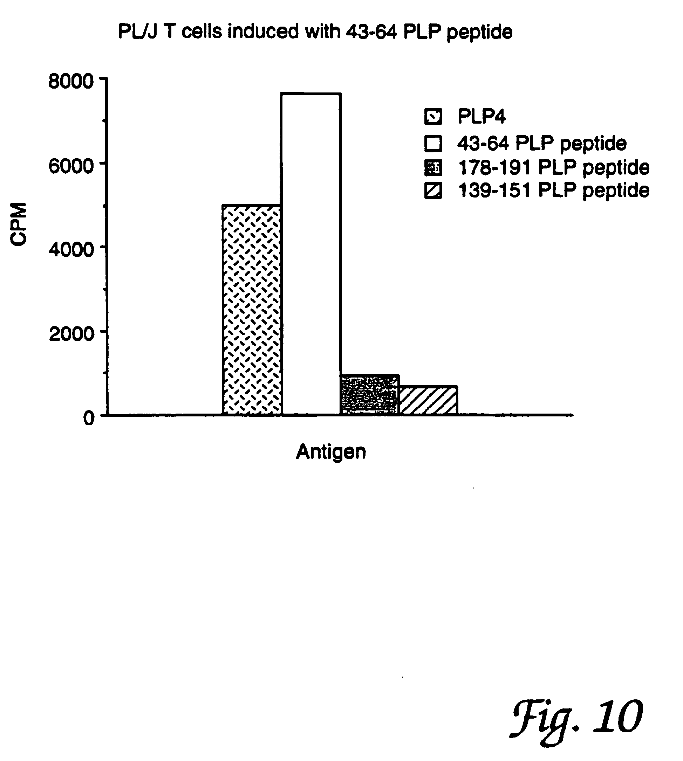

Fig. 10 Proliferative responses of T cell enriched lymph node cells from PL/J mice immunized with PLP peptide 43-64 in response to *in vitro* stimulation with synthetic PLP peptides (139-151, 43-64, or 178-191), discussed above, or intact ΔPLP4 (PLP4) at 10µg/ml. T cells were incubated for 72 h in media alone or with antigens. Proliferation was measured by ³H-thymidine incorporation following an 18 h pulse. All assays were replicated with triplicate cultures.

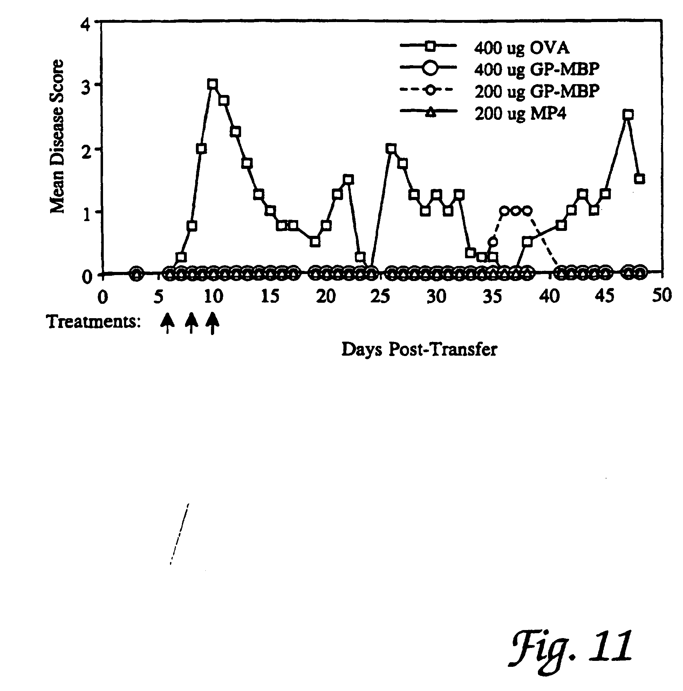

Fig. 11 Treatment of EAE induced by the transfer of 30,000,000 T cells that were activated with guinea pig MBP. Treatments were: 200µg MP4; 200µg guinea pig MBP (GP-MBP); 400µg guinea pig MBP; or 400µg ovalbumin (OVA, control); as indicated. These treatments were administered twice daily (at 6 hour intervals) i.v. on days 6, 8, and 10 after adoptive transfer of encephalitogenic T cells. Each treatment group consisted of 3 to 5 animals.

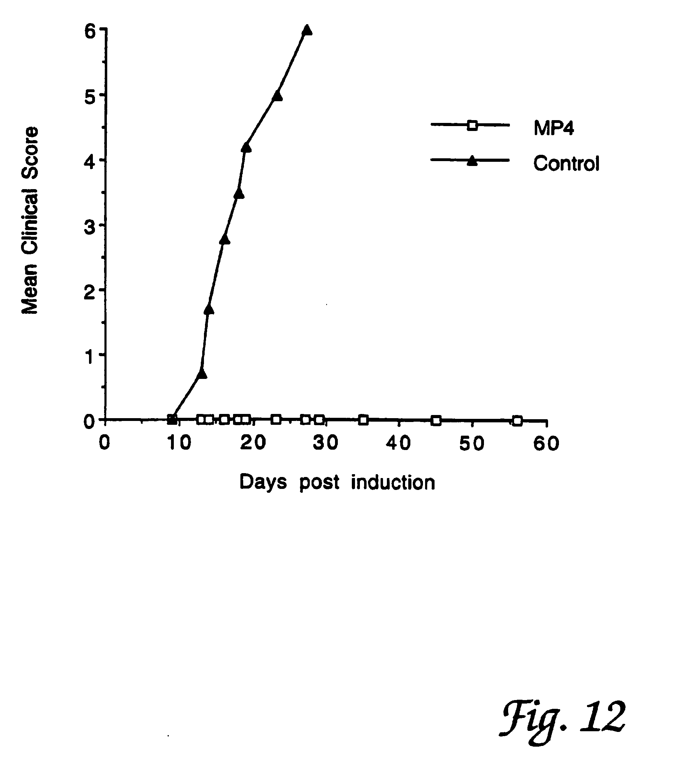

Fig. 12 Treatment of EAE induced by immunization of SJL mice with 100µg PLP peptide 139-151, discussed above for Fig. 1. Treatments were with 250µg MP4 or 250µg pigeon cytochrome c (control); as indicated. These treatments were administered twice daily (at 6 hour intervals) i.v. on days 5,7, and 9 after immunization. Each treatment group consisted of 3 animals.

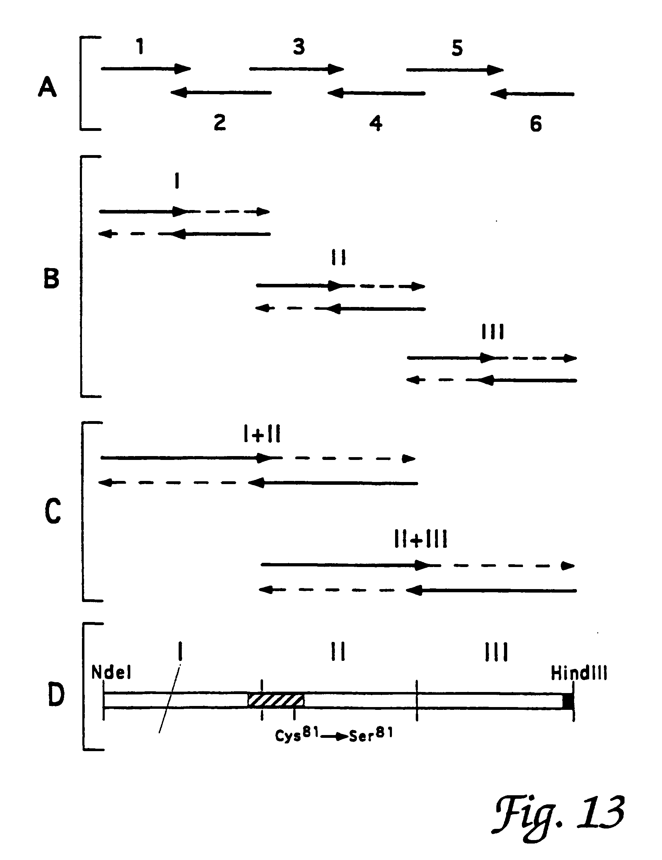

Fig. 13. PCR strategy for construction of a synthetic MBP21.5 gene (cDNA). Indicated by bracket A is the alignment of overlapping oligonucleotides 1 through 6 (SEQ ID NO:5, SEQ ID NO:6, SEQ ID NO:7, SEQ ID NO:8, SEQ ID NO:9, and SEQ ID NO:10) that were used to construct the MBP+X2^{Cys81/Bact}. gene. Three subdomains of the gene (I, II, and III as shown by the diagram indicated by bracket B) were initially synthesized. Larger domains (I+II, II+III) were formed by overlapping PCR using the appropriate outside oligonucleotides (oligonucleotides 1 and 4, and oligonucleotides 3 and 6, respectively) as shown by the diagram indicated by bracket C. The full-length molecule was completed by overlapping-PCR of domains I+II and II+III using outside oligonucleotides 1 and 6. A map of the final product is shown by the diagram indicated by bracket D. In this diagram, the hatched region in this map of the full-length molecule depicts the location of exon 2, with the cysteine at amino acid residue 81 (Cys⁸¹) shown as altered to serine (Ser⁸¹). The dark box at the 3' end of the gene (right hand side of the diagram) illustrates the addition of sequences encoding the histidine tag that was added to facilitate purification.

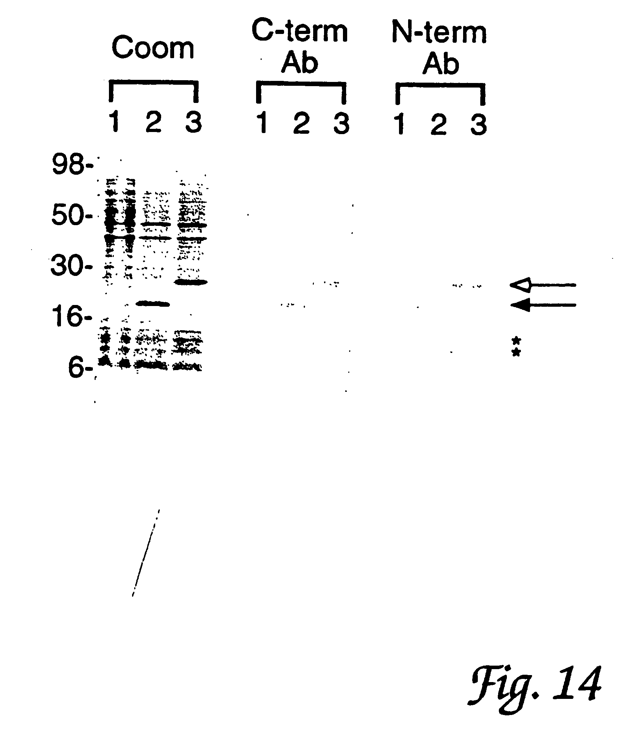

Fig. 14. Recombinant MBP expression and subcellular localization in bacterial cells -- unfractionated whole cell lysates. Cell lysates were prepared from induced cultures of BL21 (DE3) cells that were transformed with control pET22b vector without added insert ("1"), pET22b/MBP18.5^{hum.} ("2") or pET22b/MBP+X2^{Cys81/Bact.} ("3"). Whole cell lysates were separated by 16% SDS-PAGE under reducing conditions (note that under these conditions, no dimers are seen), then Coomassie stained (Coom) or immunoblotted with monoclonal antibodies that recognize either a carboxy-terminal epitope ("C-term Ab") or an amino-terminal epitope ("N-term Ab") of human brain MBP. Asterisks highlight the position of two fragments of MBP+X2^{Cys81} that are recognized by only the "N-term Ab" mAb. Molecular weights in kilodaltons (as determined by electrophoreising marker proteins) appear on the left. The open and closed arrows mark the positions of MBP+X2^{Cys81} and MBP18.5, respectively.

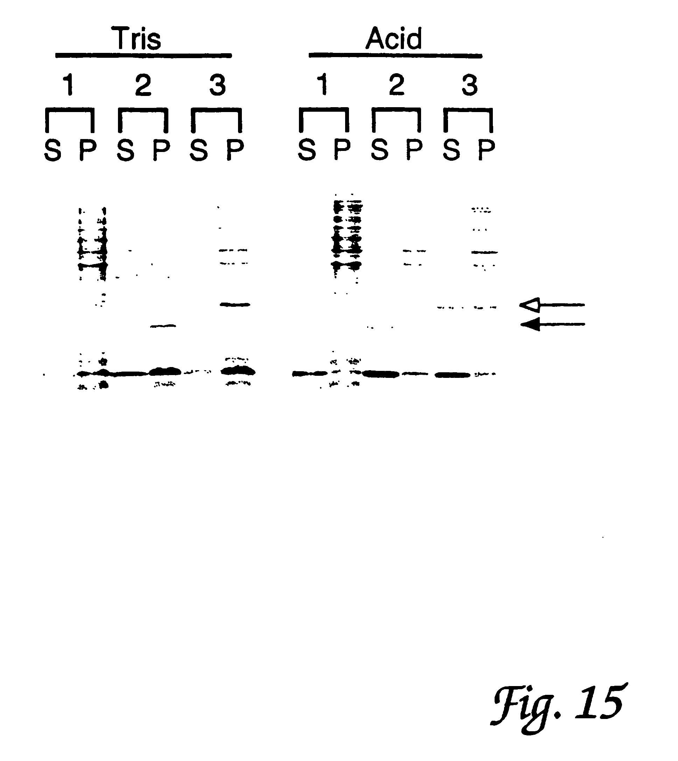

Fig. 15. Recombinant MBP expression and subcellular localization in bacterial cells - soluble vs. insoluble fractions. Cell lysates were prepared from induced cultures of BL21 (DE3) cells that were transformed with control pET22b vector without insert ("1"), pET22b/MBP18.5^{hum.} ("2") or pET22b/MBP+X2^{Cys81/Bact.} ("3"). Bacterial lysates were fractionated into soluble ("S") or insoluble pellet ("P") fractions using either neutral buffer ("Tris") or 0.1N HCl ("Acid") conditions as described above. Shown are the Coomassie stained gels obtained by SDS-PAGE of the cell fractions under reducing conditions (note that under these conditions, no dimers are seen). The open and closed arrows mark the positions of MBP+X2^{Cys81} and MBP18.5, respectively. Note that the acid extraction (but not the neutral extraction) allowed recovery of the MBP+X2^{Cys81} and the MBP18.5 polypeptides in the soluble fractions.

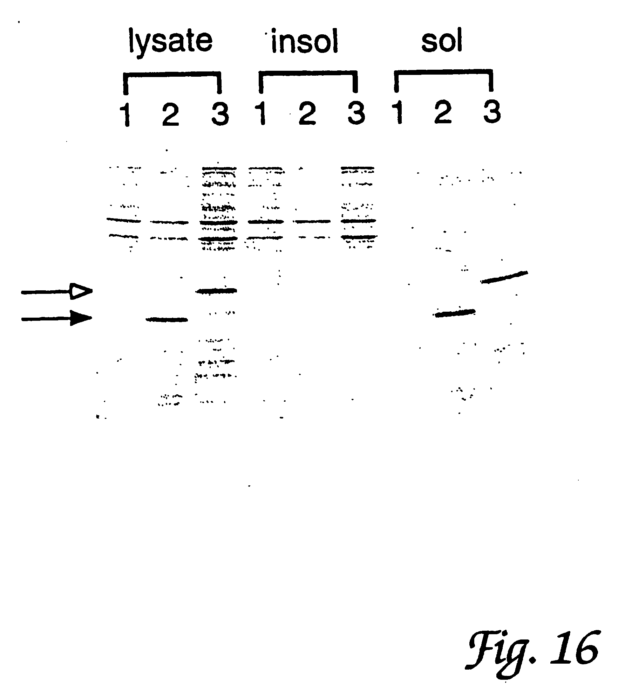

Fig. 16. Large scale acid extraction of recombinant MBP from bacterial cells. Shown is a Coomassie stained SDS/PAGE gel carried out under reducing conditions (note that under these conditions, no dimers are seen). Each group of three lanes shows whole cell lysate ("lysate") and insoluble ("insol") and soluble ("sol") fractions obtained from simultaneous acid extraction and mechanical disruption. Cells were harvested from induced cultures of BL21(DE3) cells transformed with either pET22b vector without added insert ("1"), pET22b/MBP18.5^{hum.} ("2") or pET22b/MBP+X2^{Cys81/bact.} ("3"). The positions of MBP+X2^{Cys81} (open arrows) and MBP18.5^{hum}. (closed arrows) are indicated. Note that this large scale acid extraction allowed recovery of almost all of the MBP+X2^{Cys81} and the MBP18.5 polypeptides in the soluble fractions.

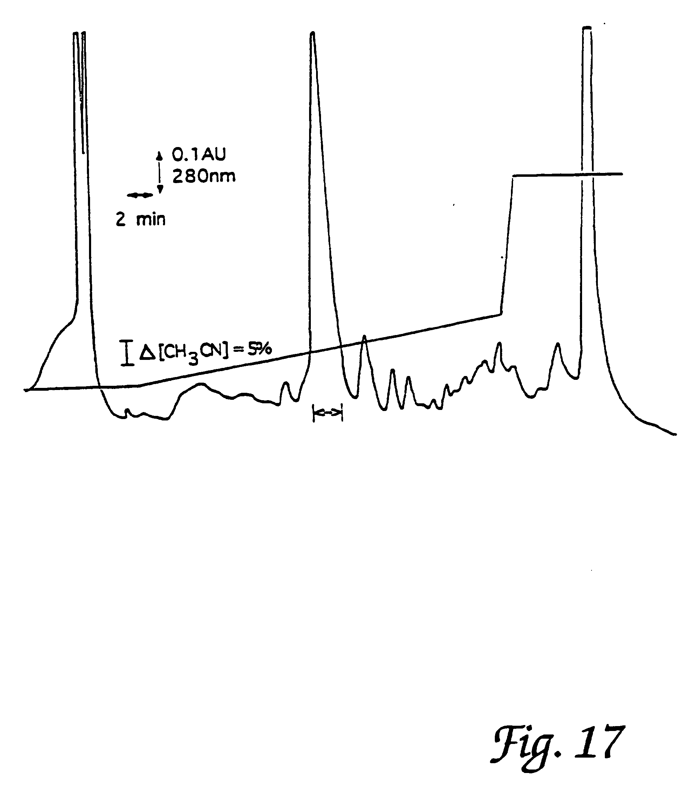

Fig. 17. Chromatograph showing reversed-phase chromatographic isolation of acid-extracted MBP+X2^{Cys81}. The soluble fraction recovered from the experiment shown in Fig. 16 ("sol" lane "3") was chromatographed over a VYDAC C4 reverse phase column and eluted via a 25-50% (CH₃CN)/0.1%TFA gradient. MBP+X2^{Cys81} is found in pooled fractions that correspond to the large peak eluting between 17 and 20 minutes. A similar chromatograph was obtained for MBP18.5.

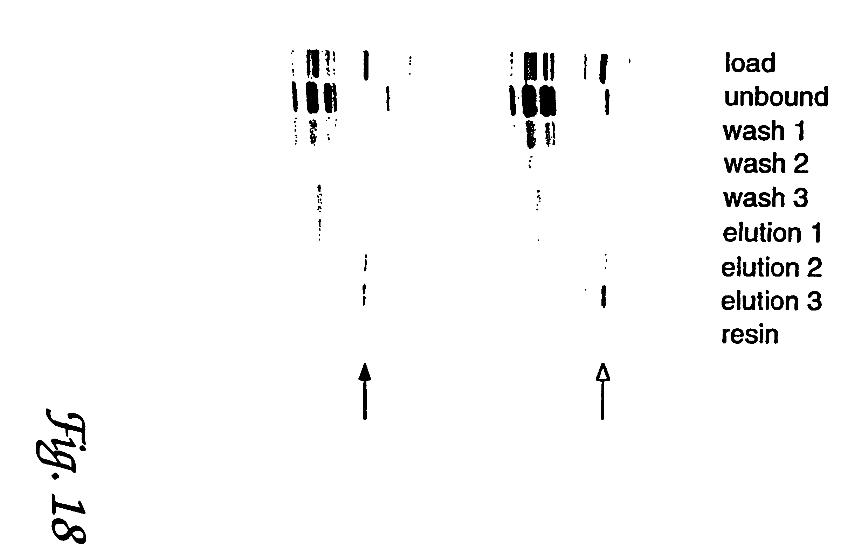

Fig. 18. Purification of MBP+X2^{Cys81} (top panel) and MBP18.5 (bottom panel) by metal chelation chromatography of acid extracts of bacterial cells. Shown are Coomassie stained gels of protein fractions collected during the affinity purification and subjected to SDS-PAGE. The positions of MBP+X2^{Cys81} (open arrow) and MBP18.5 (closed arrow) are indicated. Lanes are labeled "load" (the lysate loaded onto the column), "unbound" (the column flow-through, "wash 1", wash 2", and "wash 3" (the column eluate from each wash), "elution 1", elution 2", and "elution 3" (the column eluate from each elution step), and resin (a sample of column resin taken after the final elution, boiled in sample buffer, and loaded on the gel).

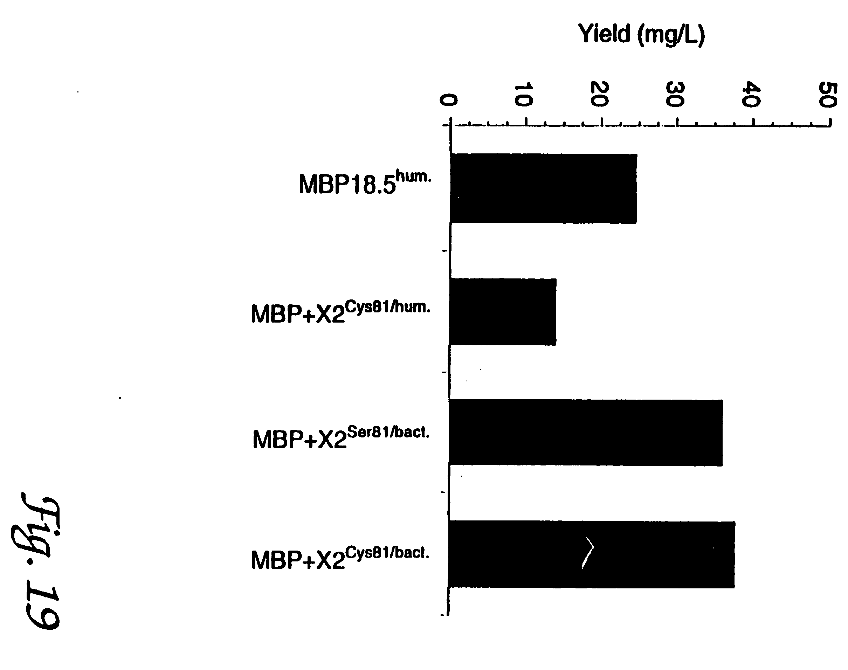

Fig. 19. Yield of bacterially expressed MBP polypeptides in bacteria transfected with nucleic acid vectors comprising the nucleic acid sequences MBP18.5^{hum.} (SEQ ID NO:4), MBP+X2^{Cys81/hum.} (SEQ ID NO:1), MBP+X2^{Ser81/bact.} (SEQ ID NO:3), and MBP+X2^{Cys81/bact.} (SEQ ID NO:2), as indicated.

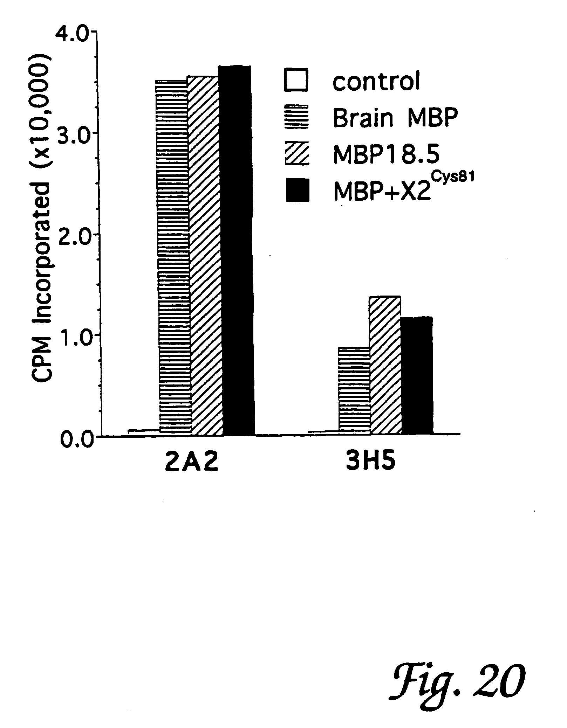

Fig. 20. MBP antigens elicit proliferative responses from human T cell clones specific for adult, brain-derived MBP. T cell lines specific for adult brain MBP18.5 were stimulated with medium alone ("control") or medium containing 10mg of either purified adult human brain MBP ("Brain MBP"), bacterially produced MBP18.5 ("MBP18.5''), or bacterially produced MBP+X2^{Cys81}("MBP+X2^{Cys81}"). Reported are total incorporated ³H-CPM from one representative proliferation assay done in triplicate as described in the Examples. "2A2" and "3H5" are human T cell lines obtained from normal individuals as described in the Examples.

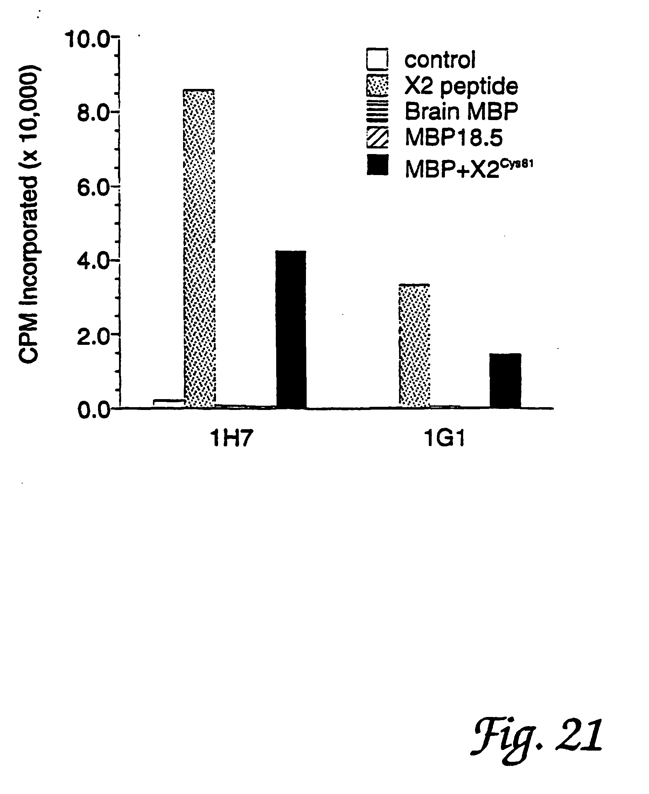

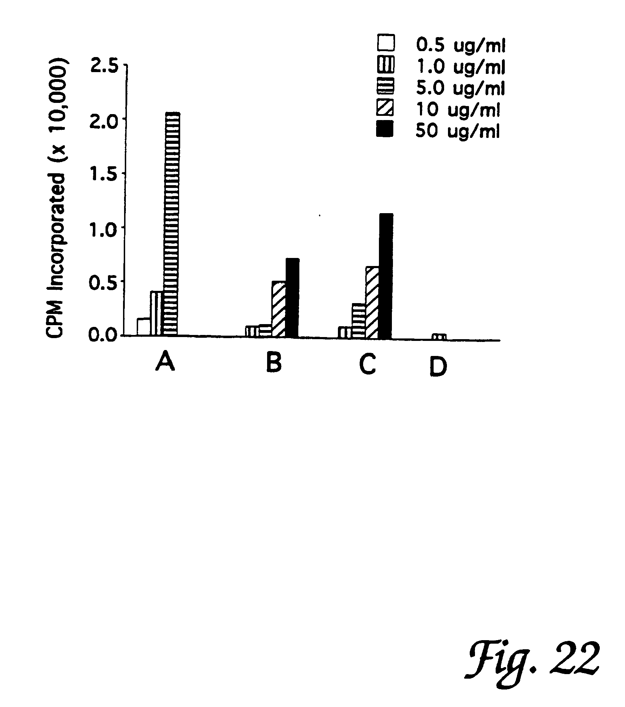

Fig. 21. Proliferative responses of exon 2-specific human T cell lines to MBP antigens. Human T cell lines 1H7 and 1G1 were stimulated with medium alone ("control") or medium containing 10µg of either purified adult human brain MBP ("Brain MBP"), bacterially produced MBP18.5 ("MBP18.5"), bacterially produced MBP+X2^{Cys81} ("MBP+X2^{Cys81}"), or exon 2-encoded peptide corresponding to amino acids 59 to 84 of SEQ ID NO:1 ("X2 peptide"). Presented are the total ³H-CPM incorporated during the proliferation assays, which were done in triplicate as described in the Examples. 1H7 and 1G1 are human T cell lines that are specific for the exon 2 encoded region of MBP and were obtained from the same MS patient as the 3A11 line used in the experiment set forth below in Fig. 22. Presented are the total ³H cpm incorporated during the proliferation assays, which were done in triplicate as described in the Examples.
Fig. 22. Proliferative responses of exon 2-specific human T cell lines to MBP+X2^{Cys81} and MBP+X2^{Ser81}. Human T cell line 3A11 was stimulated with varying doses of exon 2 peptide ("A"), MBP+X2^{Cys81} ("B"), MBP+X2^{Ser81} ("C"), or medium alone ("D"). 3A11 is a human T cell line that is specific for the exon 2 encoded region of MBP and was obtained from the same MS patient as the 1H7 and 1G1 lines used in the experiment described in Fig. 21. Presented are the total ³H cpm incorporated during the proliferation assays, which were done in triplicate as described in the Examples.

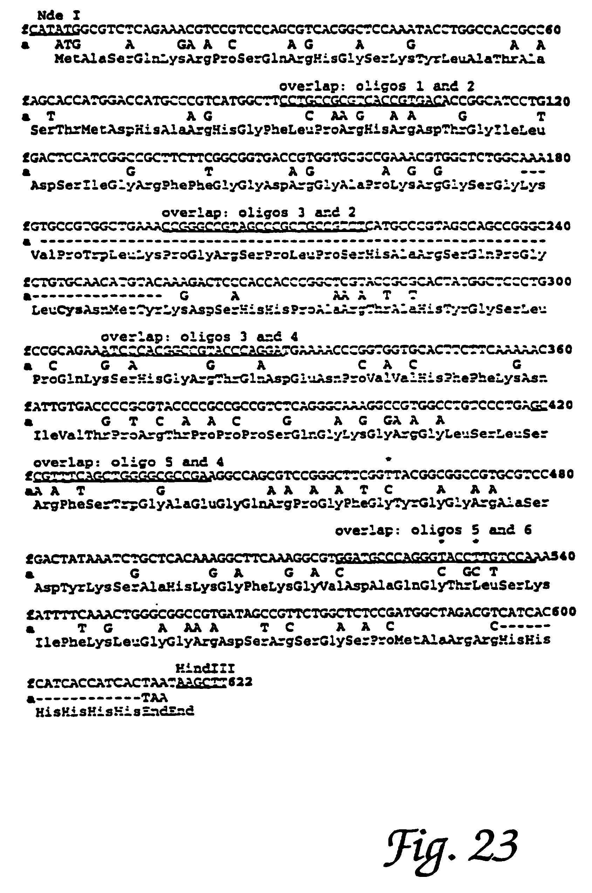

Fig. 23. Sequence comparison of recombinant human MBP+X2^{Cys81/bact.} (fetal form, "f", SEQ ID NO:1) to that of adult brain-derived human MBP (adult form "a", SEQ ID NO:4). The adult brain-derived human MBP sequence (Genbank accession #M13577) is noted only in positions that deviate from the E. coli preferred codon sequence of MBP+X2^{Cys81/bact.}. The initiator (ATG) and stop codons (TAA) are indicated for both genes. Dashes in the adult brain-derived human MBP sequence reflect the positions of exon 2 (bp 177-255) and the histidine tag (bp 595-612) additions to this version of MBP+X2^{Cys81}/bact. (i.e., MBP+X2^{Cys81}/bact. with 6 carboxy terminal histidine residues, also referred to as a histidine tag). Regions of overlap between synthetic oligonucleotides used for the construction of the MBP+X2^{Cys81/bact.} gene are underlined. C to T bp mutations from the intended MBP+X2^{Cys81/bact.} gene sequence are noted by asterisks above positions 462, 528 and 532. These changes conserve the MBP+X2^{Cys81} amino acid sequence. Sense oligonucleotide 1 (SEQ ID NO:5) includes the sequence GGAATTCCGT AAGGAGGTAT AG (not shown in this figure) located 5' to the *Nde*I cloning site, and extends through base 108. Oligonucleotide 6 (bp 516-622, SEQ ID NO:10) is an antisense oligonucleotide to the sequence shown and includes the tetranucleotide CCCC (not shown in this figure) located 3' to the *Hin*dIII site. Four other oligonucleotides used include sense oligonucleotides 3 (SEQ ID NO:7) and 5 (SEQ ID NO:9) and antisense oligonucleotides 2 (SEQ ID NO:6) and 4 (SEQ ID NO:8). The cysteine at amino acid 81 is noted in boldface type.

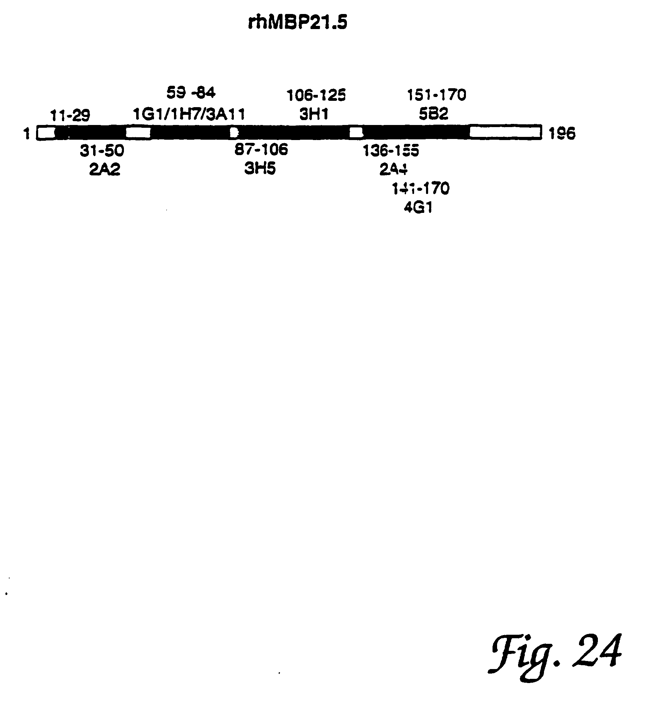

Fig. 24. Diagrammatic representation of location of MBP epitopes of recombinant human MBP 21.5 ("rhMBP21.5). numbers indicate amino acid residues of SEQ ID NO:1 corresponding to the known epitope specificity of the T cell lines tested (indicated by number letter number designations or "Gimer"). Each of the T cell lines shown gave a positive T cell response to the purified rhMBP21.5 molecules of the invention.

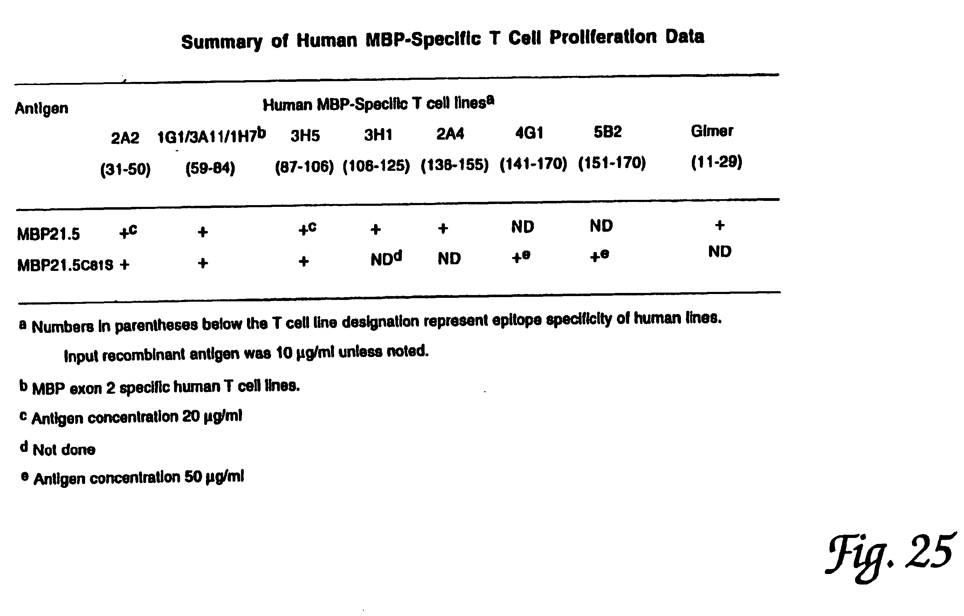

Fig. 25. Details of the specific molecules tested and results obtained with each T cell line shown in Fig. 24.

### SUMMARY OF THE INVENTION

Accordingly, it is an object of the present invention to provide compositions and methods for the diagnosis, clinical assessment, and therapeutic treatment of MS in human patients, and for the assessment of the potential responsiveness of MS patients to such therapeutic treatment.

The invention provides compositions comprising novel recombinant human PLP polypeptides. As used herein and in the claims, "PLP polypeptides" are polypeptides that contain at least one sequence corresponding to at least one hydrophilic domain of human PLP, as discussed above. In accordance with the invention, such PLP polypeptides further comprise a MBP polypeptide sequence. In some cases, the polypeptides also comprise a MOG sequence. Also provided are DNA constructs which encode PLP polypeptides and which have been engineered to optimize the production and isolation of such molecules from bacterial cells.

More specifically, the molecules of the invention include immunoreactive polypeptides comprising PLP muteins that comprise a sequence of amino acids comprising the sequence of a native PLP polypeptide minus at least one hydrophobic peptide region, and preferably minus at least two hydrophobic regions. More preferably, the sequence of amino acids comprises the sequence of a native PLP polypeptide minus at least three hydrophobic peptide regions. Most preferred are immunoreactive polypeptides comprising PLP muteins that comprise a sequence of amino acids comprising the sequence of a native PLP polypeptide minus at least some of the amino acid residues making up each of all four hydrophobic domains of PLP.

The polypeptide and nucleic acid molecules of the invention further comprise MBP sequences, i.e., sequences corresponding to any span of at least 10 contiguous amino acid residues of SEQ ID NO: 1 or SEQ ID NO:3. As used herein and in the claims, an "MBP polypeptide" is a polypeptide comprising such an MBP sequence, and "an amino acid sequence encoded by at least part of exon 2 of the human MBP gene" is a sequence of at least 10 contiguous amino acids corresponding to at least 10 contiguous amino acids from the region spanning amino acids 60-85 of SEQ ID NO:1.

Compositions are described comprising recombinant human MBP 21.5 polypeptides (i.e., MBP polypeptides that comprise an amino acid sequence encoded by at least part of exon 2 of the human MBP gene). Preferably, these MBP polypeptides include amino acid sequences encoded by all seven exons of the human MBP gene. In certain preferred embodiments, the sequence encoded by exon 2 is modified to facilitate large scale production • and purification of the polypeptide. Also provided are DNA constructs which encode MBP 21.5 polypeptides and which have been engineered to optimize the production and isolation of such molecules from bacterial cells.

The methods of the invention comprise the use of the compositions of the invention in the diagnosis and clinical assessment of MS, as well as in the therapeutic treatment of MS and in the assessment of the potential responsiveness of MS patients to such therapeutic treatment.

### DESCRIPTION OF THE PREFERRED EMBODIMENTS

As discussed above, the present invention relates to MBP and PLP polypeptides (proteins) for use in the treatment, diagnosis, and clinical assessment of MS, and to nucleic acid molecules useful in producing MBP and PLP polypeptides.

### I. MBP polypeptides

As used in this specification and in the claims "MBP 21.5 polypeptides" refers to one or more of the following polypeptides: the polypeptide of SEQ ID NO:1 (human 21.5 kDa MBP, "MBP+X2"), the polypeptide of SEQ ID NO:1 with amino acid 81 being any standard amino acid ("MBP+X2^{Xxx81}"), the polypeptide of SEQ ID NO:1 with cysteine 81 replaced with any other standard amino acid ("MBP+X2^{Xaa81}"), the polypeptide of SEQ ID NO:1 with cysteine 81 replaced with an uncharged amino acid (i.e., an amino acid that is uncharged at a pH of between 6 and 7) having a molecular weight of less than about 150 ("MBP-X2^{Xaa81<150}"), and the polypeptide of SEQ ID NO:1 with cysteine 81 replaced with serine ("MBP+X2^{Ser81}").

"MBP 21.5 polypeptides" also comprise variations of the foregoing four sequences, provided that the sequence continues to include at least some of the sequence of amino acids encoded by exon 2 of the human MBP gene, and further provided that the polypeptide can induce a "T cell response" in a population of MBP reactive T cells isolated from an MS patient. The term "T cell response" is discussed below.

A preferred MBP 21.5 polypeptide of the invention is a bacterially expressed human recombinant MBP containing amino acids encoded by exon 2 of the human MBP gene and having a molecular weight of approximately 21.5 kDa in which Cys 81 has been replaced with another standard amino acid (this polypeptide is referred to herein as "MBP+X2^{Xaa81}", and nucleic acid molecules encoding it are referred to as "MBP+X2^{Xaa81/hum.}" or "MBP+X2^{Xaa81/bact.}" with the superscript ^{hum.} or ^{bact.} indicating the codon usage in the coding region of the nucleic acid molecule, as discussed below). As used in the art, a "standard" amino acid is one of the 20 amino acids commonly found in proteins.

As used herein, the amino acid sequence encoded by exon 2 will be referred to as X2MBP or simply X2. In accordance with the invention, the X2MBP sequence may be located at any position in the MBP+X2^{Xxx81} polypeptide, although the naturally occurring position of the native exon 2 encoded sequence (as shown in SEQ ID NO:1, SEQ ID NO:2, and SEQ ID NO:3) is preferred. Other polypeptides comprising X2MBP sequences are described below.

Preferably, the replacement amino acid does not cause epitope conversion, i.e., T cell recognition of the immunodominant epitope or epitopes of X2MBP is substantially unaltered by the replacement of Cys 81 with the particular replacement amino acid. Prior to the present invention it was unknown whether replacement of amino acid residue 81 with another standard amino acid would cause such epitope conversion (i.e., whether such alterations would be epitope neutral).

Lack of epitope conversion by the substitution of any standard amino acid can be determined in accordance with the present invention by testing the responses of T cells (e.g., T cell lines) specifically reactive with X2MBP (X2MBP-specific T cells) to MBP+X2^{Xaa81} or, preferably, to a test peptide (the X2^{Xaa81} peptide) comprising the exon 2 encoded region of MBP+X2^{Xaa81} as described in detail below. The test peptide is preferably a 26 amino acid peptide with a sequence corresponding to amino acid residues 59 to 84 of SEQ ID NO:1 with Cys 81 replaced with the other standard amino acid (the "X2^{Xaa81} 26mer").

X2MBP-specific T cells can be obtained as T cell lines by conventional methods using a peptide containing the amino acid sequence encoded by exon 2 (hereinafter referred to as an "X2MBP peptide"). For example, the methods described by Voskuhl et al. 1993a may be used. See also Voskuhl *et al.*, 1993b; Segal *et al.*, 1994; Voskuhl *et al.,* 1994; and Fritz and Zhao, 1994.

Preferably human T cell lines are obtained by such standard methods following stimulation with an X2MBP peptide that has just the 26 amino acids encoded by exon 2, i.e., an X2MBP peptide whose a sequence corresponds to amino acid residues 59 to 84 of SEQ ID NO:1 (the "X2 26mer"). In particular, stimulation with the X2 26mer is preferred to stimulation with the 40 amino acid X2MBP peptide or the 18.5 kDa isoform of MBP described in the Voskuhl et al. 1993a publication.

In accordance with the present invention, X2MBP-specific T cell lines thus obtained are used, *inter alia,* to determine the epitope neutrality of a particular amino acid substitution at position 81. This is accomplished by assessing the reaction of the cells of the X2MBP-specific human T cell line to the X2^{Xaa81} peptide. (MBP+X2^{Xaa81} can also be used to test epitope neutrality, but this is less preferred.) If the X2MBP-specific T cells respond to the X2^{Xaa81} peptide containing the particular amino acid substitution to an extent that satisfies the criterion for X2MBP-specificity set forth by Voskuhl et al. 1993a, i.e. if the particular X2^{Xaa81} peptide demonstrates a stimulation index of greater than 2, as compared to medium alone controls, then epitope neutrality of a particular replacement amino acid is confirmed. Preferably the stimulation index is greater than 3.

In accordance with the present invention, such an epitope neutral replacement can generally be achieved using an uncharged amino acid that has a molecular weight of less than about 150 and that preferably is not strongly hydrophobic.

Amino acids that satisfy these requirements include Ala, Asn, Gly, Pro, Thr, and Ser. Most preferably, the replacement is Ser, resulting in an MBP 21.5 polypeptide comprising an exon 2 encoded region in which Cys 81 has been changed to Ser 81 (hereinafter this polypeptide is referred to as "MBP+X2^{Ser81"}, and nucleic acid molecules encoding it are referred to as "MBP+X2^{Ser81/hum.}" or "MBP+X2^{Ser81/bact.}", with the superscripts ^{hum.} and ^{bact.} indicating the codon usage in the coding region of the nucleic acid molecule, as discussed below).

Prior to the present invention, it was not known whether bacterially expressed MBP+X2 polypeptides would be recognized and responded to by T cells to the same extent as mammalian expressed MBP polypeptides (e.g., human derived MBP-X2). This uncertainty was due, *inter alia*, to the differences in protein folding during the expression of proteins in bacteria or mammalian cells. Bacterially expressed proteins are typically not folded into the native conformation of proteins expressed in mammalian cells. As discussed within the Background of the Invention section above under the heading "T Cells, Antigen Presenting Cells, and T Cell Epitopes", protein folding can determine whether a specific epitope is appropriately processed by APCs. For this reason, bacterially expressed proteins may not be processed and presented by APCs in the same manner as native proteins, and may therefore not be recognized by T cells.

The exon 2 sequences in MBP+X2^{Cys81} were cause for additional uncertainty, as such sequences had only been shown to stimulate T cells when added to T cells as synthetic peptides, (which do not have to be processed by APCs in order to be recognized by TCRs and responded to by T cells). Prior to the present invention, it had never been shown that the 21.5 kDa isoform of MBP (regardless of source) could be correctly processed by APCs so as to stimulate encephalitogenic T cells, a question of particular interest with regard to the role of X2 epitopes in MS pathogenesis. The present invention has allowed the demonstration that this is the case, demonstrating the clinical relevance of the previously reported X2MBP peptide work.

### II. PLP Polypeptides

A preferred PLP polypeptide of the invention is a bacterially expressed human recombinant PLP containing hydrophilic domains 2, 3 and 4. Such PLP polypeptides may include one or more hydrophobic domains. More preferrably, PLP polypeptides comprise the PLP epitopes associated with MS shown in Table 1. Such preferred PLP polypeptides include the amino acid sequence set forth in SEQ ID NO:25, SEQ ID NO:26 (preferably amino acid residues 1 to 368, inclusive, of SBQ ID NO:26), SEQ ID NO:27 (preferably amino acid residues 6 to 374, inclusive, of SEQ ID NO:27), or SEQ ID NO:28 (preferably amino acid residues 1 to 487, inclusive, of SEQ ID NO:28).

In a particularly preferred embodiment, the immunoreactive polypeptides comprise at least 10 contiguous amino acids (i.e., a linear polymer of amino acids sufficient in size to comprise an epitope), all but one target amino acid residue of which correspond to a region of the 21.5 kDa isoform of human MBP (SEQ ID NO:1) comprising amino acid residue 81 of SEQ ID no: 1. In this embodiment, the target amino acid residue is located in a position within the MBP amino acid sequence corresponding to the position of amino acid residue 81 of SEQ ID NO: 1 and the target amino acid residue is any standard amino acid other than cysteine.

Certain preferred immunoreactive polypeptides of the invention further comprise a myelin oligodendrocyte glycoprotein amino acid sequence corresponding to at least 10 contiguous amino acids of the amino acid sequence of human myelin oligodendrocyte glycoprotein (amino acid residues 199 to 319, inclusive, of SEQ ID NO:28).

Preferably, the immunoreactive polypeptides of the invention are expressed in bacteria at higher levels than the native PLP polypeptide and/or are more soluble in aqueous solution than the native PLP polypeptide.

PLP-specific T cells can be obtained as T cell lines by conventional methods using a peptide containing a PLP amino acid sequence. For example, the methods described by Voskuhl et al. 1993a may be used. See also Voskuhl et al., 1993b; Segal et al., 1994; Voskuhl et al., 1994; Fritz and Zhao, 1994; Pelfrey et al. 1993; Pelfrey et al. 1994; and Correale et al. 1995.

Prior to the present invention, it was not known whether bacterially expressed PLP polypeptides would be recognized and responded to by T cells in a manner that would allow their use as therapeutic agents. This uncertainty was due, *inter alia,* to the differences in protein folding during the expression of proteins in bacteria or mammalian cells. Bacterially expressed proteins are typically not folded into the native conformation of proteins expressed in mammalian cells. As discussed within the Background of the Invention section above under the heading "T Cells, Antigen Presenting Cells, and T Cell Epitopes", protein folding can determine whether a specific epitope is appropriately processed by APCs. For this reason, bacterially expressed proteins may not be processed and presented by APCs in the same manner as native proteins. Therefore, some or all of the epitopes in such a bacterially expressed protein may not be recognized by T cells.

### III. Nucleic Acid Molecules Encoding MBP and PLP Polypeptides

Nucleic acid molecules useful in the practice of the present invention can be prepared using a variety of techniques now known or subsequently developed in the art. For example, using techniques well known in the art they can be produced using cloned genes. The terms gene and genes, as used herein, encompass expressed (e.g., protein-encoding) nucleic acid molecules, either with intron-comprising sequences or without introns, e.g. cDNAs. The cloned genes are manipulated by conventional techniques, e.g., PCR amplification and/or restriction digestion of nucleic acid molecules to generate restriction fragments encoding portions of the MBP or PLP polypeptides. These fragments can be assembled using, for example, PCR fusion (overlapping PCR) or enzymatic ligation of the restriction digestion products. The assembled constructions or fragments thereof can be modified by mutagenic techniques such as oligonucleotide mediated site-directed mutagenesis.

Numerous publications are available that teach these conventional methods, including Sambrook, et al. 1989; Ho et al. Gene 1989; Farrell 1993; Ausubel et al. 1994; Griffin and Griffin 1994; Mullis et al. 1994; Harwood 1994; and Davis et al. 1994. Alternatively, the nucleic acid molecules encoding the MBP or PLP polypeptides used in the practice of the invention or any or all of the nucleic acid fragments used to assemble such nucleic acid molecules can be synthesized by chemical means (see, for example, Talib et al. 1991 and Ausubel et al. 1994).

In accordance with the present invention, codons for various of the amino acids of the MBP and PLP polypeptides of the invention may be "bacterialyzed" to enhance the production of the protein in bacteria. As known in the art, bacteria tend to use certain codons for particular amino acids in preference to other possible codons which encode the same amino acid. Accordingly, it is believed that the protein synthetic machinery of the bacteria may work more effectively when processing the preferred codons. Bacterialization and other alterations of myelin protein-encoding codons will now be discussed in greater detail as exemplified by specific reference to the MBP molecules of the invention.

SEQ ID NO:1 sets forth the amino acid and nucleotide sequences for the native human 21.5 kDa fetal isoform of MBP. A nucleic acid molecule encoding MBP+X2^{Xaa81} can be produced by modifying at least one of nucleotides 241 through 243 (i.e., codon 81) of SEQ ID NO: 1 so that the codon corresponds to the desired replacement amino acid. Such modification can be achieved using a variety of nucleic acid manipulation techniques now known or subsequently developed in the art, including conventional recombinant DNA techniques such as oligonucleotide mediated site-directed mutagenesis, PCR mutagenesis, or de novo synthesis of the desired polynucleotide, as discussed above.

For MBP+X2^{Ser81}, the native TGC codon can be changed to any of AGC, AGT, TCA, TCC, TCG, and TCT. In general, the change is preferably to TCG, as this change results in the creation of a new TCGA restriction site at this location. The creation of a new restriction site at this location facilitates the identification and separation of a nucleic acid molecule comprising the desired modification from the mixture of modified and unmodified nucleic acid molecules that is typically obtained as an intermediate step in the overall process of producing a nucleic acid molecule encoding MBP+X2^{Xaa81}, such as a nucleic acid molecule encoding MBP+X2^{Ser81}. When considerations of optimization of protein production override considerations of ease of nucleic acid manipulation, and when MBP+X2^{Ser81} is to be produced in bacteria, e.g., *E*. *coli* (where the TCG codon is not a bacterially preferred codon) the change is preferably to TCC, TCT, or AGC, since these codons are preferred in bacteria.

SEQ ID NO:2 sets forth the amino acid sequence for the native human 21.5 kDa fetal isoform of MBP and a modified nucleotide sequence encoding this protein wherein the codons for various of the amino acids have been "bacterialyzed" to enhance the production of the protein in bacteria. As known in the art, bacteria tend to use certain codons for particular amino acids in preference to other possible codons which encode the same amino acid. Accordingly, it is believed that the protein synthetic machinery of the bacteria may work more effectively when processing the preferred codons. However, as also known in the art, it is unpredictable whether substituting preferred codons for non-preferred codons will in fact result in a substantial enhancement in production of a particular protein in bacteria. As discussed in detail in the Examples, below, the bacterialization of SEQ ID NO:2 increased production of MBP in *E. coli* by at least 50 percent.

In SEQ ID NO:2, the bacterialization has been performed by substituting bacterially preferred codons for native human codons which did not already correspond to bacterially preferred codons (criterion 1). In selecting which codons to change, particular attention was paid to the following seven amino acids: Arg (17 of 21 codons changed); Gly (13 of 28 codons changed); Pro (10 of 17 codons changed); Lys (12 of 14 codons changed); Leu (3 of 11 codons changed); Thr (6 of 8 codons changed); and Val (3 of 5 codons changed). These amino acids were emphasized because of a strong bias for the use of certain of their redundant codons in *E. coli*. (Wada et al., 1992.). Of these seven, Arg, Pro, and Lys were considered the most important since they constitute 26% of the amino acid residues in MBP 21.5. As an alternate criterion, some codons were changed to a codon which is preferentially used in highly expressed bacterial genes (criterion 2, see Grosjean and Fiers, 1982). A complete listing of codon changes incorporated in the nucleic acid molecule corresponding to SEQ ID NO:3 (except for the native cysteine codon 81 being retained in this comparison instead of the Ser codon for amino acid number 81 found in SEQ ID NO:3) is given in Table 4, where the native (fetal) human MBP21.5 sequence data are indicated as "huMBP 21.5" and the bacterialized recombinant MBP (MBP+X2^{Cys81/bact.}) sequence data are indicated as "recMBP 21.5".

As used herein and in the claims, the expression "bacterially preferred codon" refers to a codon selected on the basis of either of the above two criteria, and the superscripts (1) ^{"hum."} and (2) ^{"bact. "} designate MBP-encoding nucleic acid sequences with (1) native human codons and (2) at least some codons that have been changed from native human codons to bacterially preferred codons.

More or less bacterialization can be performed if desired, the criterion being whether a desired level of production increase is achieved. Also, with regard to MBP, the bacterialyzed sequence can be further altered to produce MBP+X2^{Xaa81/bact.}, or preferably MBP+X2^{Ser81/bact.}. The bacterialization and the further alterations at codon 81 can be performed using the nucleic acid manipulation techniques discussed above and in the Examples.

As discussed above, SEQ ID NO:3 shows such a bacterialyzed nucleotide sequence encoding MBP+X2^{Ser81}, and further comprising an additional 18 nucleotide sequence at the 3' end (immediately preceding the termination codon, i.e., nucleotides 592-609 of SEQ ID NO:3) that encodes six histidine residues at the carboxy terminus of the encoded polypeptide (such a multiple histidine addition of at least four residues being referred to as a histidine tag). This histidine tag is not found in the native MBP+X2^{Cys81/hum.} protein, and has been added to facilitate purification of the polypeptide product of the expression of this MBP+X2^{Ser81/bact.} gene.

Histidine tags are groups of at least five consecutive histidine residues that act as metal chelators and allow the use of metal chelation chromatography or the like to rapidly and efficiently purify polypeptides containing such tags from mixtures of proteins. In accordance with the invention, such a histidine tag may be added to any of the polypeptides of the invention, or a sequence encoding such a tag may be added to any of the nucleic acid molecules of the invention so as to allow the ready purification of the polypeptides of the invention.

Preferred nucleic acid molecules of the invention are isolated nucleic acid molecules that comprise a nucleotide sequence (and/or a nucleotide sequence complementary thereto) which, when expressed in a suitable host, directs the expression of the PLP polypeptides of the invention.

The protein-encoding nucleic acid molecules of the invention can be inserted into an appropriate expression vector, i.e., a vector that contains the necessary elements for the transcription and translation of the inserted protein-encoding sequence, and then used to produce MBP and/or PLP polypeptides. A variety of host vector systems may be utilized to express the protein encoding sequence. These include, but are not limited to, mammalian cell systems infected with a virus such as vaccinia virus, adenovirus, a retrovirus, etc.; mammalian cell systems transfected with plasmids; insect cell systems infected with a virus such as baculovirus; microorganisms such as yeast containing yeast expression vectors, or bacteria transformed with bacteriophage DNA, plasmid DNA, cosmid DNA, or the like.

Useful expression vectors for bacterial use can comprise a selectable marker and bacterial origin of replication derived from commercially available plasmids including those comprising genetic elements of the well-known cloning vector pBR322 (American Type Culture Collection, 12301 Parklawn Drive, Rockville, Maryland 20852, United States of America; ATCC Accession No. 37017). These pBR322 "backbone sections," or functionally equivalent sequences, are combined with an appropriate promoter and the structural gene to be expressed.

Preferred bacterial expression vectors include, but are not limited to, the phage T7 promoter plasmids pET14b, and pET22b (Novagen, Madison, WI). These vectors are preferably expressed in *E. coli* BL21(DE3) (Novagen, Madison, WI). This strain is lysogenic for a recombinant bacteriophage DE3 lysogen, which contains the gene for T7 polymerase behind the *E. coli* lacUV5 promoter (Studier et al., 1990). Other preferred bacterial expression vectors are Trc vectors including the pET Trc S05/NI vector (SEQ ID NO:21) the pTrc 99A vector (Pharmacia) and the pSE vectors (Invitrogen, San Diego, CA).

Other promoters commonly used in recombinant microbial expression vectors include, but are not limited to, the lactose promoter system (Chang, et al., 1978), the tryptophan (trp) promoter (Goeddel, et al., 1980) and the tac promoter, or a fusion between the tac and trp promoters referred to as the trc promoter (see Sambrook, et al., 1989, and Maniatis, et al., 1982, particualrly page 412). Particularly preferred promoters are bacteriophage promoters, e.g., the T7 promoter discussed above, that can be used in conjunction with the expression of the corresponding bacteriophage RNA polymerase, e.g., T7 RNA polymerase, in the host cell.

Recombinant MBP and PLP polypeptides may also be expressed in fungal hosts, preferably yeast of the genus *Saccharomyces* such as *S. cerevisiae.* Fungi of other genera such as *Aspergillus, Pichia* or *Kluyveromyces* may also be employed. Fungal vectors will generally contain an origin of replication from the 2 µm yeast plasmid or another autonomously replicating sequence (ARS), a promoter, DNA encoding the MBP and/or PLP polypeptide, sequences directing polyadenylation and transcription termination, and a selectable marker gene. Preferably, fungal vectors will include origins of replication and selectable markers permitting transformation of both *E. coli* and fungi.

Suitable promoter systems in fungi include the promoters for metallothionein, 3-phosphoglycerate kinase, or other glycolytic enzymes such as enolase, hexokinase, pyruvate kinase, and glucokinase, as well as the glucose-repressible alcohol dehydrogenase promoter (ADH2), the constitutive promoter from the alcohol dehydrogenase gene, ADH1, and others. See, for example, Schena, et al. 1991. Secretion signals, such as those directing the secretion of yeast alpha-factor or yeast invertase, can be incorporated into the fungal vector to promote secretion of the MBP and/or PLP polypeptide into the fungal growth medium. See Moir, et al., 1991.

Preferred fungal expression vectors can be constructed using DNA sequences from pBR322 for selection and replication in bacteria, and fungal DNA sequences, including the ADH1 promoter and the alcohol dehydrogenase ADH1 termination sequence, as found in vector pAAH5 (Ammerer, 1983).

Various mammalian or insect cell culture systems can be employed to express the recombinant MBP and/or PLP polypeptides of the invention. Suitable baculovirus systems for production of heterologous proteins in insect cells are reviewed by Luckow, et al., 1988. Examples of suitable mammalian host cell lines include the COS cell of monkey kidney origin, mouse C127 mammary epithelial cells, mouse BALB/c-3T3 cells, mouse MOP8 cells, Chinese hamster ovary cells (CHO), human 293T cells, HeLa, myeloma, and baby hamster kidney (BHK) cells. Mammalian expression vectors may comprise non-transcribed elements such as an origin of replication, a suitable promoter and an enhancer linked to the MBP and/or PLP encoding sequence to be expressed, and other 5' or 3' flanking sequences such as ribosome binding sites, polyadenylation sequences, splice donor and acceptor sites, and transcriptional termination sequences.

The transcriptional and translational control sequences in mammalian expression vector systems to be used in transforming vertebrate cells may be provided by viral sources. For example, commonly used promoters and enhancers are derived from Polyoma virus, Adenovirus, Simian Virus 40 (SV40), vaccinia, and human cytomegalovirus (CMV), including the cytomegalovirus immediate-early gene 1 promoter and enhancer.

Particularly preferred eukaryotic vectors for the expression of recombinant MBP and/or PLP polypeptides are pAPEX-1 (SEQ ID NO:11 and, more preferably, pAPEX-3p, SEQ ID NO:12. The vector pAPEX-1 is a derivative of the vector pcDNAI/Amp (Invitrogen) which was modified to increase protein expression levels. First, the 3'-untranslated SV40 small-t antigen intron was removed by deletion of a 601 base pair *Xba*I/*Hpa*I fragment since this intron is susceptible to aberrant splicing into upstream coding regions (Evans and Scarpulla, 1989; Huang and Gorman, 1990). Second, a chimeric adenovirus-immunoglobulin hybrid intron was introduced into the 5'-untranslated region by replacing a 484 base pair *Nde*I-*Not*I fragment with a corresponding 845 base pair *Nde*I-*Not*I fragment from the vector pRc/CMV7SB (Sato et al., 1994, J. Biol. Chem. 269:17267 et *seq*). Finally, to increase plasmid DNA yields from E. coli, the resulting CMV promoter expression cassette was shuttled into the vector pGEM-4Z (Promega Corp. Madison, WI).

The vector pAPEX-3 is a derivative of the vector pDR2 (Clontech Laboratories, Inc. Palo Alto, CA) in which the EBNA gene was first removed by deletion of a 2.4 kb *Cla*I/*Acc*I fragment. The RSV promoter was then replaced with the CMV promoter and the adenovirus/immunoglobulin chimeric intron by exchanging a 450 bp *Mlu*I/*Bam*HI fragment from pDR2 with a 1.0 kb *Ml*uI/*Bam*HI fragment from the vector pAPEX-1. For construction of pAPEX-3P, a 1.7 kb *Bst*BI/*Swa*I fragment containing the HSV tk promoter and hygromycin phosphotransferase (hyg) gene was removed from pAPEX-3 and replaced with a 1.1 kb *Sna*BI/*Nh*eI fragment containing the SV40 early promoter and puromycin acetyltransferase (pac) gene (Morgenstern and Land, 1990, Nucleic Acids Res. 18:3587-3596) plus a 137 bp *Xba*I/*Cla*I fragment containing an SV40 polyadenylation signal from the vector pAPEX-1.

A particularly preferred host cell for the expression of recombinant MBP- and/or PLP-encoding inserts in the pAPEX vectors is the human 293 EBNA cell line (Invitrogen, San Diego, CA).

Another preferred eukaryotic vector for the expression of recombinant MBPs and/or PLPs is pcDNAI/Amp (Invitrogen Corporation, San Diego, California). The pcDNAI/Amp expression vector contains the human cytomegalovirus immediate-early gene I promoter and enhancer elements, the Simian Virus 40 (SV40) consensus intron donor and acceptor splice sequences, and the SV40 consensus polyadenylation signal. This vector also contains an SV40 origin of replication that allows for episomal amplification in cells (e.g., COS cells, MOPS cells, etc.) transformed with SV40 large T antigen, and an ampicillin resistance gene for propagation and selection in bacterial hosts.

### III. Preparation of the Polypeptides of the Invention

Purified recombinant MBPs and PLPs are prepared by culturing suitable host/vector systems (preferably bacterial systems) to express the recombinant MBP and/or PLP translation products of the nucleic acid molecules of the present invention, which are then purified from the culture media or cell extracts of the host system, e.g., the bacteria, insect cells, fungal, or mammalian cells. The invention thus provides a method for producing MBP and PLP polypeptides comprising growing a recombinant host containing a nucleic acid molecule of the invention, such that the nucleic acid molecule is expressed by the host, and isolating the expressed polypeptide.

Fermentation of cells that express recombinant MBP and/or PLP proteins containing one or more histidine tag sequences (a sequence comprising a stretch of at least 5 histidine residues) as a secreted product greatly simplifies purification. Such a histidine tag sequence enables binding under specific conditions to metals such as nickel, and thereby to nickel (or other metal) columns for purification.

In general terms, the purification is performed using a suitable set of concentration and fractionation (e.g., chromatography) steps. For purification of MBP polypeptides, a particularly preferred purification step involves acid extraction, as described in the examples, below, under the heading "Purification and characterization of MBP Polypeptides".

The purified MBP and PLP polypeptides of the invention, however prepared, will in general be characterized by the presence of some impurities. These impurities may include proteins, carbohydrates, lipids, or other molecules in amounts and of a character which depend on the production and purification processes used. These components will ordinarily be of viral, prokaryotic, eukaryotic, or synthetic origin, and preferably are non-pyrogenic and present in innocuous contaminant quantities, on the order of less than about 1% by weight.

### IV. Clinical Applications

As discussed above, the MBP and PLP polypeptides encoded by the MBP and/or PLP nucleic acid molecules of the invention can be used in the diagnosis, clinical assessment, and treatment of MS, and for the assessment of the potential responsiveness of MS patients to therapeutic treatment involving the administration of the PLP polypeptides. Procedures for such diagnosis and assessment involve an assay entailing the incubation of replicate cultures of T cells in the presence and absence of one or more of the MBP and PLP polypeptides discussed herein, and the detection of T cell activation and/or T cell apoptosis (referred to in this specification and in the claims as a "T cell response") resulting from incubation in the presence, but not the absence, of the one or more polypeptides.

More specifically, such an assay preferably comprises isolating and partially purifying T cells from a patient, combining the isolated T cells with a PLP and/or MBP polypeptide such as a polypeptide selected from the group consisting of the polypeptide of SEQ ID NO:1, the polypeptide of SEQ ID NO:1 with cysteine 81 replaced with any other standard amino acid, the polypeptide of SEQ ID NO:1 with cysteine 81 replaced with an uncharged amino acid having a molecular weight of less than about 150, and the polypeptide of SEQ ID NO:1 with cysteine 81 replaced with serine; and/or with the polypeptide of SEQ ID NO:23, the polypeptide of SEQ ID NO:24, the polypeptide of SEQ ID NO:26, the polypeptide of SEQ ID NO:27, the polypeptide of SEQ ID NO:28, or one of the other preferred MBP or PLP polypeptides described above, and measuring the level of a T cell response induced by the polypeptide. Methods for measuring T cell responses are described below under the subheading "Detection of T Cell Responses."

In accordance with the present invention, such an assay may be provided as a kit for the detection of MBP or PLP reactive T cells comprising an isolated PLP or MBP 21.5 polypeptide in close confinement and/or proximity with an agent for use in the detection of a T cell response, such as any of the agents described below under the subheading "Detection of T Cell Responses". In a preferred embodiment of such a kit, the kit further comprises a label indicating that the kit is for use in the diagnosis and/or clinical assessment of multiple sclerosis.

A finding of T cells in a patient's CSF that exhibit a T cell response when incubated with PLP or MBP 21.5 polypeptides in this fashion is taken as an indication that the patient is suffering from MS. A finding of such MBP or PLP responsive T cells in CSF and/or blood of an MS patient is an indication that the patient is an appropriate candidate for treatment with MBP and/or PLP polypeptides. The levels of such T cells in the blood or CSF may be monitored as an indication of disease progression and response to treatment.

The number of such reactive T cells in a patient's blood and/or CSF (the "precursor frequency" or "reactive T cell index") can be monitored over time, and can be used as an indicator of the clinical progression of the disease, with increasing numbers indicating exacerbation and decreasing numbers indicating improvement. The reactive T cell index also serves as a predictor of when a therapeutic treatment would be appropriate, e.g., a sudden increase in the index would suggest that therapeutic intervention should be commenced or intensified. If the index is monitored during a course of treatment, whether or not the treatment involves the administration of PLP chimène a significant decline in the reactive T cell index is an indication of therapeutic success, while a significant rise in the index indicates therapeutic failure, and suggests that the therapeutic regimen should be adjusted.

The invention thus provides an assay comprising isolating and partially purifying T cells from a patient, combining the isolated T cells with an immunoreactive MBP 21.5 polypeptide or PLP polypeptide (the PLP polypeptide comprising a PLP mutein amino acid sequence having the amino acid sequence of a native PLP polypeptide minus at least one or two hydrophobic peptide regions, preferably minus at least three hydrophobic peptide regions) and measuring the level of a T cell response induced by the polypeptide.

### A. Detection of T Cell Responses

Assays of T cell activation and of apoptosis are well known to those of skill in the art. Detailed discussions of and protocols for such assays can be found in numerous publications including, Wier, 1978; Klaus, 1987; Voskuhl et al., 1993; and Ormerod, 1994. Such assays measure alterations of certain key indicators of T cell activation, and/or apoptosis.

For T cell activation, these indicators generally include reagents for the detection of T cell proliferation, cytokine release, and expression of cytokine receptors and other activation-associated cell surface markers. For apoptosis, these indicators generally include dyes, stains, and other reagents for the observation/detection of nuclear shrinkage and/or cell death; metabolic inhibitors capable of inhibiting apoptotic cell death; stains, enzymes, labeled nucleic acid precursors, and other indicators of DNA degradation.

All assays of T cell activation and of apoptosis involve the use of cell culture (tissue culture) supplies, typically including culture vessels such as multi-well plates, dishes, and flasks, as well as test tubes and centrifuge tubes, liquid measuring devices such as pipettes, droppers, and dropper bottles, cell culture media, and buffer solutions. Many of these assays also involve a readout that involves a labeled antibody, often a secondary antibody against a primary, unlabeled antibody that specifically binds to the indicator being measured. In addition, these assays involve numerous other reagents and instruments, as discussed below and in the Examples. As used in this specification, and in the claims, an "agent for use in the detection of a T cell response" is any of the reagents (including antibodies), supplies, media, and instruments discussed herein that can be used for such detection.

Unless reagents specific for T cells are used as indicators, the measurements of T cell responses will generally involve the labeling and/or further purification of T cells from preparations of white blood cells, which are typically obtained (i.e., partially purified) by centrifugation and/or filtration of the body fluid (e.g., cerebrospinal fluid or decoagulated blood) in which they are isolated. As used hereinafter, and in the claims, "isolated T cells" are T cells that have been removed from the body of a living subject, but not necessarily further purified (e.g., by centrifugation to remove white blood cells from a body fluid or by separation of T cells from other blood cells). The isolation of T cells thus involves lancets, needles, syringes, evacuated blood collection tubes, and other blood and/or CSF collection supplies, and may further involve the use of filtration and centrifugation supplies.

Methods for specifically labeling T cells typically involve conventional immunohistochemical and/or FACS techniques involving antibodies to T cell specific markers, which are generally T cell receptors, subunits thereof, and associated molecules such as CD3. Such antibodies are commercially available from numerous sources.

Methods for at least partially purifying T cells include cell sorting by FACS using the above-mentioned antibodies, various affinity purification methods, including passage over glass beads and/or nylon wool, the use of antibodies to markers for other white blood cell types to remove cells other than T cells from mixtures of white blood cells, and differential centrifugation, e.g., centrifugal elutriation and/or density gradient centrifugation using density gradient media such as polysucrose (FICOLL), albumin, colloidal silica, and the like.

Detection of T cell proliferation can be accomplished by labeling or partially purifying T cells as discussed above and applying methods used to detect cell proliferation generally. One such method involves labeling newly synthesized DNA by culturing the T cells in the presence of detectable nucleic acid precursor molecules that can be incorporated into nascent DNA by living cells. Such precursors include ³H thymidine and other radioactively labeled precursors, and BrdU and other conveniently detectable non-radioactive precursors. When radioactively labeled precursors are used, unincorporated precursors are washed away and levels of incorporated precursors are measured by autoradiography, scintillation counting, or other conventional methods of radiation quantification.

When BrdU and the like are used, unincorporated precursors are washed away and antibodies or other reagents capable of specifically binding to the precursor are used to detect precursor that has been incorporated into nuclear DNA. Additionally, reagents that label metabolically active cells can be used to follow increases in cell number. Such reagents include MTT, XTT, MTS, and WST-1. which are cleaved by mitochondrial enzymes to yield products that can be readily detected and measured spectrophotometrically, with the level of cleavage products thus measured being proportional to the number of metabolically active cells in the sample being tested. Such reagents are commercially available from many sources.

Numerous cell surface markers of T cell activation are known in the art, and are generally detected by antibodies (which are commercially available from numerous sources) using conventional immunohistochemical and/or FACS techniques. These markers include CD25 (the IL-2 receptor), CD26, CD30, CD69, and CD71 (the transferrin receptor).

T cell activation can also be detected by measuring cytokine release into culture medium (see, for example, Correale et al. 1995). Inactive T cells do not release cytokines, while at least some active T cells release IL-2, IL-4, IL-5, IL-6, IL-10, IL-11, IL-12, IL-13, IL-14, gamma interferon, TNF alpha, and the TNF-related cytokine known as the FAS ligand. In addition, T cell activation may be detected by T cell surface expression of activation-specific markers including CD95 (the FAS receptor). Antibodies for detecting each of these cytokines and markers are well known in the art and are commercially available; assays using such antibodies to measure cytokines, e.g., in culture medium, are also well known in the art and are items of commerce.

A particularly sensitive assay for T cell activation is the recently developed enzyme-linked immunospot (ELISPOT) assay, which typically detects cytokine release by single T cells as spots on an antibody coated substrate upon which the T cells are cultured. Such assays are described in Taguchi et al., 1990, and Sun et al., 1991. Preferably the ELISPOT assay is used to detect the secretion of gamma interferon.

Materials and methods for determining whether cellular morbidity is a result of an ongoing process of apoptosis are also well known to workers in the art. In addition to conventional histochemical stains, which allow the detection of apoptosis-associated ultrastructural changes, apoptosis detection procedures, including assays and staining techniques, have been in use in the art for many years. These procedures typically determine if cell death depends upon active metabolism (e.g., protein synthesis) or whether dying cells exhibit DNA degradation (fragmentation).

The former type of procedure involves growing replicate cultures containing dying cells in the presence or absence of a metabolic inhibitor, e.g., a protein synthesis inhibitor such as cycloheximide, an RNA synthesis inhibitor such as actinomycin D, or an immune-specific inhibitor such as cyclosporin, and determining whether such inhibition delays cell death; if it does then apoptosis is almost certainly involved. See, for example, Dhein et al., 1995, in which cell death is detected as the ability of the dye propidium iodide to enter the cell.

Procedures for the detection of DNA fragmentation may involve the isolation and size separation of DNA, typically by phenol extraction and gel electrophoresis. A newer technique involves the use of the enzyme terminal deoxynucleotidyl transferase ("TdT" or "terminal transferase"), an appropriate buffer (e.g., cacodylate buffer containing a cobalt salt and a reducing agent such as DTT, DTE, or BME) and a labeled deoxynucleotide triphosphate (dNTP) or a labeled derivative or analog thereof (e.g., BrdUTP, a biotynilated dNTP, a digoxigen labeled dNTP, or a radiolabeled dNTP, collectively referred to as a "labeled XTP").

TdT incorporates labeled XTPs onto free ends of DNA molecules. Since DNA degradation associated with apoptosis involves the generation of a great many free ends compared with a much smaller number in healthy cells, the incorporation of high levels of labeled XTPs relative to healthy cells indicates ongoing apoptosis. TdT methods for detecting apoptosis thus involve the detection of the incorporated labeled XTP (usually following washing of the cells to remove unincorporated labeled XTPs) typically using conventional techniques such as autoradiography or immunohistochemistry (e.g., using antibodies against the labeled XTP -- either tagged, e.g., fluorescently or enzymatically tagged antibodies, or in conjunction with tagged secondary antibodies). A commercial kit for the practice of this method is available from ONCOR, Inc., Gaithersburg. MD, as the "APOPTAG" kit.

Another recently developed technique involves an ELISA using an anti-histone capture antibody and an anti-DNA detection antibody. This assay depends on the conventional separation of intact chromatin from fragmented chromatin, with the levels of fragmented chromatin so separated being measured by the above mentioned ELISA. A commercial kit for the practice of this method is available from Boehringer Mannheim Corporation, Indianapolis, IN, as the "cell death detection" kit.

### B. Treatment

With regard to treatment using the MBP polypeptides of the invention, it should be noted that the MBP 21.5 polypeptides of the invention, (e.g., MBp+X2^{Ser81}) have various advantages in comparison to non-human-derived MBP antigens used in prior approaches for obtaining antigen tolerization in MS patients. Such advantages include the inclusion of the full spectrum of MBP immunodominant regions, and the consequent ability of these polypeptides to induce tolerance in T cells reactive with any such MBP immunodominant regions.

Intra-antigenic and inter-antigenic spread of autoreactivity are related phenomena associated with autoimmune diseases in which additional epitopes within an antigen, or additional antigens within a target tissue, become targeted by autoreactive T cells during disease progression. Such antigen spreading has been observed during the course of the inflammatory autoimmune process in the murine models of experimental allergic encephalomyelitis (EAE) and insulin-dependent diabetes (Lehmann et al. 1992; McCarron et al. 1990; Kaufman et al. 1993; Tisch et al. 1993).

These findings of antigen spreading, as well as the demonstration of variability in the immunodominant epitopes recognized by MBP reactive activated T cells in MS patients, indicate that an effective MBP-specific therapy will need to target a heterogeneous population of MBP-specific autoreactive T cells. Therefore, in order for parenteral MBP administration to be maximally effective in the treatment of MS, the complete repertoire of its immunodominant epitopes must be presented to T lymphocytes.

In accordance with certain aspects of the present invention, their is provided a medicament as per claim 1 for treating a patient suffering from multiple sclerosis Preferably the PLP polypeptide comprises the complete repertoire of known human PLP immunodominant epitopes. The PLP polypeptide is administered in an amount sufficient to achieve a concentration of the polypeptide in a relevant compartment (i.e., body fluid or tissue compartment) of the patient's body, e.g., the patient's blood, cerebrospinal fluid, lymph, reticuloendothelial system, liver, lymph nodes, spleen, thymus, and the like, sufficient to induce apoptosis of PLP reactive T cells. Preferably the polypeptide is administered repeatedly to the patient at least two times at an interval of at least twelve hours and not more than four days between administrations. The polypeptide is preferably administered without the concomitant administration of an adjuvant, so that tolerance, rather than exacerbation of disease, will result.

In accordance with the present invention, the concentration of the PLP polypeptide in the patient's body fluid or tissue compartment that is sufficient to induce apoptosis of PLP reactive T cells is determined using the materials, methods, and assays described above under "Clinical Applications" and "Detection of T Cell Responses". A concentration is considered sufficient to induce apoptosis of MBP or PLP reactive T cells when a substantial decrease in the number of T cells from peripheral blood exhibiting responses to MBP or PLP epitopes (the "precursor frequency" or "reactive T cell index") is seen following treatment (compared to T cells from blood samples taken before treatment) in response to the polypeptide, as compared to control assays, which are performed using irrelevant polypeptides (e.g., albumin). An at least 25% reduction in reactive T cell index will, in general, comprise a "substantial reduction". Smaller reductions are also considered "substantial" if they represent a statistically significant reduction, i.e., a reduction that, when analyzed by a standard statistical test, such as the student's T test, will give a probability value, p, less than or equal to 0.05 and, preferably, less than or equal to 0.015.

Alternatively, the concentration of the polypeptide in the patient's blood and/or cerebrospinal fluid that is sufficient to induce apoptosis of MBP or PLP reactive T cells may be determined by routine *in vivo* experimentation as the amount required to stabilize the clinical course or improve the clinical symptoms of EAE or MS.

In accordance with the invention, PLP and/or MPB 21.5 polypeptides may also be used to induce tolerization of PLP and/or MBP reactive T cells in an MS patient by administration on a schedule designed to induce tolerization without inducing apoptosis (e.g., by inducing T cell anergy). Such schedules are typically used to tolerize patients to allergens, and generally involve administration of smaller doses (typically ranging from micrograms to hundreds of micrograms) of the tolerizing agent (in this case the MBP 21.5 preparation) on a weekly, biweekly, or monthly basis.

The amount of administered polypeptide that is sufficient to achieve a desired concentration of the polypeptide in a body fluid or tissue compartment of the patient can be readily determined from routine human and animal study data using standard pharmacokinetic calculations well known to those of skill in the art. Initial *in vivo* studies are done in mice that have been treated to induce EAE. Preferably the dose of polypeptide is subsequently determined in a primate, e.g., a human patient or a marmoset (a monkey that is known to have MBP reactive T cells in its peripheral blood). Preferably the dosage is adjusted to achieve a clinical improvement (preferably in animals) or a substantial reduction in the number of T cells from peripheral blood exhibiting responses to MBP or PLP epitopes.

The dose will also vary depending on the manner of administration, the particular symptoms of the patient being treated, the overall health, condition, size, and age of the patient, and the judgment of the prescribing physician.

Subject to the judgment of the physician, a typical therapeutic treatment includes a series of doses, which will usually be administered concurrently with the monitoring of clinical severity of disease and reactive T cell index. Administration of the polypeptides will generally be performed by an intravascular route, e.g., via intravenous infusion by injection. Other routes of administration (e.g., subcutaneous injection, intradermal injection, intramuscular injection, inhaled aerosol, oral, nasal, vaginal, rectal, and the like) may be used if desired as determined by the physician.

Formulations suitable for injection are found in Remington's Pharmaceutical Sciences, Mack Publishing Company, Philadelphia, PA, 17th ed. (1985). Such formulations must be sterile and non-pyrogenic, and generally will include a pharmaceutically effective carrier, such as saline, buffered (e.g., phosphate buffered) saline, Hank's solution, Ringer's solution, dextrose/saline, glucose solutions, and the like. The formulations may contain pharmaceutically acceptable auxiliary substances as required, such as, tonicity adjusting agents, wetting agents, bactericidal agents, preservatives, stabilizers, and the like.

The formulations of the invention can be distributed as articles of manufacture comprising packaging material and the polypeptides. The packaging material will include a label which indicates that the formulation is for use in the treatment of neurologic disease and may specifically refer to multiple sclerosis.

Without intending to limit it in any manner, the present invention will be more fully described by the following examples.

### EXAMPLES

### Construction of bacterial vectors directing the expression of MBP 21.5 polypeptides and native MBP19.5

A full-length cDNA coding for the 18.5 kDa isoform of human MBP was obtained from the ATCC (#5748; ATCC, Rockville, MD). Plasmid pKBP-1 was used as a template in a standard PCR reaction using AmpliTaq (Perkin-Elmer, Norwalk, CT.) for 30 cycles with denaturation at 94°C for 1 min, annealing at 52°C for 1 min and extension at 72°c for 1 min. The sense oligonucleotide primer (5'-CATATGGCGT CACAGAAGAG AC-3', SEQ ID NO:13) encodes the N-terminus of hMBP18.5 (MASQKR) and contains an *Nde*I cloning site, whereas the antisense primer (5'-GGATCCTTAG CGTCTAGCCA TGGGTG-3', SEQ ID NO:14) encodes the C-terminal residues (PMARR) and contains a *Bam*HI cloning site. Following an additional extension at 72°C for 10 min, the resulting 526 base pair (bp) fragment was subcloned into pCRII (Invitrogen, San Diego, CA) as described by the supplier. Kanamycin-resistant *E. coli* DH10B (Gibco/BRL, Gaithersburg, MD) transformants were selected and the insert identified by restriction analysis and verified by dideoxy sequence analysis. The MBP coding region was subcloned into the *Nde*I and *Xho*I sites of the phage T7 promoter plasmid pET14b (Novagen, Madison, WI) and later recloned into pET22b (Novagen, Madison, WI). The resulting recombinant MBP18.5 gene contains only unmodified native codons, except for an additional 18 nucleotide sequence that encodes a histidine tag at the 3' end (immediately preceding the termination codon) that is not found in the native human MBP18.5 protein, and has been added to facilitate purification of the product of this MBP18.5^{hum.} gene. The resulting recombinant vector (pET22b/MBP18.5^{hum.}) was transformed into *E. coli* BL21(DE3) (Novagen, Madison, WI) where the DE3 lysogen contains the gene for T7 polymerase behind the *E. coli* lacUv5 promoter (Studier et al., 1990).

A synthetic recombinant gene encoding the 21.5 kDa isoform of human MBP was constructed in three rounds of overlapping PCR (Ho et al. 1989) (see Fig. 13). Each of three gene subdomains was synthesized in a 100µl reaction using 5 pmole of each the appropriate pair of HPLC purified oligonucleotides and 0.5 units of Taq polymerase (Perkin-Elmer). Thirty cycles of denaturation for 1 minute at 95°C, annealing at 50°C for 1 minute and DNA strand extension at 72°C for 1 minute were carried out. Five percent of each purified PCR fragment was then used as a template in a second round of PCR, where two subdomains were combined using flanking oligonucleotides. Purification of these DNA fragments and a third round of PCR resulted in amplification of a 648bp product. The PCR product was digested with *Eco*RI and *Hin*dIII, subcloned into pBS(-), and transformed into *E. coli* XL-1 Blue (Stratagene, LaJolla, CA). Ampicillin-resistant transformants were selected and the desired constructions identified by restriction and sequence analysis. Restriction fragments from several independent clones were combined to remove undesired mutations that occurred during PCR cloning, and the resulting MBP+X2^{Cys81/Bact.} gene was cloned into pET22b at the *Nde*I and *Hin*dIII sites.

An altered gene encoding a cysteine to serine substitution at amino acid residue 81 of the 21.5 kDa isoform of human MBP was constructed by the following steps. PCR amplification of an internal MBP fragment was carried out using pET22b/MBP21.5^{hum}. as template along with the mutagenic antisense primer (5'-GTCTTTGTAC ATGTTCGACA GGCCCGGCTG GCTACG-3', SEQ ID NO:15, Ser⁸¹ codon underlined, *Nsp*I site in italics) in combination with a sense oligonucleotide primer (5'-CAGCACCATG GACC-3', SEQ ID NO:16, *Nco*I site in italics). The *Nsp*I*-Nco*I restriction fragment in MBP+X2^{Cys81/Bact}. was then exchanged with the mutated fragment to create MBP+X2^{Ser81}/Bact.

By using the MBP18. 5^{hum}. gene as template in overlapping PCR, a version of MBP+X2^{Cys81} was created with native human codons. A PCR fragment that includes human exon 2 sequence was generated from pET22b/rhMBP18.5 by utilizing sense oligonucleotide 5'-GGTGCGCCAA AGCGGGGCTC TGGCAAGGTA CCCTGGCTAA AGCCGGGCCG GAGCCCTCTG CCCTCTCATG CCCGCAGCCA GCCTGGGCTG TGCAACATGT ACAAGGACTC ACACCACCCG GCAAGAAC-3', SEQ ID NO:17, in combination with an antisense oligonucleotide (SEQ ID NO:18) that hybridizes to the T7 terminator of plasmid pET22b. A second PCR fragment was generated using the same template but with a T7 promoter oligonucleotide (SEQ ID NO:19) in combination with an antisense oligonucleotide (5'GGCTTTAGCC AGGGTACCTT GCCAGAGCCC CGCTTTGGC 3', SEQ ID NO:20) that hybridized to the 5' end of exon 2. Fusion of both PCR products by amplification with T7 promoter and terminator oligonucleotides in a second round of PCR completed the construction of a PCR product containing the MBP+X2^{Cys81/hum.} gene. A restriction fragment obtained from this PCR product was then subcloned into pET22b at the *Nde*I and *Hin*dIII sites and the selection of the desired clone was confirmed by sequence analysis.

### Bacterial expression and identification of recombinant MBP

For expression of recombinant MBP polypeptides, *E. coli* strain BL21(DE3) was transformed with the expression plasmids and ampicillin-resistant colonies selected and grown in Terrific Broth (TB) medium (Sambrook et al. 1989) to an OD₆₀₀ of 0.6. Protein expression was induced for 4 hours with 1mM isopropylthiogalactoside (IPTG). Analytical characterization of recombinantly expressed MBP polypeptides was carried out by removing 1ml of induced cells at an OD₆₀₀ of 1.5. Cell pellets were lysed by boiling in 100µl of 20 mM Tris-HCl, pH7.5 with 10% of the lysate analyzed by 16% SDS-PAGE (Novex, San Diego, CA). recombinantly expressed MBP polypeptides were identified by either Coomassie R-250 staining or immunoblotting with rat monoclonal antibodies specific to either the human MBP amino-terminal residues 36-50 corresponding to MBP exon 1 (MCA 408, SeroTec, Indianapolis, IN) or carboxy-terminal residues 129-138 corresponding to MBP exon 6 (MCA 70, SeroTec, Indianapolis, IN) .

For fractionation of *E*. *coli* cells into soluble and insoluble fractions, cell pellets from two ml of each induced culture was collected at an OD₆₀₀ of 1.5 and resuspended in 400ml of 20mM Tris-HCl pH 8.0. To prepare a total cell lysate, the suspension was made 100mg/ml with lysozyme and 1mM with phenylmethylsulfonyl fluoride, then incubated at 30°C for 15 minutes. This was followed by the addition of 10mM MgCl₂ and 200 mg/ml of DNase I (Sigma, St. Louis, MO) and incubation for 20 minutes at room temperature. The cell lysate was divided, one-half receiving additional Tris buffer and the other half made 0.1N HCl and extracted at room temperature for 30 minutes. After centrifugation, the soluble supernatant was removed from the insoluble pellet and each fraction boiled for 5 minutes in SDS-containing loading dye. SDS-PAGE gels of 20% of each fraction were analyzed for recombinantly expressed MBP polypeptides as described above.

### Purification and characterisation of recombinant MBPs

For purification of recombinantly expressed MBP polypeptides, 1L cultures of induced cells were harvested by centrifugation and pellets homogenized in 10 ml/g (10% w/v) of 0.1N HCl using a TEKHAR homogenizer (The Tekmar Co., Cincinnati, OH). Cells were mechanically disrupted by 3 passes (at 10,000 psi with nitrogen) through a MICROFLUIDIZER (Model M110-T, Microfluidics Corp., Newton, MA) with all manipulations performed on ice. The soluble fraction containing recombinantly expressed MBP was collected as the supernatant following centrifugation of the cell lysate at 10,000xg for 30 min at 4°C in a Beckman JA-10 rotor. The supernatant was filtered through a WHATMAN POLYCAP TF (0.45µm) membrane (Whatman LabSales, Hillsboro, OR) and concentrated 5-10 fold using a PM-10 membrane in an AMICON stir cell apparatus (Amicon, Beverly, MA). Particulates were removed from the concentrated fraction by passing through a MILLEX GV (0.2mm) syringe filter (Millipore Corporation, Bedford, MA) and the filtered sample loaded onto a VYDAC C4 reverse phase column (1.0cm dia/25cm length, VYDAC, Hesperia, CA) at 4.1 ml/minute. Proteins were eluted using a linear 25-40% acetonitrile/0.1% trifluoroacetic acid (TFA) gradient for 30 minutes, then lyophilized.

For purification of recombinantly expressed MBP polypeptides, the lyophilized material was resuspended in binding buffer (8M urea, 10mM beta-mercaptoethanol, 0.1M NaH₂PO₄, 0.01 M Tris-HCl, pH 8.0) and bound to Ni-NTA resin according to the manufacturers instructions (Qiagen Inc., Chadsworth, CA). The column was washed twice with the same binding buffer, and contaminating *E. coli* proteins were removed with binding buffer that was adjusted to pH 6.3 (wash 3). rhMBP was eluted with a step gradient that included binding buffer at pH 5.9 (elution 1) and pH 4.5 (elution 2), and finally 6M guanidine hydrochloride, 0.2M acetic acid (elution 3). All fractions and a portion of the column resin were analyzed by 16% SDS-PAGE in the presence of reductant.

MBP polypeptides were quantified using a rapid analytical reversed-phase HPLC assay. A 4.6x50mm C18 column (C18 HYTACH, Glycotech, Branford, CT) was used and assays were performed at 80°C in a manner similar to the HPLC described by Kalghatgi and Horvath, 1987. Recombinantly expressed MBP polypeptides were extracted from disrupted cells with O.1N HCl and fractionated on the C18 HYTACH reversed-phase column using a linear 10-30% acetonitrile/0.1% triflouroacetic acid (TFA) gradient over 1 minute. In the linear assay range, measurement of the MBP polypeptide peak height is directly proportional to the quantity of MBP polypeptide. The concentration of an MBP+X2^{Cys81} standard was determined by amino acid composition. The molecular weight for MBP+X2^{Cys81} was determined by mass spectrophotometry to be 22,188 daltons. N-terminal sequencing of the purified MBP+X2^{Cys81} protein gave the amino acid sequence Ala Ser Gln Lys Arg Pro Ser Gln Arg His Gly Ser Lys Tyr Leu Ala Thr Ala Ser Thr Met Asp His Ala Arg, corresponding to the first 25 amino acids predicted from the nucleotide sequence of MBP+X2^{Cys81/hum.} (SEQ ID NO:1).

### Establishment of MBP18.5- and exon 2-specific T cell lines and proliferation assays

Native human MBP was prepared as described previously (Voskuhl et al. 1993a). MBP exon 2-encoded synthetic peptide was purchased from Synthecell Corp. (Rockville, MD) and was greater than 95% pure by HPLC analysis. Peripheral blood lymphocytes were isolated by leukapheresis and separation on FICOLL gradients. Cells were then cryopreserved in RPMI 1640 (Whittaker Bioproducts, Walkersville, MD) with 10% DMSO and stored in liquid nitrogen until use. T cell lines were generated using a limiting cell concentration, as described previously (Voskuhl et al. 1993a). 2A2 and 3H5 are human T cell lines that were obtained from normal individuals. 1H7, 1G1 and 3A11 are human T cell lines obtained from MS patients and are specific for the exon 2-encoded region of MBP. T cell lines were rested for 10 days after the last restimulation, then used as responders at a concentration of 2x10⁵ cells/ml. Autologous irradiated (3000 rad) peripheral blood lymphocytes (PBL) were used as stimulators at a concentration of 1x10⁶/ml. Fifty microliters of both responder and stimulator cells were mixed in each well of a round bottomed 96-well microtiter plate (Nunc, Roskilde, Denmark) with 100µl of the particular MBP antigen or medium alone. For the recombinant MBPs, lyophilized preparations from the reversed-phase HPLC purification were resuspended in PBS at a concentration of 8-10 mg/ml then diluted with medium immediately prior to use. Assays were done in triplicate and carried out in Iscove's Modified Dulbecco's Medium (IMDM, Gibco, Grand Island, NY) containing 2mM L-glutamine, 100U/ml penicillin and 100mg/ml streptomycin (all Whittaker Bioproducts, Walkersville, MD) supplemented with 10% pooled human serum (obtained from 4-7 normal AB NIH blood bank donors, heat inactivated and sterile filtered before use). Cultures were incubated for 72h at 37°C in 5% CO₂. During the last 18h of culture, cells were pulsed with 1mCi/well ³[H]-thymidine, harvested onto glass fiber filters, and thymidine incorporation measured by scintillation counting.

### Construction and bacterial expression of recombinant human MBP genes

A synthetic gene was constructed to encode the fetal isoform of adult human MBP (21.5 kDa isoform, MBP+X2^{Cys81}) (see Figs. 1 and 2). While others have typically constructed synthetic genes by ligating numerous oligonucleotides that encompass the complete sense and antisense strands of a particular coding region (Jayarman et al. 1991; Williams et al. 1988; Hernan et al. 1992; Wosnick et al. 1987), only six oligonucleotides (SEQ ID NO:5, SEQ ID NO:6, SEQ ID NO:7, SEQ ID NO:8, SEQ ID NO:9, and SEQ ID NO:10) were utilized here to synthesize the 644bp gene encoding recombinant human MBP+X2^{Cys81}. The HPLC-purified oligonucleotides ranged in size from 110 to 130 bp, with 20-25 bp overlapping regions designed for hybridization of sense and antisense strands during 3 rounds of PCR (Fig. 13). For optimal bacterial expression of the recombinant MBP gene, many of the human codons were converted to preferred bacterial codons based on codon bias tables created for all known (Wada et al. 1992) or highly expressed (Grosjean and Fiers, 1982) *E. coli* genes. Significant codon changes were employed, especially for those encoding arginine, proline and lysine, which comprise 26% of the amino acid residues in MBP21.5.

Several independent clones were sequenced and each had multiple nucleotide substitutions or deletions attributed to either rejection of the synthetic DNA by the bacterial cloning strain or PCR-based errors. All of these errors were corrected except for cytosine to thymine substitutions that were identified at nucleotide positions 462, 528 and 532. These changes were not corrected, as they conserve the encoded MBP+X2^{Cys81} amino acid sequence and are not deleterious to the bacterial codon preference (Wada et al. 1992). For recombinant expression of the adult human brain derived (18.5 kDa) isoform of MBP, a cDNA clone with native human codons encoding this isoform (MBP18.5^{/hum.}, encoding MBP18.5) was modified by PCR to include the appropriate restriction sites for cloning into the same expression vector. The expression of recombinant MBP polypeptides in bacteria was initially characterized using small-scale shake flask cultures grown in rich TB medium. Following induction of 10ml cultures with IPTG, both recombinant forms of MBP were expressed to high levels in BL21(DE3) cells. MBP18.5 and MBP+X2^{Cys81} were the major proteins identified by Coomassie dye staining of total bacterial proteins separated by SDS-PAGE (Fig. 14, "Coom") and were recognized specifically by antibodies directed to either the carboxy- (Fig. 14, "C-term Ab") or amino-(Fig. 14, "N-term Ab") terminus of human MBP. Two smaller MBP-immunoreactive polypeptides (between 6-16 kDa) could be identified in the MBP+X2^{Cys81} lysate, but only by immunoblot analysis with the N-terminal antibody, indicating that premature termination of translation near the carboxy terminus, rather that proteolysis, was responsible for their presence. This was confirmed in pulse-chase labeling experiments which showed that the smaller polypeptides were stable during the course of the experiment.

Although inclusion bodies were not evident in shake flask experiments, recombinant MBPs were observed in the insoluble fraction of lysed bacterial cells (Fig. 15, "Tris"). Previously, a homogeneous protein purified from bovine spinal cord was shown to have encephalitogenic activity and be soluble at pH 2-3 (Einstein et al. 1962). This encephalitogenic protein was subsequently identified as MBP, and consists almost exclusively of the 18.5 kDa isoform (Deibler et. al. 1972). Since MBP is acid soluble, we reasoned that it might be possible to streamline purification by direct acid extraction of bacterial lysates. We therefore attempted to solublize rhMBPs under acidic conditions. Treatment of total cellular lysates with 0.1N HCl (Fig. 15, "Acid") released most of the rhMBPs into the soluble fraction (S). The inability to extract all of the rhMBPs from the insoluble pellet fraction (P) may be due to incomplete lysis of cells during this particular sample preparation.

### Purification and characterization of MBP Polypeptides

For purification of recombinantly expressed MBP polypeptides, cells from 1L shake flask cultures were mechanically disrupted in the acidic conditions described above. Following simultaneous cell disruption and acid extraction, all of the recombinantly expressed MBP polypeptides were found in the soluble fraction (Fig. 16, "sol"). The soluble acid fraction was applied directly onto a VYDAC C4 reversed-phase column and rhMBPs eluted as a single, sharp peak at 17-20 min with a 25-40% acetonitrile/0.1% TFA gradient (Fig. 17). N-terminal sequencing of the peak fraction verified the correct amino-terminal sequence for the MBP polypeptides, as described above. The predicted molecular weight of MBP+X2^{Cys81} with an additional carboxy-terminal histidine tag agreed with the mass of 22,185 daltons obtained by mass spectrophotometric analysis of the peak fraction. Coomassie stained gels of the pooled peak fractions identified the recombinant MBP polypeptides, but also showed a heterogeneous mix of truncated MBP fragments apparently produced by limited acid hydrolysis of full-length MBP polypeptides (Fig. 18, "load'). By exploiting the C-terminal histidine tag, full-length MBP material was obtained by metal chelation chromatography using denaturing conditions and acidic pH elutions (Fig. 18). The majority of the full-length MBP polypeptides eluted with either elution 2 (8M urea, 10mM beta-mercaptoethanol, 0.1M NaH₂PO₄, 0.01 M Tris, pH 4.5) or elution 3 (6M guanidine hydrochloride, 0.2M acetic acid), although contaminating *E. coli* proteins were observed in the eluate from the less stringent second elution (Fig. 18, "elution 2").

To quantitatively compare the expression of the MBP+X2^{Cys81/bact}. to that of MBP18.5^{/hum.}, soluble acid lysates were prepared from three sets of one liter bacterial cultures and analyzed using the rapid analytical reversed-phase HPLC assay described above. Using a standard amount of MBP+X2^{Cys81}, as determined by amino acid analysis, and relating the peak height to protein concentration, we observed that 1.5 to 2.0-fold more MBP 21.5 polypeptide was expressed from the synthetic MBP+X2^{Cys81/bact.} gene compared to the expression from the MBP18.5^{/hum} gene. The average expression level of recombinant protein from MBP genes with bacterial codons was 50 mg/L compared to 30 mg/L from genes with human codons. This reflects bacterial codon bias and not an effect of exon 2-related sequences, as a strain that expressed the MBP+X2^{Cys81/hum.} gene produced a similar amount of MBP polypeptide as the strain expressing the MBP18.5^{/hum.} (see Fig. 19 and Table 5).

Under physiological conditions, a fraction of MBP+X2^{Cys81}, but not MBP18.5, formed an apparent dimeric molecule that was identified by Coomassie staining and Western blotting of nonreduced samples on SDS-PAGE gels. Dimers are not observed under similar conditions with reduced samples. MBP dimers also have been observed after reversed-phase HPLC fractionation of myelin proteins from bovine CNS (van Noort et al. 1994).

Such dimers are particularly undesirable in a protein preparation that is to be formulated for pharmaceutical administration, as, for such use, such proteins are generally preferred as single molecular entities with defined characteristics, including a unique molecular weight. It was thus important to devise a means by which single, monomeric forms of MBP 21.5 polypeptides could be conveniently and efficiently prepared. In order to test whether dimer formation of MBP+X2^{Cys81} was mediated through the single cysteine residue at position 81 (Cys⁸¹) of exon 2, the cysteine (Cys⁸¹) was converted to a serine (Ser⁸¹) by site-directed mutagenesis.

Reversed-phase HPLC showed that MBP+X2^{Ser81} was expressed in bacteria at a level similar to MBP+X2^{Cys81} (on average 50 mg/L, see Fig. 19) and remained monomeric in physiological solution, without reductant. As an alternative method of testing such an amino acid substitution for effective elimination of dimer formation, X2MPB peptides may be prepared and tested for dimer formation in physiological solution, without reductant.

### MBP18.5- and MBP exon 2-specific T cells recognize Recombinant Human (rh) MBPs

To assess the biological activity of recombinant forms of MBP, we tested the in vitro proliferation response of human MBP-specific T cell lines when challenged with the recombinant proteins. T cell lines were generated that respond to brain-derived human MBP18.5 or a synthetic exon 2 peptide (amino acid residues 60-85 of MBP21.5, SEQ ID NO:1). Two MBP18.5-specific lines, 2A2 (recognizing residues 31-50) and 3H5 (recognizing residues 87-106), were stimulated by incubation for 72 hours in vitro with either MBP18.5 or the MBP+X2 polypeptides. During the final 18 hours of this incubation the cells were pulsed with ³H-thymidine to allow measurement of cell proliferation. As shown in Fig. 20, both T cell lines responded equally well to MBP+X2^{Cys81} and MBP18.5, regardless of whether purified from human brain or bacteria. We also analyzed the antigen recognition of additional human T cell lines that respond to MBP epitopes that hve been described in the art. As designated in the art, and described herein, these MBP 18.5 epitopes are contained within residues 106-125, 136-155, 141-170, and 151-170 of MBP18.5, with the numbering being that used in the art, which is based on the amino acid sequence of the porcine MBP molecule. In each case, significant T cell proliferation was observed in response to native MBP18.5 and recombinant MBP+X2^{Cys81}.

The MBP+X2 molecules were engineered to include exon 2-encoded peptide sequences. In addition to providing a means to prepare therapeutic agents containing X2, the molecules allowed the determination of whether or not APCs could display exon 2 epitopes derived from full length MPB 21.5 in a manner that allowed recognition by T cells. This was also important for the MBP+X2^{Ser81} polypeptide, as it was not known if the single cysteine residue in exon 2 was essential for T cell recognition.

Proliferation assays with two independent exon 2-peptide-specific human T cell lines clearly demonstrated that only synthetic exon 2 peptide, MBP+X2^{Cys81} (Fig. 21) and MBP+X2^{Ser81} (Fig. 22) could elicit a T cell response. In addition, dose response assays (Fig. 22) revealed that both MBP+X2^{Cys81} and MBP+X2^{Ser81} were efficiently displayed to the T cells *in vitro*. This indicates that Cys⁸¹ is dispensable for presentation of the exon 2-encoded epitope recognized by the clones tested. T cell proliferation data are also summarized in Fig. 24 and Fig. 25.

These results demonstrate that human T cells can respond to processed X2 epitopes derived from full length MBP 21.5 molecules, and that the bacterially expressed recombinant forms of MBP, including MBP18.5, MBP+X2^{Cys81}, and MBP+X2^{Ser81}, can be as effective in stimulating encephalitogenic T cells as the native MBP18.5 protein.

### Synthesis. Expression and Purification of APLP4 and other PLP Muteins

A DNA template consisting of a 1.5 kb fragment containing full-length human PLP target sequence cloned in plasmid pUC8 (ATCC# 57466) was extensively modified to produce ΔPLP4. Three polynucleotides, each encoding a peptide comprising hydrophilic domain 2, 3, or 4 were synthesized independently by PCR and subsequently fused by overlapping PCR. The sequence integrity of the DNA containing the entire ΔPLP4 open reading frame was verified by dideoxy sequence analysis. The ΔPLP4 coding region was subcloned into plasmid pET22b (Novagen) as an *Nde*I/*Hind*III fragment. The ΔPLP4 protein contains five additional amino acids (Met-Leu-Glu-Asp-Pro) fused to the N terminus and five additional histidines (a histidine tag) fused to the C-terminal histidine.

Plasmid pΔPLP4 was transformed into *E. coli* strain W3110 (DE3) comprising a lambda DE3 lysogen (Studier et al 1990) and the ΔPLP4 polypeptide produced by the transformed bacteria was identified by Coomassie Blue staining, and by probing Western blots with rabbit polyclonal serum raised against a synthetic peptide (amino acids 118-130) of human PLP (Serotec, AHP261) followed by detection with horseradish perxoidase labeled goat anti-rabbit antibody and an enhanced chemiluminescence (ECL) detection system (Amersham). Following induction with 1 mM IPTG, ΔPLP4 accounted for approximately 50% of the total cell protein and appeared to be exclusively located in inclusion bodies. Selective extraction of the inclusion bodies is carried out with a buffer containing 6M guanidine HCL, or, preferably, 5M guanidine HCL, 20mM sodium citrate, pH 5.0. Such extraction resulted in a preparation with a purity of up to 90% as judged by SDS-PAGE and analytical reverse phase HPLC. N-Terminal amino acid sequence determination confirmed that the sequence of the isolated polypeptide contained the predicted amino terminal residues of ΔPLP4.

Using similar conventional techniques, nucleic acid molecules encoding other PLP mutein polypeptides were constructed and expressed in W3110 (DE3). These include ΔPLP3 (SEQ ID NO:23), in which hydrophobic domains 1, 3, and 4 are absent, and a similar construct encoding a PLP mutein lacking only hydrophobic domains 1 and 4 (ΔPLP2, SEQ ID NO:29). ΔPLP2 also includes a His tag sequence attached to its amino terminus, which as linked to and separated from the amino terminus of the second hydrophilic domain of PLP by a linker containing a thrombin cleavage site (amino acid residues 14-19 of SEQ ID NO:29). Expression of the encoded PLP muteins revealed that ΔPLP3 was expressed at levels comparable to those for ΔPLP4, discussed above, while ΔPLP2 was expressed at levels so low that they could only be detected by pulse chase radiolabeling analysis. A native PLP construct tested in the same expression system did not yield any detectable PLP polypeptide, even when analyzed by pulse chase radiolabeling.

### Construction, Expression, and Purification of MP4 and other Chimeric PLP Molecules

An MBP21.5 - ΔPLP4 fusion protein, MP4 was constructed as follows. A synthetic DNA fragment encoding MBP21.5 (SEQ ID NO:1) was placed under the control of the T7 promoter in the expression vector pET22b as described above. Next, the DNA fragment containing an appropriately spaced ribosome binding site and the ΔPLP4 gene was ligated downstream of the MBP21.5 gene, creating a dicistronic operon for independent expression of MBP21.5 and ΔPLP4. The dicistronic construct was digested with *Aat*II-*Xho*I and ligated to a synthetic *Aat*II-*Xho*I linker/adapter (corresponding to the sequence spanning nucleotides 588 to 605 of SEQ ID NO:26) creating a gene fusion encoding the MBP21.5/ΔPLP4 chimeric protein designated MP4 (SEQ ID NO:26). The sequence integrity of the resulting expression construct for the MP4 fusion protein was confirmed and the MP4-encoding plasmid was used to transform *E. coli* W3110 (DE3), referred to above harboring a lysogenic chromosomal copy of the bacteriophage T7 RNA polymerase.

Using similar conventional techniques, nucleic acid molecules encoding other chimeric PLP polypeptides were constructed and expressed in W3110 (DE3). These include MP3, (SEQ ID NO:25), PM4 (SEQ ID NO:27), and MMOGP4 (SEQ ID NO:28). MP3 was an analogous chimera to MP4 except that the PLP mutein moiety was the ΔPLP3 chimera of SEQ ID NO:23. PM4 was analogous to MP4 except that a different linker (corresponding to the sequence spanning nucleotides 508 to 519 of SEQ ID NO:27) was used in an overlapping PCR procedure to link MBP21.5 and ΔPLP4 in the opposite orientation to that in MP4. MMOGP4 was constructed by inserting a sequence encoding the extracellular domain of human myelin oligodendrocyte glycoprotein (Pham-Dinh et al. J Neurochem 1994, 63:2353 *et seq*) into MP4 between the MBP and PLP derived sequences. Expression of the encoded chimeric PLP polypeptides revealed that they were expressed at levels comparable to those for MP4, as discussed below.

MP4 synthesis was induced in *E. coli* W3110 (DE3) carrying the MP4 plasmid by the addition of 1 mM IPTG, and the protein appeared to be exclusively located in an insoluble fraction, accounting for approximately 20% of the total cell protein. MP4 was isolated as follows. *E. coli* paste from a 20L IPTG induced fermentation was resuspended in 10 volumes (10mL/g wet weight) of lysis buffer (20mM Na Citrate, 1mM EDTA, pH 5.0). The paste was uniformly suspended using an UltraTurrax T 50 homogenizer on ice. HCl was added to pH 5.0 and cells were lysed on ice using a Microfluidizer Model M-110T homogenizer operated at a pressure of 15,000-20,000 psi at the interaction chamber. The resultant lysate was centrifuged at approx. 10,000x g to separate soluble and insoluble fractions. The soluble fraction was discarded and the insoluble fraction was resuspended in 10 volumes (10mL/gram wet wt.) of extraction buffer (6M Guanidine-HCl, 0.5M NaCl, 20mM sodium phosphate, pH 5.0) using a homogenizer (Tekmar TP 18/1051). The extract was allowed to incubate with stirring for 60 min. at 2-8 degrees C. The extract was then centrifuged at 10,000x g for 30 min. The supernatant from the centrifugation was sonicated on ice with a Branson Sonifier 450 for 5 min to shear contaminating nucleic acids and filtered through a 0.45 micron filter (Whatman 75AS Polycap) to yield a filtered supernatant.

Column chromatography was performed in two steps. In the first step, metal chelate chromatography was employed as follows: A column with dimensions of 5 cm diameter x 20 cm length containing Chelating SEPHAROSE Fast Flow (Pharmacia Biotech) was packed in deionized water. Approximately the upper two thirds of the column was charged with Ni⁺⁺ by loading 1 mL of 0.1M NiCl2 per 7.8 mL of resin. The column was then washed with 5 CV of deionized water. The column was equilibrated with 2 CV of Buffer A (6M Guanidine-HCl, 0.5M NaCl, 20 mM sodium phosphate, 1mM 2-mercaptoethanol, pH 7.2) and a baseline optical density at 280nm was measured. The filtered supernatant was adjusted to pH 7.2 with NaOH and 2-mercaptoethanol was added to a final concentration of 1mM. This reduced sample was warmed to room temperature. The reduced sample was loaded at a flow rate of 50mL/min. The flow rate was then adjusted to 100mL/min and the column was washed with Buffer A until the column outflow reached the baseline optical density at 280nm. The column was then washed three times successively with 6M Urea, 0.5M NaCl, 0.02M sodium phosphate, first at pH 7.2, then at pH 6.3, and finally at pH 5.5, with each wash being continued until the optical density returned to baseline.

MP4 was eluted from the column with 6M Urea, 0.5M NaCl, 0.02M sodium phosphate, pH 3.5, while monitoring optical density at 280nm. Protein containing fractions were pooled and MP4 was further purified as follows: An aliquot of pooled fractions containing approximately thirty-five mg of MP4 (as estimated by analytical HPLC and SDS PAGE) was fully reduced by adding dithiothreitol to a final concentration of 50mM, guanidine HCL to a final concentration of 6M, and adjusting the pH to 8.0. The sample was then incubated at 37 degrees C for 0.5 hr. The resulting reduced and denatured preparation was then filtered through a 0.45 micron filter and applied to a 1 cm diameter x 25 cm length C4 VYDAC (Hesperia, CA) reversed phase HPLC column equilibrated in 55% solvent A (50% Formic Acid/50% H₂O) and 45% solvent B (50% Acetonitrile/50% Formic Acid) at room temperature. (A baseline optical density reading at 280nm is taken prior to loading the sample on the column.) After loading, the column effluent was monitored at 280nm until the reading returned to baseline. The column was then eluted with a linear gradient increasing solvent B concentration from 55% solvent A, 45% solvent B to 0% solvent A, 100% Solvent B.

Pooled protein containing fractions were concentrated in a Rotavap concentrator (Buchi Corp.) until brought to a concentration of approximately 2-3 mg/mL. Deionized water was then added to the flask to bring the sample to approximately its original volume and the sample was again concentrated to remove residual formic acid. This process was repeated until approximately five to ten times the original volume of water was added and removed. The sample was then transferred to a stirred cell concentrator (Amicon) equipped with a 10,000 dalton cutoff PM-10 membrane. Diafiltration was performed at 4 degrees C with three additions of deionized water until a total of twelve diavolumes of deionized water was passed through the sample. (The final pH of the sample was 3.5.)

The resulting concentrated material had a purity of up to 90% MP4 as judged by SDS-PAGE and analytical reversed phase HPLC. N-Terminal amino acid sequence determination confirmed that the sequence of the isolated polypeptide contained the predicted amino terminal residues of SEQ ID NO:26).

Further purification of MP4 may be desired. Additional purification steps include gel filtration chromatography and ion exchange chromatography (preferably cation exchange chromatography). These steps are facilitated by the addition of a non-ionic detergent (preferably TWEEN 20) to a concentration of 0.1% to 1.0%. The non-ionic detergent may be added at any point in the purification subsequent to cell lysis, as it does not interfere with metal chelate chromatography.

As with any pharmaceutical preparation derived from bacteria, the MP4 preparation is tested for toxicity in animals before administration to humans, with any toxic preparations being further purified or discarded. Such toxicity testing is preferably done using mice, and most preferably using mice in which EAE has been induced as described below, either by injection of encephalitogenic proteins or, preferably, by adoptive transfer of T cells from animals suffering from EAE. Testing in this manner has the additional benefit of allowing efficacy of treatment to be assessed in the animal model system. For such testing, 300 µg doses of bacterially produced polypeptide are preferably administered according to the appropriate treatment schedule described below under the subheading "Treatment of Mice with EAE."

### T Cell Responses Induced by the PLP Polypeptides of the Invention

The ΔPLP4 and MP4 polypeptides of the invention, were tested in *in vitro* and *in vivo* systems for their ability to stimulate T cell responses and to prevent and treat EAE/MS. The results of these studies are set forth in Figures 1-12 and Table 3. These results demonstrate that the PLP polypeptides of the invention can induce T cell responses and affect T cell reactivity to a variety of MBP and PLP epitopes, and can induce and prevent and treat EAE.

### Induction of EAE by Active Immunization

Female SJL/J mice were purchased from The Jackson Laboratories (Bar Harbor, ME). All mice were used between 8 and 12 weeks of age. All mice were maintained on standard laboratory food and water *ad libitum.* Feeding was adjusted to assure that paralyzed animals were afforded easier access to food and water.

Female SJL/J mice were immunized by subcutaneous injection with 150 µl of an incomplete Freund's adjuvant emulsion containing 150 µg of *Mycobacterium* tuberculosis H37Ra (Difco; complete Freund's adjuvant) and antigen. Antigens included 100 µg ovalbumin, 100 µg recombinant ΔPLP4, 300 µg MP4, or 150 µg of PLP peptide 1CS (amino acid residues 139-151 of SEQ ID NO:22 with a serine substituted for the cysteine at position 140). Each 150 µl injection was distributed over three sites on the dorsal flank.

All mice also received subsequent injections of 300 ng of pertussis toxin (List Biologicals, Campbell, CA) on days 0 and 3, as a higher incidence of disease was obtained when pertussis toxin was coadministered in preliminary tests. The immunomodulating effect of pertussis toxin on EAE induction is well known, although the precise mode of action is unknown. Pertussis toxin is a vasoactive substance believed to produce blood-brain barrier permeability and thus facilitate the entry of encephalitogenic cells into the CNS.

Initial clinical signs of disease were usually observed between day 12 and 16 post-immunization. Mice were monitored daily and a mean clinical score was assigned to each group. Mean day of onset was calculated based upon the initial appearance of clinical signs.

### Adoptive Transfer of EAE

Donor SJL/J mice were immunized subcutaneously with 100 µg of ΔPLP4 as described above. Nine to eleven days later, draining lymph node cells were harvested and stimulated with 25 µg/ml of PLP peptide 1CS for 4 days in the presence of syngeneic SJL/J antigen presenting cells (APCs). The peptide activated T cells (1.6x10⁷ in 0.1 ml PBS) were harvested, washed twice, and injected intravenously into syngeneic naive recipients.

### Treatment of Mice with EAE

Mice were divided into treatment groups that included untreated mice and mice receiving intravenous injections of either 125 µg of ΔPLP4 or pigeon cytochrome c (as a control) twice a day (separated by 6-8 hours) on days 2, 4, and 6 in the adoptive transfer experiments or days 5, 7, and 9 in the active immunization experiments.

Although preferred and other embodiments of the invention have been described herein, further embodiments may be perceived and practiced by those skilled in the art without departing from the scope of the invention as defined by the following claims.

**Table 1**

| Autoreactive Human PLP Peptides In MS Patients | | |
|---|---|---|
| PLP Peptide Name | Residues in SEQ ID NO:24 | PLP Peptide Amino Acid Sequence |
| PLP 38-49 | 9-20 | ALTGTERLIETY^{a} |
| PLP 40-60 | 11-31 | TGTEKLIETYFSKNYQDYEYL^{b,c} |
| PLP 88-108 | 42-62 | EGFYTTGAVRQIFGDYKTTIC^{e} |
| PLP 89-106 | 43-60 | GFYTTGAVRQIFGDYKTT^{b} |
| PLP 91-104 | 45-58 | YTTGAVRQIGDYK^{a} |
| PLP 95-116 | 49-70 | AVRQIFGDYKTTICGKGLSATV^{d} |
| PLP 103-116 | 57-70 | YXTTTCGKGLSATV^{a,e} |
| PLP 104-117 | 58-71 | KTTICGKGLSATVT^{f} |
| PLP 115-128 | 69-82 | TVTGGQKGRGSRGQ^{a} |
| PLP 117-150 | 71-104 | TGGQKGRGSRGQHQAHSLERVCHCLGKWLGHPDK^{c} |
| PLP 139-151 | 93-105 | HCLGKMLGHPDKF^{g,a} |
| PLP 139-154 | 93-108 | HCLGKWLGHPDKFVGI^{e} |
| PLP 142-153 | 96-107 | GKWLGHPOKFVG^{f} |
| PLP 183-195 | 115-127 | COSIAFPSKTSAS^{a} |
| PLP 195-206 | 127-138 | SIGSLCADARMY^{c} |
| PLP 195-208 | 127-140 | SIGSLCADARMYGV^{a} |
| PLP 220-234 | 152-166 | GSNLLSICKTAEFQM^{a} |

| | | |
|---|---|---|
| The numbers following the letters PLP in the PLP peptide names (left hand column) indicate that the sequence of that peptide spans and corresponds to the amino acid residues of those numbers (inclusive) of SEQ ID NO: 22. Superscript lower case letters at the ends of the peptide sequences indicate references discussing the peptides, as follows: ^{a} Kinkel et al., 1992. Neurology 42 (Suppl. 3): 159 (abstr. 87P). ^{b} Pelfrey et al., 1993. J Neuroimmunol 46: 33-42. ^{c} Trotter et al., 1993. J immunol 150:196A (abstr. 1117). ^{d} Inobe et al., 1992. Neurology 42 (Suppl. 3): 159-160 (abstr. 87P). ^{e} Trotter et al., 1991 J Neuroimmunol 33: 55-62. ^{f} Correale et al., 1995. J Immunol 154: 2959-2968. ^{g} Chou et al., 1992. J Neuroimmunol 38: 105-113. | | |

**Table 2**

| Encephalitogenic Epitopes of PLP in Inbred Mouse strains | | | |
|---|---|---|---|
| PLP peptide | Residues In SEQ ID NO:24 | Amino Acid Sequence | Strain |
| 43-64 | 14-35 | EKLIETYFSKNYQDYEYLINVI | PL/J (H-2^{d}) |
| 103-116 | 57-70 | YRTTICGKGLSATV | SWR (H-2^{q}) |
| 139-151 | 93-105 | HCLGKWLGHPDKF | SJL/J (H-2^{s}) |

| | | | |
|---|---|---|---|
| The numbers in the left hand column ("PLP Peptide") indicate that the sequence of that peptide spans and corresponds to the amino acid residues of those numbers (inclusive) of SEQ ID NO:22. | | | |

**Table 3. Induction of EAE in SJL/J Mice**

| | | | | |
|---|---|---|---|---|
| Group | Antigen* | Incidence | Mean Day of Onset^{†} | Mean Clin. Score‡ |
| A | Ovalbumin | 0/6 | | |
| B | ΔPLP4 | 6/6 | 12.6 (12-15) | 3.7 (3-4) |
| C | 139-151 | 6/6 | 14.2 (13-22) | 4.6 (3-5) |
| D | MP4 | 5/5 | 12.8 (12-16) | 2.3 (2-3) |

| | | | | |
|---|---|---|---|---|
| *Immunizations were performed on day 0 with CFA (150 µg H37Ra). Antigens used were either 100 µg ovalbumin (Sigma), 100 µg ΔPLP4, 150 µg PLP peptide 139-151, or 300 µg MP4. All groups received 300 ng pertussis toxin injected i.v. on day 0 and 3. ^{†} The mean number of days between immunization and the first signs of EAE is shown for each group of animals, with the range in brackets. ^{‡}The mean clinical grade at the height of disease severity is shown, with the range in brackets. | | | | |

**TABLE 4**

| amino acid | codon | huMBP 21.5 | recMBP 21.5 | amino acid | codon | huMBP 21.5 | recMBP 21.5 |
|---|---|---|---|---|---|---|---|
| Arg | CGT | 2 | 19 | Ser | TCT | 3 | 7 |
| | CGC | 4 | 1 | | TCC | 7 | 9 |
| | CGA | | | | TCA | 4 | |
| | CGG | 2 | | | TCG | 2 | |
| | AGA | 9 | 1 | | AGT | 2 | |
| | AGG | 4 | | | AGC | 4 | 6 |
| Gly | GGT | 2 | 4 | Ala | GCT | 3 | 3 |
| | GGC | 13 | 24 | | GCC | 5 | 6 |
| | GGA | 10 | | | GCA | 2 | |
| | GGG | 3 | | | GCG | 3 | 4 |
| Lys | AAA | 2 | 14 | Val | GIT | | |
| | AAG | 12 | | | GTC | 2 | |
| Leu | CTT | 2 | | | GTA | 1 | |
| | CTC | 1 | | | GTG | 2 | 5 |
| | CTA | | | His* | CAT | 3 | 6 |
| | CTG | 8 | 10 | | CAC | 8 | 11 |
| | TTA | | | Gln | CAA | 1 | |
| | TTG | | 1 | | CAG | 7 | 8 |
| Pro | CCT | 1 | | Asn | AAT | | |
| | CCC | 5 | | | AAC | 3 | 3 |
| | CCA | 4 | | Asp | GAT | 3 | 3 |
| | CCG | 7 | 17 | | GAC | 6 | 6 |
| Thr | ACT | 1 | | Glu | GAA | 2 | 2 |
| | ACC | 2 | 8 | | GAG | | |
| | ACA | 2 | | Ile | ATT | 2 | 2 |
| | ACG | 3 | | | ATC | 2 | 2 |
| Phe | TTT | 4 | | | ATA | | |
| | TTC | 5 | 9 | Tyr | TAT | 2 | 2 |
| Cys | TGT | | | | TAC | 3 | 3 |
| | TGC | 1 | 1 | Trp | TGG | 2 | 2 |
| Met | ATG | 4 | 4 | | | | |

| | | | | | | | |
|---|---|---|---|---|---|---|---|
| *recMBP21.5 contains six additional Histidines at the C-terminus. | | | | | | | |

**TABLE 5**

| GENE | OD₆₀₀ | WET WT (g) | 0.1 N HCL (g/ml) | PEAK HT (cm) | LYSATE VOL (ml) |
|---|---|---|---|---|---|
| MBP+X2^{Cys81/bact.} | 2.70 | 8.0 | 0.080 | 4.3 | 126 |
| MBP+X2^{Ser81/bact.} | 1.89 | 8.8 | 0.088 | 3.6 | 126 |
| MBP1 8.5^{hum.} | 1.96 | 8.0 | 0.080 | 2.8 | 126 |
| MBP+X2^{Cys81/hum.} | 1.76 | 6.0 | 0.060 | 1.6 | 126 |

### References

Abbas et. al. 1994. Cell and Mol Immunology, pp. 377-391.
Adorini et al. 1993. Immunol Today 14, pp. 285-289.
Allegretta et. al. 1990. Science 247, pp. 718-722.
Alvord, et al. 1984. Prog Clin Biol Res 146, pp. 359-363.
Ammerer, 1983. Meth Enzymol 101, pp. 192 et seq*.*
Amor et al., 1994. J Immunol 153, pp. 4349-4356.
Ausubel, et al., 1994. Current Protocols in Molecular Biology, John Wiley & Sons, New York.
Boehme and Lenardo 1993. Eur J Immunol 23, pp. 1552-1560.
Bruck et al. 1994. Ann Neurol 35, pp.65.
Brunner et al., 1995. Nature 373, pp. 441-444.
Chang, et al., 1978. Nature 275, pp. 615 et seq*.*
Chou et al. 1989 J Neurosci Res 23, pp. 207 et seq*.*
Chou et al. 1992. J Neuroimmunol 38, pp. 105-114.
Cohen et al., 1992. Ann Rev Immunol 10, pp. 267 et seq*.*
Correale et al., 1995. J Immunol 154, pp. 2959-2968.
Cotter et al., 1990. Anticancer Research 10, pp. 1153 et seq*.*
Crispe 1994. Immunity 1, pp. 347-349.
Critchfield et al. 1994. Science 263, pp. 1139-1143.
Davis et al., 1994. Basic methods in molecular biology, 2nd ed. Appleton and Lange, Norwalk, CT.
Dhein et al., 1995. Nature 373, pp. 438-441.
Deibler et al. 1972. Prep Biochem 2, pp. 139-165.
Duvall and Wyllie, 1986. Immunol Today 7, pp. 115.
Einstein et al.1962. J Neurochem 9, 353.
Endoh et al. 1986. J of Immunol 137, pp. 3832-3835.
Evans and Scarpulla, 1989. Gene 84, pp. 135 et seq*.*
Farrell, Jr., 1993. RNA Methodologies: A laboratory guide for isolation and characterization. Academic Press Inc., San Diego, CA.
Fritz and Zhao. 1994. J. Neuroimmunol 51, pp. 1-6.
Goeddel, et al., 1980. Nucl Acids Res 8, pp. 4057 et seq*.*
Greer et al. 1992. J of Immunol 149, pp. 783-788.
Griffin and Griffin, Eds., 1994. PCR technology, current innovations. CRC Press, Boca Raton, FL.
Grosjean and Fiers, 1982. Gene 18, pp. 199 et seq*.*
Harwood, Ed., 1994. Protocols for gene analysis: Methods in molecular biology, Vol. 31. The Humana Press, Totowa, NJ.
Hauser 1994. Harrison's Principles of Int Med, Thirteenth Ed., pp. 2287-2295.
Hernan et al. 1992 Biochemistry 31, pp. 8619 et seq*.*
Ho et al. 1989. Gene 77, pp. 51-59.
Huang and Gorman, 1990. Mol Cell Biol 10, pp. 1805 et seq*.*
Jayarman et al. 1991. Proc Natl Acad Sci USA 88, 4084.
Johns et al., 1995. J Immunol 154, pp. 5536-5541.
Ju et al., 1995. Nature 373, pp. 444-448.
Kalghatgi and Horvath. 1987 J Chromatogr. 398, 335.
Kamholtz et al. 1988. J Neurosci Res 21, pp. 62-70.
Kaufman et al. 1993. Nature 366, pp. 69 et seq*.*
Kawabe and Ochi, 1991. Nature 349, pp. 245-248.
Kennedy et al. 1990. J of Immunol 144, pp. 909-915.
Kerlero de Rosbol et al. 1993. J Clin Invest. 92, pp. 2602 et seq*.*
Kerr et al., 1991. Apoptosis: the molecular basis of cell death, Tomei and Cope (eds.), Cold Spring Harbor Laboratory Press, Plainview, New York, pp. 5 et seq*.*
Klaus, Ed., 1987. Lymphocytes: A practical approach. IRL Press, Oxford, England.
Kuchroo et al. 1992. J of Immunol 148, pp. 3776-3782.
Kuchroo et al. 1994. J of Immunol 150, pp. 3326-3336.
Lees and Mackin. 1988. Neuronal and Glial Proteins. Academic Press, San Diego, pp. 267-298
Lehmann et al. 1992. Nature 358, pp. 155-157.
Lenardo 1991. Nature 353, pp. 858-860.
Lockshin and Zakeri, 1991. Apoptosis: the molecular basis of cell death, Tomei and Cope (eds.), Cold Spring Harbor Laboratory Press, Plainview, New York, pp. 47 et seq*.*
Luckow, et al., 1988. Bio/Technology 6, pp. 47 et seq*.*
Maniatis, 1982. Molecular Cloning: A Laboratory Manual. Cold Spring Harbor Laboratory, Cold Spring Harbor, NY.
Marrack and Kappler, 1987. Science 238, pp. 1073 et seq*.*
Martin et al. 1990. J Immunol 145, pp. 540 et seq*.*
Martin et al. 1992. Ann Rev Immunol 10, pp. 153-187.
McCarron et al. 1990. J Neuroimmunol 29, pp. 73 et seq*.*
McRae et al. 1992. J of Neuroimmunol 38, pp. 229-240.
Meinl et al. 1993. J Clin Invest 92, pp. 2633 et seq*.*
Miller et al. 1992. J Neuroimmunol 39, pp. 243 et seq*.*
Miller and Karpus. 1994. Immunol Today, pp. 356-361.
Moir, et al., 1991. Meth Enzymol 194, pp. 491-507.
Morgenstern and Land, 1990. Nucl Acids Res 18, pp. 3587 et seq*.*
Mullis et al., Eds., 1994. The Polymerase Chain Reaction. Springer-Verlag, New York, NY.
Nagata and Suda, 1995. Immunol Today 16, pp. 39 et seq*.*
Oettinger et al. 1993. J Neuroimmunol 44, pp. 157 et seq*.*
Ormerod, Ed., 1994. Flow cytometry : A practical approach, 2nd ed. IRL Press at Oxford University Press, Oxford, England.
Ota et al. 1990. Nature 346, pp. 183 et seq*.*
Paul, 1989. Fundamental Immunology, 2nd Ed. Paul (ed.), Raven Press, New York.
Pette et al. 1990. Neurology 40, pp. 1770 et seq*.*
Pelfry et al. 1993. J of Neuroimmunol 46, pp. 33-42.
Pelfry et al. 1994. J of Neuroimmunol 53, pp. 153-161.
Prineas et al. 1993. Ann Neurol 33, pp. 137.
Raine and Wu. 1993. J Neuropathol Exp Neurol 52, pp. 199.
Racke et al., 1995. J Immunol, 154, pp. 450-458.
Remington's Pharmaceutical Sciences, Mack Publishing Co., Philadelphia, PA, 17th ed. (1985)
Richert et al. 1989. J Neuroimmunol 23, pp. 55 et seq.
Roth et al. 1987. J of Neurosci Res 17, pp. 321-328.
Russell et. al. 1993 Proc Natl Acad Sci USA 90, pp. 4409-4413.
Saeki et. al. 1992. Proc Natl Acad Sci USA 89, pp. 6157-6161.
Sambrook et al. 1990. Molecular Cloning: A Laboratory Manual (Cold Spring Harbor Press, Cold Spring Harbor, NY)
Sato et al. 1994. J Biol Chem 269, pp. 17267 et seq*.*
Schena, et al., 1991. Meth Enzymol 194, pp. 389-398.
Schwartz, 1993. Schwartz, RS, "Autoimmunity and Autoimmune Diseases" in Paul, Fundamental Immunology, 3rd Ed. Raven Press, New York, 1993, pp. 1033-1097.
Segal et al. 1994. J of Neuroimmunol 51, pp. 7-19.
Sercarz et. al. 1959. Nature 184, pp. 1080-1082.
Singer et. al. 1994. Immunity 1, pp. 365-371.
Smith et al., 1989. Nature 337, pp. 181-184.
Sobel et al. 1992. J of Immunol 149, pp. 1444-1451.
Sobel et al., 1994. Neurochem Res 19, pp. 915-921.
Smith et al., 1989. Nature 337, pp. 181-184.
Sprent 1994. Cell 76, pp. 315-322.
Steinman, 1995. Cell 80, pp. 7-10.
Studier et al. 1990. Meth Enzymol 185, pp. 60-89.
Strasser, 1995. Nature. 373, pp. 385-386.
Sun et al. 1991. Eur J Immunol 21, pp. 1461-1468.
Tabira, 1988. Ann NY Acad Sci 540, pp. 187-201.
Taguchi et al., 1990. J Immunol Meth 128, pp. 65-73.
Talib, et al., 1991. Gene 98:289-293.
Tisch et al. 1993 Nature 366, pp. 72-75.
Trotter et al. 1987. J of Neurosci Res 79, pp. 173-188.
Trotter et al. 1991. J of Neuroimmunol 33, pp. 55-62.
Tuohy et al. 1988. J of Immunol 141, pp. 1126-1130.
Tuohy et al. 1989. J of immunol 142, pp. 1523-1527.
Tuohy et al. 1992. J of Neuroimmunol 39, pp. 67-74.
Touhy, 1994. Neurochem Res 19, pp. 935-944.
Van der Venn et al. 1989. J of Neuroimmunol 21, pp. 183-191.
Van der Venn et al. 1990. J of Neuroimmunol 26, pp. 139-145.
Van der Venn et al. 1992. J of Neuroimmunol 38, pp. 139-146.
van Noort et al. 1994. J Chromatogr B 653, pp. 155 et seq*.*
von Boehmer, 1988. Ann Rev Immunol 6, pp. 309 et seq*.*
Voskuhl et al. 1993a. J of Neuroimmunol 42, pp. 187-192.
Voskuhl et al. 1993b. J of Neuroimmunol 46, pp. 137-144.
Voskuhl et al. 1994. J Immunol 149, pp. 4834 et seq*.*
Wada et al. 1992. Nucl Acids Res 20 (Supplement), pp. 2111-2118.
Weimbs and Stoffel. 1992. Biochemistry 31, pp. 12289-12296.
Weiner et al. 1993. Science 259, pp. 1321 et seq*.*
Weir, 1978. Handbook of experimental immunology, 3rd ed. Volume 2, Cellular immunology, Blackwell Scientific Publications, Oxford, England.
Whitham et al. 1991. J of Immunol 147, pp. 101-107.
Whitham et al. 1991. J of Immunol 147, pp. 3803-3808.
Williams et al. 1988. Nucl Acids Res 16, pp. 10453 et seq*.*
Wosnick et al. 1987. Gene 60, 115.
Yoon et al. 1993. Science 259, pp. 1263 et seq*.*
Zhang et al. 1993. Science 261, pp. 1451-1454.
Zhang et al. 1994. J Exp Med 179, pp. 973-984.

## Claims

1. An isolated immunoreactive polypeptide **characterised in that** it comprises any one of the amino acid sequences selected from the group consisting of the following:: SEQ ID NO: 25, SEQ ID NO: 26, SEQ ID NO: 27 and SEQ ID NO: 28; these polypeptides being designated MP3 chimera, MP4 chimera, PM4 chimera and MMOGP4 chimera respectively.

2. An isolated immunoreactive polypeptide according to claim 1 **characterised in that** it comprises any one of the amino acid sequences selected from the group consisting of the following:: amino acid residues 1 to 385 of SEQ ID NO: 25, amino acid residues 1 to 368 of SEQ ID NO: 26, amino acid residues 6 to 374 of SEQ ID NO: 27 and amino acid residues 1 to 487 of SEQ ID NO: 28 these polypeptides being designated MP3 chimera, MP4 chimera, PM4 chimera and MMOGP4 chimera respectively.

3. An isolated immunoreactive polypeptide according to claim 1 or claim 2 which comprises amino acid residues 1 to 368 of SEQ ID NO: 26 and is designated MP4.

4. A nucleic acid molecule **characterised in that** it comprises a nucleotide sequence which when expressed in a suitable host directs the expression of a polypeptide as claimed in claim 1, claim 2, or claim 3.

5. A composition suitable for administration to a human patient **characterised in that** it comprises an immunoreactive polypeptide according to any of claims 1 to 3 and a pharmaceutically acceptable carrier.

6. A tolerance inducing composition suitable for administration to a human patient **characterised in that** it comprises one or more immunoreactive polypeptides according to any of claims I to 3 and a pharmaceutically acceptable carrier.

7. A method for producing an isolated immunoreactive polypeptide according to any of claims 1 to 3 comprising the step of growing a recombinant host containing a nucleic acid molecule according to claim 4, such that the nucleic acid is expressed by the host and isolating the expressed polypeptide.

8. A method according to claim 7 wherein the recombinant host cell is a bacterial host cell.

9. A method as claimed in claim 8 wherein isolating the expressed polypeptide comprises disruption of the host to yield a disruptate, followed by fractionation of the disruptate involving acid extraction thereof.

10. An isolated immunoreactive polypeptide according to any of claims 1 to 3 or a composition according to claim 5 for use in preventing or treating a patient suffering from MS.

11. Use of an isolated immunoreactive polypeptide according to any of claims 1 to 3 in the manufacture of a medicament for preventing or treating a patient suffering from multiple sclerosis.

12. Use as claimed in claim 11 wherein the polypeptide is administered to the patient according to a regimen comprising administration of the polypeptide at least twice at an interval of at least twelve hours and not more than 4 days.

13. A method for assessing the potential responsiveness of a human MS patient to therapeutic treatment which method comprises the steps of:
a) incubating replicate cultures of T cells isolated from the patient in the presence and absence of one or more polypeptides according to any of claims 1 to 3, and
b) assaying for the presence of T cell activation and/or T cell apoptosis resulting from incubation in the presence, but not in the absence, of one or more of those polypeptides according to claims 1 to 3.

14. A use according to claim 12 wherein the one or more polypeptides are selected from the group consisting of the following:: amino acid residues 1 to 385 of SEQ ID NO: 25, amino acid residues 1 to 368 of SEQ ID NO: 26, amino acid residues 6 to 374 of SEQ ID NO: 27 and amino acid residues 1 to 487 SEQ ID NO: 28, these polypeptides being designated MP3 chimera, MP4 chimera, PM4 chimera and MMOGP4 chimera respectively.

15. Use of an isolated immunoreactive polypeptide according to any of claims 1 to 3 in the manufacture of a medicament for assessing the potential responsiveness of a human MS patient to therapeutic treatment.

## Patentansprüche

1. Isoliertes immunreaktives Polypeptid, **dadurch gekennzeichnet, dass** es eine der Aminosäuresequenzen umfasst, die aus der Gruppe ausgewählt sind, welche aus den Folgenden besteht: SEQ ID No: 25, SEQ ID No: 26, SEQ ID No: 27 und SEQ ID No: 28, wobei diese Polypeptide als MP3-Chimäre, MP4-Chimäre, PM4-Chimäre bzw. MMOGP4-Chimäre bezeichnet werden.

2. Isoliertes immunreaktives Polypeptid nach Anspruch 1, **dadurch gekennzeichnet, dass** es eine der Aminosäuresequenzen umfasst, die aus der Gruppe ausgewählt sind, welche aus den Folgenden besteht: Aminosäurereste 1 bis 385 von SEQ ID No: 25, Aminosäurereste 1 bis 368 von SEQ ID No: 26, Aminosäurereste 6 bis 374 von SEQ ID No: 27 und Aminosäurereste 1 bis 487 von SEQ ID No: 28, wobei diese Polypeptide als MP3-Chimäre, MP4-Chimäre, PM4-Chimäre bzw. MMOGP4-Chimäre bezeichnet werden.

3. Isoliertes immunreaktives Polypeptid nach Anspruch 1 oder Anspruch 2, welches die Aminosäurereste 1 bis 368 von SEQ ID No: 26 umfasst und als MP4 bezeichnet wird.

4. Nukleinsäuremolekül, **dadurch gekennzeichnet, dass** es eine Nukleotidsequenz umfasst, welche, wenn sie in einem geeigneten Wirt exprimiert wird, die Expression eines Polypeptides nach Anspruch 1, Anspruch 2 oder Anspruch 3 steuert.

5. Für eine Verabreichung an einen menschlichen Patienten geeignete Zusammensetzung, **dadurch gekennzeichnet, dass** sie ein immunreaktives Polypeptid nach einem der Ansprüche 1 bis 3 und einen pharmazeutisch verträglichen Träger umfasst.

6. Für eine Verabreichung an einen menschlichen Patienten geeignete toleranzauslösende Zusammensetzung, **dadurch gekennzeichnet, dass** sie ein oder mehrere immunreaktive Polypeptide nach einem der Ansprüche 1 bis 3 und einen pharmazeutisch verträglichen Träger umfasst.

7. Verfahren zur Herstellung eines isolierten immunreaktiven Polypeptids nach einem der Ansprüche 1 bis 3, welches die Stufe umfasst, bei der man einen rekombinanten Wirt, der ein Nukleinsäuremolekül nach Anspruch 4 enthält, züchtet, so dass die Nukleinsäure von dem Wirt exprimiert wird, und das exprimierte Polypeptid isoliert.

8. Verfahren nach Anspruch 7, wobei die rekombinante Wirtszelle eine Bakterienwirtszelle ist.

9. Verfahren nach Anspruch 8, wobei das Isolieren des exprimierten Polypeptids das Aufbrechen des Wirts zum Erhalt eines Extraktes, gefolgt von Fraktionierung des Extraktes, welche eine Säureextraktion davon einbezieht, umfasst.

10. Isoliertes immunreaktives Polypeptid nach einem der Ansprüche 1 bis 3 oder Zusammensetzung nach Anspruch 5 für eine Verwendung in der Prävention gegen MS oder zur Behandlung eines Patienten, der unter MS leidet.

11. Verwendung eines isolierten immunreaktiven Polypeptids nach einem der Ansprüche 1 bis 3 bei der Herstellung eines Medikaments zur Prävention von multipler Sklerose oder zur Behandlung eines Patienten, der unter multipler Sklerose leidet.

12. Verwendung nach Anspruch 11, wobei das Polypeptid dem Patienten nach einer Vorschrift verabreicht wird, welche eine Verabreichung des Polypeptids wenigstens zweimal in einem Intervall von wenigstens zwölf Stunden und nicht mehr als vier Tagen umfasst.

13. Verfahren zum Untersuchung des potentiellen Ansprechens eines menschlichen MS-Patienten auf eine therapeutische Behandlung, wobei das Verfahren die folgenden Schritte umfasst:
a) Inkubieren von Replikaten von T-Zell-Kulturen, die aus dem Patienten isoliert wurden, in der Gegenwart und Abwesenheit von einem oder mehreren Polypeptiden nach einem der Ansprüche 1 bis 3 und
b) Untersuchen hinsichtlich des Vorhandenseins von T-Zell-Aktivierung und/oder T-Zell-Apoptose, welche aus einer Inkubation in der Gegenwart, aber nicht in der Abwesenheit von einem oder mehreren solcher Polypeptide nach den Ansprüchen 1 bis 3 resultieren.

14. Verwendung nach Anspruch 12, wobei das eine Polypeptid oder die mehreren Polypeptide aus der Gruppe ausgewählt ist/sind, welche aus den Folgenden besteht:
Aminosäurereste 1 bis 385 von SEQ ID No: 25, Aminosäurereste 1 bis 368 von SEQ ID No: 26, Aminosäurereste 6 bis 374 von SEQ ID No: 27 und Aminosäurereste 1 bis 487 von SEQ ID No: 28, wobei diese Polypeptide als MP3-Chimäre, MP4-Chimäre, PM4-Chimäre bzw. MMOGP4-Chimäre bezeichnet werden.

15. Verwendung eines isolierten immunreaktiven Polypeptides nach einem der Ansprüche 1 bis 3 bei der Herstellung eines Medikaments zur Untersuchung des potentiellen Ansprechens eines menschlichen MS-Patienten auf eine therapeutische Behandlung.

## Revendications

1. Polypeptide immunoréactif isolé, **caractérisé en ce qu'**il comprend n'importe laquelle des séquences d'aminoacides choisie dans le groupe consistant en les suivantes : SEQ ID N° 25, SEQ ID N° 26, SEQ ID N° 27 et SEQ ID N° 28, ces polypeptides étant appelés respectivement chimère MP3, chimère MP4, chimère PM4 et chimère MMOGP4.

2. Polypeptide immunoréactif isolé suivant la revendication 1, **caractérisé en ce qu'**il comprend n'importe laquelle des séquences d'aminoacides choisie dans le groupe consistant en les suivants : les résidus d'aminoacides 1 à 385 de la SEQ ID N° 25, les résidus d'aminoacides 1 à 368 de la SEQ ID N° 26, les résidus d'aminoacides 6 à 374 de la SEQ ID N° 27 et les résidus d'aminoacides 1 à 487 de la SEQ ID N° 28, ces polypeptides étant appelés respectivement chimère MP3, chimère MP4, chimère PM4 et chimère MMOGP4.

3. Polypeptide immunoréactif isolé suivant la revendication 1 ou la revendication 2, qui comprend les résidus d'aminoacides 1 à 368 de la SEQ ID N° 26 et qui est appelé MP4.

4. Molécule d'acide nucléique, **caractérisée en ce qu'**elle comprend une séquence de nucléotides qui, lorsqu'elle est exprimée dans un hôte convenable, dirige l'expression d'un polypeptide suivant la revendication 1, la revendication 2 ou la revendication 3.

5. Composition convenable pour l'administration à un patient humain, **caractérisée en ce qu'**elle comprend un polypeptide immunoréactif suivant l'une quelconque des revendications 1 à 3 et un support pharmaceutiquement acceptable.

6. Composition induisant une tolérance, convenable pour l'administration à un patient humain, **caractérisée en ce qu'**elle comprend un ou plusieurs polypeptides immunoréactifs suivant l'une quelconque des revendications 1 à 3 et un support pharmaceutiquement acceptable.

7. Procédé pour la production d'un polypeptide immunoréactif isolé suivant l'une quelconque des revendications 1 à 3, comprenant l'étape de croissance d'un hôte recombinant contenant une molécule d'acide nucléique suivant la revendication 4, de telle sorte que l'acide nucléique soit exprimé par l'hôte, et l'isolement du polypeptide exprimé.

8. Procédé suivant la revendication 7, dans lequel la cellule hôte recombinante est une cellule hôte bactérienne.

9. Procédé suivant la revendication 8, dans lequel l'isolement du polypeptide exprimé comprend la rupture de l'hôte pour obtenir un produit de rupture, puis le fractionnement du produit de rupture par extraction avec un acide.

10. Polypeptide immunoréactif isolé suivant l'une quelconque des revendications 1 à 3 ou composition suivant la revendication 5, destiné à être utilisé dans la prévention ou le traitement de la sclérose en plaques (MS) chez un patient.

11. Utilisation d'un polypeptide immunoréactif isolé suivant l'une quelconque des revendications 1 à 3, dans la production d'un médicament destiné à la prévention ou au traitement de la sclérose en plaques chez un patient.

12. Utilisation suivant la revendication 11, dans laquelle le polypeptide est administré au patient par un schéma d'administration comprenant l'administration du polypeptide au moins deux fois à un intervalle d'au moins douze heures et non supérieur à 4 jours.

13. Méthode pour déterminer la capacité de réponse potentielle d'un patient humain atteint de MS à un traitement thérapeutique, méthode qui comprend les étapes de :
a) mise en incubation de cultures parallèles de lymphocytes T isolés du patient en la présence et en l'absence d'un ou plusieurs polypeptides suivant l'une quelconque des revendications 1 à 3, et
b) détermination de la présence d'une activation des lymphocytes T et/ou d'une apoptose des lymphocytes T résultant de l'incubation en la présence, mais non en l'absence, d'un ou plusieurs de ces polypeptides suivant les revendications 1 à 3.

14. Utilisation suivant la revendication 12, dans laquelle le ou les polypeptides sont choisis dans le groupe consistant en les suivants : les résidus d'aminoacides 1 à 385 de la SEQ ID N° 25, les résidus d'aminoacides 1 à 368 de la SEQ ID N° 26, les résidus d'aminoacides 6 à 374 de la SEQ ID N° 27 et les résidus d'aminoacides 1 à 487 de la SEQ ID N° 28, ces polypeptides étant appelés respectivement chimère MP3, chimère MP4, chimère PM4 et chimère MMOGP4.

15. Utilisation d'un polypeptide immunoréactif isolé suivant l'une quelconque des revendications 1 à 3, dans la production d'un médicament destiné à déterminer la capacité de réponse potentielle d'un patient humain atteint de MS à un traitement thérapeutique.
